(19) Europäisches Patentamt
European Patent Office
Office européen des brevets

(11) EP 1 481 972 A1

(12) **EUROPEAN PATENT APPLICATION**
published in accordance with Art. 158(3) EPC

(43) Date of publication:
**01.12.2004 Bulletin 2004/49**

(21) Application number: **03743574.0**

(22) Date of filing: **04.03.2003**

(51) Int Cl.[7]: **C07D 251/22**, C07D 273/04, C07D 285/34, C07D 413/12, C07D 413/06, C07D 417/12, C07D 417/06, C07D 417/14, C07D 413/14, C07D 401/12, C07D 401/06, C07D 401/14, A01N 43/88, A01N 43/64, A01N 47/16, A01N 47/06

(86) International application number:
**PCT/JP2003/002461**

(87) International publication number:
**WO 2003/074498 (12.09.2003 Gazette 2003/37)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **05.03.2002 JP 2002058993**
**09.10.2002 JP 2002296007**

(71) Applicant: **Sumitomo Chemical Takeda Agro Company, Limited**
**Tokyo 104-0033 (JP)**

(72) Inventors:
- **ITOH, Shigeyuki**
**Tsukuba-shi, Ibaraki 305-0821 (JP)**
- **KONOBE, Masato**
**Tsukuba-shi, Ibaraki 305-0821 (JP)**
- **SAKAMOTO, Norihisa**
**Tsukuba-shi, Ibaraki 300-2635 (JP)**

(74) Representative:
**von Kreisler, Alek, Dipl.-Chem. et al**
**Deichmannhaus am Dom,**
**Postfach 10 22 41**
**50462 Köln (DE)**

# (54) CYCLIC COMPOUND, PROCESS FOR PRODUCING THE SAME, AND PEST CONTROL AGENT

(57) A compound represented by the formula:

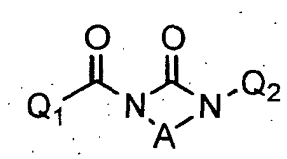

wherein, A represents a group represented by the formula: $CY_1Y_2OCY_3Y_4$, $CY_1Y_2SO_nCY_3Y_4$, $CY_1Y_2NRCY_3Y_4$, $CY_1{=}NCY_3Y_4$ or $CY_1Y_2N{=}CY_3$ (wherein n is 0, 1, or 2, and $Y_1$, $Y_2$, $Y_3$, and $Y_4$ each independently represents hydrogen, alkyl, or haloalkyl); R represents hydrogen, halogen, an optionally substituted hydrocarbon group, etc.; $Q_1$ represents substituted phenyl or a substituted aromatic heterocyclic group; and $Q_2$ represents substituted phenyl, alkyl, or haloalkyl, or a salt. thereof; a process for producing the. compound or a salt thereof; and an insecticide containing. the same. Besides being applicable by spraying, the insecticide can be used for soil treatment and seed treatment.

EP 1 481 972 A1

## Description

### Technical Field

The present invention relates to a cyclic compound, a process for producing the same, and a pest control agent (pest controller), in particular, a cyclic compound useful as an insecticide which contains heterocyclic ring having at least two nitrogen atoms as ring constituting atoms and having an oxo group, a process for producing the same and a pest controller using the same.

### Background Art

Numerous cyclic compounds useful as an agricultural chemical have been known. For example, JP 46-6550A and NL 710535A disclose a compound having insecticidal activity represented by the formula:

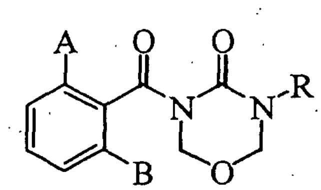

wherein A represents a hydrogen atom, a halogen atom, methyl group or methoxy group, B represents a hydrogen atom, a halogen atom, methyl group or methoxy group, provided that A and B are not a hydrogen atom at the same time, and R represents an optionally substituted phenyl and the like. However, the compound disclosed specifically is only a compound wherein A and B are chloro and R is chlorophenyl, in addition, the test results for insecticidal activity of these compounds are not described therein. On the other hand, the insecticidal activities of the compounds represented by the formula:

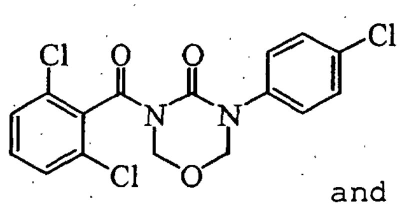

and

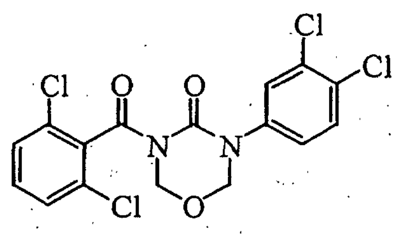

are specifically disclosed in J. Agr. Food Chem., Vol.21, No.3, 1973, 348-354. In WO 99/16316 and WO 97/45017, an invention of use wherein the compounds disclosed in the above-mentioned document and JP 46-6550A are used for soybean and cotton, respectively, is disclosed.

Further, in WO 01/14373, the compound represented by the formula:

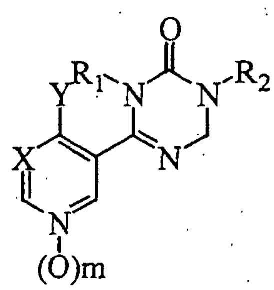

wherein, X represents CH or N, Y represents a halogen atom, $R_1$ and $R_2$ represent independently an optionally halogenated aryl group andthe like, is disclosed as an insecticide.

In JP 51-56480A, the compound represented by the formula:

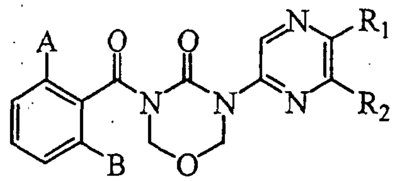

wherein, A and B represent a halogen atom and the like, $R_1$ and $R_2$ represent independently a hydrogen atom, a halogen atom and the like, is disclosed as an insecticide.

Furthermore, in JP 60-11482A, the compound represented by the formula:

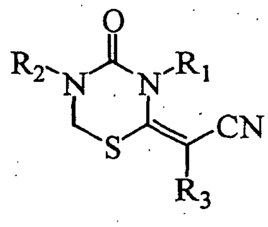

wherein, $R_1$ represents a substituted phenyl and the like,. $R_2$ represents an alkyl or aryl, $R_3$ represents a nitrile and the like, is disclosed as a herbicide.

Disclosure of Invention

In each of the above-mentioned known arts, only a spray treatment is disclosed as application of insecticide. However, if soil treatment and seed treatment for crops are made possible, it can make a significant contribution to the laborsaving in agriculture and improvement of safety in workers and environment. Thus, the main purpose of the present invention is to provide a new insecticide useful for each of spray treatment, soil treatment and seed treatment.

The present inventors variously studied a compound useful as an insecticide, and found out that the compounds represented by the following formula (I) have an insecticidal activity by spray treatment as well as a. potent insecticidal activity by irrigation treatment of pesticide solution and these compounds have systemic action (penetration and translocation ability).

As a treatment method for crops, a spray treatment is used for an insecticidal ingredient having no systemic action (penetration and translocation ability). However, when an insecticidal ingredient has permeable translocation ability, not only spray treatment for crops but also soil treatment and seed treatment are made possible. Here, the spray treatment means a treatment that exerts an. effect by. treating plant surface and insect body with an active ingredient, such as foliage application, spraying to tree trunk, and the like. Further, in this specification, the soil treatment means treatment to planting holes (planting hole spraying, soil-incorporation after planting hole treatment), plant foot treatment (plant foot spraying, plant foot soil-incorporation, plant foot irrigation, plant foot treatment at latter half. of raising seeding. period), planting furrow treatment (planting furrow spraying, planting furrow soil-incorporation), planting row treatment (planting row spraying, planting row .soil-incorporation, planting row spraying at growing period), planting row treatment at sowing (planting row spraying at sowing, planting row soil-incorporation at. sowing), overall treatment (overall spraying, overall soil-incorporation), other spray treatment (foliar granule spraying at growing period, spraying under tree crown or around main stem, soil surface spraying, soil surface .incorporation, sowing hole spraying, spraying on the ribbing ground, inter-plant spraying), other irrigation treatment (irrigation into soil, irrigation during raising seeding, injection treatment of. pesticide solution, irrigation on plant foot), nursery box treatment (nursery box spraying, nursery box irrigation), nursery tray treatment (nursery tray spraying, nursery tray irrigation), nursery bed treatment (nursery bed spraying, nursery bed irrigation, nursery bed spraying in paddy field, immersion of nursery plant), seed bed soil-incorporation treatment (seed bed soil-incorporation, seed bed soil-incorporation before sowing), other treatment (growing media incorporation, plowing, surface soil-incorporation, soil incorporation into rain dropping,planting spot treatment, flower cluster granule spraying, paste fertilizer mixing) and the like as well as a treatment that exerts an effect by treating the soil with an active ingredient.

In addition, the seed treatment means a treatment that exerts an effect by. treating seed with an active ingredient, such as blowing treatment, painting treatment, immersion treatment, impregnation treatment, application treatment, film coating, and the like, and also includes a treatment for seed tubers and bulbs.

Furthermore the present inventors have found out that the compounds represented by the following formula

(I) are. useful not only as agricultural chemicals but also as pest controller in other field. The present inventors variously studied based on these knowledges, which resulted in completion of the present invention.

That is, the present invention provides:

(1) A compound represented by the formula:

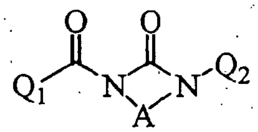

(I)

wherein, A represents a group represented by the formula:

1) $CY_1Y_2OCY_3Y_4$
2) $CY_1Y_2SO_nCY_3Y_4$
3) $CY_1Y_2NRCY_3Y_4$
4) $CY_1=NCY_3Y_4$ or
5) $CY_1Y_2N=CY_3$

wherein n represents 0, 1, or 2, and $Y_1$, $Y_2$, $Y_3$ and $Y_4$ represent independently a hydrogen atom, a $C_{1-6}$ alkyl group or a halo$C_{1-6}$ alkyl group; R represents a hydrogen atom, a halogen atom, a group via optionally oxidized sulfur atom, a nitro group, a nitroso group, a formyl group, an optionally substituted hydroxy group, a cyano group, an optionally substituted hydrocarbon group, an optionally substituted amino group, an optionally substituted heterocyclic group, an optionally substituted $C_{2-7}$ acyl group, an optionally substituted $C_{1-6}$ alkoxycarbonyl group,. an optionally substituted $C_{7-11}$ aralkyloxycarbonyl. group, an optionally substituted $C_{6-10}$ aryloxycarbonyl group, an optionally substituted carbamoyl group, an optionally substituted thiocarbamoyl group, an optionally substituted hydroxyoxalyl group, an optionally substituted aminooxalyl group or an optionally substituted hydroxyaminooxalyl group; $Q_1$ represents a substituted phenyl group or a substituted aromatic heterocyclic group; and $Q_2$ represents a substituted phenyl group, a $C_{1-6}$ alkyl group or a halo$C_{1-6}$ alkyl group; provided that when A is a group represented by $CH_2OCH_2$, $Q_1$ is a group other than dichlorophenyl; (hereinafter, in the present specification sometimes referred to as Compound(I)), or a salt thereof,

(2) the compound according to the above-mentioned (1), which is any one of the compounds represented by. the formulas:

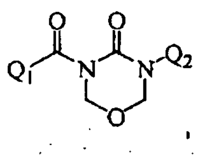

(Ia) ,

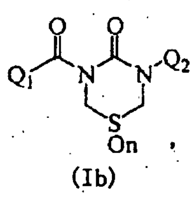

(Ib) ,

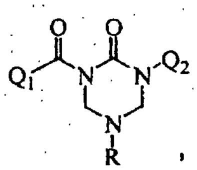

(Ic) ,

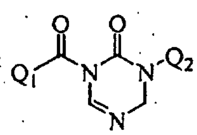

(Id) or

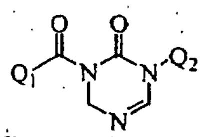

(Ie)

wherein respective symbols are as defined in the above-mentioned (1),

(3) the. compound according to the above-mentioned (2), wherein R is a hydrogen atom, a nitroso group, a. hydroxyoxalyl group, an aminooxalyl group, or a $C_{1-6}$ alkyl. group, a $C_{3-10}$ cycloalkyl group, a $C_{2-6}$ alkenyl group, a $C_{2-6}$ alkynyl group, a $C_{6-10}$ aryl group, a $C_{7-11}$ aralkyl group, a $C_{1-6}$ alkoxycarbonyl group, a $C_{6-10}$ aryloxycarbonyl group, a $C_{7-11}$ aralkyloxycarbonyl group, a $C_{1-6}$ alkoxycarbonylthio group, a $C_{2-7}$ acyl group, a $C_{1-6}$ alkylsulfonyl group, a $C_{6-10}$ arylsulfonyl group, a $C_{1-6}$ alkoxyoxalyl group, a N-$C_{1-6}$ alkylcarbamoyl group, a N,N-di($C_{1-6}$ alkyl) carbamoyl group, a N-($C_{1-6}$ alkoxy, $C_{7-11}$ aralkyloxy or $C_{1-6}$ alkyl)aminooxalyl group, a N,N-di($C_{1-6}$ alkyl)aminooxalyl group, or a N,O-di($C_{1-6}$ alkyl)hydroxyaminooxalyl group, each of which may be substituted,

(4) the compound according to the above-mentioned (1), wherein the substituent of the substituted phenyl group for $Q_2$ is at least one selected from the group consisting of a halogen atom, a cyano group, a $C_{1-6}$ alkyl group, a halo$C_{1-6}$ alkyl group, a $C_{1-6}$ alkoxycarbonyl group, a group represented by the formula: $Z_1$-W- (wherein $Z_1$ represents a benzene ring or an aromatic heterocyclic ring, each of which is substituted with a halogen atom, a $C_{1-6}$ alkyl group or a halo$C_{1-6}$ alkyl group, and -W- represents -O-, -S-, -SO- or $-SO_2-$. Such as phenyloxy group, phenylthio group, phenylsulfinyl group, phenylsulfonyl group, aromatic heterocyclic oxy group, aromatic heterocyclic

thio group, aromatic heterocyclic sulfinyl group and aromatic heterocyclic sulfonyl group, each of which has a halogen, a $C_{1-6}$ alkyl group or a halo$C_{1-6}$ alkyl group as a substituent), or a group represented by the formula: $Z_2$-W- (wherein $Z_2$ represents a $C_{1-6}$ alkyl, a $C_{2-6}$ alkenyl or a $C_{2-6}$ alkynyl, each of which may be substituted with a halogen atom, and -W- is as defined above. Such as alkoxy group, alkylthio group, alkylsulfinyl group, alkylsulfonyl group, alkenyloxy group, alkenylthio group, alkenylsulfinyl group, alkenylsulfonyl group, alkynyloxy group, alkynylthio group, alkynylsulfinyl group and alkynylsulfonyl group, each of which may be halogenated),

(5) the compound according to the above-mentioned (1), wherein $Q_1$ is a phenyl group or an aromatic heterocyclic group, each of which is substituted with a halogen atom, a $C_{1-6}$ alkyl group, a halo$C_{1-6}$ alkyl group, a $C_{1-6}$ alkoxy group or a halo$C_{1-6}$ alkoxy group,

(6) the compound according to the above-mentioned (2), wherein $Q_1$ is difluorophenyl, chlorofluorophenyl or 3-chloropyridin-2-yl,

$Q_2$ is a phenyl group which has at least one substituent selected from the group consisting of a halogen atom, cyano group, $C_{1-3}$ alkyl group, halo$C_{1-3}$ alkyl group, $C_{1-4}$ alkoxycarbonyl group; phenyloxy group, phenylthio group, phenylsulfinyl group and phenylsulfonyl group, each of which is substituted with halogen atom, $C_{1-3}$ alkyl group or halo$C_{1-3}$ alkyl group; pyridyloxy group, pyridylthio group, pyridylsulfinyl group, pyridylsulfonyl group, each of which is substituted with halogen atom, $C_{1-3}$ alkyl group or halo$C_{1-3}$ alkyl group; and $C_{1-3}$ alkoxy group, $C_{1-4}$ alkylthio group, $C_{1-4}$ alkylsulfinyl group, $C_{1-4}$ alkylsulfonyl group, $C_{2-4}$ alkenylthio group, $C_{2-4}$ alkenylsulfinyl group, $C_{2-4}$ alkenylsulfonyl group, $C_{2-4}$ alkynylthio group, $C_{2-4}$ alkynylsulfinyl group and $C_{2-4}$ alkynylsulfonyl group, each of which may be substituted with halogen atom; or 2,2,2-trifluoroethyl,

R is a hydrogen atom, $C_{1-3}$ alkyl group, allyl group, propargyl group, phenyl group, benzyl group, phenethyl group, methoxycarbonyl group, benzyloxycarbonyl group, methoxycarbonylthio group, N-methylcarbamoyl group, N,N-dimethylcarbamoyl group, acetyl group (which may be substituted with halogen atom, methylthio group, methylsulfinyl group or methylsulfonyl group), methylsulfonyl group, 4-methylphenylsulfonyl group, trifluoromethylsulfonyl group, nitroso group, methoxyoxalyl group, hydroxyoxalyl group, aminooxalyl group, N-(methoxy, benzyloxy or methyl)aminooxalyl group, N,N-dimethylaminooxalyl group or N,O-dimethylhydroxyaminooxalyl group,

(7) a process for producing the compound according to the above-mentioned (1) or a salt thereof, which comprises reacting a compound represented by the formula:

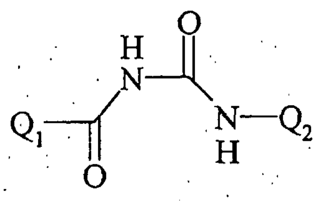

(II)

wherein respective symbols are as defined in the above-mentioned (1), or a salt thereof, with a compound represented by the formula:

$$L\text{-}A\text{-}L \qquad (III)$$

wherein L represents a leaving group, or

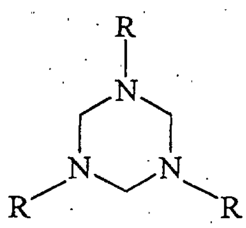

(IV)

wherein R is as defined in the above-mentioned (1); and if desired, converting the substituent of the obtained compound to other substituent,

(8) a process for producing the compound represented by the formula:

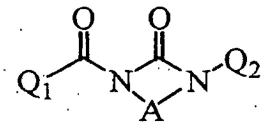

(I)

wherein, A represents a group represented by the formula:

1) $CY_1Y_2OCY_1Y_2$
2) $CY_1Y_2SO_nCY_1Y_2$ or
3) $CY_1Y_2NRCY_1Y_2$

wherein n represents 0, 1 or 2, and $Y_1$ and $Y_2$ represent independently a hydrogen atom, a $C_{1-6}$ alkyl group or a halo$C_{1-6}$ alkyl group; R represents a hydrogen atom, a halogen atom, a group via optionally oxidized sulfur atom, a nitro group, a. nitroso group, a formyl group, an optionally substituted hydroxy group, a cyano group, an optionally substituted hydrocarbon group, an optionally substituted amino group, an optionally substituted heterocyclic group, an optionally substituted $C_{2-7}$ acyl group, an optionally substituted $C_{1-6}$ alkoxycarbonyl group, an optionally substituted $C_{7-11}$ aralkyloxycarbonyl group, an optionally substituted $C_{6-10}$ aryloxycarbonyl group, an optionally substituted carbamoyl group, an optionally substituted thiocarbamoyl group, an optionally substituted hydroxyoxalyl group, an optionally substituted aminooxalyl group or an optionally substituted hydroxyaminooxalyl group; $Q_1$ represents a substituted phenyl group or a substituted aromatic heterocyclic group; and $Q_2$ represents a substituted phenyl group, a $C_{1-6}$ alkyl group or a halo$C_{1-6}$ alkyl group; provided that when A is a group represented by $CH_2OCH_2$, $Q_1$ is a group other than dichlorophenyl; or a salt thereof, which comprises subjecting the compound represented by the formula:

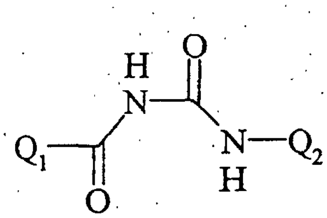

(II)

wherein respective symbols are as defined in the above-mentioned (1), or a salt thereof, to dehydration reaction with an aldehyde or a ketone, if necessary, in the presence of hydrogen sulfide or amine represented by the. formula $RNH_2$ (wherein R is as defined in the above-mentioned (1)); and if desired, converting the substituent of the obtained compound to other substituent,

(9) a pest controller for soil treatment which comprises compound (I) (provided that when A is a group represented by $CH_2OCH_2$, $Q_1$ may be dichlorophenyl) or a salt thereof as an active ingredient,

(10) a pest controller according to the above-mentioned (9) which is for seed treatment, and

(11) a pest controller for spraying which comprises the compound according to any one of the above-mentioned (1)-(6) or a salt thereof as an active ingredient.

Detailed Description of the Invention

In Compound (I), A is a group represented by the formula:

1) $CY_1Y_2OCY_3Y_4$
2) $CY_1Y_2SO_nCY_3Y_4$
3) $CY_1Y_2NRCY_3Y_4$
4) $CY_1{=}NCY_3Y_4$ or
5) $CY_1Y_2N{=}CY_3$

wherein n represents 0, 1 or 2, and $Y_1$, $Y_2$, $Y_3$ and $Y_4$ represent independently a hydrogen atom, an alkyl group or a haloalkyl group.

Examples of the "alkyl group" represented by $Y_1$, $Y_2$, $Y_3$ and $Y_4$ include a straight or branched alkyl group having 1 to 6 carbons (e.g. methyl, ethyl, n-propyl, isopropyl, n-butyl, isobutyl, sec-butyl, t-butyl, heptyl, hexyl, etc.).

Examples of the haloalkyl group include the above-mentioned $C_{1-6}$ alkyl group which is substituted with one or more, preferably 1 to 3 halogens, for example, fluorine, chlorine, bromine, iodine and the like at substitutable position (trifluoromethyl, 2,2,2-trifluoroethyl).

$Q_1$ represents a substituted phenyl group or a substituted aromatic heterocyclic group.

Examples of the substituent for the "substituted phenyl group" and "substituted aromatic heterocyclic group" include a halogen atom (e.g. fluorine, chlorine,. bromine, iodine and the like), a nitro group, a cyano. group, a hydroxy group, an optionally halogenated lower. alkyl group (e.g. optionally halogenated $C_{1-6}$ alkyl group such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl, etc.), an optionally halogenated lower alkoxy group (e.g. $C_{1-6}$ alkoxy group such as methoxy, ethoxy, propoxy, isopropoxy, butoxy, isobutoxy, pentyloxy, hexyloxy etc., difluoromethoxy etc.), an amino group, a mono-lower alkylamino group (e.g. mono-$C_{1-6}$ alkylamino group such as methylamino, ethylamino, etc.), a.di-lower alkylamino group (e.g. di-$C_{1-6}$ alkylamino group such as dimethylamino, diethylamino, etc.), a carboxyl group, a lower alkylcarbonyl group (e.g. $C_{1-6}$ alkylcarbonyl group such as acetyl, propionyl, etc.), a lower alkoxycarbonyl group (e.g. $C_{1-6}$ alkoxycarbonyl group such as methoxycarbonyl, ethoxycarbonyl, propoxycarbonyl, butoxycarbonyl, etc.), an aralkyloxycarbonyl group (e.g. $C_{7-11}$ aralkyloxycarbonyl group such as benzyloxycarbonyl, etc.), a carbamoyl group, a thiocarbamoyl group, a mono-lower alkylcarbamoyl group (e.g. mono-$C_{1-6}$ alkylcarbamoyl group such as methylcarbamoyl, ethylcarbamoyl, etc.), a di-lower alkylcarbamoyl group (e.g. di-$C_{1-6}$ alkylcarbamoyl group such as dimethylcarbamoyl, diethylcarbamoyl, etc.), an aryl-carbamoyl group (e.g. $C_{6-10}$ aryl-carbamoyl group such as phenylcarbamoyl, naphthylcarbamoyl, etc.), an aryl group (e.g. $C_{6-10}$ aryl group such as phenyl, naphthyl,. etc.), an aryloxy group (e.g. $C_{6-10}$ aryloxy group such as phenyloxy, naphthyloxy, etc.), an optionally halogenated lower alkylcarbonylamino group (e.g. optionally halogenated $C_{1-6}$ alkylcarbonylamino group such as acetylamino, trifluoroacetylamino, etc.), a mercapto group, a sulfinyl group, a sulfonyl group, a $C_{1-6}$ alkylsulfinyl group, a $C_{1-6}$ alkylsulfonyl group, a $C_{1-6}$ alkylthio group, an arylthio group, an arylsulfinyl group, an arylsulfonyl group, (said aryl group may be substituted with a halogen, an optionally substituted hydrocarbon group, an alkoxy group, a cyano group, a nitro group, a hydroxy group, etc.), an aromatic heterocyclic group, an aromatic heterocyclic-oxy group, an aromatic heterocyclic-thio group, an aromatic heterocyclic-sulfinyl group, an aromatic heterocyclic-sulfonyl group (said aromatic heterocyclic group may be substituted with a halogen, an optionally substituted hydrocarbon group, an alkoxy group, a cyano group, a nitro group, a hydroxy group, etc.). Here, examples of the "optionally substituted hydrocarbon group" include a group hereinafter exemplified for R, and examples of the aromatic heterocyclic group include a group exemplified below.

Examples of the "aromatic heterocyclic group" include a 5- to 6-membered aromatic monocyclic heterocyclic group. having one. or more hetero atoms selected from a nitrogen atom, a sulfur atom and an oxygen atom in addition to carbon atoms as a ring constituent atom (e.g. 2- or 3-furyl, 2- or 3- thienyl,. 2- or 3-pyrrolyl, 2-, 4- or 5-oxazolyl, 3-, 4- or 5-isoxazolyl, 2-, 4- or 5-thiazolyl, 3-, 4- or 5-isothiazolyl, 2-, 4- or 5-imidazolyl, 3-, 4-or 5-pyrazolyl, 4- or 5-1,2,3-oxadiazolyl, 3- or 5-1,2,4-oxadiazolyl, 2-1,3,4-oxadiazolyl, furazanyl, 4- or 5-1,2,3-thiadiazolyl, 3- or 5-1,2,4-thiadiazolyl, 2-1,3,4-thiadiazolyl, 4- or 5-1H-1,2,3-triazolyl, 3-1,2,4-triazolyl, 5-1H-tetrazolyl, 2-, 3- or 4-pyridyl, 2-, 4-or 5-pyrimidinyl, 3- or 4-pyridazinyl, 2-pyrazinyl, 3-, 5- or 6-1,2,4-triazinyl, 2-1,3,5-triazinyl, etc.).

Said phenyl group or aromatic heterocyclic group may be substituted with. one or more, preferably 1 to 3. substituents described above at a substitutable position, and when the number of substituents is 2 or more,. respective substituents may be the same or different. As these "substituted phenyl group", for example, 2,6-difluorophenyl, 2-chloro-6-fluorophenyl, 2,6-dichlorophenyl.and the like are exemplified, and as the " substituted aromatic heterocyclic group", 3-chloropyridin-2-yl and the like are exemplified.

$Q_2$ represents a substituted phenyl group, an alkyl group or a haloalkyl group.

As the substituent for the "substituted phenyl. group", for example, a halogen atom (e.g. fluorine, chlorine, bromine, iodine etc.), a nitro group, a cyano group, a hydroxy group, an optionally halogenated lower alkyl group (e. g. optionally halogenated $C_{1-6}$ alkyl group such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl, etc.), an optionally halogenated lower alkoxy group (e.g. $C_{1-6}$ alkoxy group such as methoxy, ethoxy, propoxy, isopropoxy, butoxy, isobutoxy, pentyloxy, hexyloxy etc., halo$C_{1-6}$ alkoxy group such as difluoromethoxy, 1,1,2,2-tetrafluoroethoxy, 1,1,2,3,3,3-hexafluoro-1-propoxy, etc.), an amino group, a mono-lower alkylamino group (e.g. mono-$C_{1-6}$ alkylamino group such as methylamino, ethylamino, etc.), a di-lower alkylamino group (e.g. di-$C_{1-6}$ alkylamino group such as dimethylamino, diethylamino, etc.), a carboxyl group, a lower alkylcarbonyl group (e.g. $C_{1-6}$ alkylcarbonyl group such as acetyl, propionyl, etc.), a lower alkoxycarbonyl group (e.g. $C_{1-6}$ alkoxycarbonyl group such as methoxycarbonyl, ethoxycarbonyl,. propoxycarbonyl, butoxycarbonyl, etc.), an aralkyloxycarbonyl group, a carbamoyl group, a thiocarbamoyl group, a mono-lower alkylcarbamoyl group (e.g. mono-$C_{1-6}$ alkylcarbamoyl group such as methylcarbamoyl, ethylcarbamoyl, etc.), a di-lower alkylcarbamoyl group (e.g. di-$C_{1-6}$ alkylcarbamoyl group such as dimethylcarbamoyl, diethylcarbamoyl, etc.), an aryl-carbamoyl group (e.g. $C_{6-10}$ aryl-carbamoyl group such as phenylcarbamoyl, naphthylcarbamoyl, etc.), an aryl group (e.g. $C_{6-10}$ aryl group such as phenyl, naphthyl, etc.), an optionally substituted aryloxy group (e.g. $C_{6-10}$ aryloxy group such as phenyloxy, naphthyloxy, etc., phenyloxy substituted with halogen such as fluorine, chlorine etc., $C_{1-6}$ alkyl such as methyl, ethyl etc., or halo$C_{1-6}$ alkyl such as trifluoromethyl, etc.), an optionally halogenated lower alkylcarbonylamino group. (e.g. optionally halogenated $C_{1-6}$ alkylcarbonylamino group such as acetylamino, trifluoroacetylamino, etc.), a mercapto group, a sulfinyl group, a sulfonyl group, a $C_{1-6}$ alkylsulfinyl group which may be substituted with a halogen and the like (e.g. methylsulfinyl, ethylsulfinyl, 1-propylsulfinyl, 2-propylsulfinyl, t-butylsulfinyl, difluoromethylsulfinyl, trifluoromethylsulfinyl, 2,2,2-trifluoroethylsulfinyl, 1,1,2,2-tetrafluoroethylsulfinyl, 2-chloro-1,1,2-trifluoroethylsulfinyl, 1,1,2,2,2-pentafluoroethylsulfinyl, 1,1,2,2,3,3,3-heptafluoro-1-propylsulfinyl, 1,1,2,3,3,3-hexafluoro-1-propylsulfinyl, 1,1,1,2,3,3,3-heptafluoro-2-propylsulfinyl, etc.), a $C_{1-6}$ alkylsulfonyl group which may be substituted with a. halogen and the like (e.g. methylsulfonyl, ethylsulfonyl, 1-propylsulfonyl, 2-propylsulfonyl, t-butylsulfonyl, difluoromethylsulfonyl, trifluoromethylsulfonyl, 2,2,2-trifluoroethylsulfonyl, 1,1,2,2-tetrafluoroethylsulfonyl, 2-chloro-1,1,2-trifluoroethylsulfonyl, 1,1,2,2,2-pentafluoroethylsulfonyl, 1,1,2,2,3,3,3-heptafluoro-1-propylsulfonyl, 1,1,2,3,3,3-hexafluoro-1-propylsulfonyl, 1,1,1,2,3,3,3-heptafluoro-2-propylsulfonyl, etc.), a $C_{1-6}$ alkylthio group which may be substituted with a halogen and the like (e.g. methylthio, ethylthio, 1-propylthio, 2-propylthio, t-butylthio, difluoromethylthio, trifluoromethylthio, 2,2,2-trifluoroethylthio, 1,1,2,2-tetrafluoroethylthio, 2-chloro-1,1,2-trifluoroethylthio, 1,1,2,2,2-pentafluoroethylthio, 1,1,2,2,3,3,3-heptafluoro-1-propylthio, 1,1,2,3,3,3-hexafluoro-1-propylthio, 1,1,1,2,3,3,3-heptafluoro-2-propylthio, etc.); arylthio group, arylsulfinyl group and arylsulfinyl group (said aryl group may be substituted with a halogen, an optionally substituted hydrocarbon group, an alkoxy group, a cyano group, a nitro group, a hydroxy group, etc.); a $C_{2-6}$ alkenyloxy group, $C_{2-6}$ alkenylthio group, $C_{2-6}$ alkenylsulfinyl group, $C_{2-6}$ alkenylsulfonyl group, each of which may be substituted with a halogen and the like (e.g. 3,3-dichloroarylthio, 3,3-dichloroarylsulfinyl, 3,3-dichloroarylsulfonyl); a $C_2$-$_6$ alkynyloxy group, $C_{2-6}$ alkynylthio group, $C_{2-6}$ alkynylsulfinyl group, $C_{2-6}$ alkynylsulfonyl group, each of which may be substituted with a halogen and the like (e.g. propargylthio, propargylsulfinyl, propargylsulfonyl); an aromatic heterocyclic group, aromatic heterocyclic-oxy. group, aromatic heterocyclic-thio group, aromatic heterocyclic-sulfinyl group and aromatic heterocyclic-sulfonyl group (these aromatic heterocyclic group (pyridine ring, etc.) may be substituted with a halogen (fluorine, chlorine etc.) an optionally substituted hydrocarbon group (haloalkyl such as trifluoromethyl etc.), an alkoxy. group, a cyano group, a nitro group, a. hydroxy group, etc.), and the like are used. Here, examples of the "optionally substituted hydrocarbon group" include a group hereinafter exemplified for R, and examples of the aromatic heterocyclic group include a group exemplified above.

The "phenyl group" may have one or more, preferably 1 to 3 substituents described above at. a substitutable position, and when the number of substituents is 2 or more, respective substituents may be the same or different.

Examples of the "alkyl group" and "haloalkyl group" represented by $Q_2$ include the same groups as those exemplified with respect to $Y_1$-$Y_4$.

Representative examples of compound (I) include a compound represented by the formula (Ia) to (Ie) above.

R in the compound represented by the formula (Ic) represents a hydrogen atom, a halogen atom, a group via optionally oxidized sulfur atom, a nitro group, a nitroso group, a formyl group, an optionally substituted hydroxy group, a cyano group, an optionally substituted hydrocarbon group, an optionally substituted amino group, an optionally substituted heterocyclic group, an optionally substituted $C_{7-11}$ aralkyl group, an optionally substituted $C_{2-7}$ acyl group, an optionally substituted $C_{1-6}$ alkoxycarbonyl group, an optionally substituted $C_{7-11}$ aralkyloxycarbonyl group, an optionally substituted $C_{6-10}$ aryloxycarbonyl group, an optionally substituted carbamoyl group, an optionally substituted thiocarbamoyl group, an optionally substituted hydroxyoxalyl group, an optionally substituted aminooxalyl group or an optionally substituted hydroxyaminooxalyl group.

Examples of. the "halogen atom" represented by R include fluorine, chlorine, bromine, iodine, etc.

Examples of the "group via optionally oxidized sulfur. atom" include an optionally substituted thio group, an optionally substituted sulfinyl group, an optionally substituted sulfonyl group, and the like. For example, $C_{1-6}$ alkylthio, $C_{1-6}$ alkylsulfinyl, $C_{1-6}$ alkylsulfonyl (e.g. methylsulfonyl etc.), $C_{6-10}$ arylthio, $C_{6-10}$ arylsulfinyl, $C_{6-10}$ arylsulfonyl (e.g. 4-methylphenylsulfonyl etc.), $C_{1-7}$ acylthio, $C_{1-7}$ acylsulfinyl, $C_{1-7}$ acylsulfonyl, $C_{1-6}$ alkoxycarbonylthio (e.g. methoxycarbonylthio etc.), $C_{1-6}$ alkoxycarbonylsulfinyl, $C_{1-6}$ alkoxycarbonylsulfonyl, $C_{6-10}$ aryloxycarbonylthio, $C_{6-10}$ aryloxycarbonylsulfinyl, $C_{6-10}$ aryloxycarbonylsulfonyl, $C_{1-6}$ alkylmono- and di-substituted aminothio, $C_{1-6}$ alkylmono- and di-substituted aminosulfinyl, $C_{1-6}$ alkylmono- and di-substituted aminosulfonyl, $C_{6-10}$ arylmono- and di-substituted aminothio, $C_{6-10}$ arylmono- and di-substituted aminosulfinyl, $C_{6-10}$ arylmono- and di-substituted aminosulfonyl, and the like are exemplified. These groups may have further one or more substituents at a substitutable position such as haloalkylsulfonyl group (e.g. trifluoromethylsulfonyl), and the substituents include the same groups as those hereinafter exemplified with respect to the "substituents" for the "optionally. substituted hydrocarbon group".

Examples of the "optionally substituted hydroxy group" include, in addition to a hydroxy group, a $C_{1-6}$ alkoxy group (e.g. methoxy, ethoxy, propoxy, isopropoxy, butoxy, pentyloxy, hexyloxy. etc.), $C_{6-14}$ aryloxy group (e.g. phenoxy, naphthoxy etc.), $C_{7-15}$ aralkyloxy group (e.g. benzyloxy, naphthylmethyloxy etc.).

Examples of the "hydrocarbon group" for the "optionally substituted hydrocarbon group" include an aliphatic hydrocarbon group, a monocyclic saturated hydrocarbon group and an aromatic hydrocarbon group, and those having

1 to 16 carbons are preferred. Specifically, for example, alkyl group, alkenyl group, alkynyl group, cycloalkyl group, aralkyl group and aryl group are used.

The "alkyl group" is preferably, for example, a lower alkyl group, and a $C_{1-6}$ alkyl group such as methyl, ethyl, propyl, isopropyl, butyl, isobutyl, sec-butyl, t-butyl, pentyl, hexyl, etc. are used generally.

The "alkenyl group" is preferably, for example, a lower alkenyl group, and a $C_{2-6}$ alkenyl group such as vinyl, 1-propenyl, allyl, isopropenyl, butenyl, isobutenyl, etc. are used generally.

The "alkynyl group" is preferably, for example, a lower alkynyl group, and a $C_{2-6}$ alkynyl group such as ethynyl, propargyl, 1-propynyl, etc. are used generally.

The "cycloalkyl group" is preferably, for example, a lower cycloalkyl group, and a $C_{3-6}$ cycloalkyl group such as cyclopropyl, cyclobutyl, cyclopentyl, cyclohexyl, etc. are used generally.

The "aralkyl group" is preferably a $C_{7-11}$ aralkyl group such as benzyl, phenethyl, etc., and benzyl group is used generally.

The "aryl group" is preferably $C_{6-14}$ aryl group such as phenyl, 1-naphthyl, 2-naphthyl, biphenylyl, 2-anthryl, etc., and for example, phenyl group is used generally.

Examples of the substituents which may be possessed by the "hydrocarbon group" of the "optionally substituted hydrocarbon group" include a halogen atom (e.g. fluorine, chlorine, bromine, iodine and the like), a nitro group, a cyano group, a hydroxy group, an optionally halogenated lower alkyl group (e.g. optionally halogenated $C_{1-6}$ alkyl group such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl, etc.), a lower alkoxy group (e.g. $C_{1-6}$ alkoxy group such as methoxy, ethoxy, propoxy, isopropoxy, butoxy, isobutoxy, pentyloxy, hexyloxy etc.), an amino group, a mono-lower alkylamino group (e.g., mono-$C_{1-6}$ alkylamino group such as. methylamino, ethylamino, etc.), a di-lower alkylamino group (e.g. di-$C_{1-6}$ alkylamino group such as dimethylamino, diethylamino, etc.), a carboxyl group, a lower alkylcarbonyl group (e.g. $C_{1-6}$ alkylcarbonyl group such as acetyl, propionyl, etc.), a lower alkoxycarbonyl group (e.g. $C_{1-6}$ alkoxycarbonyl group such as methoxycarbonyl, ethoxycarbonyl, propoxycarbonyl, butoxycarbonyl, etc.), a carbamoyl group, a thiocarbamoyl group, a mono-lower alkylcarbamoyl group (e.g. mono-$C_{1-6}$ alkylcarbamoyl group such as methylcarbamoyl, ethylcarbamoyl, etc.), a di-lower alkylcarbamoyl group (e.g. di-$C_{1-6}$ alkylcarbamoyl group such as dimethylcarbamoyl, diethylcarbamoyl, etc.), an aryl-carbamoyl group (e.g. $C_{6-10}$ aryl-carbamoyl group such as phenylcarbamoyl, naphthylcarbamoyl, etc.), an aryl group (e.g. $C_{6-10}$ aryl group such as phenyl, naphthyl, etc.), an aryloxy group (e.g. $C_{6-10}$ aryloxy group such as phenyloxy, naphthyloxy, etc.), an optionally halogenated lower. alkylcarbonylamino group (e.g. optionally halogenated $C_{1-6}$ alkylcarbonylamino group such as acetylamino, trifluoroacetylamino, etc.), an oxo group, a $C_{1-6}$ alkylthio group (e.g. methylthio etc.), a $C_{1-6}$ alkylsulfinyl group (e.g. methylsulfinyl etc.), a $C_{1-6}$ alkylsulfonyl group (e.g. methylsulfonyl etc.), a $C_{6-10}$ arylthio group, a $C_{6-10}$ arylsulfinyl group, a $C_{6-10}$ arylsulfonyl group, and the like. The "hydrocarbon group" of the "optionally substituted hydrocarbon group" may have one or more, preferably 1 to 3 above-mentioned substituents at a substitutable position of the hydrocarbon group, and when the number of substituents is 2 or.. more, respective substituents may be the same or different.

Preferable examples of the "hydrocarbon group" of the. "optionally substituted hydrocarbon group" represented by R include an alkyl group (e.g. $C_{1-6}$ alkyl group such as methyl, ethyl, propyl, isopropyl, etc.), an alkenyl group (e.g. $C_{2-6}$ alkenyl group such as vinyl, etc.), an alkynyl group (e.g. $C_{2-6}$ alkynyl group such as ethynyl, etc.), a cycloalkyl group (e.g. $C_{3-6}$ cycloalkyl group such as cyclopropyl, cyclobutyl, cyclopentyl, cyclohexyl, etc.), an aralkyl group (e.g. $C_{7-11}$ aralkyl group such as benzyl, phenethyl, etc.), and an aryl group (e.g. $C_{6-14}$ aryl group such as phenyl. etc.), and particularly an alkyl group (e.g. $C_{1-6}$ alkyl group such as methyl, etc.) and cycloalkyl group (e.g. $C_{3-6}$ cycloalkyl group such as cyclopropyl, etc.) are used generally. The "alkyl group", "alkenyl group", "alkynyl group", "cycloalkyl group", "aralkyl group" and "aryl group" may. have 1 to 5, preferably 1 to 3 substituents (preferably. halogen atom such as fluorine, etc.) similar to those optionally possessed by the above-mentioned "hydrocarbon group".

Examples of the "optionally substituted amino group" represented by R include an amino group which may have 1 or 2 of the above-mentioned "optionally substituted hydrocarbon group" as a substituent. Preferable examples of the substituent which may be possessed by the "amino group" include 1 or 2 selected from an optionally substituted lower alkyl group and an optionally substituted aryl group, and particularly one of optionally substituted lower alkyl group is preferred. As the "lower alkyl group", for example, a $C_{1-6}$ alkyl group such as methyl, ethyl, propyl, isopropyl, butyl, isobutyl, sec-butyl, tert-butyl, etc. is used. The "lower alkyl group" may have 1 to 3 substituents similar to those optionally possessed by the above-mentioned "hydrocarbon group". As the "aryl group", a $C_{6-14}$ aryl group such as. phenyl etc. is used. The "aryl group" may have 1 to 5, preferably 1 to 3 substituents (preferably, halogen atom such as fluorine, chlorine etc., $C_{1-6}$ alkoxy group such as methoxy, ethoxy, etc.) similar to those optionally possessed by the above-mentioned "hydrocarbon group". As the "optionally substituted amino group", phenylamino which is substituted with 1 to 3 lower alkoxy groups (e.g. $C_{1-4}$ alkoxy group such as methoxy, etc.) or a mono-alkylamino group which is substituted with lower alkyl group (e.g. $C_{1-4}$ alkyl group such as methyl, ethyl, propyl, butyl, tert-butyl, etc.) is used generally.

Examples of the "heterocyclic group" for the "optionally substituted heterocyclic group" represented by R include a 5- to 14-membered (preferably 5- to 10-membered) (monocyclic to tricyclic, preferably monocyclic or bicyclic) heterocyclic group containing 1 to 4 (preferably 1 to 3) of one. or two kinds of hetero atoms selected from a nitrogen atom, an oxygen atom and. a sulfur atom in addition to carbon atoms. For example, a 5-membered cyclic ring group containing 1 to 4 hetero atoms selected from an oxygen atom, a sulfur atom and a nitrogen atom in addition to carbon atoms such as 2- or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4- or 5-isothiazolyl, 3-, 4- or 5-pyrazolyl, 2-, 3-, or 4-pyrazolidinyl, 2-, 4- or 5-imidazolyl, 4- or 5-1H-1,2,3-triazolyl, 3- or 5-1,2,4-triazolyl, 5-1H- or 5-2H-tetrazolyl, etc.; a 6-membered cyclic ring group containing 1 to 4 hetero atoms selected from an oxygen atom, a sulfur atom and a nitrogen atom in addition to carbon atoms such as 2-, 3- or 4-pyridyl, N-oxido-2-, 3-or 4-pyridyl, 2-, 4- or 5-pyrimidinyl, N-oxido-2-, 4- or 5-pyrimidinyl, 1-, 2- or 3-thiomorpholinyl, 1-, 2- or 3-morpholinyl, 1-, 2-, 3- or 4-piperidino, 2-, 3- or 4-piperidyl, 2-, 3- or 4-thiopyranyl, 2-, 3- or 4-4H-1,4-oxazinyl, 2-, 3- or 4-4H-1,4-thiazinyl, 1, 3-thiazinyl, 1-or 2-piperazinyl, 3-, 5- or 6-1,2,4-triazinyl, 2-1,3,5-triazinyl, 3- or 4-pyridazinyl, 2-pyrazinyl, N-oxido-3-or 4-pyridazinyl, etc.; a bicyclic or tricyclic. condensed ring group containing 1 to 4 hetero atoms selected from an oxygen atom, a sulfur atom and a nitrogen atom in addition to carbon atoms (preferably a group formed by condensing the above-mentioned 5- to 6-membered cyclic ring with 1 or 2 of a 5- to 6-membered cyclic ring group optionally containing 1 to 4 hetero: atoms selected from an oxygen atom, a sulfur atom and a nitrogen atom in addition to carbon atoms) such as 1-, 2-, 3-, 4-, 5-, 6- or 7-indolyl, 2-, 3-, 4-, 5-, 6- or 7-benzofuryl, 2-; 4-, 5-, 6- or 7-benzothiazolyl, 2-, 4-, 5-, 6- or 7-benzoxazolyl, 1-, 2-, 4-, 5-, 6- or 7-benzimidazolyl, 2-, 3-, 4-, 5-, 6-, 7- or 8-quinolyl, 1-, 3-, 4-,. 5-, 6-, 7- or 8-isoquinolyl, 1-, 4-, 5-, 6- or 7-phthalazinyl, 2-, 4-, 5-, 6-, 7- or 8-quinazolinyl, 2-, 3-, 5-, 6-, 7- or 8-quinoxalinyl, 1-, 2-, 3-, 5-, 6-, 7-or 8-indolizinyl, 1-, 2-, 3-, 4-, 6-, 7-, 8- or 9- quinolizinyl, 2-, 3-, 4-, 5-, 6- or 7-1,8-naphthyridinyl, 1-, 2-, 3-, 4-, 5-, 6-, 7- or 8-dibenzofuranyl, 1-, 2-, 3-, 4-, 5-, 6-, 7-, 8- or 9-carbazolyl, 1-, 2-, 3-, 4-, 5-, 6-, 7- or 8-acridinyl, 1-, 2-, 3-, 4-, 5-, 6-, 7-, 8-or 9-phenanthridinyl, 1-, 3-, 4-, 5-, 6-, 7- or 8-chromanyl, 1-, 2-, 3-, 4-, 6-, 7-, 8-, 9- or 10-phenothiazinyl, 1-, 2-, 3-, 4-, 6-, 7-, 8-. or. 9-phenoxazinyl, and the like are used. Among these, a 5- to 7-membered (preferably 5- or 6-membered) heterocyclic group containing 1 to 3 hetero atoms selected from an oxygen atom, a sulfur atom and a nitrogen atom in addition to carbon atoms is preferred.

Preferable examples of the "heterocyclic group" for the "optionally substituted heterocyclic group" represented by R include a 5- or 6-membered heterocyclic group containing 1 to 3 hetero atoms selected from a nitrogen atom, an oxygen atom and a sulfur atom in addition to carbon atoms. Specifically, for example, 1-, 2- or 3-pyrrolidinyl, 2- or 4-imidazolinyl, 2-, 3-, or 4-pyrazolidinyl, 1-, 2-, 3- or 4-piperidyl, 2-, 3- or 4-piperidyl, 1- or 2-piperazinyl, morpholinyl, 2- or 3-thienyl, 2-, 3- or 4-pyridyl, 2-furyl or 3-furyl, pyrazinyl, 2-pyrimidinyl, 3-pyrrolyl, 3-pyridazinyl, 3-isothiazolyl, 3-isoxazolyl and the like are exemplified. Particularly preferably, a 6-membered nitrogen-containing heterocyclic group (e.g. pyridyl, etc.) is used.

As the substituent that may be possessed by the "heterocyclic group" of the "optionally substituted heterocyclic group", for example, a halogen atom (e.g.. fluorine, chlorine, bromine, iodine and the like), a lower alkyl group (e.g. $C_{1-6}$ alkyl group such as methyl, ethyl, propyl, isopropyl, butyl, isobutyl, sec-butyl, tert-butyl, pentyl, hexyl, etc.), a cycloalkyl group (e.g. $C_{3-6}$ cycloalkyl group such as cyclopropyl, cyclobutyl, cyclopentyl, cyclohexyl, etc.), a lower alkynyl group (e.g. $C_{2-6}$ alkynyl group such as ethynyl, 1-propynyl, propargyl, etc.), a lower alkenyl group (e.g. $C_{2-6}$ alkenyl group such as vinyl, allyl, isopropenyl, butenyl, isobutenyl, etc.), an aralkyl group (e.g. $C_{7-11}$ aralkyl group such as benzyl, $\alpha$-methylbenzyl, phenethyl, etc.), an aryl group (e.g. $C_{6-10}$ aryl group such as phenyl, naphthyl, etc., preferably phenyl, etc.), a lower alkoxy group (e.g. $C_{1-6}$ alkoxy group such as methoxy, ethoxy, propoxy, isopropoxy, butoxy, isobutoxy, sec-butoxy, tert-butoxy, etc.), an aryloxy group (e.g. $C_{6-10}$ aryloxy group such as phenyloxy, etc.), a lower alkanoyl group (e.g. $C_{1-6}$ alkylcarbonyl group such as formyl, acetyl, propionyl, butyryl, isobutyryl, etc.), an arylcarbonyl (e.g. $C_{6-10}$ arylcarbonyl group such as benzoyl, naphthoyl, etc.), a lower alkanoyloxy group (e.g. $C_{1-6}$ alkylcarbonyloxy group such as formyloxy, acetyloxy, propionyloxy, butyryloxy, isobutyryloxy, etc.), an arylcarbonyloxy group (e.g. $C_{6-10}$ arylcarbonyloxy group such as benzoyloxy, naphthoyloxy, etc.), a carboxyl group, a lower alkoxycarbonyl group (e.g. $C_{1-6}$ alkoxycarbonyl group such as methoxycarbonyl, ethoxycarbonyl, propoxycarbonyl, isopropoxycarbonyl, butoxycarbonyl, isobutoxycarbonyl, tert-butoxycarbonyl, etc.), an aralkyloxycarbonyl group (e.g. $C_{7-11}$ aralkyloxycarbonyl group such as benzyloxycarbonyl, etc.), a carbamoyl group, mono-, di- or tri-halogeno-lower alkyl group (e.g. mono-, di- or tri-halogeno-$C_{1-4}$ alkyl group such as chloromethyl, dichloromethyl, trifluoromethyl, 2,2,2-trifluoroethyl, etc.), an oxo group, an amidino group, an imino group, an amino group, a mono-lower alkylamino group (e.g. mono-$C_{1-4}$ alkylamino group such as methylamino, ethylamino, propylamino, isopropylamino, butylamino, etc.), a di-lower alkylamino group (e.g. di-$C_{1-4}$ alkylamino group such as dimethylamino, diethylamino, dipropylamino, diisopropylamino, dibutylamino, methylethylamino, etc.), 3- to 6-membered cyclic amino group which may contain 1 to 3 hetero atoms selected from an oxygen atom, a sulfur atom and a nitrogen atom in addition to carbon atoms and one nitrogen atom (e.g. 3-to 6-membered cyclic amino group such' as aziridinyl, azetidinyl, pyrrolidinyl, pyrrolinyl, pyrrolyl, imidazolyl, pyrazolyl, imidazolidinyl, piperidinyl, morpholinyl, dihydropyridyl, pyridyl, N-methylpiperazinyl, N-ethylpiperazinyl, etc.), alkylenedioxy group (e.g. $C_{1-3}$ alkylenedioxy such as methylenedioxy, ethylenedioxy, etc.), a hydroxy group, a nitro group, a cyano group, a mercapto group, a sulfo group, a sulfino group, a phosphono group, a sulfamoyl group, a

mono-alkylsulfamoyl group (e.g. mono-$C_{1-6}$ alkylsulfamoyl group such as N-methylsulfamoyl, N-ethylsulfamoyl, N-propylsulfamoyl, N-isopropylsulfamoyl, N-butylsulfamoyl, etc.), a dialkylsulfamoyl group (e.g. di- $C_{1-6}$ alkylsulfamoyl group such as N,N-dimethylsulfamoyl, N,N-diethylsulfamoyl, N,N-dipropylsulfamoyl, N,N-dibutylsulfamoyl, etc.), an alkylthio group (e.g. $C_{1-6}$ alkylthio group such as methylthio, ethylthio, propylthio, isopropylthio, butylthio, sec-butylthio, tert-butylthio, etc.), an arylthio group (e.g. $C_{6-10}$ arylthio group such as phenylthio, naphthylthio, etc.), a lower alkylsulfinyl group (e.g. $C_{1-6}$ alkylsulfinyl group such as methylsulfinyl, ethylsulfinyl, propylsulfinyl, butylsulfinyl, etc.), an arylsulfinyl group (e.g. $C_{6-10}$ arylsulfinyl group such as phenylsulfinyl, naphthylsulfinyl, etc.), a lower alkylsulfonyl group (e.g. $C_{1-6}$ alkylsulfonyl group such as methylsulfonyl, ethylsulfonyl, propylsulfonyl, butylsulfonyl, etc.), an arylsulfonyl group (e. g. $C_{6-10}$ arylsulfonyl group such as phenylsulfonyl, naphthylsulfonyl, etc.), and the like are used.

The "heterocyclic group" of the "optionally substituted heterocyclic group" may have one or more, preferably 1 to 3 above-mentioned substituents at a substitutable position of heterocyclic group, and when the number of substituents is 2 or more, respective substituents may be the same or different.

Examples of the "acyl group" for the "optionally substituted acyl group" represented by R include a lower alkylcarbonyl group (e.g. $C_{1-6}$ alkylcarbonyl group such as acetyl, propionyl, pivaloyl, etc.). In addition, as its substituent, the same substituents as those for the above-mentioned "optionally substituted hydrocarbon group" are exemplified, and one or more substituents may be present at a substitutable position.

The "substituted acyl group" includes, specifically, trifluoromethylcarbonyl, bromomethylcarbonyl, methylthiomethylcarbonyl, methylsulfinylmethylcarbonyl, methylsulfonylmethylcarbonyl, and the like.

Examples of the "alkoxycarbonyl group" for the "optionally substituted alkoxycarbonyl group" represented by R include a $C_{1-6}$ alkoxycarbonyl group (e.g. methoxycarbonyl, ethoxycarbonyl, propoxycarbonyl, isopropoxycarbonyl, butoxycarbonyl, t-butoxycarbonyl, pentyloxycarbonyl, hexyloxycarbonyl, etc.). In addition, as its substituent, the same substituents as those for the above-mentioned "optionally substituted hydrocarbon group" are exemplified, and one or more substituents may be. present at a substitutable position.

Examples of the "aralkyloxycarbonyl group" for the "optionally substituted aralkyloxycarbonyl group" represented by R include $C_{7-11}$ aralkyloxycarbonyl group (e.g. benzyloxycarbonyl, etc.). In addition, as its substituent, the same substituents as those for the above-mentioned "optionally substituted hydrocarbon group" are exemplified, and one or more substituents may be present at a substitutable position.

Examples of the "aryloxycarbonyl group" for the "optionally substituted aryloxycarbonyl group" represented by R include $C_{6-14}$ aryloxycarbonyl group (e.g. phenoxycarbonyl, naphthoxycarbonyl, etc.). In addition, as its substituent, the same substituents as those for the above-mentioned "optionally substituted hydrocarbon group" are exemplified, and one or more substituents may be present at a substitutable position.

Examples of the "substituent" for the "optionally substituted carbamoyl" and "optionally substituted thiocarbamoyl" represented by R include the same groups as the substituents exemplified for the above-mentioned "optionally substituted hydrocarbon group", and one or more substituents may be present at a substitutable position. As the "substituted carbamoyl", specifically, dimethylaminocarbonyl and the like are. exemplified.

Examples of the "optionally substituted hydroxyoxalyl group" represented by R include a hydroxyoxalyl group, an alkoxyoxalyl group (e.g. $C_{1-6}$ alkoxyoxalyl group such as methoxyoxalyl, etc.), an aralkyloxyoxalyl group (e.g. $C_{7-11}$ aralkyloxyoxalyl group such as benzyloxyoxalyl, etc.), an aryloxyoxalyl group (e.g. $C_{6-14}$ aryloxyoxalyl such as phenoxyoxalyl, etc.). In addition, the "optionally substituted hydroxyoxalyl group" may have further one or more substituents at a substitutable position, and the "substituent" includes the same groups as the substituents exemplified for the above-mentioned "optionally substituted hydrocarbon group". As such the "optionally substituted hydroxyoxalyl group" having a further substituent, benzyloxyoxalyl and the like are exemplified.

Examples of the "optionally substituted aminooxalyl group" represented by R include aminooxalyl group, N-alkylaminooxalyl group (e.g. N-$C_{1-6}$ alkylaminooxalyl group such as N-methylaminooxalyl, etc.), N,N-dialkylaminooxalyl group (e.g. N,N-di$C_{1-6}$ alkylaminooxalyl group such as N,N-dimethylaminooxalyl, N-methyl-N-ethylaminooxalyl, etc.). In addition, the "optionally substituted aminooxalyl group" may have further one or more substituents at a. substitutable position, and the "substituent" includes the same groups as the. substituents exemplified for the above-mentioned "optionally substituted hydrocarbon group".

Examples of the "optionally substituted hydroxyaminooxalyl group" represented by R include hydroxyaminooxalyl group, $C_{1-6}$ alkoxyaminooxalyl group (e.g. methoxyaminooxalyl, etc.), $C_{7-11}$ aralkyloxyaminooxalyl group (e. g. benzyloxyaminooxalyl, etc.), $C_{6-14}$ aryloxyaminooxalyl (e.g. phenoxyaminooxalyl, etc.), N-$C_{1-6}$ alkoxy-N-$C_{1-6}$ alkylaminooxalyl group (e.g. N-methoxy-N-methylaminooxalyl, etc.), N-$C_{1-6}$ alkylhydroxyaminooxalyl group (e.g. N-methylhydroxyaminooxalyl, etc.), and the like. In addition, the "optionally substituted hydroxyaminooxalyl group" may have further one or more substituents at a substitutable position, and the "substituent" includes the same groups as the substituents exemplified for the above-mentioned "optionally substituted hydrocarbon. group". As such the "optionally substituted hydroxyaminooxalyl group" having a further substituent, benzyloxyaminooxalyl and the like are exemplified.

R is preferably a hydrogen atom, $C_{1-6}$ alkyl group, $C_{2-6}$ alkenyl group, $C_{2-6}$ alkynyl group, $C_{7-11}$ aralkyl group,

$C_{6-10}$ aryl group, $C_{1-6}$ alkoxycarbonyl group, $C_{7-11}$ aralkyloxycarbonyl group, $C_{6-10}$ aryloxycarbonyl group, $C_{1-6}$ alkoxycarbonylthio group, N-$C_{1-6}$ alkylcarbamoyl group, N,N-di($C_{1-6}$ alkyl)carbamoyl group, $C_{2-7}$ acyl group (which may be substituted with a halogen atom, $C_{1-6}$ alkylthio group, $C_{1-6}$ alkylsulfinyl group or $C_{1-6}$ alkylsulfonyl group), $C_{1-6}$ alkylsulfone group, $C_{6-10}$ arylsulfone group, halo$C_{1-6}$ alkylsulfone group, nitroso group, $C_{1-6}$ alkoxyoxalyl group, hydroxyoxalyl group, aminooxalyl group, N-($C_{1-6}$ alkoxy, $C_{7-11}$ aralkyloxy or $C_{1-6}$ alkyl)aminooxalyl group, N,N-di($C_{1-6}$ alkyl) aminooxalyl group, or N,O- di($C_{1-6}$ alkyl)hydroxyaminooxalyl group.

In compound (I), the substituent of the substituted phenyl group for $Q_2$ is preferably at least the one selected from a halogen atom; $C_{1-6}$ alkyl group or halo$C_{1-6}$ alkyl group; phenyloxy group, phenylthio group, phenylsulfinyl group or phenylsulfonyl group, each of which is substituted with a halogen atom, $C_{1-6}$ alkyl group or halo$C_{1-6}$ alkyl group; pyridyloxy group, pyridylthio group, pyridylsulfinyl group or pyridylsulfonyl group, each of which is substituted with a halogen atom, $C_{1-6}$ alkyl group or halo$C_{1-6}$ alkyl group; $C_{1-6}$ alkoxy group, $C_{1-6}$ alkylthio group, $C_{1-6}$ alkylsulfinyl group, $C_{1-6}$ alkylsulfonyl group, $C_{2-4}$ alkenyloxy group, $C_{2-4}$ alkenylthio group, $C_{2-4}$ alkenylsulfinyl group, $C_{2-4}$ alkenylsulfonyl group, $C_{2-4}$ alkynyloxy group, $C_{2-4}$ alkynylthio group, $C_{2-4}$ alkynylsulfinyl group and $C_{2-4}$ alkynylsulfonyl group.

Further, the compound (I) wherein $Q_1$ is difluorophenyl or chlorofluorophenyl is also preferred.

Compound (I) can occur. as isomers such as tautomer, steric isomer and the like, and. in the present specification, the compound represented by the formula (I) encompasses such isomers and mixtures thereof.

Acidic groups such as sulfonyl group, carboxyl group etc. in the substituents in the molecule of Compound (I) can form agrochemically acceptable basic salts with an inorganic base, organic base etc., and basic nitrogen atoms in the molecule and basic groups such as amino acid groups in substituent groups can form agrochemically acceptable acid addition salts with an inorganic acid, organic acid etc. The inorganic basic salts include, for example, salts with alkali metals (e.g., sodium, potassium etc.), alkaline earth metals (e.g., calcium etc.) and ammonia etc., and the organic basic salts include salts with e.g. dimethylamine, triethylamine, N,N-dimethylaniline, piperazine, pyrrolidine, piperidine, pyridine, 2-phenylethylamine, benzylamine, ethanolamine, diethanolamine, 1,8-diazabicyclo[5,4,0]undecene (abbreviated hereinafter as DBU). etc.' The inorganic acid addition salts of Compound (I) include salts with e.g. hydrochloric acid, hydrobromic acid, hydriodic acid, sulfuric acid, nitric acid, phosphoric acid, perchloric acid etc., and the organic acid addition salts of Compound (I) include salts with e.g. formic acid, acetic acid, propionic acid, oxalic acid, succinic acid, benzoic acid, p-toluenesulfonic acid, methanesulfonic acid, trifluoroacetic acid etc.

Compound (I) or a salt thereof can be produced according to a method described in e.g. JP-A 46-6650 or following Examples, by allowing to react a compound represented by the above-mentioned formula (II) with a compound represented by the formula (III) in the presence of base such as. alkali metal alcoholate such as sodium ethylate, sodium methylate, potassium tert-butoxide etc., organic base such as triethylamine, diisopropylethylamine, pyridine, 4-dimethylaminopyridine, N,N-dimethylaniline etc., inorganic base such as potassium carbonate, sodium carbonate, sodium hydroxide, potassium hydroxide, sodium hydrogen carbonate, potassium hydrogen carbonate etc.,. metal hydride such as lithium hydride, sodium hydride, potassium hydride etc. and organic lithium reagent such as butyl lithium, lithium diisopropylamide etc., in solvent such as aliphatic hydrocarbons such as pentane, hexane, heptane, petroleum ether etc., aromatic hydrocarbons such as benzene, toluene, xylene etc., esters such as methyl acetate, ethyl acetate, ethyl formate, ethyl propionate etc., ketones. such as acetone, methyl ethyl ketone etc., ethers such as diethyl ether, dipropyl ether, diisopropyl ether, dibutyl ether, tetrahydrofuran, dioxane etc.,. nitriles such as acetonitrile, propionitrile, etc., acid amides such as dimethylformamide, dimethylacetamide, etc., cyclic amides such as 1-methyl-2-pyrrolidone etc., cyclic ureas such as 2,3-dimethyl-2-imidazolidinone etc., sulfoxides such as dimethylsulfoxide etc., sulfones such as sulfolane etc., phosphoric acid amides such as hexamethylphosphoramide etc., halogenated hydrocarbons such as dichloromethane, chloroform, 1,2-dichloroethane, carbon tetrachloride etc., aromatic amines such as pyridine, picoline, lutidine, quinoline etc., and mixtures thereof, water, and further mixed solvents thereof with water. An amount of 'the base to be used is not particularly limited as far as it does not. adversely affect the reaction, and it can be used in large excess also doubling as a solvent.

The reaction temperature is usually about -50 to. 200°C, preferably about -30 to 150°C. The reaction time is generally about 0.1 to 96 hours, preferably 0.1 to 72 hours, more preferably about 0.1 to 24 hours.

In the compound of formula (III), the leaving group represented by L includes the above-mentioned halogen atom and the like.

As shown in the following Examples as another method, the compound of formula (III) is produced by allowing to react a compound represented by the formula (IV) in the presence of halogenation reagent such as phosphorus pentachloride etc. in a solvent such as aliphatic hydrocarbons such as pentane, hexane, heptane, petroleum ether etc., aromatic hydrocarbons such as benzene, toluene, xylene etc., esters such as methyl acetate, ethyl acetate, ethyl formate, ethyl propionate etc., ketones such as acetone, methyl ethyl ketone etc., ethers such as diethyl ether, dipropyl ether, diisopropyl ether, dibutyl ether, tetrahydrofuran, dioxane etc., nitriles such as acetonitrile, propionitrile, etc., acid amides such as dimethylformamide, dimethylacetamide, etc., cyclic amides such as 1-methyl-2-pyrrolidone etc., cyclic ureas such as 1,3-dimethyl-2-imidazolidinone etc., sulfoxides such as dimethylsulfoxide etc., sulfones such as sulfolane etc., phosphoric acid amides such as hexamethylphosphoramide etc., halogenated hydrocarbons such as dichloromethane, chloroform, 1,2-dichloroethane, carbon tetrachloride etc., aromatic amines such as pyridine, picoline, lutidine, quinoline etc., and mixtures thereof, water, and further mixed solvents thereof with water, and then without isolating, followed by allowing to react with a compound of formula (II) in the presence of base such as alkali metal alcoholate such as sodium ethylate, sodium methylate, potassium tert-butoxide etc., organic base such as triethylamine, diisopropylethylamine, pyridine, 4-dimethylaminopyridine, N,N-dimethylaniline etc., inorganic base such as potassium carbonate, sodium carbonate, sodium hydroxide, potassium hydroxide, sodium hydrogen carbonate, potassium hydrogen carbonate etc., metal hydride such as lithium hydride, sodium hydride, potassium hydride etc. and organic lithium reagent such as butyl lithium, lithium diisopropylamide etc., to produce compound (I) or a salt thereof.

The reaction temperature is usually about -50 to 200°C, preferably about -30 to 150°C. The reaction time is generally about 0.1 to 96 hours, preferably 0.1 to 72 hours, more preferably about 0.1 to 24 hours.

By subjecting the resulting compound to a reaction known per se such as alkylation, alkenylation, alkynylation, acylation, amination, sulfidation, sulfinylation, sulfonation, oxidation, reduction, halogenation, nitration, nitrosylation etc., its substituent can be converted to other desired substituent.

Particularly, when n is 0 in the compound represented by the above-mentioned formula (Ib), the compound of. formula (Ib) wherein n is 1 or 2 can be produced by oxidizing with a known oxidant in a suitable solvent.

Examples of the oxidant include m-chloroperbenzoic acid, hydrogen peroxide, etc.

Examples of the solvent include aliphatic hydrocarbons such as pentane, hexane, heptane, petroleum ether etc., aromatic hydrocarbons such as benzene, toluene, xylene etc., esters such as methyl acetate, ethyl acetate, ethyl formate, ethyl propionate etc., ketones such as acetone, methyl ethyl ketone etc., ethers such as diethyl ether, dipropyl. ether, diisopropyl ether, dibutyl ether, tetrahydrofuran, dioxane etc., nitriles such as acetonitrile, propionitrile, etc., acid amides such as dimethylformamide, dimethylacetamide, etc., cyclic amides such as 1-methyl-2-pyrrolidone etc., cyclic ureas such as 1,3-dimethyl-2-imidazolidinone etc., sulfones such as sulfolane etc., phosphoric acid amides such as hexamethylphosphoramide etc., halogenated hydrocarbons such as dichloromethane, chloroform, 1,2-dichloroethane, carbon tetrachloride etc., aromatic amines such as pyridine, picoline, lutidine, quinoline etc., organic acids such as acetic acid etc., .. and mixtures thereof, water, and further mixed solvents thereof with water.

The reaction temperature is usually about -50 to. 200°C, preferably about -30 to 150°C. The reaction time is generally about 0.1 to 96 hours, preferably 0.1 to 72 hours, more preferably about 0.1 to 24 hours.

In addition, the compound represented by the above-mentioned formula (Ic) wherein the substituent R is a hydrogen can be produced by a method known per se such as reduction (hydrogenation etc.), hydrolysis and the like from the compound of formula (Ic) wherein the substituent R is alkoxycarbonyl, aralkyl, aralkyloxycarbonyl,. aryloxycarbonyl, alkylsulfonyl, arylsulfonyl or the like.

When the substituent R of the compound represented by the above-mentioned formula (Ic) is a hydrogen, the compound (I) represented by the above-mentioned formula (Id) and (Ie) can be produced by halogenating with a known halogenation agent in a suitable solvent, followed by dehydrogen halide with a base.

Examples of the halogenation agent include alkyl hypochlorite, chlorine, bromine and the like.

Examples of the base include alkali metal alcoholate such as sodium ethylate, sodium methylate, potassium tert-butoxide etc., organic base such as triethylamine, diisopropylethylamine, pyridine, 4-dimethylaminopyridine, N,N-dimethylaniline etc., inorganic base such as potassium carbonate, sodium carbonate, sodium hydroxide, potassium hydroxide, sodium hydrogen carbonate, potassium hydrogen carbonate etc., Metal hydride such as lithium hydride, sodium hydride, potassium hydride etc. and organic lithium reagent such as butyl lithium, lithium diisopropylamide etc.

Examples of the solvent include aliphatic hydrocarbons such as pentane, hexane, heptane, petroleum ether etc., aromatic hydrocarbons such as. benzene, toluene, xylene etc., esters such as methyl acetate, ethyl acetate, ethyl formate, ethyl propionate etc., ketones such as acetone, methyl ethyl ketone etc., ethers such as diethyl ether, dipropyl ether, diisopropyl ether, dibutyl ether, tetrahydrofuran, dioxane etc., nitriles such as acetonitrile, propionitrile, etc., acid amides such as dimethylformamide, dimethylacetamide, etc., cyclic amides such as 1-methyl-2-pyrrolidone etc., cyclic ureas such as 1,3-dimethyl-2-imidazolidinone etc., sulfoxides such as dimethylsulfoxide etc., sulfones such as sulfolane etc., phosphoric acid amides such as hexamethylphosphoramide etc., halogenated hydrocarbons such as dichloromethane, chloroform, 1,2-dichloroethane, carbon tetrachloride etc., aromatic amines such as pyridine, picoline, lutidine, quinoline etc., and mixtures thereof, water, and further mixed solvents thereof with water.

The amount of the base to be used is not particularly limited as far as it does not adversely affect the reaction, and it can be used in large excess also doubling as a solvent.

The reaction temperature is usually about -50 to 200°C, preferably about -30 to 150°C. The reaction time is generally about. 0.1 to 96 hours, preferably 0.1 to 72 hours, more preferably about 0.1 to 24 hours.

When the substituent R of the compound represented by. the above-mentioned formula (Ic) is a hydrogen, the substituent R can be converted to other desired substituent R by subjecting to a method known per se such as. alkylation, alkenylation, alkynylation, acylation,. amination, sulfidation, sulfinylation, sulfonation, oxidation, reduction, halogenation, nitration, nitrosylation etc.

When A is $CY_1Y_2OCY_1Y_2$, $CY_1Y_2SCY_1Y_2$ or $CY_1Y_2NRCY_1Y_2$, compound (I) or a salt thereof can be produced

according to the reaction scheme:

Reaction Scheme 1

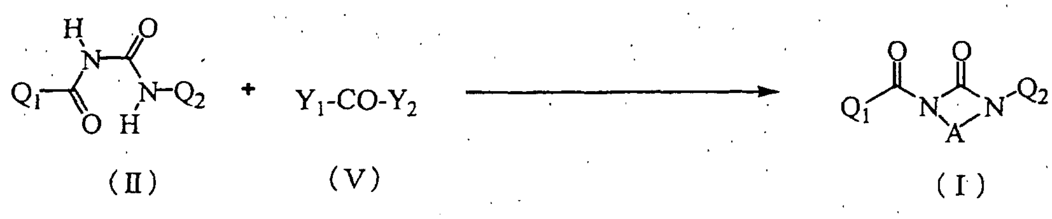

wherein respective symbols are as defined above, for example,. by allowing to react a compound represented by the above-mentioned formula (II) with various aldehydes or ketones (V) such as formaldehyde, acetone, acetaldehyde etc. in a solvent such as pentane, hexane, heptane, petroleum ether etc., aromatic hydrocarbons such as benzene, toluene, xylene etc., esters such as methyl acetate, ethyl acetate, ethyl formate, ethyl propionate etc., ketones such as acetone, methyl ethyl ketone etc., ethers such as diethyl ether, dipropyl ether, diisopropyl ether, dibutyl ether, tetrahydrofuran, dioxane etc., nitriles such as acetonitrile, propionitrile, etc., acid amides such as. dimethylformamide, dimethylacetamide, etc., cyclic amides such as 1-methyl-2-pyrrolidone etc., cyclic ureas such as 1,3-dimethyl-2-imidazolidinone etc., sulfoxides such as dimethylsulfoxide etc., sulfones such as sulfolane etc., phosphoric acid amides such as hexamethylphosphoramide etc., halogenated hydrocarbons such as dichloromethane, chloroform, 1,2-dichloroethane, carbon tetrachloride etc., aromatic amines such as pyridine, picoline, lutidine, quinoline etc., and mixtures thereof, water, and further mixed solvents thereof with water.

In addition, the reaction can be carried out with mixing hydrogen sulfide, various amines ($RNH_2$: R is as defined above) and the like.

In this reaction, an acidic catalyst such as p-toluenesulfonic acid etc. or a basic catalyst such as triethylamine etc. can be added with an amount of 0.01 to 0.5 equivalent relative to the substrate in order to accelerate the reaction.

The reaction temperature is usually about -50 to 200°C, preferably about -30 to 150°C. The reaction time is generally about 0.1 to 96 hours, preferably 0.1 to. 72 hours, more preferably about 0.1 to 24 hours.

The resulting compound can be purified by a method known per se (e.g. recrystalization, chromatography, etc.).

The compounds represented by the formula (II), formula (III) and formula (IV) is known, or can be produced according to a method known per se or a method shown in the following Reference Examples.

Compound (I) of the present invention is. effective for controlling a hygiene pest and an animal and plant parasitic pest, and exhibit a strong insecticidal activity by treating an animal and a plant which are parasitized by a pest. In addition, Compounds (I) of the present invention have little phytotoxic effects on a plant, and have little toxicity to fishes and, thus, have both safe and advantageous nature as an agent for controlling pests for hygiene, the livestock industry, pets, horticulture and agriculture.

When Compound (I) is used as an agricultural chemical, in particular, as an insecticide, the compound is used in a form which general agricultural chemicals and veterinary drugs can take, that is, a dosage form such as an emulsion, a solution, a microemulsion, a flowable formulation, an oil solution, a wettable powder, a powder, a granule, a fine granule, a seed coating agent, a smoking agent, a tablet, a microcapsule, a spray formulation, an EW agent, an ointment, a poison bait, a capsule, a pellet, an injectable, a shampoo preparation and the like, by dissolving or dispersing one kind or. two kinds or more (preferably, one kind or more, and not more. than three kinds) of Compound (I) or a salt thereof as an active ingredient in a suitable liquid carrier, or mixing. with or being adsorbed on a suitable solid carrier depending on a use purpose. To .these preparations, if needed, an emulsifying agent, a suspending agent, a developer, a penetrant, a wetting agent, a thickener, a stabilizer or the like may be added, and they can be prepared by a method known per se.

As a liquid carrier (solvent) to be used, for example, solvents such as water, alcohols (e.g. methyl alcohol, ethyl alcohol, n-propyl alcohol, isopropyl alcohol, ethylene glycol etc.), ketones (e.g. acetone, methyl ethyl ketone, methyl isobutyl ketone, cyclohexanone etc.), ethers (e.g. tetrahydrofuran, ethylene glycol monomethyl ether, diethylene glycol monomethyl ether, propylene glycol monomethyl ether etc,), aliphatic hydrocarbons (e.g. kerosine, kerosene, fuel oil, machine oil etc.), aromatic hydrocarbons (e.g. toluene, xylene, solvent naphtha, methylnaphthalene etc.), halogenated hydrocarbons (e.g. dichloromethane, chloroform, carbon tetrachloride etc.), acid amides (e.g. *N*,*N*-dimethylformamide; *N*,*N*-dimethylacetamide, *N*-methylpyrrolidone etc.), esters (e.g. ethyl acetate, butyl acetate, fatty acid glycerin ester, γ-butyrolactone etc.), nitriles (e.g. acetonitrile, propionitrile etc.), and the like are suitable. These can be appropriately used by mixing one kind or two kinds or more (preferably one kind or more, and not more than three

kinds) at a. suitable ratio.

As a solid carrier (diluent, bulking agent), a vegetable powder (e.g. soybean powder, tobacco powder, wheat powder, woodmeal etc.), a mineral powder (e.g. clays 'such as kaolin, bentonite, acid clay etc., talcs such as talc powder, agalmatolite powder etc., silicas such as diatomaceous earth, mica powder etc.), alumina, a sulfur powder, an active carbon, calcium carbonate, ammonium sulfate, sodium hydrogen carbonate, lactose, urea and the like are used, and these can be appropriately used by mixing one kind or two kinds or more (preferably one kind or more, and not more than three kinds) at a suitable ratio.

In addition, as an ointment base materials, for example, one kind or two kinds or more (preferably, one kind or more, and not more than three kinds) of materials selected from the group consisting of polyethylene glycol, pectin, polyhydric alcohol ester of higher fatty acid such as monostearic acid glycerin ester and the like, cellulose derivative such as methylcellulose and the like, sodium alginate, bentonite, higher alcohol, polyhydric alcohol such as glycerin and the like,. vaseline, white vaseline, liquid paraffin, lard, various vegetable oils, lanolin, dehydrated lanolin, hardened oil, resins and the like, or these materials wherein following various surfactants are added thereto are appropriately used.

As a surfactant used as an emulsifying agent, a developer, a penetrant, a dispersant and the like, depending on the necessity, nonionic and anionic surfactants such as soaps, polyoxyethylene alkyl aryl ethers [e.g. Neugen (trade name), E·A142 (trade name); manufactured by Dai-ichi Kogyo Seiyaku Co., Ltd., Nonal (trade name); manufactured by Toho Chemical Industries Co., Ltd.], alkyl sulfate salts [e.g. Emar 10 (trade name); Emar 40 (trade name); manufactured by Kao Corporation], alkylbenzene sulfonic acid salts [e.g. Neogen (trade name), Neogen T(trade name); manufactured by Dai-ichi Kogyo Seiyaku Co., Ltd., Neoperex; manufactured by Kao Corporation], polyethylene glycol ethers [e.g., Nonipol 85 (trade name), Nonipol 100 (trade name), Nonipol 160 (trade name); manufactured by Sanyo Chemical Industries, Ltd.] polyhydric alcohol esters [e.g. Tween 20 (trade name), Tween 80 (trade name); manufactured by Kao Corporation], alkylsulfosuccinic acid salts [e.g. Sanmolin OT20 (trade name); manufactured by Sanyo Chemical Industries, Ltd., Newcalgen EX70 (trade name); manufactured by Takemoto Oil &. Fat Co., Ltd.], alkylnaphthalene sulfonic acid salts [e.g. Newcalgen EX70 (trade name); manufactured by Takemoto Oil. & Fat Co., Ltd.], alkenyl sulfonic acid salts [e.g. Solpol 5115 (trade name); manufactured by Toho Chemical Industries Co., Ltd.] and the like are appropriately used. In addition, Compound (I) can also be used appropriately by compounding with, for example, other insecticide (pyrethroid insecticide, organic phosphorus insecticide, carbamate insecticide, neonicotinoid insecticide, natural insecticide etc.), an acaricide, a machine oil, a nematodecide, a herbicide, a plant hormone agent, a plant growth regulating substance, an antibacterial agent (e.g. copper antibacterial agent, organic chlorine antibacterial agent, organic sulfur antibacterial agent, phenol antibacterial agent etc.), a synergist, an attractant, a repellent, a drug harm alleviating agent, a pigment, a fertilizer, an animal feed (feed for livestock such as cow pig and hence chicken, feed for pet animal such as dog and cat, feed for raised fish such as. young yellowtail and sea bream), veterinary medicaments (medicaments for treating or preventing diseases of livestock, pet animal, raised fish), a veterinary nutrient and the like.

The ratio of Compound (I) contained in the agricultural chemical and veterinary drug composition (insecticide and anthelmintic) of the present invention is usually about 0.1 to 80% by weight, preferably about 1 to 20% by weight relative to the total amount of the composition. Specifically, when the compound is, used as an emulsion, a solution or a wettable powder (e.g. granular wettable powder), usually about 1 to 80% by weight, preferably about 1 to 20% by weight is suitable. When used as an oil solution or a powder, usually about 0.1 to 50% by weight, preferably about 0.1 to 20% by weight is suitable. When used in a granule, usually about 5 to 50% by weight, preferably about 1 to 20% by weight is suitable.

Other agricultural chemical active ingredient (e.g. an insecticide, a herbicide, an acaricide and/or an antibacterial agent) which is compounded in the agricultural chemical composition of the present invention is used usually in the range of about 1 to 80% by weight, preferably about 1 to 20% by weight relative to the total amount of the preparation.

The content of an additive other than the aforementioned active ingredients differs depending on a kind or a content of an agricultural chemical active ingredient or a dosage form of a preparation, and is. usually about 0.001 to 99.9% by weight, preferably about 1 to 99% by weight. More specifically, it is preferable to add a surfactant at usually about 1 to 20% by weight, more preferably about 1 to 15% by weight, a flowing aid at about 1 to 20% by weight, and a carrier at about 1 to 90% by weight, preferably at about 1 to 70% by weight relative to the total amount of the composition. Specifically, when a solution is prepared, it is preferable to add a surfactant at usually about 1 to 20% by weight, preferably 1 to 10% by weight, and water at about 20 to 90% by weight. An emulsion or a wettable powder (e.g. granular wettable powder) should be diluted with water appropriately. (e.g. about 100 to 5,000-fold) for use to spray.

Typical examples of the compound (including isomers and salts thereof) which can be used by mixing with the Compound (I) or salts thereof of the present invention are shown below.

A insecticide, an acaricide and a nematocide include:

acephate, acequinocyl, acetamiprid, acetoprole, acrinathrin, alanycarb, aldrin, allethrin, Aluminium phosphide, amidoflumet, amitraz, Arsenic acid, avermectin-B, benclothiaz, bendiocarb, benfluthrin, benfuracarb, bensultap, benzoximate, bifenthrin, bistrifluron, BPMC, bromopropylate, buprofezin, butathiofos, cadusafos, Calcium cyanamide, Calcium polysulfide, carbaryl:NAC, carbofuran, carbosulfan, cartap, chlordane, chlorethoxyfos, chlorfenvinphos:CVP, chlorfluazuron, chlorphenapyr, chlorpyrifos, chlorpyrifos-methyl, chromafenozide, cloethocarb, clofentezine, clothianidin, cyanophos:CYAP, cycloprothrin, cyfluthrin, cyhalothrin, cypermethrin, cyromazine, DCIP (dichlorodiisopropyl ether), D-D (1,3-Dichloropropene), DDT, deltamethrin, diafenthiuron, diazinon, dichlofenthion, dichlorvos:DDVP, dicofol, dieldrin, dienochlor, diflubenzuron, dimethoate, dimethylvinphos, dinotefuran, disulfoton, DSP, emamectin-benzoate, endosulfan, EPN, esfenvalerate, ethiofencarb, ethion, ethiprole, ethofenprox, ethoprophos, etoxazole, etrimfos, fenazaquin, fenbutatin oxide, fenitrothion:MEP, fenobucarb, fenothiocarb, fenoxycarb, fenpropathrin, fenpyroximate, fenthion, fenvalerate, fipronil, fluacrypyrim, fluazinam, fluazuron, flucycloxuron, flucythrinate, flufenerim, flufenoprox, flufenoxuron, flumethrin, flonicamid, fluproxyfen, flupyrazofos, flurimfen, fluvalinate, formetanate, formothion, fosthiazate, furathiocarb, halfenprox, hexaflumuron, hexythiazox, Hydrogen phosphide, hydroprene, imidacloprid, imiprothrin, indoxacarb, isofenphos, isoprocarb, isoxathion, lufenuron, levamisol, machine oil, malathion, mesulfenfos, metam-ammonium, metam-sodium, methidathion, methiocarb, methomyl, methoxychlor, methoxyfenozide, Methyl bromide, metofluthrin, metolcarb:MTMC, metoxadiazone, milbemycin-A, monocrotophos, naled:BRP, nicotine-sulfate, NNI-0001, novaluron, noviflumuron, nidinotefuran, nitenpyram, oxamyl, oxydeprofos:ESP, parathion, permethrin, phenthoate:PAP, phosalone, phosmet:PMP, pirimicarb, pirimiphos-methyl, Potassium oleate, prallethrin, profenofos, profluthrin, propaphos, propargite:BPPS, propoxur, prothiofos, protrifenbute, pymetrozine, pyraclofos, pyrethrins, pyridaben, pyridafenthion, pyridalyl, pyrimidifen, pyriproxyfen, quinalphos, resmethrin, salithion, silafluofen, spinosad, spirodiclofen, spiromesifen, Sulfur, sulfluramid, sulprofos, tebufenozide, tebufenpyrad, tebupirimfos, teflubenzuron, tefluthrin, temephos, tetrachlorvinphos, tetradifon, thiacloprid, thiamethoxam, thiocyclam, thiodicarb, thiometon,. TI-809, tolfenpyrad, tralomethrin, triazamate, trichlorfon:DEP, triflumuron, vamidothion, vaniliprole, XMC, xylylcarb and the like.

An antibacterial agent includes:

acibenzolar-S-methyl, amobam, ampropylfos, anilazine, azoxystrobin, benalaxyl, benodanil, benomyl, benthiavalicarb, benthiazole, bethoxazin, bitertanol, blasticidin-S, Bordeaux mixture, boscalid, bromuconazole, buthiobate, Calcium hypochlorite, Calcium polysulfide, captan, carbendazol, carboxin, carpropamid, chlobenthiazone, chloroneb, chloropicrin, chlorothalonil:TPN, chlorthiophos, Cinnamaldehyde, clozylacon, CNA (2,6-Dichloro-4-nitroaniline), Copper hydroxide, Copper sulfate, cyazofamid, cyfluphenamid, cymoxanil, cyproconazole, cyprodinil, cyprofuram, dazomet, debacarb, dichlofluanid, D-D (1,3-Dichloropropene), diclocymet, diclomezine, diethofencarb, difenoconazole, diflumetorim, dimefluazole, dimethirimol, dimethomorph, diniconazole-M, dinocap, edifenphos, epoxiconazole, nickel dimethyldithiocarbamate,, etaconazole, ethaboxam, ethirimol, etridiazole, famoxadone, fenamidone, fenarimol, fenbuconazole, Fendazosulam, fenhexamid, fenoxanil, fenpiclonil, fenpropidin, fenpropimorph, fentiazon, fentin hydroxide, ferimzone, fluazinam, fludioxonil, flumetover, flumorph, fluoroimide, fluotrimazole, fluoxastrobin, fluquinconazole, flusilazole, flusulfamide, flutolanil, flutriafol, fosetyl-Al, fthalide, fuberidazole, furalaxyl, furametpyr, furcarbanil, furconazole-cis, hexaconazole, hymexazol, IBP, imazalil, imibenconazole, iminoctadine-albesilate, iminoctadine-triacetate, iodocarb, ipconazole, iprodione, iprovalicarb, isoprothiolane, kasugamycin, kresoxim-methyl, mancozeb, maneb, mepanipyrim, mepronil, metalaxyl, metalaxyl-M, metam-sodium, methasulfocarb, Methyl bromide, metconazole, methfuroxam, metominostrobin, metrafenone, metsulfovax, mildiomycin, milneb, myclobutanil, myclozolin, nabam, orysastrobin, ofurace, oxadixyl, oxolinic. acid, oxpoconazole, oxycarboxin, oxytetracycline, pefurazoate, penconazole, pencycuron, picoxystrobin, polycarbamate, polyoxin, Potassium hydrogen carbonate, probenazole, prochloraz, procymidone, propamocarb-hydrochloride, propiconaole, propineb, proquinazid, prothiocarb, prothioconazole, pyracarbolid, pyraclostrobin, pyrazophos, pyributicarb, pyrifenox, pyrimethanil, pyroquilon, quinoxyfen, quintozene:PCNB, silthiopham, simeconazole, sipconazole, Sodium bibarbonate, sodium hypochlorite, spiroxamine, SSF-129 ((E)-2[2-(2,5-dimethylphenoxymethyl)phenyl]-2-methoxyimino-N-methylacetamide), streptomycin, Sulfur, tebuconazole, tecloftalam, tetraconazole, thiabendazole, thiadinil, thiram:TMTD, thifluzamide, thibphanate-methyl, tolclofosmethyl, TPN, triadimefon, triadimenol, triazoxide, triclamide, tricyclazole, tridemorph, triflumizole, trifloxystrobin, triforine, triticonazole, validamycin, vinclozolin, viniconazole, zineb, ziram, zoxamide, tolnifanide and the like.

A herbicide, a plant hormone agent and a plant growth regulating substance include:

Abscisic acid, acetochlor, acifluorfen-sodium, alachlor, alloxydim, ametryn, amicarbazone, amidosulfuron, aminoethoxyvinylglycine, aminopyralid, AC94,377, amiprofosmethyl, ancymidol, asulam, atrazine, aviglycine, azimsulfuron, beflubutamid, benfluralin, benfuresate, bensulfuron-methyl, bensulide:SAP, bentazone, benthiocarb, benzamizole, benzfendizone, benzobicyclon, benzofenap, benzyl adenine, benzylaminopurine, bialaphos, bifenox, Brassinolide, bromacil, bromobutide, butachlor, butafenacil, butamifos, butylate, cafenstrole, Calcium carbonate, Calcium peroxide, carbaryl, chlomethoxynil, chloridazon, chlorimuron-ethyl, chlorphthlim, chlorpropham, chlorsulfuron, chlorthal-dimethyl, chlorthiamid:DCBN, choline chloride, cinidon-ethyl, cinmethylin, cinosulfuron, clethodim, clomeprop, cloxyfonac-sodium, chlormequat chloride, 4-CPA (4-chlorophenoxyacetic acid), cliprop, clofencet, cumyluron, cyanazine, cyclanilide, cyclosulfamron, cyhalofop-butyl, 2,4-D salts (2, 4-Dichlorophenoxyacetic acid salts), dichlorprop:2,4-DP, daimuron, dalapon:DPA, dimethenamid-P, daminozide, dazomet, n-Decyl alcohol, dicamba-sodium:MDBA, dichlobenil:DBN, diflufenican, dikegulac, dimepiperate, dimethametryn, dimethenamid, diquat, dithiopyr, diuron, endothal, epocholeone, esprocarb, ethephon, ethidimuron, ethoxysulfuron, ethychlozate, etobenzanid, fenarimol, fenoxaprop-ethyl, fentrazamide, flazasulfuron, florasulam, fluazifop-butyl, fluazolate, flucarbazone, flufenacet, flufenpyr, flumetralin, flumioxazin, flupropanate-sodium, flupyrsulfuron-methyl-sodium, flurprimidol, fluthiacetmethyl, foramsulfuron, forchlorfenuron, formesafen, gibberellin, glufosinate, glyphosate, halosulfuron-methyl, hexazinone, imazamox, imazapic, imazapyr, imazaquin, imazosulfuron, inabenfide, Indole acetic acid:IAA, Indole butyric acid, iodosulfuron, ioxynil-octanoate, isouron, isoxachlortole, isoxadifen, karbutilate, lactofen, lenacil, linuron, LGC-42153, Maleic hydrazide, mecoprop:MCPP, MCP salts (2-Methyl-4-chlorophenoxyacetic acid salts), MCPA-thioethyl, MCPB (2-Methyl-4-chlorophenoxybutanoic acid ethyl ester), mefenacet, mefluidide, mepiquat, mesosulfuron, mesotrione, methyl daimuron, metamifop, metolachlor, metribuzin, metsulfuron-methyl, molinate, naphthylacetic acid, NAD (1-naphthaleneacetamide), naproanilide, napropamide, n-decyl alcohol, nicosulfuron, n-phenylphthalamic acid, orbencarb, oxadiazon, oxaziclomefone, oxine-sulfate, paclobutrazol, paraquat, Pelargonic acid, pendimethalin, penoxsulam, pentoxazone, pethoxamide, phenmedipham, picloram, picolinafen, piperonyl butoxide, piperophos, pretilachlor, primisulfuron-methyl, procarbazone, prodiamine, profluazol, profoxydim, prohexadione-calcium, prohydrojasmon, prometryn, propanil, propoxycarbazone, propyzamide, pyraclonil, pyraflufen-ethyl, pyrazolate, pyrazosulfuron-ethyl, pyrazoxyfen, pyribenzoxim, pyributicarb, pyridafol, pyridate, pyriftalid, pyriminobac-methyl, pyrithiobac, quiclorac, quinoclamine, quizalofop-ethyl, rimsulfuron, sethoxydim, siduron, simazine, simetryn, Sodium chlorate, sulfosulfuron, swep:MCC, tebuthiuron, tepraloxydim, terbacil, terbucarb:MBPMC, thenylchlor, thiazafluron), thidiazuron, thifensulfuron-methyl, triaziflam, tributes, triclopyr, tridiphane, trifloxysulfuron, trifluralin, trinexapac-ethyl, tritosulfuron, uniconazole-P, vemolate:PPTC and the like.

In addition, the Compound (I) or salts thereof of the present invention may be used by mixture with a synergist . such as piperonyl butoxide, sesamex, sulfoxide, MGK 264, N-declyimidazole, WARF(warfarin)-antiresistant, TBPT(*S*,*S*,*S*-tributyl phosphorotrithioate), TPP(triphenyl phosphate), IBP(Iprobenfos), PSCP(pseudomonapepsin), $CH_3I$, t-phenylbutenone, diethylmaleate, DMC(Chlorfenethol), FDMC(Bis(p-chlorophenyl)(trifluoromethyl)carbinol), ETP(teniposide), ETN(endothelin) and the like, and also may be used by mixture with a drug harm alleviating agent such as benoxacor, cloquintocet-mexyl, cyometrinil, daimuron, dichlormid, fenchlorazole-ethyl, fenclorim, flurazole, fluxofenim, furilazole, mefenpyr-diethyl MG191, naphthalic anhydride, oxabetrinil and the like.

Furthermore, the Compound (I) or salts thereof of the present invention may be used by mixture with a controlling agent for an outside-parasitic arthropod such as pyrethroid agents, IGR agents (juvenile hormone-like substances such as methoprene, fenoxycarb and the like, chitin synthase inhibitors such as lufenuron, flufenoxuron, novaluron, hexaflumuron, teflubenzuron, diflubenzuron, triflumuron and the like, insect growth regulating agents such as cyromazine, pyriproxyfen and the like), and neonicotinoid compounds (nitenpyram etc.), or may be used by mixture with a controlling agent for inner parasite such as an above-mentioned IGR agent as in vivo administration agent for animal, a controlling agent for filaria (macrolide compounds such as selamectin, ivermectin, milbemycin, moxidectin etc.) or the like, and further may be used by mixture with an antibacterial agent for animal, vaccine, therapeutic agent, nutritional supplement and bait.

The preparation containing Compound (I) of the present invention or a salt thereof is particularly effective for controlling pests, specifically, such as Hemiptera such as *Eurydema rugosum, Scotinophara lurida, Riptortus clavatus, Stephanitis nashi, Laodelphax striatellus, Nilaparvata lugens, Nephotettix cincticeps, Unaspis yanonensis, Aphis glycines, Lipaphis erysimi, Brevicoryne brassicae, Aphis gossypii, Myzus persicae, Aula corthum solani, Aphis spiraecola, Bemisia tabaci, Trialeurodes vaporariorum, Sogatella furcifera, Empoasca onukii, Pseudococus comstocki, Planococcus citri, Icerya purchasi, Plautia stali, Eysarcoris parvus* and the like; Lepidoptera such as *Spodoptera litura, Plutella xylostella, Pieris rapae crucivora, Chilo supppressalis, Autographa nigrisigna, Helicoverpa assulta, Pseudaletia separata, Mamestra brassicae, Adoxophyes orana fasciata, Notarcha derograta, Cnaphalocrocis medinalis, Phthorimaea operculella, Chilo polychrysus, Typoryza incertulas, Spodoptera exigua, Agrotis segetum, Agrotis ipsilon, Heliothis armigera, Heliothis virescens, Heliothis zea, Naranga aenescens, Ostrinia nubilalis, Ostrinia, furnacalis, Parnara guttata, Adoxophyes sp., Caloptilia theivora, Phyllonorycter ringoneella, Carposina niponensis, Grapholita molesta, Cydia pomonella* and the like;

Coleoptera such as *Epilachna vigintioctopunc tata, Aulacophorafemoralis, Phyllotreta striolata, Oulema oryzae, Echinocnemus squameus, Lissorhoptrus oryzophilus, Anthonomus grandis, Callosobruchus* chinensis, *Sphenophorus venatus, Popillia japonica, Anomala cuprea, Diabrotica spp., Leptinotarsa decemlineata, Agriotes spp., Lasioderma serricorne, Anthrenus verbasci, Tribolium castaneum, Lyctus brunneus, Anoplophora malasiaca, Tomicus piniperda* and the like;

Diptera such as *Musca domestica, Culex popiens pallens, Tabanus trigonus, Delia antiqua, Delia platura, Anopheles sinensis, Agromyza oryzae, Hydrellia griseola, Chlorops oryzae, Dacus cucurbitae, Ceratitis capitata, Liriomyza trifolii, Liriomyza sativae* and the like;

Orthoptera such as *Locusta migratoria, Gryllotalpa africana, Oxya yezoensis, Oxya* japonica and the like;

Thysanoptera such as *Thrips tabaci, Thrips parmi, Frankliniella occidentalis, Baliothrips biformis, Scirtothrips dorsalis* and the like;

Hymenoptera such as *Athalia rosae, Acromyrmex spp., Solenopsis spp.* and the like;
Blattodea such as *Blattella germanica, Periplaneta fuliginosa, Periplaneta japonica, Periplaneta americana* and the like;
Acarina such as, Tetranychidae such as *Tetranychus urticae, Panonychus citri, Tetranychus kanzawai, Tetranychus cinnabarinus,* Tetranychus *viennensis, Tetranychus desertorum, Panonychus ulmi* and the like, Eriophyidae such as *Aculops pelekassi*, *Aculops lycopersici, Aceria diospyri, Aculus fockeui*, *Eriophyes chibaensis* and the like, Tarsonemidae such as *Polyphagotarsonemus latus, Phytonemus pallidus* and the like, Acaridae such as *Rhizoglyphus echinopus* and the like, Tenuipalpidae, Eupodidae and the like;
Nematoda such as *Aphelenchoides besseyi, Meloidogyne incognita, Pratylenchus penetrans, Nothotylenchus acris* and the like;
Isoptera such as *Coptotermes formosanus, Reticulitermes speratus, Odontotermes formosanus, Cryptotermes domesticus* and the like.

Furthermore, the preparation containing Compound (I). of the present invention or a salt thereof can be used in the field of treatment for disease of livestock and in livestock farming, and also for maintaining public sanitation by exterminating an arthropod and parasite which parasitize inside and/or outside of vertebrates such as human, cow, sheep, goat, pig, poultry, dog, cat, fish and the.like. Examples of pests include, for example, Ixodes spp., Boophilus spp. (e.g. *Boophilus microplus*), Amblyomma spp., Hyalomma spp., Rhipicephalus spp. (e.g. *Rhipicephalus appendiculatus*), Haemaphysalis spp., dermacentor spp., Ornithodoros spp. (e.g. *Ornithodoros moubata), Dermahyssus gallinae,* Sarcoptes spp. (e.g. *Sarcoptes scabiei*), Psoroptes spp., Chorioptes spp., Demodex spp., Eutrombicula spp., Aedes spp., Anopheles spp., Culex spp, Culicodes spp, Musca. spp., Hypoderma spp., Gasterophilus spp., Haematobia spp, Tabanus spp, Simulium spp., Triatoma spp., Phthiraptera (e.g. Damalinia spp., Linognathus spp., Haematopinus spp), Ctenocephalides spp.Xenosylla spp), monomorium pharaonis and Nematoda (for example, Trichostrongylus (e.g. *Nippostrongylus brasiliensis, Trichostrongylus axei, Trichostrongylus colubriformis*), Trichinella (e.g. *Trichinella spiralis*), *Haemonchus contortus*, Nematodirus (e.g. *Nematodirus battus*), *Ostertagia circumcincta*, Cooperia spp., Hymenolepis nana) and the like.

The agricultural chemical composition containing the Compound (I) of the present invention or a salt thereof has a superior pesticidal activity and can be used as an extremely lower toxic, therefore safe and excellent agricultural chemical composition (pesticide). And the agricultural chemical composition of the present invention can be used in a manner similar to a conventional agricultural chemical composition and consequently can yield very substantial benefits compared to existing lines. The arising above-mentioned pests (insect pests etc.) can be exterminated by spraying the agricultural chemical composition of the present invention on paddy, field, orchard, non-tillage farm, house and the. like by a method known per se and bringing the pests into contact with the composition or feeding the pests. As an alternative mode, for example, the arthropod and parasite living with said vertebrates can be exterminated by administering the agricultural chemical composition of the present invention to. inside (internal parts of the body) or outside (body surface) of the above-mentioned vertebrates. In addition, sanitary pests arising from the excrement of the animal can be exterminated by feeding a. livestock animal.

More specifically, for example, the agricultural chemical composition of the present invention can be used to the. targeting pests by a method such as seed treatment, nursery box treatment, planting holes treatment, soil-incorporation treatment, foliar spray, ULV spray, immersion treatment, application, coating, fertilizer-mixing treatment, injection to tree trunk, poison bait, smoking, irrigation, subaqueous application for paddy, mixing treatment to unmilled rice and kernel, and the like. The amount of application can be changed within a wide range depending on the application time, application place, application method and the like, and it is desirable to apply so that the active ingredient (compound or a salt thereof) per hectare comes generally to about 0.3 g to 3,000 g, preferably about 50 g to 1,000 g. When the agricultural chemical composition of the present invention is a wettable powder, it may be diluted with water to use so that the final concentration of. active ingredient comes to the range of about 0.1 to 1,000 ppm, preferably about 10 to. 500 ppm. Furthermore, recently advances have been made in the technologies of genetically-modified crops (a herbicide resistant crop, a pest resistant crop integrated a gene producing pest-control proteins, a disease resistant crop.integrated a gene producing substances resistant to diseases, a flavor-improved crop, a storage stability-improved crop, a yield-improved crop, etc.), insect pheromone (a disrupting agent of communication of *Tortricidae, Mamestra brassicae*, etc.) and IPM (integrated pests management) using a counterpest insect. The agricultural chemical composition of the present invention can be used' together with these technologies or can be used by systematizing with them.

Examples

The present invention will be further illustrated by the following Synthetic Examples, Reference Examples, Formulation Examples and Test Examples; however, the present invention is not limited to these examples.

The elution in the column chromatography for Synthetic Examples was carried out under the observation by TLC (Thin Layer Chromatography). In the TLC observation, kieselgel $60F_{254}$ (70 to 230 meshes) manufactured by

Merck & Co., Inc. was used as TLC plate; the solvent used as an elution solvent in column chromatography was used as developing solvent; and a UV detector was used for detection. Kieselgel 60 (70 to 230 meshes) manufactured by Merck & Co., Inc. was used as silica gel for column chromatography. When a mixed solvent was used as developing solvent, the numeric value in parentheses shows a mixing ratio of solvents by volume. NMR spectra were proton NMR, and were determined with Bruker AC-200P (200 MHz) spectrometer and AVANCE400 (400 MHz) spectrometer using tetramethylsilane as internal standard. All delta values were shown in ppm. IR spectra were determined with Perkin-Elmer Paragon 1000, and were shown in $cm^{-1}$. MS spectra were determined with JEOL JMS-AX505W. In addition, the abbreviations used in the following Synthetic Examples, Reference Examples and tables have the following meanings. Me: methyl group, Et: ethyl group, Ph: phenyl group, Pr-n (or n-Pr): n-propyl, Pr-i (or i-Pr, or $^{i}$Pr): isopropyl, Pr-cyclo (or cyclo-Pr): cyclopropyl, Bu-n (or n-Bu): n-butyl, Bu-i (or i-Bu): isobutyl, Bu-s (or s-Bu): sec-butyl, Bu-t (or t-Bu): tert-butyl, s: singlet, br: broad, brs: broad singlet, d: doublet, t: triplet, q: quartet, qu: quintet, sep: septet, m: multiplet, dd: double doublet, dt: double triplet, J: coupling constant, Hz: herz, %: % by weight, m.p.: melting point, and room temperature means the temperature of about 15 to 25°C.

Synthetic Example 1

Synthesis of 1-(4-chlorophenyl)-3-(2,6-difluorobenzoyl)-5-methyltetrahydro-1,3,5-triazin-2(*1H*)-one (compound No. III-1)

8.45 g (40.6 mmol) of phosphorus pentachloride (96%) was added to 45 ml of dichloromethane, and a solution obtained by dissolving 1,3,5-trimethylhexahydro-1,3,5-triazine (2.50 g, 19.3 mmol) in dichloromethane (45 -ml) under heating at 40 °C was added dropwise to the above mixture. The reaction mixture was heated at 40 °C for 2.5 hrs, and then ice-cooled. 2.00 g (6.44 mmol) of 1-(4-Chlorophenyl)-3-(2,6-difluorobenzoyl)urea was added thereto at 1 °C. After stirring for 15 mins., a solution of triethylamine 6.2 ml (4.4 mmol) in dichloromethane (25 ml) was added dropwise at 1-3 °C, and stirred at 1 °C for 1.5 hrs. After stirring at room temperature for 15 mins., the reaction solution was added into. an ice-cooled aqueous. sodium hydroxide solution (prepared from 10.9 g (272 mmol) of sodium hydroxide and 160 ml of water) at 2-15°C. After separating the layers, water layer was extracted twice with 50 ml of dichloromethane. The organic layer was washed with 50 ml of water, dried over anhydrous magnesium sulfate, and concentrated under reduced pressure. The resulting residue was purified with silica gel column chromatography (chloroform : hexane : acetone = 10 : 10 : 1), and recrystallized from ethyl acetate-hexane. The obtained crystals was further purified with silica gel column chromatography (chloroform : hexane : acetone = 10 : 5 : 1), and recrystallized from ethyl acetate-hexane to give 0.46 g (1.3 mmol), of 1-(4-chlorophenyl)-3-(2,6-difluorobenzoyl)-5-methyltetrahydro-1,3,5-triazin-2(*1H*)-one as white solid (m.p. 140.0-140.5°C, yield 19%).
$^{1}$HNMR ($CDCl_3$)δppm: 2.80 (3H, s, NMe), 4.57 (2H, s, $NCH_2N$), 5.02 (2H, s, $NCH_2N$), 6.87 (2H, t J=8.4Hz, Ph), 7.13-7.35 (5H, m, Ph).
IR (nujol)ν$cm^{-1}$: 2925, 2854, 1702, 1666, 1623, 1340, 1306, 1190, 1016, 996, 777.
MS (EI) m/z: 365 ($M^+$), 153(BP).

Synthetic Example 2

Synthesis of 3-(4-chlorophenyl)-5-(2,6-difluorobenzoyl)tetrahydro-*4H*-1,3,5-oxadiazin-4-one (compound No. I-1)

0.29 g (8.0 mmol) of Sodium hydride (60%) was suspended in 13 ml of DMF. A solution obtained by dissolving 1-(4-chlorophenyl)-3-(2,6--difluorobenzoyl)urea (1.00 g, 3.22 mmol) in DMF (30 ml) under ice-cooling was added thereto at 3°C. After stirring for 40 mins., a solution of bischloromethyl ehter (0.44 g, 3.8 mmol) in DMF (7 ml) was added at 3-5°C. The reaction mixture was stirred at room temperature for 2 hrs. The reaction solution was poured into 130 ml of ice-water, extracted with 130. ml. of ethyl acetate, and the organic layer was washed three times. with 130 ml of saturated brine. After drying over anhydrous magnesium sulfate, the solution was concentrated under reduce pressure. The resulting residue was purified with silica gel column chromatography (chloroform : hexane : acetone = 10 : 10 : 1) to give 0.22 g (0.62 mmol) of 3-(4-chlorophenyl)-5-(2,6-difluorobenzoyl)tetrahydro-4*H*-1,3,5-oxadiazin-4-one as white solid (m.p. 125-126°C, yield 19%).
$^{1}$HNMR ($CDCl_3$)δppm: 5.16 (2H, s, $OCH_2N$), 5.52(2H, s, $OCH_2N$), 6.85-6.93 (2H, m, Ph), 7.17-7.37 (5H, m, Ph).
IR (nujol)ν$cm^{-1}$: 2923, 2854, 1709, 1675, 1626, 1592, 1492, 1450, 1394, 1346, 1323, 1296, 1274, 1247, 1118, 1092, 1039, 1017, 1002, 848,831, 792, 743, 724, 594, 524, 514.
MS (EI) m/z: 352 ($M^+$), 139(BP).

Reference Example 1

Synthesis of 1,3,5-triisopropylhexahydro-1,3,5-triazine

25.0 ml (294 mmol) of isopropylamine was dissolved in 75 ml of toluene. 28.6 g (352 mmol) of 37% aqueous formaldehyde solution was added thereto under ice-cooling. After stirring at room temperature for 20 hrs, the reaction solution was washed twice with 125 ml of water. The solution was dried over anhydrous magnesium sulfate, and concentrated under reduce pressure to give 18.87 g. (88.43 mmol) of. 1,3,5-triisopropylhexahydro-1,3,5-triazine as colorless liquid.

Synthetic Example 3

Synthesis of 1-(4-chlorophenyl)-3-(2,6-difluorobenzoyl)-5-isopropyltetrahydro-1,3,5-triazin-2-(*1H*)-one (compound No. III-3)

1.34 g (6.44 mmol) of phosphorus pentachloride (96%) was added to 8 ml of dichloromethane, and a solution obtained by dissolving 1,3,5-triisopropylhexahydro-1,3,5-triazine (0.69 g, 3.2 mmol) in dichloromethane (4 ml) under heating at 40 °C was added dropwise to the above mixture. The reaction mixture was heated at 40 °C for 3 hrs, and then ice-cooled. 1.00 g (3.22 mmol) of 1-(4-Chlorophenyl)-3-(2,6-difluorobenzoyl)urea was added thereto at 1 °C. After stirring for 15 mins., a solution of triethylamine 1.0 ml (7.2 mmol) in dichloromethane (4 ml) was added dropwise at 1-3 °C, and stirred at 1 °C for 1 hr. After stirring at room temperature for 3.5 hrs, the reaction solution was added to an ice-cooled aqueous sodium hydroxide solution (prepared from 1.8 g (45 mmol) of sodium hydroxide and 30 ml of water) at 2-15°C. After separating the layers, water layer was extracted twice with 25 ml of dichloromethane. The organic layer was washed with 25 ml of water, dried over anhydrous magnesium sulfate, and concentrated under reduced pressure. The resulting residue was purified with silica gel column chromatography (chloroform : hexane : acetone = 15 : 10 : 1) to give 0.19 g (0.48 mmol) of 1-(4-chlorophenyl)-3-(2,6-difluorobenzoyl)-5-isopropyltetrahydro-1,3,5-triazin-2(*1H*)-one as white solid (m.p. 130-131°C, yield 15%).
$^1$HNMR ($CDCl_3$)δppm: 1.24 (6H, d J=6.3Hz, $CHMe_2$), 3.34 (1H, hep. J=6.3Hz, $CHMe_2$), 4.70 (2H, s, $NCH_2N$), 5.17 (2H, s, $NCH_2N$), 6.82-6.90 (2H, m, Ph), 7.12-7.35 (5H, m, Ph).
IR (nujol)ν$cm^{-1}$: 2924, 2854, 1676, 1494, 1340, 1309, 1191, 793.

Reference Example 2

Synthesis of 1,3,5-triphenylhexahydro-1,3,5-triazine

4.00 g (43.0 mmol) of aniline was dissolved in 100 ml of toluene. 6.98 g (86.0 mmol) of 37% aqueous formaldehyde solution was added thereto. After heating under reflux for 2.5 hrs with dehydrating, the reaction solution was cooled under room temperature. The reaction solution was concentrated under reduce pressure to give 2.99 g (9.48 mmol) of 1,3,5-triphenylhexahydro-1,3,5-triazine as white solid.

Synthetic Example 4

Synthesis of 1-(4-chlorophenyl)-3-(2,6-difluorobenzoyl)-5-phenyltetrahydro-1,3,5-triazin-2(*1H*)-one (compound No. III-2)

3.47 g (16.7 mmol) of phosphorus pentachloride (96%) was added to 20 ml of dichloromethane, and a solution obtained by dissolving 1,3,5-triisopropylhexahydro-1,3,5-triazine (2.50 g, 7.93 mmol) in dichloromethane (50 ml) under heating at 40 °C was added dropwise to the above mixture. The reaction mixture was heated at 40 °C for 4 hrs, and then ice-cooled. 0.82 g (2.6 mmol) of 1-(4-chlorophenyl)-3-(2,6-difluorobenzoyl)urea was added thereto at 1 °C. After stirring for 15 mins., a solution of triethylamine 2.5 ml (18 mmol) in dichloromethane (10 ml) was added dropwise at 1-3 °C, and stirred at 1 °C for 80 mins. After stirring at room temperature for 1 hr, the reaction solution was added to an ice-cooled aqueous sodium hydroxide solution (prepared from 4.6 g (115 mmol) of sodium hydroxide and 70 ml of water) at 2-15°C. After separating the layers, water layer was extracted twice with 20 ml of dichloromethane. The organic layer was washed with 20 ml of water, dried over anhydrous magnesium sulfate, and concentrated under reduced pressure. The resulting residue was purified with silica gel column chromatography (chloroform : hexane : acetone = 1 : 4 : 1) to give 0.18 g (0.42 mmol) of 1-(4-chlorophenyl)-3-(2,6-difluorobenzoyl)-5-phenyltetrahydro-1,3,5-triazin-2(*1H*)-one as white solid (m.p. 166.5-167.5°C, yield 16%).
$^1$HNMR ($CDCl_3$)δppm: 5.15 (2H, s, $NCH_2N$), 5.59 (2H, s, $NCH_2N$), 6.83 (2H, t J=8.4Hz, Ph), 6.99-7.42 (10H, m, Ph).

IR (nujol)νcm$^{-1}$: 2924, 2854, 1696, 1681, 1495, 1430, 1312, 1292, 1175, 668.
MS (EI) m/z: 427 ($M^+$), 105 (BP).

Reference Example 3

Synthesis of 1,3,5-triallylhexahydro-1,3,5-triazine

0.56 g (14 mmol) of sodium hydroxide was dissolved in 21 ml of water, and the solution was mixed with 21 ml of diethyl ether. A solution of allylamine (4.20 g, 73.6 mmol) in diethyl ether (21 ml) was added thereto under ice-cooling. 14 ml (170 mmol) of 37% aqueous formaldehyde solution was added thereto, and stirred at room temperature for 3 days. The layers of the reaction solution was separated, and extracted with 30 ml of diethyl ether. The organic layer was washed with 40 ml of water, dried over anhydrous magnesium sulfate, and concentrated under reduce pressure to give 6.68 g (33.2 mmol) of 1,3,5-triallylhexahydro-1,3,5-triazine as an oil.

Synthetic Example 5

Synthesis of 5-allyl-1-(4-chlorophenyl)-3-(2,6-difluorobenzoyl)tetrahydro-1,3,5-triazin-2(*1H*)-one (compound No. III-5)

5.4 g (6.44 mmol) of phosphorus pentachloride (96%) was added to 35 ml of dichloromethane, and a solution obtained by dissolving 1,3,5-triallylhexahydro-1,3,5-triazine (2.67 g, 12.9 mmol) in dichloromethane (10 ml) under heating at 40 °C was added dropwise to the above mixture. The reaction mixture was heated at 40 °C for 2.5 hrs, and then ice-cooled. 1.00 g (3.22 mmol) of 1-(4-chlorophenyl)-3-(2,6-difluorobenzoyl)urea was added thereto at 1 °C. After stirring for 15 mins., a solution of triethylamine 1.8 ml (13 mmol) in dichloromethane (20 ml) was added dropwise at 1-3 °C, and stirred at 1 °C for 1 hr. After stirring at room temperature for 4 hr, the reaction solution was added to an ice-cooled aqueous 10% sodium hydroxide solution (80 ml) at 2-15°C. After separating the layers, water layer was extracted twice with 10 ml of dichloromethane. The organic layer was washed with 70 ml of water, dried over anhydrous magnesium sulfate, and concentrated under reduced pressure. The resulting residue was purified with silica gel column chromatography (chloroform : hexane : acetone = 1 : 3 : 1) to give 0.95 g (2.4 mmol) of 5-allyl-1-(4-chlorophenyl)-3-(2,6-difluorobenzoyl)tetrahydro-1,3,5-triazin-2(*1H*)-one as white solid (m.p. 87-88°C, yield 75%).
$^1$HNMR ($CDCl_3$)δppm: 3.59 (2H, d J=6.5Hz, $NCH_2C$=), 4.60 (2H, s, $NCH_2N$), 5.08 (2H, s, $NCH_2N$), 5.28-5.37 (2H, m, $=CH_2$), 5.81-5.98 (1H, m, CH), 6.83-6.91 (2H, m, Ph), 7.11-7.35 (5H, m, Ph).
IR (nujol)νcm$^{-1}$: 2924, 2854, 1677, 1240, 1188, 999, 789.

Synthetic Example 6

Synthesis of 5-benzyloxycarbonyl-1-(4-chlorophenyl)-3-(2,6-difluorobenzoyl)tetrahydro-1,3,5-triazin-2(*1H*)-one (compound No. III-7)

0.54 g (14 mmol) of sodium hydride (60%) was suspended in 30 ml of DMF. With ice-cooling, 2.00 g (6.44 mmol) of 1-(4-chlorophenyl)-3-(2,6-difluorobenzoyl)urea was added thereto at 3°C. After stirring for 30 mins., a solution of benzyl N,N-bis(chlorometyl)carbamate (1.06 g, 6.45 mmol) in THF (5 ml) was added dropwise at 1-2°C. The reaction mixture was stirred at room temperature for 6 hrs. To the reaction solution was added 4 ml of water, the solution was concentrated under reduce pressure. To the resulting residue was added 50 ml of water, extracted with 80 ml of ethyl acetate, and the organic layer was washed 3 times with 100 ml of water. After drying over anhydrous magnesium sulfate, the organic layer was concentrated under reduced pressure. The obtained residue was purified with silica gel column chromatography (chloroform : ethyl acetate = 40 : 1) to give 1.46 g (3.00 mmol) of 5-benzyloxycarbonyl-1-(4-chlorophenyl)-3-(2,6-difluorobenzoyl)tetrahydro-1,3,5-triazin-2(*1H*)-one as an oil (yield 46.6%).
$^1$HNMR ($CDCl_3$)δppm: 5.17 (2H, s, $NCH_2N$), 5.27 (2H, s, $COOCH_2$), 5.57 (2H, s, $NCH_2N$), 6.84-6.92. (2H, m, Ph), 7.20-7.37 (10H, m, Ph).
IR (neat)νcm$^{-1}$: 3034, 1732, 1698, 1494, 1464, 1434, 1342, 1307, 1237, 1221, 1005, 791, 755.

Synthetic Example 7

Synthesis of 1-(4-chlorophenyl)-3-(2,6-difluorobenzoyl)tetrahydro-1,3,5-triazin-2(*1H*)-one (compound No. III-6)

0.02 g of 10% palladium carbon and 0.50 g (1.0 mmol) of 5-benzyloxycarbonyl-1-(4-chlorophenyl)-3-(2,6-difluorobenzoyl)tetrahydro-1,3,5-triazin-2(*1H*)-one was added to 15 ml of ethanol, and stirred for 7.5 hrs at room temperature under hydrogen atmosphere. The reaction solution was filtered, and the filtrate was concentrated under reduced

pressure. The resulting residue was purified with silica gel column chromatography (chloroform : ethyl acetate = 10 : 1) to give 0.17 g (0.48 mmol) of 1-(4-chlorophenyl)-3-(2,6-difluorobenzoyl)tetrahydro-1,3,5-triazin-2(*1H*)-one as a white solid (m.p. 167-168°C, yield 49%).
$^1$HNMR ($CDCl_3$)δppm: 2.73 (1H, brt, NH), 4.63 (2H, d J=8.4Hz, $NCH_2N$), 5.06 (2H, d J=8.4Hz, $NCH_2N$), 6.82-6.91 (2H, m, Ph), 7.14-7.35 (5H, m, Ph).
IR (nujol)ν$cm^{-1}$: 3326, 2954, 2925, 2854, 1683, 1666, 1490, 1312, 1014.

Synthetic Example 8

Synthesis of 5-acetyl-1-(4-chlorophenyl)-3-(2,6-difluorobenzoyl)tetrahydro-1,3,5-triazin-2(*1H*)-one (compound No. III-9)

0.20 g (0.57 mmol) of 1-(4-chlorophenyl)-3-(2,6-difluorobenzoyl)tetrahydro-1,3,5-triazin-2(*1H*)-one and 0.1 ml (0.7 mmol) of triethylamine was added to 10 ml of THF, and 0.05 ml (0.7 mmol) of acetyl chloride was added thereto under ice-cooling. Further, to the mixture was added 10 ml of THF, 0.07 g (0.6 mmol) of DMAP and 0.25 ml (3.5 mmol) of acetyl chloride, and stirred at room temperature for 20 hrs. The reaction solution was filtered, and the filtrate was concentrated under reduced pressure. The resulting residue was purified with silica gel column chromatography (chloroform : ethyl acetate = 5 : 1) to give 0.14 g (0.36 mmol) of 5-acetyl-1-(4-chlorophenyl)-3-(2,6-difluorobenzoyl) tetrahydro-1,3,5-triazin-2(*1H*)-one as a white solid (m.p. 171.0-171.5°C, yield 64%).
$^1$HNMR ($CDCl_3$)δppm: 2.33 (3H, s, COMe) , 5.25 (2H, s, $NCH_2N$), 5.61 (2H, s, $NCH_2N$), 6.86-6.94 (2H, m, Ph), 7.21-7.37 (5H, m, Ph).
IR (nujol)ν$cm^{-1}$: 2921, 1703, 1683, 1674, 1592, 1313, 1223, 1200, 1014, 1000, 722.

Synthetic Example 9

Synthesis of 1-(4-chlorophenyl)-3-(2,6-difluorobenzoyl)-5-methanesulfonyltetrahydro-1,3,5-triazin-2(*1H*)-one (compound No. III-12)

0.20 g (0.57 mmol) of 1-(4-chlorophenyl)-3-(2,6-difluorobenzoyl)tetrahydro-1,3,5-triazin-2(*1H*)-one and 0.12 g (1.2 mmol) of triethylamine was added to 5 ml of THF, and a solutiion obtained by dissolving 0.13 g (1.1 mmol) of methanesulfonyl chloride in 5 ml of THF under ice-cooling was added thereto. After stirring at room temperature for 4 hrs, the reaction solution was filtered, and the filtrate. was concentrated under reduced pressure. The resulting residue was purified with silica gel column chromatography (hexane : ethyl acetate = 1 : 1) to give 0.18 g (0.42 mmol) of 1-(4-chlorophenyl)-3-(2,-6-difluorobenzoyl)-5-methanesulfonyltetrahydro-1,3,5-triazin-2(*1H*)-one as a white solid (m. p. 178.0-178.5°C, yield 72%).
$^1$HNMR ($CDCl_3$)δppm: 3.17 (3H, s, $SO_2Me$), 5.18 (2H, s, $NCH_2N$), 5.58 (2H, s, $NCH_2N$), 6.85-6.93 (2H, m, Ph), 7.19-7.39 (5H, m, Ph).
IR (nujol)ν$cm^{-1}$: 2925, 2854, 1684, 1624, 1471, 1444, 1354, 1312, 1252, 1159, 1015, 966.

Reference Example 4

Synthesis of bisiodomethyl thioether

9.24 g (70.5 mmol) of bischloromethyl thioether was dissolved in 50 ml of acetone. 27.02 g (147 mmol) of sodium iodide was added thereto, and stirred overnight at room temperature under light shielding. The insolubles were filtered off, and the filtrate was concentrated under reduce pressure. To the obtained residue was added 30 ml of water, and extracted with 50 ml of chloroform. After drying over anhydrous magnesium sulfate, the organic layer was concentrated under reduced pressure to give 21.30 g (67.8 mmol) of bisiodomethyl thioether as an oil.

Synthetic Example 10

Synthesis of 3-(4-chlorophenyl)-5-(2,6-difluorobenzoyl)tetrahydro-*4H*-1,3,5-thiadiazin-4-one (compound No. II-1)

0.28 g (7.7 mmol) of sodium hydride (60%) was suspended in 20 ml of DMF. With ice-cooling, 1.00 g (3.11 mmol) of 1-(4-chlorophenyl)-3-(2,6-difluorobenzoyl)urea was added thereto at 3°C. After stirring for 40 mins., a solution of bisiodomethyl thioether (1.10 g, 3.50 mmol) in DMF (5 ml) was added dropwise at 3-5°C. The reaction mixture was stirred at room temperature for 18 hrs. The reaction solution was ice-cooled, and mixed solution of 20 ml of saturated aqueous ammonium chloride solution and 10 ml of water was added thereto at 2-20°C. The reaction solution was

extracted 3 times with 20 ml of ethyl acetate, and the organic layer was washed 3 times with 15 ml of saturated brine. After drying over anhydrous magnesium sulfate, the organic layer was concentrated under reduced pressure. The obtained residue was purified with silica gel column chromatography (ethyl acetate : hexane = 1 : 3), and then purified with HPLC for fractionation (column: SI-40B Φ 26x300mm, mobile phase: ethyl acetate : hexane = 25 : 75) to give 0.24 g (0.65 mmol) of 3-(4-chlorophenyl)-5-(2,6-difluorobenzoyl)tetrahydro-*4H*-1,3,5-thiadiazin-4-one as a white solid (m.p. 128.5-130.5°C, yield 20%).
$^1$HNMR ($CDCl_3$)δppm: 4.71 (2H, s, $SCH_2N$), 5.15 (2H, s, $SCH_2N$), 6.88 (2H, t J=7.9Hz, Ph), 7.17-7.37 (5H, m, Ph).
IR (neat)ν$cm^{-1}$: 1691, 1673, 1624, 1489, 1334, 1237, 1006, 790.

Synthetic Example 11

Synthesis of 3-(4-chlorophenyl)-5-(2,6-difluorobenzoyl)tetrahydro-*4H*-1,3,5-thiadiazin-4-one-1-oxide (compound No. II-2)

300.9 mg (0.816 mmol) of 3-(4-chlorophenyl)-5-(2,6-difluorobenzoyl)tetrahydro-*4H*-1,3,5-thiadiazin-4-one was dissolved in 10 ml of chloroform. 415.5 mg (3.43 mmol) of m-CPBA (70%) was added thereto, and stirred for 2 hrs at room temperature. To the reaction solution was added 10 ml of water and 0.4 g of potassium carbonate, and the layers were separated. The water layer was extracted 2 times with 10 ml of chloroform. After drying over anhydrous magnesium sulfate, the organic layer was concentrated under reduced pressure. The obtained residue was crystallized from diethyl ether-hexane to give 181.5 mg (0.472 mmol) of 3-(4-chlorophenyl)-5-(2,6-difluorobenzoyl)tetrahydro-*4H*-1,3,5-thiadiazin-4-one-1-oxide as a white solid (m.p. 220.0-220.5°C, yield 86%).
$^1$HNMR ($CDCl_3$)δppm: 4.43 (1H, d J=13.7Hz, SOCHN), 4.67 (1H, d J=13.6Hz, SOCHN), 4.76 (1H, d J=13.6Hz, SOCHN), 5.77 (1H, d J=13.7Hz,. SOCHN), 6.97 (2H, t J=8.8Hz, Ph), 7.29-7.50 (5H, m, Ph).
IR (nujol)ν$cm^{-1}$: 1713, 1677, 1624, 1445, 1366, 1338, 1068, 1008, 800.

Synthetic Example 12

Synthesis of 3-(4-chlorophenyl)-5-(2,6-difluorobenzoyl)tetrahydro-*4H*-1,3,5-thiadiazin-4-one-1,1-dioxide (compound No. II-3)

201.2 mg (0.546 mmol) of 3-(4-chlorophenyl)-5-(2,6-difluorobenzoyl)tetrahydro-*4H*-1,3,5-thiadiazin-4-one was dissolved in 10 ml of chloroform. 131.9 mg (0.535 mmol) of m-CPBA (70%) was added thereto, and stirred for 2 hrs under ice-cooling. To the reaction solution was added 10 ml of water and 0.2 g of potassium carbonate, and the layers were separated. The water layer was extracted 2 times with 10 ml of chloroform. After drying over anhydrous magnesium sulfate, the organic layer was concentrated under reduced pressure. The obtained residue was dissolved in 50 ml of chloroform, washed with aqueous potassium carbonate solution, and concentrated under reduced pressure. The obtained residue was purified with silica gel column chromatography (ethyl acetate : chloroform = 1 : 5), and then crystallized from diethyl ether-hexane to give 53.2 mg (0.133 mmol) of 3-(4-chlorophenyl)-5-(2,6-difluorobenzoyl)tetrahydro-*4H*-1,3,5-thiadiazin-4-one-1,1-dioxide as a white solid (m.p. 274-275°C, yield 16%).
$^1$HNMR ($CDCl_3$)δppm: 4.89 (2H, s, $SO_2CH_2N$), 5.27 (2H, s, $SO_2CH_2N$), 7.01 (2H, t J=7.9Hz, Ph), 7.25-7.31 (2H, m, Ph), 7.36-7.55 (3H, m, Ph).
IR (neat)ν$cm^{-1}$: 1714, 1700, 1624, 1341, 1237, 1136, 1006.

Synthetic Example 13

Synthesis of 5-benzyloxycarbonyl-1-[4-(2-chloro-4-trifluoromethylphenoxy)-2-fluorophenyl]-3-(2,6-difluorobenzoyl) tetrahydro-1,3,5-triazin-2(*1H*)-one (compound No. III-22)

0.44 g (11 mmol) of sodium hydride (60%) was suspended in 20 ml of DMF. With ice-cooling, 2.00 g (4.09 mmol) of 1-[4-(2-chloro-4-trifluoromethylphenoxy)-2-fluorophenyl]-3-(2,6-difluorobenzoyl)urea was added thereto at 3°C. After stirring for 30 mins., a solution of benzyl N,N-bis(chloromethyl)carbamate (1.22 g, 4.92 mmol) in THF (4 ml) was added dropwise at 1-2°C. The reaction mixture was stirred at room temperature for 1.5 hrs. To the reaction solution was added 4 ml of water, then 20 ml of water, and extracted with 50 ml of ethyl acetate. The organic layer was washed 3 times with 20 ml of water, and 3 times with 20 ml of saturated brine. After drying over anhydrous magnesium sulfate, the organic layer was concentrated under reduced pressure. The obtained residue was purified with silica gel column chromatography (hexane : ethyl acetate = 2 : 1) to give 1.38 g (2.08 mmol) of 5-benzyloxycarbonyl-1-[4-(2-chloro-4-trifluoromethylphenoxy)-2-fluorophenyl]-3-(2,6-difluorobenzoyl)tetrahydro-1,3,5-triazin-2(*1H*)-one as an oil (yield 50.7%).

$^1$HNMR ($CDCl_3$)δppm: 5.12 (2H, s, $NCH_2N$), 5.28 (2H, s, $COOCH_2$), 5.59 (2H, s, $NCH_2N$), 6.71-6.91 (4H, m, Ph), 7.07 (1H, d J=8.5Hz, Ph), 7.23-7.36 (7H, m, Ph), 7.50 (1H, d J=8.6Hz, Ph), 7.75 (1H, s, Ph).
IR (neat)νcm$^{-1}$: 3070, 2962, 1705, 1626, 1595, 1470, 1434, 1323, 1265, 1239, 1129, 1082.

Synthetic Example 14

Synthesis of 1-[4-(2-chloro-4-trifluoromethylphenoxy)-2-fluorophenyl]-3-(2,6-difluorobenzoyl)tetrahydro-1,3,5-triazin-2(*1H*)-one (compound No. III-27)

0.02 g of 10% palladium carbon and 1.17 g (1.76 mmol) of 5-benzyloxycarbonyl-1-[4-(2-chloro-4-trifluoromethylphenoxy)-2-fluorophenyl]-3-(2,6-difluorobenzoyl)tetrahydro-1,3,5-triazin-2(*1H*)-one was added to 25 ml of ethanol, and stirred for 27 hrs at room temperature under hydrogen atmosphere. The reaction solution was filtered, and the filtrate was concentrated under reduced pressure. The resulting residue was purified with silica gel column chromatography (chloroform : ethyl acetate = 20 : 1 → 5 : 1) to give 0.61 g (1.2 mmol) of 1-[4-(2-chloro-4-trifluoromethylphenoxy)-2-fluorophenyl]-3-(2,6-difluorobenzoyl)tetrahydro-1,3,5-triazin-2(*1H*)-one as a white solid (m.p. 135-137°C, yield 65%).
$^1$HNMR ($CDCl_3$)δppm: 4.57 (2H, s, $NCH_2N$), 5.08 (2H, s, $NCH_2N$), 6.73-6.91 (4H, m, Ph), 7.08 (1H, d J=8.8Hz, Ph), 7.20-7.32 (2H, m, Ph), 7.50 (1H, d J=8.6Hz, Ph), 7.74 (1H, s, Ph).
IR (nujol)νcm$^{-1}$: 3316, 2925, 2854, 1676, 1324, 1236, 1173, 1131, 1082, 789.

Synthetic Example 15

Synthesis of methyl 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetate (compound No. III-37)

1.00 g (1.89 mmol) of 1-[4-(2-chloro-4-trifluoromethylphenoxy)-2-fluorophenyl]-3-(2,6-difluorobenzoyl)tetrahydro-1,3,5-triazin-2(1H)-one and 0.32 ml (2.3 mmol) of triethylamine was added to 5 ml of THF, and a solutiion obtained by dissolving 0.28 g (2.3 mmol) of methyloxalyl chloride in 5 ml of THF under ice-cooling was added thereto, and stirred at room temperature for 30 mins. The reaction solution was filtered, and the filtrate was concentrated under. reduced pressure. The resulting residue was purified with silica gel column chromatography (chloroform : ethyl acetate = 40 : 1) to give 0.55 g (0.89 mmol) of methyl 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetate as an amorphous (yield 95%).
$^1$HNMR ($CDCl_3$)δppm: (mixture of rotational isomer) (major) 3.97 (3H, s, COOMe), 5.24 (2H, s, $NCH_2N$), 5.70 (2H, s, $NCH_2N$), 6.77 (2H, d J=8.9Hz, Ph), 6.87-6.95 (2H, m, Ph), 7.10 (1H, d J=8.7Hz, Ph), 7.31-7.40 (2H, m, Ph), 7.52 (1H, d J=8.4Hz, Ph), 7.76 (1H, s, Ph) + (minor) 3.91 (3H, s, COOMe), 5.33 (2H, s, $NCH_2N$), 5.67 (2H, s, $NCH_2N$), 6.77 (2H, d J=8.9Hz, Ph), 6.87-6.95 (2H, m, Ph), 7.10 (1H, d J=8.7Hz, Ph), 7.31-7.40 (2H, m, Ph), 7.52 (1H, d J=8.4Hz, Ph), 7.76 (1H, s ,Ph).
IR (nujol)νcm$^{-1}$: 1738, 1693, 1626, 1323, 1266, 1238, 1203, 1172, 1136, 1081, 1006, 970, 791, 721. MS (CI) m/z: 616 ($M^+$+1, BP).

Synthetic Example 16

Synthesis of benzyl 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetate (compound No. III-39)

0.70 g (1.3 mmol) of 1-[4-(2-chloro-4-trifluoromethylphenoxy)-2-fluorophenyl]-3-(2,6-difluorobenzoyl)tetrahydro-1,3,5-triazin-2(1H)-one and 0.22 ml (1.6 mmol) of triethylamine was added to 6 ml of THF, and a solution obtained by dissolving 0.31 g (1.6 mmol) of benzyloxalyl chloride in 3 ml of THF under ice-cooling was added thereto, and stirred at room temperature for 50 mins. The reaction solution was filtered, and the filtrate was concentrated under reduced pressure. The resulting residue was purified with silica gel column chromatography (chloroform : ethyl acetate = 40 : 1) to give 0.77 g (1.1 mmol) of benzyl 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetate as an amorphous (yield 86%).
$^1$HNMR ($CDCl_3$)δppm: (mixture of rotational isomer) (major) 5.21 (2H, s, $NCH_2N$), 5.36 (2H, s, $COOCH_2$), 5.66 (2H, s, $NCH_2N$), 6.67-6.91 (4H, m, Ph), 7.09 (1H, d J=8.5Hz, Ph), 7.25-7.55 (8H, m, Ph), 7.75 (1H, s, Ph) + (minor) 5.21 (2H, s, $NCH_2N$), 5.31 (2H, s, $COOCH_2$), 5.66 (2H, s, $NCH_2N$), 6.67-6.91 (4H, m, Ph), 7.09 (1H, d J=8.5Hz, Ph), 7.25-7.55 (8H, m, Ph), 7.75 (1H, s, Ph).
IR (nujol)νcm$^{-1}$: 1694, 1323, 1266, 1238, 1173, 1135, 1080.

Synthetic Example 17

Synthesis of 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetic acid (compound No. III-38)

0.02 g of 10% palladium carbon and 0.40 g (0.58 mmol) of benzyl 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetate was added to 15 ml of ethanol, and stirred for 1 hr at room temperature under hydrogen atmosphere. The reaction solution was filtered, and the filtrate was concentrated under reduced pressure. The resulting residue was washed with hexane-diisopropyl ether to give 0.32 g (0.53 mmol) of 2-[3-[4-(2-chloro-4-trifluoromethylphenoxy)-2-fluorophenyl]-5-(2,6-difluorobenzoyl)hexahydro-4-oxo-1,3,5-triazin-1-yl]-2-oxoacetic acid as a white solid (m.p. 67-72°C, yield 91%).
$^1$HNMR (DMSO-$d_6$)δppm: (mixture of rotational isomer) (major) 5.22 (2H, s, $NCH_2N$), 5.70 (2H, s, $NCH_2N$), 6.96 (1H, d J=8.2Hz, Ph), 7.08-7.31 (4H, m, Ph), 7.48-7.56 (2H, m, Ph), 7.72 (1H, d J=7.0Hz, Ph), 8.03 (1H, s, Ph) + (minor) 5.27 (2H, s, $NCH_2N$), 5.65 (2H, s, $NCH_2N$), 6.96 (1H, d J=8.2Hz, Ph), 7.08-7.31 (4H, m, Ph), 7.48-7.56 (2H, m, Ph), 7.72 (1H, d J=7.0Hz, Ph), 8.03 (1H, s, Ph).
IR (nujol)ν$cm^{-1}$: 3400, 1693, 1626, 1511, 1497, 1324, 1266, 1238, 1175, 1130, 1082, 1007.

Synthetic Example 18

Synthesis of 2-[3-[4-(2-chloro-4-trifluoromethylphenoxy)-2-fluorophenyl]-5-(2,6-difluorobenzoyl)hexahydro-4-oxo-1,3,5-triazin-1-yl]-N,N-dimethyl-2-oxoacetamide (compound No. III-43)

0.35 g (2.8 mmol) of oxalyl chloride and 0.09 ml. (0.6 mmol) of triethylamine was added to 7 ml of acetonitrile, and a solutiion obtained by dissolving 0.29 g (0.55 mmol) of 1-[4-(2-chloro-4-trifluoromethylphenoxy)-2-fluorophenyl]-3-(2,6-difluorobenzoyl)tetrahydro-1,3,5-triazin-2(*1H*)-one in 15 ml of acetonitrile under ice-cooling was added thereto, and stirred for 70 mins. under ice-cooling. 0.69 g (8.5 mmol) of dimethylamine hydrochloride and 2.3 ml (17 mmol) of triethylamine were added to 15 ml of acetonitrile. The previously prepared reaction solution was added thereto under ice-cooling, and stirred for 70 mins. The reaction solution was filtered, and the filtrate was concentrated under reduced pressure. The resulting residue was purified with silica gel column chromatography (chloroform : ethyl acetate = 5 : 1) to give 0.21 g (0.33. mmol) of 2-[3-[4-(2-chloro-4-trifluoromethylphenoxy)-2-fluorophenyl]-5-(2,6-difluorobenzoyl)hexahydro-4-oxo-1,3,5-triazin-1-yl]-N,N-dimethyl-2-oxoacetamide as a white solid (m.p. 98-100°C, yield 60%).
$^1$HNMR ($CDCl_3$)δppm: (mixture of rotational isomer) (major) 3.06 (3H, s, NMe), 3.08 (3H, s, NMe), 5.25 (2H, s, $NCH_2N$), 5.60 (2H, s, $NCH_2N$), 6.76-6.94 (4H, m, Ph), 7.11 (1H, d J=8.6Hz, Ph), 7.26-7.34 (2H, m, Ph) , 7.53 (1H, d J=8.6Hz, Ph), 7.76 (1H, s, Ph) + (minor) 3.03 (3H, s, NMe), 3.12 (3H, s, NMe), 5.14 (2H, s, $NCH_2N$), 5.69 (2H, s, $NCH_2N$), 6.76-6.94 (4H, m, Ph), 7.11 (1H, d J=8.6Hz, Ph), 7.26-7.34 (2H, m, Ph), 7.53 (1H, d J=8.6Hz, Ph), 7.76 (1H, s, Ph).
IR (nujol)ν$cm^{-1}$: 1723, 1686, 1672, 1646, 1326, 1271, 1122, 1083, 1008, 971, 722.

Synthetic Example 19.

Synthesis of 1-(2,6-difluorobenzoyl)-3-[4-(difluoromethylthio)-2-fluorophenyl]-5-methyltetrahydro-1,3,5-triazin-2(*1H*)-one (compound No. III-148)

2.14 g (9.87 mmol) of phosphorus pentachloride (96%) was added to 30 ml of dichloromethane, and a solution obtained by dissolving 1,3,5-trimethylhexahydro-1,3,5-triazine (0.69 g, 5.34 mmol) in dichloromethane (10 ml) under heating at 40 °C was added dropwise to the above mixture. The reaction mixture was heated at 40 °C for 2 hrs, and then ice-cooled. 1.00 g (2.66 mmol) of 1-(2,6-difluorobenzoyl)-3-[4-(difluoromethylthio)-2-fluorophenyl]urea was added thereto at 1 °C. After stirring for 15 mins., a solution of triethylamine (1.5 ml, 11 mmol) in dichloromethane (6 ml) was added dropwise at 1-3 °C, and stirred at 1 °C for 1 hr. The reaction solution was added to an aqueous sodium hydroxide solution (prepared from 2.30. g (57.5 mmol) of sodium hydroxide and 60 ml of water) at 2-15°C under ice-cooling. The reaction solution was extracted with 40 ml of dichloromethane, dried over anhydrous magnesium sulfate, and concentrated under reduced pressure. The resulting residue was purified with silica gel column chromatography (acetonitrile : chloroform = 1 : 20), and washed out with diethyl ether to give 0.61 g (1.4 mmol) of. 1-(2,6-difluorobenzoyl)-3-[4-(difluoromethylthio)-2-fluorophenyl]-5-methyltetrahydro-1,3,5-triazin-2(*1H*)-one as a white solid (m.p. 135-137°C, yield 53%).
$^1$HNMR ($CDCl_3$)δppm: 2.82 (3H, s, NMe), 4.54 (2H, s, $CH_2$), 5.04 (2H, s, $CH_2$), 6.80 (1H, t J=56.3Hz, $CF_2H$), 6.85-6.95 (2H, m, Ph), 7.28-7.38 (4H, m, Ph).
IR (nujol)ν$cm^{-1}$: 1668, 1622, 1593, 1569, 1464, 1426, 1403, 1378, 1338, 1316, 1275, 1237, 1185, 1163, 1123, 1059, 1028, 1007, 737.

Synthetic Example 20

Synthesis of 1-(2,6-difluorobenzoyl)-3-[2-fluoro-4-(trifluoromethylthio)phenyl]-5-methyltetrahydro-1,3,5-triazin-2(*1H*)-one (compound No. III-160)

1.02 g (4.70 mmol) of phosphorus pentachloride (96%) was added to 15 ml of dichloromethane, and a solution obtained by dissolving 1,3,5-trimethylhexahydro-1,3,5-triazine (0.34 g, 2.6 mmol) in dichloromethane (5 ml) under heating at 40 °C was added dropwise to the above mixture. The reaction mixture was heated at 40 °C for 2 hrs, and then ice-cooled. 0.50 g (1.3 mmol) of 1-(2,6-difluorobenzoyl)-3-[2-fluoro-4-(trifluoromethylthio)phenyl]urea was added thereto at 1 °C. After stirring for 15 mins., a solution of triethylamine (0.7 ml, 5.0 mmol) in dichloromethane (3 ml) was added dropwise at 1-3. °C, and stirred at 1 °C for 1 hr. The reaction solution was added to an aqueous sodium hydroxide solution (prepared from 1.15 g (28.8 mmol) of sodium hydroxide and 30 ml of water) at 2-15°C under ice-cooling. The reaction solution was extracted with 20 ml of dichloromethane, dried over anhydrous magnesium sulfate, and concentrated under reduced pressure. The resulting residue was purified with silica gel column chromatography (ethyl acetate : hexane = 1 : 2), and diethyl ether-hexane was added, cooled, and the precipitated crystals were washed with hexane to give 0.14 g (0.31 mmol) of 1-(2,6-difluorobenzoyl)-3-[2-fluoro-4-(trifluoromethylthio)phenyl]-5-methyltetrahydro-1,3,5-triazin-2(*1H*)-one as a white solid (m.p. 92-94°C, yield 24%).
$^1$HNMR ($CDCl_3$)δppm: 2.82 (3H, s, NMe), 4.56 (2H, s, $CH_2$), 5.05 (2H, s, $CH_2$), 6.86-6.90 (2H, m, Ph), 7.27-7.46 (4H, m, Ph).
IR (nujol)νcm$^{-1}$: 1684, 1625, 1592, 1503, 1468, 1429, 1311, 1186, 1163, 1129, 1104, 1004, 774.

Synthetic Example 21

Synthesis of 1-(2,6-difluorobenzoyl)-3-[2-fluoro-4-(trifluoromethylsulfinyl)phenyl]-5-methyltetrahydro-1,3,5-triazin-2 (*1H*)-one (compound No. III-164)

9.67 g (46.2 mmol) of phosphorus pentachloride (96%) was added to 50 ml of dichloromethane, and a solution obtained by dissolving 1,3,5,-trimethylhexahydro-1,3,5-triazine (3.00 g, 23.2 mmol) in dichloromethane (10 ml) under heating at 40 °C was added dropwise to the above mixture. The reaction mixture was heated at 40 °C for 5 hrs, and then cooled under room temperature. The reaction solution was concentrated under reduced pressure, and the resulting residue was purified by sublimation (85-90°C/20mmHg). 0.44 g (3.4 mmol) of the obtained bischloromethylmethylamine was weighed out in a stream of nitrogen, and dissolved in 20 ml of dichloromethane. The solution was ice-cooled, and 0.50 g (1.2 mmol) of 1-(2,6-difluorobenzoyl)-3-[2-fluoro-4-(trifluoromethylsulfinyl)phenyl)urea was added thereto at 1 °C. After stirring for 15 mins., a solution of triethylamine (0.95 ml, 6.9 mmol) in dichloromethane (3 ml) was added dropwise at 1-3 °C, and stirred at 1 °C for 1 hr. The reaction solution was added to an aqueous sodium hydroxide solution (prepared from 1.20 g (30.0 mmol) of sodium hydroxide and 30 ml of water) at 2-3°C under ice-cooling. The reaction solution was extracted with 20 ml of dichloromethane, dried over anhydrous magnesium sulfate, and concentrated under reduced pressure. The resulting residue was purified with silica gel column chromatography. (ethyl acetate : hexane = 1 : 1) to give 0.53 g (1.1 mmol) of 1-(2,6-difluorobenzoyl)-3-[2-fluoro-4-(trifluoromethylsulfinyl)phenyl]-5-methyltetrahydro-1,3,5-triazin-2(*1H*)-one as an amorphou (yield 92%).
$^1$HNMR ($CDCl_3$)δppm: 2.84 (3H, s, NMe), 4.60 (2H, s,. $CH_2$), 5.06 (2H, s, $CH_2$), 6.87-6.91 (2H, m, Ph), 7.26-7.32 (1H, m, Ph), 7.53-7.54 (2H, m, Ph), 7.60-7.63 (1H, m, Ph). IR (nujol)νcm$^{-1}$: 1682, 1625, 1592, 1502, 1469, 1426, 1336, 1300, 1185, 1139, 1083, 1004, 775.

Synthetic Example 22

Synthesis of 1-(2,6-difluorobenzoyl)-3-[4-(difluoromethylsulfinyl)-2-fluorophenyl]-5-methyltetrahydro-1,3,5-triazin-2 (*1H*)-one (compound No. III-152)

9.67 g (46.2 mmol) of phosphorus pentachloride (96%) was added to 50 ml of dichloromethane, and a solution obtained by dissolving 1,3,5-trimethylhexahydro-1,3,5-triazine (3.00 g, 23.2 mmol) in dichloromethane (10 ml) under heating at 40 °C was added dropwise to the above mixture. The reaction mixture was heated at 40 °C for 5 hrs, and then cooled under room temperature. The reaction solution was concentrated under reduced pressure, and the resulting residue was purified by sublimation (85-90°C/20mmHg). 0.46 g (3.6 mmol, 2.8. eq) of the obtained bischloromethylmethylamine was weighed out in a stream of nitrogen, and dissolved in 20 ml of dichloromethane. The solution was ice-cooled, and 0.50 g (1.3 mmol) of 1-(2,6-difluorobenzoyl)-3-[4-(difluoromethylsulfinyl)-2-fluorophenyl]urea was added thereto at 1 °C. After stirring for 15 mins., a solution of triethylamine (1.0 ml, 7.2 mmol) in dichloromethane (3 ml) was added dropwise at 1-3 °C, and stirred at 1 °C for 1 hr. The reaction solution was added to an aqueous sodium

hydroxide solution (prepared from 1.20 g (30.0 mmol) of sodium hydroxide and 30 ml of water) at 2-3°C under ice-cooling. The reaction solution was extracted with 20 ml of dichloromethane, dried over anhydrous magnesium sulfate, and concentrated under reduced pressure. The resulting residue was purified with silica gel column chromatography (ethyl acetate : hexane = 1 : 1) to give 0.45 g (1.0 mmol) of 1-(2,6-difluorobenzoyl)-3-[4-(difluoromethylsulfinyl)-2-fluorophenyl]-5-methyltetrahydro-1,3,5-triazin-2(*1H*)-one as a white solid (m.p. 50-55°C, yield 77%).
$^1$HNMR ($CDCl_3$)δppm: 2.83 (3H, s, NMe), 4.59 (2H, s, $CH_2$), 5.06 (2H, s, $CH_2$), 6.03 (1H, t J=55.1Hz, $CF_2H$), 6.87-6.91 (2H, m, Ph), 7.26-7.31 (1H, m, Ph), 7.47-7.56 (3H, m, Ph).
IR (nujol)νcm$^{-1}$: 1682, 1625, 1592, 1002, 889.

Synthetic Example 23

Synthesis of 1-(2,6-difluorobenzoyl)-3-[4-(difluoromethylsulfonyl)-2-fluorophenyl]-5-methyltetrahydro-1,3,5-triazin-2(*1H*)-one (compound No. III-156)

1.00 g (4.61 mmol) of phosphorus pentachloride (96%) was added to 15 ml of dichloromethane, and a solution obtained by dissolving 1,3,5-trimethylhexahydro-1,3,5-triazine (0.31 g, 2.4 mmol) in dichloromethane (5 ml) under heating at 40 °C was added dropwise to the above mixture. The reaction mixture was heated at 40 °C for 2 hrs. The reaction solution was ice-cooled, and 0.50 g (1.2 mmol) of 1-(2,6-difluorobenzoyl)-3-[4-(difluoromethylsulfonyl)-2-fluorophenyl]urea was added at 1 °C. After stirring for 15 mins., a solution of triethylamine (0.65 ml, 4.7 mmol) in dichloromethane (3 ml) was added dropwise at 1-3 °C, and stirred at 1 °C for 1 hr. The reaction solution was added to an aqueous sodium hydroxide solution (prepared from 1.00 g (25.0 mmol) of sodium hydroxide and 30 ml of water) at 2-15°C under ice-cooling. The insolbles were dissolved by adding 30 ml of dichloromethane, and the layers were separated. The water layer was extracted with 30 ml of dichloromethane. The organic layer was dried over anhydrous magnesium sulfate, and concentrated under reduced pressure. The resulting residue was purified.with silica gel column chromatography (acetonitrile : chloroform = 1 : 9), and washed out with diethyl ether to give 0.41 g (0.88 mmol) of 1-(2,6-difluorobenzoyl)-3-[4-(difluoromethylsulfonyl)-2-fluorophenyl]-5-methyltetrahydro-1,3,5-triazin-2(*1H*)-one as a white solid (m.p. 203-205°C, yield 73%).
$^1$HNMR ($CDCl_3$) δppm: 2.82 (3H, s, NMe), 4.62 (2H, s, $CH_2$), 5.06 (2H, s, $CH_2$), 6.18 (1H, t J=53.1Hz, $CF_2H$), 6.88-6.92 (2H, m, Ph), 7.27-7.33 (1H, m, Ph), 7.56-7.60 (1H, m, Ph), 7.74-7.79 (2H, m, Ph).
IR (nujol)νcm$^{-1}$: 1687, 1623, 1466; 1416, 1385, 1342, 1301, 1272, 1234, 1208, 1159, 1106, 1075, 999, 793.

Synthetic Example 24

Synthesis of 3-(2,6-difluorobenzoyl)-5-[4-(difluoromethylthio)-2-fluorophenyl]tetrahydro-*4H*-1,3,5-oxadiazin-4-one (compound No. I-41)

0.23 g (5.8 mmol) of sodium hydride (60%) was suspended in 20 ml of DMF. With ice-cooling, 1.00 g (2.66 mmol) of 1-(2,6-difluorobenzoyl)-3-[4-(difluoromethylthio)-2-fluorophenyl]urea was added thereto at 3°C. After stirring for 40 mins., a solution of bischloromethyl ether (0.36 g, 3.1 mmol) in DMF (3 ml) was added dropwise at 3-5°C. The reaction mixture was stirred at room temperature for 2.5 hrs. The reaction solution was cooled under ice-cooling, and a mixed solution of sarurated aqueous ammonium chloride solution (20 ml) and water (10 ml) was added thereto at 2-20°C. The reaction solution was extracted 3 times with 20 ml of ethyl acetate. The organic layer was washed 3 times with 20 ml of saturated brine. After drying over anhydrous magnesium sulfate, the organic layer was concentrated under reduced pressure. The obtained residue was purified with silica gel column chromatography (ethyl acetate : chloroform : hexane = 1 : 1 : 4), and recrystallized from diethyl ether-hexane to give 0.20 g (0.48 mmol) of 3-(2,6-difluorobenzoyl)-5-[4-(difluoromethylthio)-2-fluorophenyl]tetrahydro-*4H*-1,3,5-oxadiazin-4-one as a white solid (m.p. 77-79°C, yield 18%).
$^1$HNMR ($CDCl_3$)δppm: 5.14 (2H, s, $CH_2$), 5.61 (2H, s, $CH_2$), 6.82 (1H, t J=56.3Hz, $CF_2H$), 6.87-6.96 (2H, m, Ph), 7.25-7.40 (4H, m, Ph).
IR (nujol)νcm$^{-1}$: 1683, 1622, 1494, 1469, 1404, 1351, 1331, 1301, 1254, 1233, 1074, 1046, 1009, 789.

Synthetic Example 25

Synthesis of 3-(2,6-difluorobenzoyl)-5-[2-fluoro-4-(trifluoromethylthio)phenyl]tetrahydro-*4H*-1,3,5-oxadiazin-4-one (compound No. I-44)

0.21 g (5.8 mmol) of sodium hydride (60%) was suspended in 20 ml of DMF. Under ice-cooling, 1.00 g (2.54 mmol) of 1-(2,6-difluorobenzoyl)-3-[2-fluoro-4-(trifluoromethylthio)phenyl]urea was added thereto at 3°C. After stirring

for 40 mins., a solution of bischloromethyl ether (0.35 g, 3.0 mmol) in DMF (3 ml) was added dropwise at 3-5°C. The reaction mixture was stirred at room temperature for 2 hrs. The reaction solution was cooled under ice-cooling, and a mixed solution of sarurated aqueous ammonium chloride solution (20 ml) and water (10 ml) was added thereto at 2-20°C. The reaction solution was extracted 3 times with 20 ml of ethyl acetate. The organic layer was washed 3 times with 20 ml of saturated brine. After drying over anhydrous magnesium sulfate, the organic layer was concentrated under reduced pressure. The obtained residue was purified with silica gel column chromatography (ethyl acetate : chloroform : hexane = 1 : 1 : 4), and then purified with HPLC for fractionation (column: SI-40B Φ 26x300mm, mobile phase: ethyl acetate : hexane = 17 : 83) to give 0.28 g (0.64 mmol) of 3-(2,6-difluorobenzoyl)-5-[2-fluoro-4-(trifluoromethylthio)]tetrahydro-*4H*-1,3,5-oxadiazin-4-one as a white solid (m.p. 57-59°C, yield 25%).
$^1$HNMR ($CDCl_3$) δppm: 5.15 (2H, s, $CH_2$), 5.60 (2H, s, $CH_2$), 6.88-6.92 (2H, m, Ph), 7.26-7.48 (4H, m, Ph).
IR (nujol)νcm$^{-1}$: 1682, 1626, 1593, 1503, 1470, 1445, 1406, 1318, 1238, 1109, 1006, 887, 788.

Synthetic Example 26

Synthesis of 3-(2,6-difluorobenzoyl)-5-[4-(difluoromethylsulfinyl)-2-fluorophenyl]tetrahydro-*4H*-1,3,5-oxadiazin-4-one (compound No. I-42)

0.20 mg (0.48 mmol) of 3-(2,6-difluorobenzoyl)-5-[4-(difluoromethylthio)-2-fluorophenyl]tetrahydro-*4H*-1,3,5-oxadiazin-4-one was dissolved in 10 ml of chloroform. 124 mg (0.50 mmol) of m-CPBA (70%) was added thereto at 2°C under ice-cooling. The temperature of reaction solution was raised to 18°C over 7.5 hrs. To the reaction solution was added. 20 ml of chloroform and the chloroform layer was washed with 30 ml of saturated aqueous sodium bicarbonate solution. After drying over anhydrous magnesium sulfate, the chloroform layer was concentrated under reduced pressure. The obtained residue was purified with HPLC for fractionation (column: SI-40B Φ 26x300mm, mobile phase: ethyl acetate : hexane = 50 : 50) to give 0.13 g (0.30 mmol) of 3-(2,6-difluorobenzoyl)-5-[4-(difluoromethylsulfinyl)-2-fluorophenyl]tetrahydro-*4H*-1,3,5-oxadiazin-4-one as a white solid (m.p. 138-140°C, yield 63%).
$^1$HNMR ($CDCl_3$)δppm: 5.19 (2H, s, $CH_2$), 5.62 (2H, s, $CH_2$), 6.04 (1H, t J=55.1Hz, $CF_2H$), 6.89-6.93 (2H, m, Ph), 7.31-7.36 (1H, m, Ph), 7.48-7.51 (1H, m, Ph), 7.58-7.63 (2H, m, Ph).
IR (nujol)νcm$^{-1}$: 1706, 1691, 1626, 1498, 1469, 1453, 1403, 1351, 1327, 1303, 1272, 1252, 1229, 1114, 1080, 1003, 795.

Synthetic Example 27

Synthesis of 3-(2,6-difluorobenzoyl)-5-[2-fluoro-4-(trifluoromethylsulfinyl)phenyl]tetrahydro-*4H*-1,3,5-oxadiazin-4-one (compound No. I-45)

0.27 g (0.62 mmol ) of 3-(2,6-Difluorobenzoyl)-5-[2-fluoro-4-(trifluoromethylthio)phenyl]tetrahydro-*4H*-1,3,5-oxadiazin-4-one was dissolved in 16 ml of chloroform. 0.16 g (0.65 mmol) of m-CPBA (70%) was added thereto, and stirred for 24 hrs at room temperature. The reaction solution was washed with 16 ml of saturated aqueous sodium bicarbonate solution. After drying over anhydrous magnesium sulfate, the chloroform layer was concentrated under reduced pressure. The obtained residue was purified with silica gel column chromatography (ethyl acetate : hexane = 1 : 2) to give 0.13 g (0.29 mmol) of 3-(2,6-difluorobenzoyl)-5-[2-fluoro-4-(trifluoromethylsulfinyl)phenyl]tetrahydro-*4H*-1,3,5-oxadiazin-4-one as a colorless amorphous (yield 47%).
$^1$HNMR ($CDCl_3$)δppm: 5.19 (2H, s, $CH_2$), 5.62 (2H,s, $CH_2$), 6.89-6.93 (2H, m,. Ph), 7.32-7.36 (1H, m, Ph), 7.54-7.66 (3H, m, Ph).
IR (neat)νcm$^{-1}$: 1682, 1626, 1593, 1504, 1470, 1435, 1412, 1317, 1192, 1138, 1084, 1007, 885, 788.

Synthetic Example 28

Synthesis of 3-(2,6-difluorobenzoyl)-5-[4-(difluoromethylsulfonyl)-2-fluorophenyl]tetrahydro-*4H*-1,3,5-oxadiazin-4-one (compound No. I-43)

0.20 g (0.48 mmol) of 3-(2,6-difluorobenzoyl)-5-[4-(difluoromethylthio)-2-fluorophenyl]tetrahydro-*4H*-1,3,5-oxadiazin-4-one was dissolved in 10 ml of chloroform. 0.48 g (1.9 mmol) of m-CPBA (70%) was added thereto at room temperature, and stirred for 18 hrs. To the reaction solution was added 20 ml of chloroform, and washed with 30 ml of saturated aqueous sodium bicarbonate solution. After drying over anhydrous magnesium sulfate, the chloroform layer was concentrated under reduced pressure. The obtained residue was purified with silica gel column chromatography (ethyl acetate : hexane = 1 : 2) to give 0.11 g (0.24 mmol) of 3-(2,6-difluorobenzoyl)-5-[4-(difluoromethylsulfonyl)-2-fluorophenyl]tetrahydro-*4H*-1,3,5-oxadiazin-4-one as a white solid (m.p. 210-213°C, yield 50%).

$^1$HNMR ($CDCl_3$)δppm: 5.21 (2H, s, $CH_2$), 5.61 (2H, s, $CH_2$), 6.18 (1H, t J=53.1Hz, $CF_2H$), 6.90-6.95 (2H, m, Ph), 7.31-7.39 (1H, m, Ph), 7.65-7.67 (1H, m, Ph), 7.69-7.79 (2H, m, Ph).
IR (nujol)ν$cm^{-1}$: 1693, 1626, 1594, 1503, 1467, 1415, 1401, 1376, 1349, 1327, 1293, 1234, 1159, 1109, 1080, 1003, 785.

Synthetic Example 29

Synthesis of 1-(2,6-difluorobenzoyl)-3-[2-fluoro-4-(trifluoromethylthio)phenyl]-5-methylsulfonyltetrahydro-1,3,5-triazin-2(*1H*)-one (compound No. III-162)

0.21 g (5.3 mmol) of sodium hydride (60%) was suspended in 30 ml of DMF. Under ice-cooling, 1.00 g (2.54 mmol) of 1-(2,6-difluorobenzoyl)-3-[2-fluoro-4-(trifluoromethylthio)phenyl]urea was added thereto at 3°C. After stirring for 30 mins., a solution of N,N-bis(chloromethyl)methanesulfonamide (0.59 g, 3.1 mmol) in DMF (3 ml) was added dropwise at 3-5°C. The reaction mixture was stirred at 3°C for 1 hr. To the reaction solution was added a mixed solution of sarurated aqueous ammonium chloride solution (30 ml) and water (15 ml) at 4-26°C. The reaction solution was extracted 3 times with 30 ml of ethyl acetate, and the organic layer was washed 3 times with 30 ml of saturated brine. After drying over anhydrous magnesium sulfate, the organic layer was concentrated under reduced pressure. The obtained residue was purified with silica gel column chromatography (ethyl acetate : hexane = 1 : 2), and the resulting residue was purified with HPLC for fractionation (column: SI-40B Φ 26x300mm, mobile phase: ethyl acetate : hexane = 33 : 67) to give 0.18 g (0.34 mmol) of 1-(2,6-difluorobenzoyl)-3-[2-fluoro-4-(trifluoromethylthio)phenyl]-5-methylsulfonyltetrahydro-1,3,5-triazin-2(*1H*)-one as an colorless oil (yield 14%).
$^1$HNMR ($CDCl_3$)δppm: 3.21 (3H, s, $SO_2$ $CH_3$), 5.16 (2H, s, $NCH_2$), 5.62 (2H, s, $NCH_2$), 6.88-6.92 (2H, m, Ph), 7.33-7.50 (4H, m, Ph).
IR (neat)ν$cm^{-1}$: 1694, 1626, 1503, 1470, 1435, 1352, 1306, 1239, 1163, 1112, 1006, 757.

Synthetic Example 30

Synthesis of 1-(2,6-difluorobenzoyl)-3-[2-fluoro-4-(trifluoromethylthio)phenyl]-5-aceyltetrahydro-1,3,5-triazin-2(*1H*)-one (compound No. III-161)

0.11 g (2.8 mmol) of sodium hydride (60%) was suspended in 15 ml of DMF. Under ice-cooling, 0.50 g (1.3 mmol) of 1-(2,6-difluorobenzoyl)-3-[2-fluoro-4-(trifluoromethylthio)phenyl]urea was added thereto at 3°C. After stirring for 30 mins., a solution of N,N-bis(chloromethyl)acetamide (0.24 g, 1.5 mmol) in DMF (2 ml) was added dropwise at 3-5°C. The reaction mixture was stirred at 3°C for 1 hr. To the reaction solution was added a mixed solution of saturated aqueous ammonium chloride solution (20 ml) and water (10 ml) at 4-26°C. The reaction solution was extracted 3 times with 20 ml of ethyl acetate,. and the organic layer was washed 3 times with 20 ml of saturated brine. After drying over anhydrous magnesium sulfate, the organic layer was concentrated under reduced pressure. The obtained residue was purified with silica gel column chromatography (ethyl acetate : hexane = 1 : 1) to. give 0.31 g (0.65 mmol) of 1-(2,6-difluorobenzoyl)-3-[2-fluoro-4-(trifluoromethylthio)phenyl]-5-aceyltetrahydro-1,3,5-triazin-2(*1H*)-one as an amorphous (yield 50%).
$^1$HNMR ($CDCl_3$)δppm: 2.34 (3H, s, $COCH_3$), 5.23 (2H, s, $NCH_2$), 5.63 (2H, s, $NCH_2$), 6.88-6.92 (2H, m, Ph), 7.26-7.47 (4H, m, Ph).
IR (nujol)ν$cm^{-1}$: 1693, 1626, 1592, 1503, 1467, 1412, 1377, 1352, 1306, 1186, 1138, 1082, 1006, 966.

Synthetic Example 31

Synthesis of 5-benzyloxycarbonyl-1-(3,5-dichloro-2,4-difluorophenyl)-3-(2,6-difluorobenzoyl)tetrahydro-1,3,5-triazin-2(*1H*)-one (compound No. III-55)

0.57 g (14 mmol) of sodium hydride (60%) was suspended in 25 ml of DMF. Under ice-cooling, 2.00 g (5.25 mmol) of 1-(3,5-dichloro-2,4-difluorophenyl)-3-(2,6-difluorobenzoyl)urea was added thereto at 3°C. After stirring for 30 mins., a solution of benzyl N,N-bis(chloromethyl)carbamate (1.56 g, 6.29 mmol) in THF (5 ml) was added dropwise at 1-2°C. The reaction mixture was stirred for 40 mins. at room temperature. To the reaction solution was added 4 ml of water, then 25 ml of water, and extracted with 60 ml of ethyl acetate. The organic layer was washed 3 times with 25 ml of water, and 3 times with 25 ml of saturated brine. After drying over anhydrous magnesium sulfate, the organic layer was concentrated under reduced pressure. The obtained residue was purified with silica gel column chromatography (chloroform : acetone = 20 : 1) to give 0.92 g (1.7 mmol) of 5-benzyloxycarbonyl-1-(3,5-dichloro-2,4-difluorophenyl)-3-(2,6-difluorobenzoyl)tetrahydro-1,3,5-triazin-2(*1H*)-one as an amorphous (yield 32%).

$^1$HNMR ($CDCl_3$)δppm: 5.11 (2H, s, $NCH_2N$), 5.28 (2H, s, $COOCH_2$), 5.59 (2H, s, $NCH_2N$), 6.85-6.93 (2H, m, Ph), 7.26-7.37 (7H, m, Ph) .
IR (neat)ν$cm^{-1}$: 1708, 1625, 1292, 1243, 1217, 1156, 1046, 1005, 820, 791, 701.

Synthetic Example 32

Synthesis of 1-(3,5-dichloro-2,4-difluorophenyl)-3-(2,6-difluorobenzoyl)-3,4-dihydro-1,3,5-triazin-2(*1H*)-one (compound No. V-4) and 3-(3,5-dichloro-2,4-difluorophenyl)-1-(2,6-difluorobenzoyl)-3,4-dihydro-1,3,5-triazin-2(*1H*)-one (compound No. IV-4)

0.06 g of 10% palladium carbon and 1.50 g (2.70 mmol) of 5-benzyloxycarbonyl-1-(3,5-dichloro-2,4-difluorophenyl)-3-(2,6-difluorobenzoyl)tetrahydro-1,3,5-triazin-2(*1H*)-one was added to 45 ml of ethanol, and stirred for 70 mins. at room temperature under hydrogen atmosphere. The reaction solution was filtered, and the filtrate was concentrated under reduced pressure. To the resulting residue was added 60 ml of dichloromethane, and ice-cooled. A solution of 0.59 g (5.4 mmol) of tert-butyl hypochlorite in 15 ml of dichloromethane was added thereto. The reaction solution was stirred for 30 mins. at room temperature, and 0.38 ml of triethylamine (2.7 mmol) was added thereto, followed by stirring for 20 hrs at room temperature. To the reaction solution was added 60 ml of water, and the layers were separated. The organic layer was dried over anhydrous magnesium sulfate, and concentrated under reduced pressure. The obtained residue was purified with silica gel column chromatography (chloroform : ethyl acetate = 10 : 1, hexane : ethyl acetate = 2 : 1, chloroform : acetone = 20 : 1) to give 0.15 g (0.36 mmol) of 1-(3,5-dichloro-2,4-difluorophenyl)-3-(2,6-difluorobenzoyl)-3,4-dihydro-1,3,5-triazin-2(*1H*)-one (compound No. V-4) as an oil (yield 13%). In addition, 0.12 g (0.29 mmol) of 3-(3,5-dichloro-2,4-difluorophenyl)-1-(2,6-difluorobenzoyl)-3,4-dihydro-1,3,5-triazin-2(*1H*)-one (compound No. IV-4) was obtained as an amorphous (yield 11%). No. V-4
$^1$HNMR ($CDCl_3$)δppm: 5.60 (2H, d J =2.0Hz, $NCH_2N$), 6.88-6.97 (2H, m, Ph), 7.24-7.42 (3H, m, Ar+NCHN).
IR (neat)ν$cm^{-1}$: 1732, 1694, 1626, 1486, 1470, 1440, 1414, 1313, 1238, 1008. No. IV-4
$^1$HNMR ($CDCl_3$)δppm: 5.20 (2H, d J=2.1Hz, $NCH_2N$), 6.89-7.00 (2H, m, Ph), 7.26-7.48 (2H, m, Ph), 8.38 (1H, t J=2.1Hz, NCHN).
IR (neat)ν$cm^{-1}$: 1727, 1625, 1439, 1290, 1269, 1224, 1008.

Synthetic Example 33

Synthesis of 3-(2,6-difluorobenzoyl)-5-[2-fluoro-4-(1,1,2,2,2-pentafluoroethylthio)phenyl]tetrahydro-*4H*-1,3,5-oxadiazin-4-one (compound No. I-128)

1.50 g (3.38 mmol) of 1-(2,6-difluorobenzoyl)-3-[2-fluoro-4-(1,1,2,2,2-pentafluoroethylthio)phenyl]urea was dissolved in 30 ml of 1-methyl-pyrrolidone. Under ice-cooling, 0.16 g (4 mmol) of sodium hydride (60%) was added thereto at 3°C. After stirring for 30 mins., a solution of bischloromethyl ether (0.54 g, 4.7 mmol) in 1-methyl-pyrrolidone (1 ml) was added dropwise at 4°C. 0.16 g (4 mmol) of sodium hydride (60%) was added thereto at 4°C. Under ice-cooling the reaction mixture was stirred for 5 hrs at 5°C, and for 1 hr at room temperature. To the reaction solution was added a mixed solution of sarurated aqueous ammonium chloride solution (30 ml) and water (15 ml) at 3-25°C under ice-cooling. The reaction solution was extracted 3 times with 30 ml of ethyl acetate. The organic layer was washed 3 times with 30 ml of saturated brine. After drying over anhydrous magnesium sulfate, the organic layer was concentrated under reduced pressure. The obtained residue was purified with silica gel column chromatography (ethyl acetate : chloroform : hexane = 1 : 1 : 4), and the resulting residue was purified with HPLC for fractionation (column: SI-40B Φ 26x300mm, mobile phase: ethyl acetate : hexane = 17 : 83) to give 0.58 g (1.2 mmol) of 3-(2,6-difluorobenzoyl)-5-[2-fluoro-4-(1,1,2,2,2-pentafluoroethylthio)phenyl]tetrahydro-*4H*-1,3,5-oxadiazin-4-one as a white solid (m.p. 47-50°C, yield 36%).
$^1$HNMR ($CDCl_3$)δppm : 5.15 ( 2H, s, $CH_2$), 5.60 ( 2H, s, $CH_2$), 6.88-6.92 (2H, m, Ph), 7.30-7.48 ( 4H, m, Ph).
IR (nujol)ν$cm^{-1}$ : 1699, 1682, 1624, 1592, 1503, 1470, 1402, 1348, 1328, 1296, 1250, 1211, 1104, 1002, 966, 791.

Synthetic Example 34

Synthesis of 3-(2,6-difluorobenzoyl)-5-[2-fluoro-4-(1,1,2,2,2-pentafluoroethylsulfinyl)phenyl]tetrahydro-*4H*-1,3,5-oxadiazin-4-one (compound No. I-129)

0.27 g (0.56 mmol) of 3-(2,6-difluorobenzoyl)-5-[2-fluoro-4-(1,1,2,2,2-pentafluoroethylthio)phenyl]tetrahydro-*4H*-1,3,5-oxadiazin-4-one was dissolved in 5.0 ml of chloroform. 0.27 g (1.1 mmol) of m-CPBA (70%) was added thereto, and stirred for 24 hrs at room temperature. The reaction solution was washed with 10 ml of saturated aqueous

sodium bicarbonate solution. After drying over anhydrous magnesium sulfate, the chloroform layer was concentrated under reduced pressure. The obtained residue was purified with HPLC for fractionation (column: SI-40B Φ 26x300mm, mobile phase: ethyl acetate : hexane = 30 : 70) to give 0.18 g (0.36 mmol) of 3-(2,6-difluorobenzoyl)-5-[2-fluoro-4-(1,1,2,2,2-pentafluoroethylsulfinyl)phenyl]tetrahydro-*4H*-1,3,5-oxadiazin-4-one as an amorphous (yield 64%).
$^1$HNMR ($CDCl_3$)δppm : 5.20 (2H, s, $CH_2$), 5.61 ( 2H, s, $CH_2$), 6.89-6.93 ( 2H, m, Ph), 7.32-7.36 ( 1H, m, Ph), 7.56-7.66 ( 3H, m, Ph).
IR (neat)ν$cm^{-1}$ : 1713, 1693, 1626, 1593, 1503, 1470, 1434, 1413, 1317, 1222, 1125, 1099, 1007, 944, 788.

Synthetic Example 35

Synthesis of 3-(2,6-difluorobenzoyl)-5-[2-fluoro-4-(1,1,2,2,3,3,3-heptafluoro-1-propylthio)phenyl]tetrahydro-*4H*-1,3,5-oxadiazin-4-one (compound No. I-143)

1.30 g (2.63 mmol) of 1-(2,6-difluorobenzoyl)-3-[2-fluoro-4-(1,1,2,2,3,3,3-heptafluoro-1-propylthio)phenyl] urea was dissolved in 25 ml of 1-methylpyrrolidone. Under ice-cooling, 0.13 g (3.2 mmol) of sodium hydride (60%) was added thereto at 3°C. After stirring for 30 mins., a solution of bischloromethyl ether (0.42 g, 3.7 mmol) in 1-methylpyrrolidone (1 ml) was added dropwise at 4°C. 0.13 g (3.2 mmol) of sodium hydride (60%) was added thereto at 4°C. Under ice-cooling the reaction mixture was stirred for 5 hrs at 5°C, and for 1 hr at room temperature. To the reaction solution was added a mixed solution of sarurated aqueous ammonium chloride solution (25 ml) and water (12 ml) at 3-25°C under ice-cooling. The reaction solution was extracted 3 times with 25 ml of ethyl acetate. The organic layer was washed 3 times with 25 ml of saturated brine. After drying over anhydrous magnesium sulfate, the organic layer was concentrated under reduced pressure. The obtained residue was purified with silica gel column chromatography (ethyl acetate : chloroform : hexane = 1 : 1 : 4), and then purified with HPLC for fractionation (column: SI-40B Φ 26x300mm, mobile phase: ethyl acetate : hexane = 17 : 83) to give 0.29 g (0.54 mmol) of 3-(2,6-difluorobenzoyl)-5-[2-fluoro-4-(1,1,2,2,3,3,3-heptafluoro-1-propylthio)phenyl]tetrahydro-*4H*-1,3,5-oxadiazin-4-one as a white solid (m. p. 44-46°C, yield 21%).
1HNMR (CDCl3) δppm : 5.15 ( 2H, s, CH2), 5.60 ( 2H, s, CH2), 6.88-6.92 (2H, m, Ph), 7.26-7.48 ( 4H, m, Ph).
IR (neat)ν$cm^{-1}$ : 1687, 1625, 1503, 1471, 1405, 1323, 1215, 1115, 1006, 852 .

Synthetic Example 36

Synthesis of 3-(2,6-difluorobenzoyl)-5-[2-fluoro-4-(1,1,2,2,3,3,3-heptafluoro-1-propylsulfinyl)phenyl]tetrahydro-*4H*-1,3,5-oxadiazin-4-one (compound No. I-144)

0.13 g (0.24 mmol) of 3-(2,6-difluorobenzoyl)-5-[2-fluoro-4-(1,1,2,2,3,3,3-heptafluoro-1-propylthio)phenyl]tetrahydro-*4H*-1,3,5-oxadiazin-4-one was dissolved in 5.0 ml of chloroform. 0.12 g (0.48 mmol) of m-CPBA (70%) was added thereto, and stirred for 2 days at room temperature. The reaction solution was washed with 10 ml of saturated aqueous sodium bicarbonate solution. After drying over anhydrous magnesium sulfate, the chloroform layer was concentrated under reduced pressure. The obtained residue was purified with HPLC for fractionation (column: SI-40B Φ 26x300mm, mobile phase: ethyl acetate : hexane = 30 : 70) to give 0.11 g (0.20 mmol) of 3-(2,6-difluorobenzoyl)-5-[2-fluoro-4-(1,1,2,2,3,3,3-heptafluoro-1-propylsulfinyl)phenyl]tetrahydro-*4H*-1,3,5-oxadiazine-4-one as an amorphous (yield 83%).
$^1$HNMR ($CDCl_3$)δppm : 5.20 (2H, s, $CH_2$), 5.61 ( 2H, s, $CH_2$), 6.89-6.93 ( 2H, m, Ph), 7.32-7.36 ( 1H, m, Ph), 7.54-7.66 ( 3H, m, Ph).
IR (neat)ν$cm^{-1}$ : 1715, 1693, 1626, 1594, 1503, 1470, 1434, 1412, 1318, 1235, 1131, 1105, 1081, 1007, 789.

Synthetic Example 37

Synthesis of 3-(2,6-difluorobenzoyl)-5-[2-fluoro-4-(1,1,1,2,3,3,3-heptafluoro-2-propylthio)phenyl]tetrahydro-*4H*-1,3,5-oxadiazin-4-one (compound No. I-158)

1.94 g (3.92 mmol) of 1-(2,6-difluorobenzoyl)-3-[2-fluoro-4-(1,1,1,2,3,3,3-heptafluoro-2-propylthio)phenyl] urea was dissolved in 40 ml of 1-methyl-pyrrolidone. Under ice-cooling, 0.19 g (4.6 mmol) of sodium hydroxide (96%) was added thereto at 3°C. After stirring for 30 mins., a solution of bischloromethyl ether (0.62 g, 5.4 mmol) in 1-methylpyrrolidone (1 ml) was added dropwise at 4°C. 0.19 g (4.6 mmol) of sodium hydroxide (96%) was added thereto at 4°C. Under ice-cooling the reaction mixture was stirred for 5 hrs at 5°C, and for 1 hr at room temperature. To the reaction solution was added a mixed solution of sarurated aqueous ammonium chloride solution (40 ml) and water (20 ml) at 3-25°C under ice-cooling. The reaction solution was extracted 3 times with 40 ml. of ethyl acetate. The organic

layer was washed 3 times with 40 ml of saturated brine. After drying over anhydrous magnesium sulfate, the organic layer was concentrated under reduced pressure.. The obtained residue was purified with silica gel column chromatography (ethyl acetate : chloroform : hexane = 1 : 1 : 4), and then purified with HPLC for fractionation (column: SI-40B Φ 26x300mm, mobile phase: ethyl acetate : hexane = 17 : 83) to. give 1.10 g (2.05 mmol) of 3-(2,6-difluorobenzoyl)-5-[2-fluoro-4-(1,1,1,2,3,3,3-heptafluoro-2-propylthio)phenyl]tetrahydro-*4H*-1,3,5-oxadiazin-4-one as a white solid (m. p. 85-86°C, yield 52%).
$^1$HNMR ($CDCl_3$)δppm : 5.15 ( 2H, s, $CH_2$), 5.60 ( 2H, s, $CH_2$), 6.88-6.92 (2H, m, Ph), 7.33-7.48 ( 4H, m, Ph).
IR (nujol)ν$cm^{-1}$ : 1711, 1680, 1625, 1504, 1470, 1435, 1410, 1320, 1276, 1224, 1169, 1075, 1002, 788.

Synthetic Example 38

Synthesis of 3-(2,6-difluorobenzoyl)-5-[2-fluoro-4-(1,1,1,2,3,3,3-heptafluoro-2-propylsulfinyl)phenyl]tetrahydro-*4H*-1,3,5-oxadiazin-4-one (compound No. I-159)

0.76 g (1.4 mmol) of 3-(2,6-difluorobenzoyl)-5-[2-fluoro-4-(1,1,1,2,3,3,3-heptafluoro-2-propylthio)phenyl]tetrahydro-*4H*-1,3,5-oxadiazin-4-one was dissolved in 10 ml of chloroform. 0.69 g (2.8 mmol) of m-CPBA (70%) was added thereto, and stirred for 2 days at room temperature. The reaction solution was washed with 20 ml of saturated aqueous sodium bicarbonate solution. After drying over anhydrous magnesium sulfate, the chloroform layer was concentrated under reduced pressure. The obtained residue was purified with HPLC for fractionation (column: SI-40B Φ 26x300mm, mobile phase: ethyl acetate : hexane = 30 : 70) to give 0.37 g (0.67 mmol) of 3-(2,6-difluorobenzoyl)-5-[2-fluoro-4-(1,1,1,2,3,3,3-heptafluoro-2-propylsulfinyl)phenyl]tetrahydro-*4H*-1,3,5-oxadiazin-4-one as a white solid (m.p. 40-45°C, yield 48%).
$^1$HNMR ($CDCl_3$)δppm : 5.20 (2H, s, $CH_2$), 5.61 ( 2H, s, $CH_2$), 6.89-6.94 ( 2H, m, Ph), 7.32-7.37 ( 1H, m, Ph), 7.53-7.55 ( 1H, m, Ph), 7.63-7.65 ( 2H, m, Ph).
IR (neat)ν$cm^{-1}$ : 1682, 1626, 1594, 1503, 1470, 1413, 1276, 1220, 1130, 1007, 970.

The compounds of the above Synthetic Examples and other compounds obtained by.the similar method are shown in the following Tables 1 to 5.

Table 1

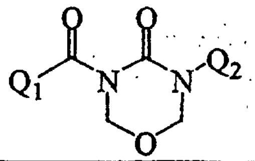

(Ia)

| compound No. | $Q_1$ | $Q_2$ | m. p. (°C) |
|---|---|---|---|
| I - 1 | 2,6-$F_2$-Ph | 4-Cl-Ph | 125-126 |
| I - 2 | 2,6-$F_2$-Ph | 3,5-$Cl_2$-4-(2-Cl-4-$CF_3$-pyridin-2yl-O)-Ph | 184-185 |
| I - 3 | 2,6-$F_2$-Ph | 2-F-4-(2-Cl-4-$CF_3$-Ph-O)-Ph | oil (1) |
| I - 4 | 2,6-$F_2$-Ph | 3,5-$Cl_2$-2,4-$F_2$-Ph | 78-82 |
| I - 5 | 2,6-$F_2$-Ph | 3,5-$Cl_2$-4-$CF_2HCF_2O$-Ph | 128-130 |
| I - 6 | 2,6-$F_2$-Ph | 2,5-$Cl_2$-4-$CF_3CFHCF_2O$-Ph | 162.0-162.5 |
| I - 7 | 2,6-$F_2$-Ph | 2-F-4-$CFClHCF_2S$-Ph | 130-132 |
| I - 8 | 2,6-$F_2$-Ph | 2-F-4-$CFClHCF_2SO$-Ph | white amorphous (2) |
| I - 9 | 2,6-$F_2$-Ph | 2-F-4-$CFClHCF_2SO_2$-Ph | |
| I - 10 | 2,6-$F_2$-Ph | 4-$CFClHCF_2S$-Ph | |
| I - 11 | 2,6-$F_2$-Ph | 4-$CFClHCF_2SO$-Ph | amorphous (3) |
| I - 12 | 2,6-$F_2$-Ph | 4-$CFClHCF_2SO_2$-Ph | |
| I - 13 | 2,6-$F_2$-Ph | 2-Cl-4-$CFClHCF_2S$-Ph | |
| I - 14 | 2,6-$F_2$-Ph | 2-Cl-4-$CFClHCF_2SO$-Ph | oil (4) |
| I - 15 | 2,6-$F_2$-Ph | 2-Cl-4-$CFClHCF_2SO_2$-Ph | |
| I - 16 | 2,6-$F_2$-Ph | 2-Cl-4-$CF_2HCF_2S$-Ph | |
| I - 17 | 2,6-$F_2$-Ph | 2-Cl-4-$CF_2HCF_2SO$-Ph | oil (5) |
| I - 18 | 2,6-$F_2$-Ph | 2-Cl-4-$CF_2HCF_2SO_2$-Ph | |
| I - 19 | 2,6-$F_2$-Ph | 4-$CF_2HCF_2S$-Ph | |
| I - 20 | 2,6-$F_2$-Ph | 4-$CF_2HCF_2SO$-Ph | oil (6) |
| I - 21 | 2,6-$F_2$-Ph | 4-$CF_2HCF_2SO_2$-Ph | |
| I - 22 | 2,6-$F_2$-Ph | 2-F-4-$CF_2HCF_2S$-Ph | 142-144 |
| I - 23 | 2,6-$F_2$-Ph | 2-F-4-$CF_2HCF_2SO$-Ph | white amorphous (7) |
| I - 24 | 2,6-$F_2$-Ph | 2-F-4-$CF_2HCF_2SO_2$-Ph | |
| I - 25 | 2,6-$F_2$-Ph | 4-$CF_3S$-Ph | 150.5-153.0 |
| I - 26 | 2,6-$F_2$-Ph | 4-$CF_3SO$-Ph | oil (8) |
| I - 27 | 2,6-$F_2$-Ph | 4-$CF_3SO_2$-Ph | |
| I - 28 | 2,6-$F_2$-Ph | 2-F-4-MeS-Ph | 85-88 |
| I - 29 | 2,6-$F_2$-Ph | 2-F-4-MeSO-Ph | oil (9) |

(1) $H^1$-NMR ($CDCl_3$)δppm : 5.12 (2H, s, $NCH_2O$), 5.61 ( 2H, s, $NCH_2O$), 6.74-7.75 ( 9H, m, Ph).
(2) $H^1$-NMR ($CDCl_3$)δppm : 5.20 ( 2H, s, $OCH_2N$), 5.62 ( 2H, s, $OCH_2N$), 6.35-6.75 ( 1H, m, CHClF), 6.91 ( 2H, t J=8.0Hz, Ph), 7.30-7.42 ( 1H, m, Ph), 7.50-7.65 ( 3H, m, Ph).
(3) $H^1$-NMR ($CDCl_3$)δppm : 5.28 ( 2H, s, $OCH_2N$), 5.59 ( 2H, s, $OCH_2N$), 6.32-6.74 ( 1H, m, CHClF), 6.88-7.05 ( 2H, m, Ph), 7.31-7.54 ( 3H, m, Ph), 7.76 ( 2H, t J=8.3Hz, Ph).
(4) $H^1$-NMR ($CDCl_3$)δppm : 4.92 ( 2H, s, $OCH_2N$), 5.16 ( 2H, s, $OCH_2N$), 6.36-6.76 ( 1H, m, CHClF), 6.90 ( 2H, t J=8.3Hz, Ph), 7.29-7.45 ( 1H, m, Ph), 7.58-7.64 ( 2H, m, Ph), 7.87 ( 1H, s ,Ph).
(5) $H^1$-NMR ($CDCl_3$)δppm : 5.16 ( 2H, def.s, $OCH_2N$), 5.64 ( 2H, def.s, $OCH_2N$), 6.19 ( 1H, tdd J=52.2, 8.4,2.0Hz, $CHF_2$), 6.90 ( 2H, t J=8.3Hz, Ph), 7.26-7.40 ( 1H, m, Ph), 7.53-7.71 ( 2H, m, Ph), 7.87 ( 1H ,s, Ph).
(6) $H^1$-NMR ($CDCl_3$)δppm : 5.28 ( 2H, s, $OCH_2N$), 5.59 ( 2H, s, $OCH_2N$), 6.16 ( 1H, tdd J=52.8,8.2,1.7Hz, $CHF_2$), 6.92 ( 2 H, t J=8.2Hz, Ph), 7.31-7.47 ( 1H ,m, Ph) ,7.52 ( 2H, d J=8.7Hz, Ph), 7.76 ( 2H ,d J=8.7Hz ,Ph).
(7) $H^1$-NMR ($CDCl_3$)δppm : 5.20 ( 2H, s, $NCH_2N$), 5.62 ( 2H, s, $NCH_2N$), 6.35-6.75 ( 1H, m, CHClF), 6.91 ( 2H, t J=8.0Hz, Ph), 7.30-7.42 ( 1H, m, Ph), 7.50-7.65 ( 3H ,m, Ph).
(8) $H^1$-NMR ($CDCl_3$)δppm : 5.28 ( 2H, s, $OCH_2N$), 5.60 ( 2H, s, $OCH_2N$), 6.92 ( 2H, t J=7.9Hz, Ph), 7.35 ( 1H, t J=8.5Hz, Ph), 7.53 ( 2H, d J=8.5Hz, Ph), 7.80 ( 2H, d J=8.5Hz ,Ph).
(9) $H^1$-NMR ($CDCl_3$) δppm : 2.70 ( 3H, s, SOMe), 5.17 ( 2H, s, $OCH_2N$), 5.62 ( 2H, s, $OCH_2N$), 6.90 ( 2H, t J=8.0Hz, Ph), 7.28-7.56 ( 4H, m, Ph).

| compound No. | $Q_1$ | $Q_2$ | m. p. (°C) |
|---|---|---|---|
| I - 30 | 2,6-$F_2$-Ph | 2-F-4-$MeSO_2$-Ph | |
| I - 31 | 2,6-$F_2$-Ph | 2,6-$F_2$-4-MeS-Ph | 89-91 |
| I - 32 | 2,6-$F_2$-Ph | 2,6-$F_2$-4-MeSO-Ph | 160-163 |
| I - 33 | 2,6-$F_2$-Ph | 2,6-$F_2$-4-$MeSO_2$-Ph | |
| I - 34 | 2,6-$F_2$-Ph | 2-F-4-$CF_3CH_2S$-Ph | 73.5-75.5 |
| I - 35 | 2,6-$F_2$-Ph | 2-F-4-$CF_3CH_2SO$-Ph | 136.5-138.5 |
| I - 36 | 2,6-$F_2$-Ph | 2-F-4-$CF_3CH_2SO_2$-Ph | |
| I - 37 | 2,6-$F_2$-Ph | 2-F-4-$CCl_2$=$CHCH_2S$-Ph | oil (10) |
| I - 38 | 2,6-$F_2$-Ph | 2-F-4-$CCl_2$=$CHCH_2SO$-Ph | |
| I - 39 | 2,6-$F_2$-Ph | 2-F-4-$CCl_2$=$CHCH_2SO_2$-Ph | |
| I - 40 | 2,6-$F_2$-Ph | $CF_3CH_2$ | 97.5-99 |
| I - 41 | 2,6-$F_2$-Ph | 2-F-4-$CF_2HS$-Ph | 77-79 |
| I - 42 | 2,6-$F_2$-Ph | 2-F-4-$CF_2HSO$-Ph | 138-140 |
| I - 43 | 2,6-$F_2$-Ph | 2-F-4-$CF_2HSO_2$-Ph | 210-213 |
| I - 44 | 2,6-$F_2$-Ph | 2-F-4-$CF_3S$-Ph | 57-59 |
| I - 45 | 2,6-$F_2$-Ph | 2-F-4-$CF_3SO$-Ph | white amorphous (11) |
| I - 46 | 2,6-$F_2$-Ph | 2-F-4-$CF_3SO_2$-Ph | 135 |
| I - 47 | 2,6-$F_2$-Ph | 2-F-4-$CH_3CH_2S$-Ph | 60-63 |
| I - 48 | 2,6-$F_2$-Ph | 2-F-4-$CH_3CH_2SO$-Ph | 143-145 |
| I - 49 | 2,6-$F_2$-Ph | 2-F-4-$CH_3CH_2SO_2$-Ph | |
| I - 50 | 2,6-$F_2$-Ph | 2-F-4-$CH_3CH_2CH_2S$-Ph | oil (12) |
| I - 51 | 2,6-$F_2$-Ph | 2-F-4-$CH_3CH_2CH_2SO$-Ph | amorphous (13) |
| I - 52 | 2,6-$F_2$-Ph | 2-F-4-$CH_3CH_2CH_2SO_2$-Ph | |
| I - 53 | 2,6-$F_2$-Ph | 2-F-4-i-PrS-Ph | 85-90 |
| I - 54 | 2,6-$F_2$-Ph | 2-F-4-i-PrSO-Ph | amorphous (14) |
| I - 55 | 2,6-$F_2$-Ph | 2-F-4-i-$PrSO_2$-Ph | |
| I - 56 | 2,6-$F_2$-Ph | 2-F-4-t-BuS-Ph | 152-153 |
| I - 57 | 2,6-$F_2$-Ph | 2-F-4-t-BuSO-Ph | 220-221 |
| I - 58 | 2,6-$F_2$-Ph | 2-F-4-t-$BuSO_2$-Ph | |
| I - 59 | 2,6-$F_2$-Ph | 2-F-4-$CH_2$=$CHCH_2S$-Ph | oil (15) |
| I - 60 | 2,6-$F_2$-Ph | 2-F-4-$CH_2$=$CHCH_2SO$-Ph | amorphous (16) |

(10) $H^1$-NMR ($CDCl_3$)δppm : 3.63 ( 2H, d J=7.5Hz, $SCH_2$), 5.11 ( 2H, s, $OCH_2N$), 5.60 ( 2H, s, $OCH_2N$), 5.93 ( 1H, t J=7.5Hz ,$CHCCl_2$), 6.89 ( 2H, t J=8.0Hz, Ph), 7.06-7.13 ( 2H, m, Ph),7 .21-7.35 ( 2H, m, Ph).

(11) $H^1$-NMR ($CDCl_3$)δppm : 5.19 (2H, s, $CH_2$), 5.62 ( 2H, s, $CH_2$), 6.89-6.93 ( 2H, m, Ph), 7.32-7.36 ( 1H, m, Ph), 7.54-7.66(3H, m, Ph).

(12) $H^1$-NMR ($CDCl_3$)δppm : 0.99-1.03 ( 3H, m, $CH_3$), 1.63-1.69 ( 2H, m, $CH_2$), 2.84-2.88 ( 2H, m, $SCH_2$), 5.09 ( 2H, s, $OCH_2N$), 5.59 ( 2H, s, $OCH_2N$), 6.86-6.90 ( 2H, m, Ph), 7.02-7.05 ( 2H, m, Ph), 7.17-7.30 ( 2H, m, Ph).

(13) $H^1$-NMR ($CDCl_3$)δppm : 1.02-1.06 ( 3H, m, $CH_3$), 1.62-1.84 ( 2H, m, $CH_2$), 2.70-2.74 ( 2H, m, $SCH_2$), 5.16 ( 2H, s, $OCH_2N$), 5.61 ( 2H, s, $OCH_2N$), 6.88-6.92 (2H, m, Ph), 7.32-7.37 ( 2H, m, Ph), 7.47-7.52 ( 2H, m, Ph).

(14) $H^1$-NMR ($CDCl_3$)δppm : 1.10 ( 3H, d J=6.8Hz, $CH_3$), 1.25 ( 3H, d J=6.8Hz, CH3 ), 1.25 ( 1H, hept J=6.8Hz, SCH), 5.15 ( 2H, s, $OCH_2N$), 5.61 ( 2H, s, $OCH_2N$), 6.88-6.92 (2H, m, Ph), 7.31-7.34 ( 2H, m, Ph), 7.44-7.51 ( 2H, m, Ph).

(15) $H^1$-NMR ($CDCl_3$)δppm : 3.53-3.55 ( 2H, m, $SCH_2$), 5.09 ( 2H, s, $OCH_2N$), 5.12-5.24 ( 2H, m, $C=CH_2$), 5.59 ( 2H, s, $OCH_2N$), 5.80-5.84 ( 1H, m, CH), 6.86-6.90 ( 2H, m, Ph), 7.04-7.08 ( 2H, m, Ph), 7.18-7.30 ( 2H, m, Ph).

(16) $H^1$-NMR ($CDCl_3$)δppm : 3.47-3.54 ( 2H, m, $SCH_2$), 5.16 ( 2H, s, $OCH_2N$), 5.24-5.40 ( 2H, m, $C=CH_2$), 5.59-5.63 ( 3H, m, $CH+OCH_2N$), 6.88-6.92 (2H, m, Ph), 7.26-7.52 ( 4H, m, Ph).

| compound No. | $Q_1$ | $Q_2$ | m. p. (°C) |
|---|---|---|---|
| I - 61 | 2,6-$F_2$-Ph | 2-F-4-$CH_2$=$CHCH_2SO_2$-Ph | |
| I - 62 | 2,6-$F_2$-Ph | 2-F-4-CH≡$CCH_2S$-Ph | oil (17) |
| I - 63 | 2,6-$F_2$-Ph | 2-F-4-CH≡$CCH_2SO$-Ph | amorphous (18) |
| I - 64 | 2,6-$F_2$-Ph | 2-F-4-CH≡$CCH_2SO_2$-Ph | |
| I - 65 | 2,6-$F_2$-Ph | 2-F-4-(2-Cl-4-$CF_3$-pyridin-2yl-S)-Ph | 101-102 |
| I - 66 | 2,6-$F_2$-Ph | 2-F-4-(2-Cl-4-$CF_3$-pyridin-2yl-SO)-Ph | 90-94 |
| I - 67 | 2,6-$F_2$-Ph | 2-F-4-(2-Cl-4-$CF_3$-pyridin-2yl-$SO_2$)-Ph | 77-80 |
| I - 68 | 2,6-$F_2$-Ph | 2-Me-4-$CF_3S$-Ph | 103-105 |
| I - 69 | 2,6-$F_2$-Ph | 2-Me-4-$CF_3SO$-Ph | 109-112 |
| I - 70 | 2,6-$F_2$-Ph | 2-Me-4-$CF_3SO_2$-Ph | |
| I - 71 | 2,6-$F_2$-Ph | 3-Me-4-$CF_3S$-Ph | 69-71 |
| I - 72 | 2,6-$F_2$-Ph | 3-Me-4-$CF_3SO$-Ph | 173 |
| I - 73 | 2,6-$F_2$-Ph | 3-Me-4-$CF_3SO_2$-Ph | |
| I - 74 | 2,6-$F_2$-Ph | 2-Cl-4-$CF_3S$-Ph | 119-121 |
| I - 75 | 2,6-$F_2$-Ph | 2-Cl-4-$CF_3SO$-Ph | amorphous (19) |
| I - 76 | 2,6-$F_2$-Ph | 2-Cl-4-$CF_3SO_2$-Ph | |
| I - 77 | 2,6-$F_2$-Ph | 3-Cl-4-$CF_3S$-Ph | 122-124 |
| I - 78 | 2,6-$F_2$-Ph | 3-Cl-4-$CF_3SO$-Ph | 174-175 |
| I - 79 | 2,6-$F_2$-Ph | 3-Cl-4-$CF_3SO_2$-Ph | |
| I - 80 | 2,6-$F_2$-Ph | 3-F-4-$CF_3S$-Ph | 147-148 |
| I - 81 | 2,6-$F_2$-Ph | 3-F-4-$CF_3SO$-Ph | 164-168 |
| I - 82 | 2,6-$F_2$-Ph | 3-F-4-$CF_3SO_2$-Ph | |
| I - 83 | 2,6-$F_2$-Ph | 2,3-$Me_2$-4-$CF_3S$-Ph | 120-130 |
| I - 84 | 2,6-$F_2$-Ph | 2,3-$Me_2$-4-$CF_3SO$-Ph | 190-194 |
| I - 85 | 2,6-$F_2$-Ph | 2,3-$Me_2$-4-$CF_3SO_2$-Ph | |
| I - 86 | 2,6-$F_2$-Ph | 2,5-$Me_2$-4-$CF_3S$-Ph | 175-179 |
| I - 87 | 2,6-$F_2$-Ph | 2,5-$Me_2$-4-$CF_3SO$-Ph | 165-170 |
| I - 88 | 2,6-$F_2$-Ph | 2,5-$Me_2$-4-$CF_3SO_2$-Ph | |
| I - 89 | 2,6-$F_2$-Ph | 5-Cl-2-Me-4-$CF_3S$-Ph | 180-187 |
| I - 90 | 2,6-$F_2$-Ph | 5-Cl-2-Me-4-$CF_3SO$-Ph | 215-217 |
| I - 91 | 2,6-$F_2$-Ph | 5-Cl-2-Me-4-$CF_3SO_2$-Ph | |
| I - 92 | 2,6-$F_2$-Ph | 2,3-$Cl_2$-4-$CF_3S$-Ph | 132 |
| I - 93 | 2,6-$F_2$-Ph | 2,3-$Cl_2$-4-$CF_3SO$-Ph | 182-183 |
| I - 94 | 2,6-$F_2$-Ph | 2,3-$Cl_2$-4-$CF_3SO_2$-Ph | |
| I - 95 | 2,6-$F_2$-Ph | 3-Cl-2-F-4-$CF_3S$-Ph | 102-103 |
| I - 96 | 2,6-$F_2$-Ph | 3-Cl-2-F-4-$CF_3SO$-Ph | amorphous (20) |
| I - 97 | 2,6-$F_2$-Ph | 3-Cl-2-F-4-$CF_3SO_2$-Ph | |
| I - 98 | 2,6-$F_2$-Ph | 5-Cl-2-F-4-$CF_3S$-Ph | 125 |
| I - 99 | 2,6-$F_2$-Ph | 5-Cl-2-F-4-$CF_3SO$-Ph | 198 |
| I - 100 | 2,6-$F_2$-Ph | 5-Cl-2-F-4-$CF_3SO_2$-Ph | |

(17) $H^1$-NMR ($CDCl_3$)δppm : 2.24 ( 1H, t J=2.7Hz, CH), 3.58 ( 2H, d J=2.7Hz, $SCH_2$), 5.11 ( 2H, s, $OCH_2N$), , 5.59 ( 2H, s, $OCH_2N$), 6.86-6.90 ( 2H, m, Ph), 7.16-7.30 ( 4H, m, Ph).
(18) $H^1$-NMR ($CDCl_3$)δppm : 2.39 ( 1H, d J=2.6Hz, CH), 3.58-3.67 ( 2H, m, $SCH_2$), 5.17 ( 2H, s, $OCH_2N$), 5.61 ( 2H, s, $OCH_2N$), 6.88-6.92 (2H, m, Ph), 7.25-7.60 ( 4H, m, Ph).
(19) $H^1$-NMR ($CDCl_3$)δppm : 5.05-5.20 (2H, br, $CH_2$), 5.50-5.80 ( 2H, br, $CH_2$), 6.88-6.92 ( 2H, m, Ph), 7.29-7.36 ( 1H, m, Ph), 7.59-7.61 ( 1H, m, Ph), 7.68-7.71 ( 1H, m, Ph), 7.91-7.92 ( 1H, m, Ph).
(20) $H^1$-NMR ($CDCl_3$)δppm : 5.17-5.22 (2H, s, $CH_2$), 5.59-5.64 ( 2H, m, $CH_2$), 6.90-6.94 ( 2H, m, Ph), 7.32-7.39 ( 1H, m, Ph), 7.56-7.60 ( 1H, m, Ph), 7.84 ( 1H, d J=8.6Hz, Ph).

| compound No. | $Q_1$ | $Q_2$ | m. p. (°C) |
|---|---|---|---|
| I - 101 | 2,6-$F_2$-Ph | 2-Cl-6-F-4-$CF_3$S-Ph | 77-78 |
| I - 102 | 2,6-$F_2$-Ph | 2-Cl-6-F-4-$CF_3$SO-Ph | 141-142 |
| I - 103 | 2,6-$F_2$-Ph | 2-Cl-6-F-4-$CF_3SO_2$-Ph | |
| I - 104 | 2,6-$F_2$-Ph | 2,6-$F_2$-4-$CF_3$S-Ph | 79-82 |
| I - 105 | 2,6-$F_2$-Ph | 2,6-$F_2$-4-$CF_3$SO-Ph | amorphous (21) |
| I - 106 | 2,6-$F_2$-Ph | 2,6-$F_2$-4-$CF_3SO_2$-Ph | |
| I - 107 | 2,6-$F_2$-Ph | 2,3-$F_2$-4-$CF_3$S-Ph | 112-113 |
| I - 108 | 2,6-$F_2$-Ph | 2,3-$F_2$-4-$CF_3$SO-Ph | amorphous (22) |
| I - 109 | 2,6-$F_2$-Ph | 2,3-$F_2$-4-$CF_3SO_2$-Ph | |
| I - 110 | 2,6-$F_2$-Ph | 2,5-$F_2$-4-$CF_3$S-Ph | 100-105 |
| I - 111 | 2,6-$F_2$-Ph | 2,5-$F_2$-4-$CF_3$SO-Ph | 197-199 |
| I - 112 | 2,6-$F_2$-Ph | 2,5-$F_2$-4-$CF_3SO_2$-Ph | |
| I - 113 | 2,6-$F_2$-Ph | 3,5-$F_2$-4-$CF_3$S-Ph | 113-114 |
| I - 114 | 2,6-$F_2$-Ph | 3,5-$F_2$-4-$CF_3$SO-Ph | |
| I - 115 | 2,6-$F_2$-Ph | 3,5-$F_2$-4-$CF_3SO_2$-Ph | |
| I - 116 | 2,6-$F_2$-Ph | 2,3,6-$F_3$-4-$CF_3$S-Ph | 112-114 |
| I - 117 | 2,6-$F_2$-Ph | 2,3,6-$F_3$-4-$CF_3$SO-Ph | 131 |
| I - 118 | 2,6-$F_2$-Ph | 2,3,6-$F_3$-4-$CF_3SO_2$-Ph | |
| I - 119 | 2,6-$F_2$-Ph | 2,3,5,6-$F_4$-4-$CF_3$S-Ph | 107 |
| I - 120 | 2,6-$F_2$-Ph | 2,3,5,6-$F_4$-4-$CF_3$SO-Ph | |
| I - 121 | 2,6-$F_2$-Ph | 2,3,5,6-$F_4$-4-$CF_3SO_2$-Ph | |
| I - 122 | 2,6-$F_2$-Ph | 3-Cl-2-Me-4-$CF_3$S-Ph | 96-97 |
| I - 123 | 2,6-$F_2$-Ph | 3-Cl-2-Me-4-$CF_3$SO-Ph | 182 |
| I - 124 | 2,6-$F_2$-Ph | 3-Cl-2-Me-4-$CF_3SO_2$-Ph | |
| I - 125 | 2,6-$F_2$-Ph | 5-F-2-Me-4-$CF_3$S-Ph | 176-177 |
| I - 126 | 2,6-$F_2$-Ph | 5-F-2-Me-4-$CF_3$SO-Ph | 199-200 |
| I - 127 | 2,6-$F_2$-Ph | 5-F-2-Me-4-$CF_3SO_2$-Ph | |
| I - 128 | 2,6-$F_2$-Ph | 2-F-4-$CF_3CF_2$S-Ph | 47-50 |
| I - 129 | 2,6-$F_2$-Ph | 2-F-4-$CF_3CF_2$SO-Ph | amorphous (23) |
| I - 130 | 2,6-$F_2$-Ph | 2-F-4-$CF_3CF_2SO_2$-Ph | |
| I - 131 | 2,6-$F_2$-Ph | 2-Cl-4-$CF_3CF_2$S-Ph | |
| I - 132 | 2,6-$F_2$-Ph | 2-Cl-4-$CF_3CF_2$SO-Ph | |
| I - 133 | 2,6-$F_2$-Ph | 2-Cl-4-$CF_3CF_2SO_2$-Ph | |
| I - 134 | 2,6-$F_2$-Ph | 2-Me-4-$CF_3CF_2$S-Ph | |
| I - 135 | 2,6-$F_2$-Ph | 2-Me-4-$CF_3CF_2$SO-Ph | |
| I - 136 | 2,6-$F_2$-Ph | 2-Me-4-$CF_3CF_2SO_2$-Ph | |
| I - 137 | 2,6-$F_2$-Ph | 5-Cl-2-Me-4-$CF_3CF_2$S-Ph | |
| I - 138 | 2,6-$F_2$-Ph | 5-Cl-2-Me-4-$CF_3CF_2$SO-Ph | |
| I - 139 | 2,6-$F_2$-Ph | 5-Cl-2-Me-4-$CF_3CF_2SO_2$-Ph | |
| I - 140 | 2,6-$F_2$-Ph | 2,3-$Me_2$-4-$CF_3CF_2$S-Ph | |

(21) $H^1$-NMR ($CDCl_3$)δppm : 5.15 (2H, s, $CH_2$), 5.63 ( 2H, s, $CH_2$), 6.89-6.93 ( 2H, m, Ph), 7.34-7.45 ( 3H, m, Ph) .

(22) $H^1$-NMR ($CDCl_3$)δppm : 5.18-5.23 (2H, m, $CH_2$), 5.59-5.64 ( 2H, m, $CH_2$), 6.90-6.94 ( 2H, m, Ph), 7.32-7.46 ( 2H, m, Ph), 7.72-7.75 ( 1H, m, Ph) .

(23) $H^1$-NMR ($CDCl_3$)δppm : 5.20 (2H, s, $CH_2$), 5.61 ( 2H, s, $CH_2$), 6.89-6.93 ( 2H, m, Ph), 7.32-7.36 ( 1H, m, Ph), 7.56-7.66 ( 3H, m, Ph).

| compound No. | $Q_1$ | $Q_2$ | m. p. (°C) |
|---|---|---|---|
| I - 141 | 2,6-$F_2$-Ph | 2,3-$Me_2$-4-$CF_3CF_2SO$-Ph | |
| I - 142 | 2,6-$F_2$-Ph | 2,3-$Me_2$-4-$CF_3CF_2SO_2$-Ph | |
| I - 143 | 2,6-$F_2$-Ph | 2-F-4-$CF_3CF_2CF_2S$-Ph | 44-46 |
| I - 144 | 2,6-$F_2$-Ph | 2-F-4-$CF_3CF_2CF_2SO$-Ph | amorphous (24) |
| I - 145 | 2,6-$F_2$-Ph | 2-F-4-$CF_3CF_2CF_2SO_2$-Ph | |
| I - 146 | 2,6-$F_2$-Ph | 2-Cl-4-$CF_3CF_2CF_2S$-Ph | |
| I - 147 | 2,6-$F_2$-Ph | 2-Cl-4-$CF_3CF_2CF_2SO$-Ph | |
| I - 148 | 2,6-$F_2$-Ph | 2-Cl-4-$CF_3CF_2CF_2SO_2$-Ph | |
| I - 149 | 2,6-$F_2$-Ph | 2-Me-4-$CF_3CF_2CF_2S$-Ph | |
| I - 150 | 2,6-$F_2$-Ph | 2-Me-4-$CF_3CF_2CF_2SO$-Ph | |
| I - 151 | 2,6-$F_2$-Ph | 2-Me-4-$CF_3CF_2CF_2SO_2$-Ph | |
| I - 152 | 2,6-$F_2$-Ph | 5-Cl-2-Me-4-$CF_3CF_2CF_2S$-Ph | |
| I - 153 | 2,6-$F_2$-Ph | 5-Cl-2-Me-4-$CF_3CF_2CF_2SO$-Ph | |
| I - 154 | 2,6-$F_2$-Ph | 5-Cl-2-Me-4-$CF_3CF_2CF_2SO_2$-Ph | |
| I - 155 | 2,6-$F_2$-Ph | 2,3-$Me_2$-4-$CF_3CF_2CF_2S$-Ph | |
| I - 156 | 2,6-$F_2$-Ph | 2,3-$Me_2$-4-$CF_3CF_2CF_2SO$-Ph | |
| I - 157 | 2,6-$F_2$-Ph | 2,3-$Me_2$-4-$CF_3CF_2CF_2SO_2$-Ph | |
| I - 158 | 2,6-$F_2$-Ph | 2-F-4-$(CF_3)_2CFS$-Ph | 85-86 |
| I - 159 | 2,6-$F_2$-Ph | 2-F-4-$(CF_3)_2CFSO$-Ph | 40-45 |
| I - 160 | 2,6-$F_2$-Ph | 2-F-4-$(CF_3)_2CFSO_2$-Ph | |
| I - 161 | 2,6-$F_2$-Ph | 2-Me-4-$(CF_3)_2CFS$-Ph | |
| I - 162 | 2,6-$F_2$-Ph | 2-Me-4-$(CF_3)_2CFSO$-Ph | |
| I - 163 | 2,6-$F_2$-Ph | 2-Me-4-$(CF_3)_2CFSO_2$-Ph | |
| I - 164 | 2,6-$F_2$-Ph | 2-Cl-4-$(CF_3)_2CFS$-Ph | |
| I - 165 | 2,6-$F_2$-Ph | 2-Cl-4-$(CF_3)_2CFSO$-Ph | |
| I - 166 | 2,6-$F_2$-Ph | 2-Cl-4-$(CF_3)_2CFSO_2$-Ph | |
| I - 167 | 2,6-$F_2$-Ph | 5-Cl-2-Me-4-$(CF_3)_2CFS$-Ph | |
| I - 168 | 2,6-$F_2$-Ph | 5-Cl-2-Me-4-$(CF_3)_2CFSO$-Ph | |
| I - 169 | 2,6-$F_2$-Ph | 5-Cl-2-Me-4-$(CF_3)_2CFSO_2$-Ph | |
| I - 170 | 2,6-$F_2$-Ph | 2,3-$Me_2$-4-$(CF_3)_2CFS$-Ph | |
| I - 171 | 2,6-$F_2$-Ph | 2,3-$Me_2$-4-$(CF_3)_2CFSO$-Ph | |
| I - 172 | 2,6-$F_2$-Ph | 2,3-$Me_2$-4-$(CF_3)_2CFSO_2$-Ph | |
| I - 173 | 2,6-$F_2$-Ph | 2-F-4-MeO-Ph | 121-122 |
| I - 174 | 2,6-$F_2$-Ph | 2-Me-4-MeO-Ph | |
| I - 175 | 2,6-$F_2$-Ph | 2-F-4-CN-Ph | 90 |
| I - 176 | 2,6-$F_2$-Ph | 2-F-4-COOMe-Ph | |
| I - 177 | 2,6-$F_2$-Ph | 2-F-4-COOEt-Ph | 123-125 |
| I - 178 | 2-Cl-6-F-Ph | 4-Cl-Ph | |
| I - 179 | 2-Cl-6-F-Ph | 3,5-$Cl_2$-4-(2-Cl-4-$CF_3$-pyridin-2yl-O)-Ph | |
| I - 180 | 2-Cl-6-F-Ph | 2-F-4-(2-Cl-4-$CF_3$-Ph-O)-Ph | |

(24) $H^1$-NMR ($CDCl_3$)δppm : 5.20 (2H, s, $CH_2$), 5.61 ( 2H, s, $CH_2$), 6.89-6.93 ( 2H, m, Ph), 7.32-7.36 ( 1H, m, Ph), 7.54-7.66 ( 3H, m, Ph).

| compound No. | $Q_1$ | $Q_2$ | m. p. (°C) |
|---|---|---|---|
| I - 181 | 2-Cl-6-F-Ph | 3,5-$Cl_2$-2,4-$F_2$-Ph | |
| I - 182 | 2-Cl-6-F-Ph | 3,5-$Cl_2$-4-$CF_2HCF_2O$-Ph | |
| I - 183 | 2-Cl-6-F-Ph | 2,5-$Cl_2$-4-$CF_3CFHCF_2O$-Ph | |
| I - 184 | 2-Cl-6-F-Ph | 2-F-4-$CFClHCF_2S$-Ph | |
| I - 185 | 2-Cl-6-F-Ph | 2-F-4-$CFClHCF_2SO$-Ph | |
| I - 186 | 2-Cl-6-F-Ph | 2-F-4-$CFClHCF_2SO_2$-Ph | |
| I - 187 | 2-Cl-6-F-Ph | 4-$CFClHCF_2S$-Ph | |
| I - 188 | 2-Cl-6-F-Ph | 4-$CFClHCF_2SO$-Ph | |
| I - 189 | 2-Cl-6-F-Ph | 4-$CFClHCF_2SO_2$-Ph | |
| I - 190 | 2-Cl-6-F-Ph | 2-Cl-4-$CFClHCF_2S$-Ph | |
| I - 191 | 2-Cl-6-F-Ph | 2-Cl-4-$CFClHCF_2SO$-Ph | |
| I - 192 | 2-Cl-6-F-Ph | 2-Cl-4-$CFClHCF_2SO_2$-Ph | |
| I - 193 | 2-Cl-6-F-Ph | 2-Cl-4-$CF_2HCF_2S$-Ph | |
| I - 194 | 2-Cl-6-F-Ph | 2-Cl-4-$CF_2HCF_2SO$-Ph | |
| I - 195 | 2-Cl-6-F-Ph | 2-Cl-4-$CF_2HCF_2SO_2$-Ph | |
| I - 196 | 2-Cl-6-F-Ph | 4-$CF_2HCF_2S$-Ph | |
| I - 197 | 2-Cl-6-F-Ph | 4-$CF_2HCF_2SO$-Ph | |
| I - 198 | 2-Cl-6-F-Ph | 4-$CF_2HCF_2SO_2$-Ph | |
| I - 199 | 2-Cl-6-F-Ph | 2-F-4-$CF_2HCF_2S$-Ph | |
| I - 200 | 2-Cl-6-F-Ph | 2-F-4-$CF_2HCF_2SO$-Ph | |
| I - 201 | 2-Cl-6-F-Ph | 2-F-4-$CF_2HCF_2SO_2$-Ph | |
| I - 202 | 2-Cl-6-F-Ph | 4-$CF_3S$-Ph | |
| I - 203 | 2-Cl-6-F-Ph | 4-$CF_3SO$-Ph | |
| I - 204 | 2-Cl-6-F-Ph | 4-$CF_3SO_2$-Ph | |
| I - 205 | 2-Cl-6-F-Ph | 2-F-4-MeS-Ph | 90-92 |
| I - 206 | 2-Cl-6-F-Ph | 2-F-4-MeSO-Ph | 155-158 |
| I - 207 | 2-Cl-6-F-Ph | 2-F-4-$MeSO_2$-Ph | |
| I - 208 | 2-Cl-6-F-Ph | 2,6-$F_2$-4-MeS-Ph | 90-93 |
| I - 209 | 2-Cl-6-F-Ph | 2,6-$F_2$-4-MeSO-Ph | 172-175 |
| I - 210 | 2-Cl-6-F-Ph | 2,6-$F_2$-4-$MeSO_2$-Ph | |
| I - 211 | 2-Cl-6-F-Ph | 2-F-4-$CF_3CH_2S$-Ph | |
| I - 212 | 2-Cl-6-F-Ph | 2-F-4-$CF_3CH_2SO$-Ph | |
| I - 213 | 2-Cl-6-F-Ph | 2-F-4-$CF_3CH_2SO_2$-Ph | |
| I - 214 | 2-Cl-6-F-Ph | 2-F-4-$CCl_2$=$CHCH_2S$-Ph | |
| I - 215 | 2-Cl-6-F-Ph | 2-F-4-$CCl_2$=$CHCH_2SO$-Ph | |
| I - 216 | 2-Cl-6-F-Ph | 2-F-4-$CCl_2$=$CHCH_2SO_2$-Ph | |
| I - 217 | 2-Cl-6-F-Ph | $CF_3CH_2$ | |
| I - 218 | 2-Cl-6-F-Ph | 2-F-4-$CF_2HS$-Ph | 118-120 |
| I - 219 | 2-Cl-6-F-Ph | 2-F-4-$CF_2HSO$-Ph | 55-60 |
| I - 220 | 2-Cl-6-F-Ph | 2-F-4-$CF_2HSO_2$-Ph | |

| compound No. | $Q_1$ | $Q_2$ | m. p. (°C) |
|---|---|---|---|
| I - 221 | 2-Cl-6-F-Ph | 2-F-4-$CF_3$S-Ph | 77-80 |
| I - 222 | 2-Cl-6-F-Ph | 2-F-4-$CF_3$SO-Ph | amorphous (25) |
| I - 223 | 2-Cl-6-F-Ph | 2-F-4-$CF_3SO_2$-Ph | |
| I - 224 | 2-Cl-6-F-Ph | 2-F-4-$CH_3CH_2$S-Ph | |
| I - 225 | 2-Cl-6-F-Ph | 2-F-4-$CH_3CH_2$SO-Ph | |
| I - 226 | 2-Cl-6-F-Ph | 2-F-4-$CH_3CH_2SO_2$-Ph | |
| I - 227 | 2-Cl-6-F-Ph | 2-F-4-$CH_3CH_2CH_2$S-Ph | |
| I - 228 | 2-Cl-6-F-Ph | 2-F-4-$CH_3CH_2CH_2$SO-Ph | |
| I - 229 | 2-Cl-6-F-Ph | 2-F-4-$CH_3CH_2CH_2SO_2$-Ph | |
| I - 230 | 2-Cl-6-F-Ph | 2-F-4-i-PrS-Ph | |
| I - 231 | 2-Cl-6-F-Ph | 2-F-4-i-PrSO-Ph | |
| I - 232 | 2-Cl-6-F-Ph | 2-F-4-i-$PrSO_2$-Ph | |
| I - 233 | 2-Cl-6-F-Ph | 2-F-4-t-BuS-Ph | |
| I - 234 | 2-Cl-6-F-Ph | 2-F-4-t-BuSO-Ph | |
| I - 235 | 2-Cl-6-F-Ph | 2-F-4-t-$BuSO_2$-Ph | |
| I - 236 | 2-Cl-6-F-Ph | 2-F-4-$CH_2$=$CHCH_2$S-Ph | |
| I - 237 | 2-Cl-6-F-Ph | 2-F-4-$CH_2$=$CHCH_2$SO-Ph | |
| I - 238 | 2-Cl-6-F-Ph | 2-F-4-$CH_2$=$CHCH_2SO_2$-Ph | |
| I - 239 | 2-Cl-6-F-Ph | 2-F-4-$CHCCH_2$S-Ph | |
| I - 240 | 2-Cl-6-F-Ph | 2-F-4-$CHCCH_2$SO-Ph | |
| I - 241 | 2-Cl-6-F-Ph | 2-F-4-$CHCCH_2SO_2$-Ph | |
| I - 242 | 2-Cl-6-F-Ph | 2-F-4-(2-Cl-4-$CF_3$-pyridin-2yl-S)-Ph | |
| I - 243 | 2-Cl-6-F-Ph | 2-F-4-(2-Cl-4-$CF_3$-pyridin-2yl-SO)-Ph | |
| I - 244 | 2-Cl-6-F-Ph | 2-F-4-(2-Cl-4-$CF_3$-pyridin-2yl-$SO_2$)-Ph | |
| I - 245 | 2-Cl-6-F-Ph | 2-Me-4-$CF_3$S-Ph | |
| I - 246 | 2-Cl-6-F-Ph | 2-Me-4-$CF_3$SO-Ph | |
| I - 247 | 2-Cl-6-F-Ph | 2-Me-4-$CF_3SO_2$-Ph | |
| I - 248 | 2-Cl-6-F-Ph | 3-Me-4-$CF_3$S-Ph | 102 |
| I - 249 | 2-Cl-6-F-Ph | 3-Me-4-$CF_3$SO-Ph | 187 |
| I - 250 | 2-Cl-6-F-Ph | 3-Me-4-$CF_3SO_2$-Ph | |
| I - 251 | 2-Cl-6-F-Ph | 2-Cl-4-$CF_3$S-Ph | 129-131 |
| I - 252 | 2-Cl-6-F-Ph | 2-Cl-4-$CF_3$SO-Ph | amorphous (26) |
| I - 253 | 2-Cl-6-F-Ph | 2-Cl-4-$CF_3SO_2$-Ph | |
| I - 254 | 2-Cl-6-F-Ph | 3-Cl-4-$CF_3$S-Ph | 112-113 |
| I - 255 | 2-Cl-6-F-Ph | 3-Cl-4-$CF_3$SO-Ph | 204-205 |
| I - 256 | 2-Cl-6-F-Ph | 3-Cl-4-$CF_3SO_2$-Ph | |
| I - 257 | 2-Cl-6-F-Ph | 3-F-4-$CF_3$S-Ph | 95-96 |
| I - 258 | 2-Cl-6-F-Ph | 3-F-4-$CF_3$SO-Ph | 197-198 |
| I - 259 | 2-Cl-6-F-Ph | 3-F-4-$CF_3SO_2$-Ph | |
| I - 260 | 2-Cl-6-F-Ph | 2,3-$Me_2$-4-$CF_3$S-Ph | |

(25) $H^1$-NMR ($CDCl_3$)δppm : 5.19 ( 2H, s, $OCH_2N$), 5.64-5.65 ( 2H, m, $OCH_2N$), 7.02-7.04 (1H, m, Ph), 7.16-7.18 ( 1H, m, Ph), 7.26-7.29 ( 1H, m, Ph), 7.60-7.65 ( 3H, m, Ph).

(26) $H^1$-NMR ($CDCl_3$)δppm : 5.05-5.30 (2H, br, $CH_2$), 5.50-5.85 ( 2H, br, $CH_2$), 6.98-7.03 ( 1H, m, Ph), 7.15-7.17 ( 1H, m, Ph), 7.24-7.30 ( 1H, m, Ph), 7.58-7.61 ( 1H, m, Ph), 7.67-7.69 ( 1H, m, Ph), 7.90-7.91 ( 1H, m, Ph).

| compound No. | $Q_1$ | $Q_2$ | m. p. (℃) |
|---|---|---|---|
| I - 261 | 2-Cl-6-F-Ph | 2,3-$Me_2$-4-$CF_3SO$-Ph | |
| I - 262 | 2-Cl-6-F-Ph | 2,3-$Me_2$-4-$CF_3SO_2$-Ph | |
| I - 263 | 2-Cl-6-F-Ph | 2,5-$Me_2$-4-$CF_3S$-Ph | |
| I - 264 | 2-Cl-6-F-Ph | 2,5-$Me_2$-4-$CF_3SO$-Ph | |
| I - 265 | 2-Cl-6-F-Ph | 2,5-$Me_2$-4-$CF_3SO_2$-Ph | |
| I - 266 | 2-Cl-6-F-Ph | 5-Cl-2-Me-4-$CF_3S$-Ph | 180-186 |
| I - 267 | 2-Cl-6-F-Ph | 5-Cl-2-Me-4-$CF_3SO$-Ph | |
| I - 268 | 2-Cl-6-F-Ph | 5-Cl-2-Me-4-$CF_3SO_2$-Ph | |
| I - 269 | 2-Cl-6-F-Ph | 2,3-$Cl_2$-4-$CF_3S$-Ph | 170 |
| I - 270 | 2-Cl-6-F-Ph | 2,3-$Cl_2$-4-$CF_3SO$-Ph | 228-229 |
| I - 271 | 2-Cl-6-F-Ph | 2,3-$Cl_2$-4-$CF_3SO_2$-Ph | |
| I - 272 | 2-Cl-6-F-Ph | 3-Cl-2-F-4-$CF_3S$-Ph | 99-101 |
| I - 273 | 2-Cl-6-F-Ph | 3-Cl-2-F-4-$CF_3SO$-Ph | 151 |
| I - 274 | 2-Cl-6-F-Ph | 3-Cl-2-F-4-$CF_3SO_2$-Ph | |
| I - 275 | 2-Cl-6-F-Ph | 5-Cl-2-F-4-$CF_3S$-Ph | 139-140 |
| I - 276 | 2-Cl-6-F-Ph | 5-Cl-2-F-4-$CF_3SO$-Ph | 217 |
| I - 277 | 2-Cl-6-F-Ph | 5-Cl-2-F-4-$CF_3SO_2$-Ph | |
| I - 278 | 2-Cl-6-F-Ph | 2-Cl-6-F-4-$CF_3S$-Ph | 108-109 |
| I - 279 | 2-Cl-6-F-Ph | 2-Cl-6-F-4-$CF_3SO$-Ph | 165 |
| I - 280 | 2-Cl-6-F-Ph | 2-Cl-6-F-4-$CF_3SO_2$-Ph | |
| I - 281 | 2-Cl-6-F-Ph | 2,6-$F_2$-4-$CF_3S$-Ph | 128-130 |
| I - 282 | 2-Cl-6-F-Ph | 2,6-$F_2$-4-$CF_3SO$-Ph | 193-195 |
| I - 283 | 2-Cl-6-F-Ph | 2,6-$F_2$-4-$CF_3SO_2$-Ph | |
| I - 284 | 2-Cl-6-F-Ph | 2,3-$F_2$-4-$CF_3S$-Ph | 78-80 |
| I - 285 | 2-Cl-6-F-Ph | 2,3-$F_2$-4-$CF_3SO$-Ph | 136-137 |
| I - 286 | 2-Cl-6-F-Ph | 2,3-$F_2$-4-$CF_3SO_2$-Ph | |
| I - 287 | 2-Cl-6-F-Ph | 2,5-$F_2$-4-$CF_3S$-Ph | 90-98 |
| I - 288 | 2-Cl-6-F-Ph | 2,5-$F_2$-4-$CF_3SO$-Ph | 214-216 |
| I - 289 | 2-Cl-6-F-Ph | 2,5-$F_2$-4-$CF_3SO_2$-Ph | |
| I - 290 | 2-Cl-6-F-Ph | 3,5-$F_2$-4-$CF_3S$-Ph | 111 |
| I - 291 | 2-Cl-6-F-Ph | 3,5-$F_2$-4-$CF_3SO$-Ph | |
| I - 292 | 2-Cl-6-F-Ph | 3,5-$F_2$-4-$CF_3SO_2$-Ph | |
| I - 293 | 2-Cl-6-F-Ph | 2,3,6-$F_3$-4-$CF_3S$-Ph | 118 |
| I - 294 | 2-Cl-6-F-Ph | 2,3,6-$F_3$-4-$CF_3SO$-Ph | 146-147 |
| I - 295 | 2-Cl-6-F-Ph | 2,3,6-$F_3$-4-$CF_3SO_2$-Ph | |
| I - 296 | 2-Cl-6-F-Ph | 2,3,5,6-$F_4$-4-$CF_3S$-Ph | 126 |
| I - 297 | 2-Cl-6-F-Ph | 2,3,5,6-$F_4$-4-$CF_3SO$-Ph | |
| I - 298 | 2-Cl-6-F-Ph | 2,3,5,6-$F_4$-4-$CF_3SO_2$-Ph | |
| I - 299 | 2-Cl-6-F-Ph | 3-Cl-2-Me-4-$CF_3S$-Ph | 138 |
| I - 300 | 2-Cl-6-F-Ph | 3-Cl-2-Me-4-$CF_3SO$-Ph | 237 |

| compound No. | $Q_1$ | $Q_2$ | m. p. (°C) |
|---|---|---|---|
| I - 301 | 2-Cl-6-F-Ph | 3-Cl-2-Me-4-$CF_3SO_2$-Ph | |
| I - 302 | 2-Cl-6-F-Ph | 5-F-2-Me-4-$CF_3S$-Ph | 177 |
| I - 303 | 2-Cl-6-F-Ph | 5-F-2-Me-4-$CF_3SO$-Ph | 226-228 |
| I - 304 | 2-Cl-6-F-Ph | 5-F-2-Me-4-$CF_3SO_2$-Ph | |
| I - 305 | 2-Cl-6-F-Ph | 2-F-4-$CF_3CF_2S$-Ph | 77-79 |
| I - 306 | 2-Cl-6-F-Ph | 2-F-4-$CF_3CF_2SO$-Ph | 40-43 |
| I - 307 | 2-Cl-6-F-Ph | 2-F-4-$CF_3CF_2SO_2$-Ph | 119-120 |
| I - 308 | 2-Cl-6-F-Ph | 2-Cl-4-$CF_3CF_2S$-Ph | |
| I - 309 | 2-Cl-6-F-Ph | 2-Cl-4-$CF_3CF_2SO$-Ph | |
| I - 310 | 2-Cl-6-F-Ph | 2-Cl-4-$CF_3CF_2SO_2$-Ph | |
| I - 311 | 2-Cl-6-F-Ph | 2-Me-4-$CF_3CF_2S$-Ph | |
| I - 312 | 2-Cl-6-F-Ph | 2-Me-4-$CF_3CF_2SO$-Ph | |
| I - 313 | 2-Cl-6-F-Ph | 2-Me-4-$CF_3CF_2SO_2$-Ph | |
| I - 314 | 2-Cl-6-F-Ph | 5-Cl-2-Me-4-$CF_3CF_2S$-Ph | |
| I - 315 | 2-Cl-6-F-Ph | 5-Cl-2-Me-4-$CF_3CF_2SO$-Ph | |
| I - 316 | 2-Cl-6-F-Ph | 5-Cl-2-Me-4-$CF_3CF_2SO_2$-Ph | |
| I - 317 | 2-Cl-6-F-Ph | 2,3-$Me_2$-4-$CF_3CF_2S$-Ph | |
| I - 318 | 2-Cl-6-F-Ph | 2,3-$Me_2$-4-$CF_3CF_2SO$-Ph | |
| I - 319 | 2-Cl-6-F-Ph | 2,3-$Me_2$-4-$CF_3CF_2SO_2$-Ph | |
| I - 320 | 2-Cl-6-F-Ph | 2-F-4-$CF_3CF_2CF_2S$-Ph | 79-81 |
| I - 321 | 2-Cl-6-F-Ph | 2-F-4-$CF_3CF_2CF_2SO$-Ph | 109-112 |
| I - 322 | 2-Cl-6-F-Ph | 2-F-4-$CF_3CF_2CF_2SO_2$-Ph | |
| I - 323 | 2-Cl-6-F-Ph | 2-Cl-4-$CF_3CF_2CF_2S$-Ph | 144-145 |
| I - 324 | 2-Cl-6-F-Ph | 2-Cl-4-$CF_3CF_2CF_2SO$-Ph | 142-143 |
| I - 325 | 2-Cl-6-F-Ph | 2-Cl-4-$CF_3CF_2CF_2SO_2$-Ph | |
| I - 326 | 2-Cl-6-F-Ph | 2-Me-4-$CF_3CF_2CF_2S$-Ph | |
| I - 327 | 2-Cl-6-F-Ph | 2-Me-4-$CF_3CF_2CF_2SO$-Ph | |
| I - 328 | 2-Cl-6-F-Ph | 2-Me-4-$CF_3CF_2CF_2SO_2$-Ph | |
| I - 329 | 2-Cl-6-F-Ph | 5-Cl-2-Me-4-$CF_3CF_2CF_2S$-Ph | 153 |
| I - 330 | 2-Cl-6-F-Ph | 5-Cl-2-Me-4-$CF_3CF_2CF_2SO$-Ph | 158 |
| I - 331 | 2-Cl-6-F-Ph | 5-Cl-2-Me-4-$CF_3CF_2CF_2SO_2$-Ph | |
| I - 332 | 2-Cl-6-F-Ph | 2,3-$Me_2$-4-$CF_3CF_2CF_2S$-Ph | |
| I - 333 | 2-Cl-6-F-Ph | 2,3-$Me_2$-4-$CF_3CF_2CF_2SO$-Ph | |
| I - 334 | 2-Cl-6-F-Ph | 2,3-$Me_2$-4-$CF_3CF_2CF_2SO_2$-Ph | |
| I - 335 | 2-Cl-6-F-Ph | 2-F-4-$(CF_3)_2CFS$-Ph | 134-135 |
| I - 336 | 2-Cl-6-F-Ph | 2-F-4-$(CF_3)_2CFSO$-Ph | 106-110 |
| I - 337 | 2-Cl-6-F-Ph | 2-F-4-$(CF_3)_2CFSO_2$-Ph | |
| I - 338 | 2-Cl-6-F-Ph | 2-Me-4-$(CF_3)_2CFS$-Ph | |
| I - 339 | 2-Cl-6-F-Ph | 2-Me-4-$(CF_3)_2CFSO$-Ph | |
| I - 340 | 2-Cl-6-F-Ph | 2-Me-4-$(CF_3)_2CFSO_2$-Ph | |

| compound No. | $Q_1$ | $Q_2$ | m. p. (°C) |
|---|---|---|---|
| I - 341 | 2-Cl-6-F-Ph | 2-Cl-4-$(CF_3)_2CFS$-Ph | |
| I - 342 | 2-Cl-6-F-Ph | 2-Cl-4-$(CF_3)_2CFSO$-Ph | |
| I - 343 | 2-Cl-6-F-Ph | 2-Cl-4-$(CF_3)_2CFSO_2$-Ph | |
| I - 344 | 2-Cl-6-F-Ph | 5-Cl-2-Me-4-$(CF_3)_2CFS$-Ph | |
| I - 345 | 2-Cl-6-F-Ph | 5-Cl-2-Me-4-$(CF_3)_2CFSO$-Ph | |
| I - 346 | 2-Cl-6-F-Ph | 5-Cl-2-Me-4-$(CF_3)_2CFSO_2$-Ph | |
| I - 347 | 2-Cl-6-F-Ph | 2,3-$Me_2$-4-$(CF_3)_2CFS$-Ph | |
| I - 348 | 2-Cl-6-F-Ph | 2,3-$Me_2$-4-$(CF_3)_2CFSO$-Ph | |
| I - 349 | 2-Cl-6-F-Ph | 2,3-$Me_2$-4-$(CF_3)_2CFSO_2$-Ph | |
| I - 350 | 2-Cl-6-F-Ph | 2-F-4-MeO-Ph | |
| I - 351 | 2-Cl-6-F-Ph | 2-Me-4-MeO-Ph | |
| I - 352 | 2-Cl-6-F-Ph | 2-F-4-CN-Ph | |
| I - 353 | 2-Cl-6-F-Ph | 2-F-4-COOMe-Ph | |
| I - 354 | 2-Cl-6-F-Ph | 2-F-4-COOEt-Ph | |
| I - 355 | 2,6-$Cl_2$-Ph | 4-Cl-Ph | |
| I - 356 | 2,6-$Cl_2$-Ph | 3,5-$Cl_2$-4-(2-Cl-4-$CF_3$-pyridin-2yl-O)-Ph | |
| I - 357 | 2,6-$Cl_2$-Ph | 2-F-4-(2-Cl-4-$CF_3$-Ph-O)-Ph | |
| I - 358 | 2,6-$Cl_2$-Ph | 3,5-$Cl_2$-2,4-$F_2$-Ph | |
| I - 359 | 2,6-$Cl_2$-Ph | 3,5-$Cl_2$-4-$CF_2HCF_2O$-Ph | |
| I - 360 | 2,6-$Cl_2$-Ph | 2,5-$Cl_2$-4-$CF_3CFHCF_2O$-Ph | |
| I - 361 | 2,6-$Cl_2$-Ph | 2-F-4-$CFClHCF_2S$-Ph | |
| I - 362 | 2,6-$Cl_2$-Ph | 2-F-4-$CFClHCF_2SO$-Ph | |
| I - 363 | 2,6-$Cl_2$-Ph | 2-F-4-$CFClHCF_2SO_2$-Ph | |
| I - 364 | 2,6-$Cl_2$-Ph | 4-$CFClHCF_2S$-Ph | |
| I - 365 | 2,6-$Cl_2$-Ph | 4-$CFClHCF_2SO$-Ph | |
| I - 366 | 2,6-$Cl_2$-Ph | 4-$CFClHCF_2SO_2$-Ph | |
| I - 367 | 2,6-$Cl_2$-Ph | 2-Cl-4-$CFClHCF_2S$-Ph | |
| I - 368 | 2,6-$Cl_2$-Ph | 2-Cl-4-$CFClHCF_2SO$-Ph | |
| I - 369 | 2,6-$Cl_2$-Ph | 2-Cl-4-$CFClHCF_2SO_2$-Ph | |
| I - 370 | 2,6-$Cl_2$-Ph | 2-Cl-4-$CF_2HCF_2S$-Ph | |
| I - 371 | 2,6-$Cl_2$-Ph | 2-Cl-4-$CF_2HCF_2SO$-Ph | |
| I - 372 | 2,6-$Cl_2$-Ph | 2-Cl-4-$CF_2HCF_2SO_2$-Ph | |
| I - 373 | 2,6-$Cl_2$-Ph | 4-$CF_2HCF_2S$-Ph | |
| I - 374 | 2,6-$Cl_2$-Ph | 4-$CF_2HCF_2SO$-Ph | |
| I - 375 | 2,6-$Cl_2$-Ph | 4-$CF_2HCF_2SO_2$-Ph | |
| I - 376 | 2,6-$Cl_2$-Ph | 2-F-4-$CF_2HCF_2S$-Ph | |
| I - 377 | 2,6-$Cl_2$-Ph | 2-F-4-$CF_2HCF_2SO$-Ph | |
| I - 378 | 2,6-$Cl_2$-Ph | 2-F-4-$CF_2HCF_2SO_2$-Ph | |
| I - 379 | 2,6-$Cl_2$-Ph | 4-$CF_3S$-Ph | |
| I - 380 | 2,6-$Cl_2$-Ph | 4-$CF_3SO$-Ph | |

| compound No. | $Q_1$ | $Q_2$ | m. p. (°C) |
|---|---|---|---|
| I - 381 | 2,6-$Cl_2$-Ph | 4-$CF_3SO_2$-Ph | |
| I - 382 | 2,6-$Cl_2$-Ph | 2-F-4-MeS-Ph | |
| I - 383 | 2,6-$Cl_2$-Ph | 2-F-4-MeSO-Ph | |
| I - 384 | 2,6-$Cl_2$-Ph | 2-F-4-$MeSO_2$-Ph | |
| I - 385 | 2,6-$Cl_2$-Ph | 2,6-$F_2$-4-MeS-Ph | |
| I - 386 | 2,6-$Cl_2$-Ph | 2,6-$F_2$-4-MeSO-Ph | |
| I - 387 | 2,6-$Cl_2$-Ph | 2,6-$F_2$-4-$MeSO_2$-Ph | |
| I - 388 | 2,6-$Cl_2$-Ph | 2-F-4-$CF_3CH_2S$-Ph | |
| I - 389 | 2,6-$Cl_2$-Ph | 2-F-4-$CF_3CH_2SO$-Ph | |
| I - 390 | 2,6-$Cl_2$-Ph | 2-F-4-$CF_3CH_2SO_2$-Ph | |
| I - 391 | 2,6-$Cl_2$-Ph | 2-F-4-$CCl_2$=$CHCH_2S$-Ph | |
| I - 392 | 2,6-$Cl_2$-Ph | 2-F-4-$CCl_2$=$CHCH_2SO$-Ph | |
| I - 393 | 2,6-$Cl_2$-Ph | 2-F-4-$CCl_2$=$CHCH_2SO_2$-Ph | |
| I - 394 | 2,6-$Cl_2$-Ph | $CF_3CH_2$ | |
| I - 395 | 2,6-$Cl_2$-Ph | 2-F-4-$CF_2HS$-Ph | 153-155 |
| I - 396 | 2,6-$Cl_2$-Ph | 2-F-4-$CF_2HSO$-Ph | 143-145 |
| I - 397 | 2,6-$Cl_2$-Ph | 2-F-4-$CF_2HSO_2$-Ph | |
| I - 398 | 2,6-$Cl_2$-Ph | 2-F-4-$CF_3S$-Ph | 130-132 |
| I - 399 | 2,6-$Cl_2$-Ph | 2-F-4-$CF_3SO$-Ph | 172-175 |
| I - 400 | 2,6-$Cl_2$-Ph | 2-F-4-$CF_3SO_2$-Ph | |
| I - 401 | 2,6-$Cl_2$-Ph | 2-F-4-$CH_3CH_2S$-Ph | |
| I - 402 | 2,6-$Cl_2$-Ph | 2-F-4-$CH_3CH_2SO$-Ph | |
| I - 403 | 2,6-$Cl_2$-Ph | 2-F-4-$CH_3CH_2SO_2$-Ph | |
| I - 404 | 2,6-$Cl_2$-Ph | 2-F-4-$CH_3CH_2CH_2S$-Ph | |
| I - 405 | 2,6-$Cl_2$-Ph | 2-F-4-$CH_3CH_2CH_2SO$-Ph | |
| I - 406 | 2,6-$Cl_2$-Ph | 2-F-4-$CH_3CH_2CH_2SO_2$-Ph | |
| I - 407 | 2,6-$Cl_2$-Ph | 2-F-4-i-PrS-Ph | |
| I - 408 | 2,6-$Cl_2$-Ph | 2-F-4-i-PrSO-Ph | |
| I - 409 | 2,6-$Cl_2$-Ph | 2-F-4-i-$PrSO_2$-Ph | |
| I - 410 | 2,6-$Cl_2$-Ph | 2-F-4-t-BuS-Ph | |
| I - 411 | 2,6-$Cl_2$-Ph | 2-F-4-t-BuSO-Ph | |
| I - 412 | 2,6-$Cl_2$-Ph | 2-F-4-t-$BuSO_2$-Ph | |
| I - 413 | 2,6-$Cl_2$-Ph | 2-F-4-$CH_2$=$CHCH_2S$-Ph | |
| I - 414 | 2,6-$Cl_2$-Ph | 2-F-4-$CH_2$=$CHCH_2SO$-Ph | |
| I - 415 | 2,6-$Cl_2$-Ph | 2-F-4-$CH_2$=$CHCH_2SO_2$-Ph | |
| I - 416 | 2,6-$Cl_2$-Ph | 2-F-4-CH≡$CCH_2S$-Ph | |
| I - 417 | 2,6-$Cl_2$-Ph | 2-F-4-$CHCCH_2SO$-Ph | |
| I - 418 | 2,6-$Cl_2$-Ph | 2-F-4-$CHCCH_2SO_2$-Ph | |
| I - 419 | 2,6-$Cl_2$-Ph | 2-F-4-(2-Cl-4-$CF_3$-pyridin-2yl-S)-Ph | |
| I - 420 | 2,6-$Cl_2$-Ph | 2-F-4-(2-Cl-4-$CF_3$-pyridin-2yl-SO)-Ph | |

| compound No. | $Q_1$ | $Q_2$ | m. p. (°C) |
|---|---|---|---|
| I - 421 | 2,6-$Cl_2$-Ph | 2-F-4-(2-Cl-4-$CF_3$-pyridin-2yl-$SO_2$)-Ph | |
| I - 422 | 2,6-$Cl_2$-Ph | 2-Me-4-$CF_3$S-Ph | |
| I - 423 | 2,6-$Cl_2$-Ph | 2-Me-4-$CF_3$SO-Ph | |
| I - 424 | 2,6-$Cl_2$-Ph | 2-Me-4-$CF_3SO_2$-Ph | |
| I - 425 | 2,6-$Cl_2$-Ph | 3-Me-4-$CF_3$S-Ph | |
| I - 426 | 2,6-$Cl_2$-Ph | 3-Me-4-$CF_3$SO-Ph | |
| I - 427 | 2,6-$Cl_2$-Ph | 3-Me-4-$CF_3SO_2$-Ph | |
| I - 428 | 2,6-$Cl_2$-Ph | 2-Cl-4-$CF_3$S-Ph | |
| I - 429 | 2,6-$Cl_2$-Ph | 2-Cl-4-$CF_3$SO-Ph | |
| I - 430 | 2,6-$Cl_2$-Ph | 2-Cl-4-$CF_3SO_2$-Ph | |
| I - 431 | 2,6-$Cl_2$-Ph | 3-Cl-4-$CF_3$S-Ph | |
| I - 432 | 2,6-$Cl_2$-Ph | 3-Cl-4-$CF_3$SO-Ph | |
| I - 433 | 2,6-$Cl_2$-Ph | 3-Cl-4-$CF_3SO_2$-Ph | |
| I - 434 | 2,6-$Cl_2$-Ph | 3-F-4-$CF_3$S-Ph | |
| I - 435 | 2,6-$Cl_2$-Ph | 3-F-4-$CF_3$SO-Ph | |
| I - 436 | 2,6-$Cl_2$-Ph | 3-F-4-$CF_3SO_2$-Ph | |
| I - 437 | 2,6-$Cl_2$-Ph | 2,3-$Me_2$-4-$CF_3$S-Ph | |
| I - 438 | 2,6-$Cl_2$-Ph | 2,3-$Me_2$-4-$CF_3$SO-Ph | |
| I - 439 | 2,6-$Cl_2$-Ph | 2,3-$Me_2$-4-$CF_3SO_2$-Ph | |
| I - 440 | 2,6-$Cl_2$-Ph | 2,5-$Me_2$-4-$CF_3$S-Ph | |
| I - 441 | 2,6-$Cl_2$-Ph | 2,5-$Me_2$-4-$CF_3$SO-Ph | |
| I - 442 | 2,6-$Cl_2$-Ph | 2,5-$Me_2$-4-$CF_3SO_2$-Ph | |
| I - 443 | 2,6-$Cl_2$-Ph | 5-Cl-2-Me-4-$CF_3$S-Ph | |
| I - 444 | 2,6-$Cl_2$-Ph | 5-Cl-2-Me-4-$CF_3$SO-Ph | |
| I - 445 | 2,6-$Cl_2$-Ph | 5-Cl-2-Me-4-$CF_3SO_2$-Ph | |
| I - 446 | 2,6-$Cl_2$-Ph | 2,3-$Cl_2$-4-$CF_3$S-Ph | |
| I - 447 | 2,6-$Cl_2$-Ph | 2,3-$Cl_2$-4-$CF_3$SO-Ph | |
| I - 448 | 2,6-$Cl_2$-Ph | 2,3-$Cl_2$-4-$CF_3SO_2$-Ph | |
| I - 449 | 2,6-$Cl_2$-Ph | 3-Cl-2-F-4-$CF_3$S-Ph | |
| I - 450 | 2,6-$Cl_2$-Ph | 3-Cl-2-F-4-$CF_3$SO-Ph | |
| I - 451 | 2,6-$Cl_2$-Ph | 3-Cl-2-F-4-$CF_3SO_2$-Ph | |
| I - 452 | 2,6-$Cl_2$-Ph | 5-Cl-2-F-4-$CF_3$S-Ph | |
| I - 453 | 2,6-$Cl_2$-Ph | 5-Cl-2-F-4-$CF_3$SO-Ph | |
| I - 454 | 2,6-$Cl_2$-Ph | 5-Cl-2-F-4-$CF_3SO_2$-Ph | |
| I - 455 | 2,6-$Cl_2$-Ph | 2,6-$F_2$-4-$CF_3$S-Ph | |
| I - 456 | 2,6-$Cl_2$-Ph | 2,6-$F_2$-4-$CF_3$SO-Ph | |
| I - 457 | 2,6-$Cl_2$-Ph | 2,6-$F_2$-4-$CF_3SO_2$-Ph | |
| I - 458 | 2,6-$Cl_2$-Ph | 2,3-$F_2$-4-$CF_3$S-Ph | |
| I - 459 | 2,6-$Cl_2$-Ph | 2,3-$F_2$-4-$CF_3$SO-Ph | |
| I - 460 | 2,6-$Cl_2$-Ph | 2,3-$F_2$-4-$CF_3SO_2$-Ph | |

| compound No. | $Q_1$ | $Q_2$ | m. p. (°C) |
|---|---|---|---|
| I - 461 | 2,6-$Cl_2$-Ph | 2,5-$F_2$-4-$CF_3S$-Ph | |
| I - 462 | 2,6-$Cl_2$-Ph | 2,5-$F_2$-4-$CF_3SO$-Ph | |
| I - 463 | 2,6-$Cl_2$-Ph | 2,5-$F_2$-4-$CF_3SO_2$-Ph | |
| I - 464 | 2,6-$Cl_2$-Ph | 3,5-$F_2$-4-$CF_3S$-Ph | |
| I - 465 | 2,6-$Cl_2$-Ph | 3,5-$F_2$-4-$CF_3SO$-Ph | |
| I - 466 | 2,6-$Cl_2$-Ph | 3,5-$F_2$-4-$CF_3SO_2$-Ph | |
| I - 467 | 2,6-$Cl_2$-Ph | 2,3,6-$F_3$-4-$CF_3S$-Ph | |
| I - 468 | 2,6-$Cl_2$-Ph | 2,3,6-$F_3$-4-$CF_3SO$-Ph | |
| I - 469 | 2,6-$Cl_2$-Ph | 2,3,6-$F_3$-4-$CF_3SO_2$-Ph | |
| I - 470 | 2,6-$Cl_2$-Ph | 2,3,5,6-$F_4$-4-$CF_3S$-Ph | |
| I - 471 | 2,6-$Cl_2$-Ph | 2,3,5,6-$F_4$-4-$CF_3SO$-Ph | |
| I - 472 | 2,6-$Cl_2$-Ph | 2,3,5,6-$F_4$-4-$CF_3SO_2$-Ph | |
| I - 473 | 2,6-$Cl_2$-Ph | 3-Cl-2-Me-4-$CF_3S$-Ph | |
| I - 474 | 2,6-$Cl_2$-Ph | 3-Cl-2-Me-4-$CF_3SO$-Ph | |
| I - 475 | 2,6-$Cl_2$-Ph | 3-Cl-2-Me-4-$CF_3SO_2$-Ph | |
| I - 476 | 2,6-$Cl_2$-Ph | 5-F-2-Me-4-$CF_3S$-Ph | |
| I - 477 | 2,6-$Cl_2$-Ph | 5-F-2-Me-4-$CF_3SO$-Ph | |
| I - 478 | 2,6-$Cl_2$-Ph | 5-F-2-Me-4-$CF_3SO_2$-Ph | |
| I - 479 | 2,6-$Cl_2$-Ph | 2-F-4-$CF_3CF_2S$-Ph | |
| I - 480 | 2,6-$Cl_2$-Ph | 2-F-4-$CF_3CF_2SO$-Ph | |
| I - 481 | 2,6-$Cl_2$-Ph | 2-F-4-$CF_3CF_2SO_2$-Ph | |
| I - 482 | 2,6-$Cl_2$-Ph | 2-Cl-4-$CF_3CF_2S$-Ph | |
| I - 483 | 2,6-$Cl_2$-Ph | 2-Cl-4-$CF_3CF_2SO$-Ph | |
| I - 484 | 2,6-$Cl_2$-Ph | 2-Cl-4-$CF_3CF_2SO_2$-Ph | |
| I - 485 | 2,6-$Cl_2$-Ph | 2-Me-4-$CF_3CF_2S$-Ph | |
| I - 486 | 2,6-$Cl_2$-Ph | 2-Me-4-$CF_3CF_2SO$-Ph | |
| I - 487 | 2,6-$Cl_2$-Ph | 2-Me-4-$CF_3CF_2SO_2$-Ph | |
| I - 488 | 2,6-$Cl_2$-Ph | 5-Cl-2-Me-4-$CF_3CF_2S$-Ph | |
| I - 489 | 2,6-$Cl_2$-Ph | 5-Cl-2-Me-4-$CF_3CF_2SO$-Ph | |
| I - 490 | 2,6-$Cl_2$-Ph | 5-Cl-2-Me-4-$CF_3CF_2SO_2$-Ph | |
| I - 491 | 2,6-$Cl_2$-Ph | 2,3-$Me_2$-4-$CF_3CF_2S$-Ph | |
| I - 492 | 2,6-$Cl_2$-Ph | 2,3-$Me_2$-4-$CF_3CF_2SO$-Ph | |
| I - 493 | 2,6-$Cl_2$-Ph | 2,3-$Me_2$-4-$CF_3CF_2SO_2$-Ph | |
| I - 494 | 2,6-$Cl_2$-Ph | 2-F-4-$CF_3CF_2CF_2S$-Ph | |
| I - 495 | 2,6-$Cl_2$-Ph | 2-F-4-$CF_3CF_2CF_2SO$-Ph | |
| I - 496 | 2,6-$Cl_2$-Ph | 2-F-4-$CF_3CF_2CF_2SO_2$-Ph | |
| I - 497 | 2,6-$Cl_2$-Ph | 2-Cl-4-$CF_3CF_2CF_2S$-Ph | |
| I - 498 | 2,6-$Cl_2$-Ph | 2-Cl-4-$CF_3CF_2CF_2SO$-Ph | |
| I - 499 | 2,6-$Cl_2$-Ph | 2-Cl-4-$CF_3CF_2CF_2SO_2$-Ph | |
| I - 500 | 2,6-$Cl_2$-Ph | 2-Me-4-$CF_3CF_2CF_2S$-Ph | |

| compound No. | $Q_1$ | $Q_2$ | m. p. (°C) |
|---|---|---|---|
| I - 501 | 2,6-$Cl_2$-Ph | 2-Me-4-$CF_3CF_2CF_2SO$-Ph | |
| I - 502 | 2,6-$Cl_2$-Ph | 2-Me-4-$CF_3CF_2CF_2SO_2$-Ph | |
| I - 503 | 2,6-$Cl_2$-Ph | 5-Cl-2-Me-4-$CF_3CF_2CF_2S$-Ph | |
| I - 504 | 2,6-$Cl_2$-Ph | 5-Cl-2-Me-4-$CF_3CF_2CF_2SO$-Ph | |
| I - 505 | 2,6-$Cl_2$-Ph | 5-Cl-2-Me-4-$CF_3CF_2CF_2SO_2$-Ph | |
| I - 506 | 2,6-$Cl_2$-Ph | 2,3-$Me_2$-4-$CF_3CF_2CF_2S$-Ph | |
| I - 507 | 2,6-$Cl_2$-Ph | 2,3-$Me_2$-4-$CF_3CF_2CF_2SO$-Ph | |
| I - 508 | 2,6-$Cl_2$-Ph | 2,3-$Me_2$-4-$CF_3CF_2CF_2SO_2$-Ph | |
| I - 509 | 2,6-$Cl_2$-Ph | 2-F-4-$(CF_3)_2CFS$-Ph | |
| I - 510 | 2,6-$Cl_2$-Ph | 2-F-4-$(CF_3)_2CFSO$-Ph | |
| I - 511 | 2,6-$Cl_2$-Ph | 2-F-4-$(CF_3)_2CFSO_2$-Ph | |
| I - 512 | 2,6-$Cl_2$-Ph | 2-Me-4-$(CF_3)_2CFS$-Ph | |
| I - 513 | 2,6-$Cl_2$-Ph | 2-Me-4-$(CF_3)_2CFSO$-Ph | |
| I - 514 | 2,6-$Cl_2$-Ph | 2-Me-4-$(CF_3)_2CFSO_2$-Ph | |
| I - 515 | 2,6-$Cl_2$-Ph | 2-Cl-4-$(CF_3)_2CFS$-Ph | |
| I - 516 | 2,6-$Cl_2$-Ph | 2-Cl-4-$(CF_3)_2CFSO$-Ph | |
| I - 517 | 2,6-$Cl_2$-Ph | 2-Cl-4-$(CF_3)_2CFSO_2$-Ph | |
| I - 518 | 2,6-$Cl_2$-Ph | 5-Cl-2-Me-4-$(CF_3)_2CFS$-Ph | |
| I - 519 | 2,6-$Cl_2$-Ph | 5-Cl-2-Me-4-$(CF_3)_2CFSO$-Ph | |
| I - 520 | 2,6-$Cl_2$-Ph | 5-Cl-2-Me-4-$(CF_3)_2CFSO_2$-Ph | |
| I - 521 | 2,6-$Cl_2$-Ph | 2,3-$Me_2$-4-$(CF_3)_2CFS$-Ph | |
| I - 522 | 2,6-$Cl_2$-Ph | 2,3-$Me_2$-4-$(CF_3)_2CFSO$-Ph | |
| I - 523 | 2,6-$Cl_2$-Ph | 2,3-$Me_2$-4-$(CF_3)_2CFSO_2$-Ph | |
| I - 524 | 2,6-$Cl_2$-Ph | 2-F-4-MeO-Ph | |
| I - 525 | 2,6-$Cl_2$-Ph | 2-Me-4-MeO-Ph | |
| I - 526 | 2,6-$Cl_2$-Ph | 2-F-4-CN-Ph | |
| I - 527 | 2,6-$Cl_2$-Ph | 2-F-4-COOMe-Ph | |
| I - 528 | 2,6-$Cl_2$-Ph | 2-F-4-COOEt-Ph | |
| I - 529 | 3-Cl-pyridin-2yl | 4-Cl-Ph | amorphous (27) |
| I - 530 | 3-Cl-pyridin-2yl | 3,5-$Cl_2$-4-(2-Cl-4-$CF_3$-pyridin-2yl-O)-Ph | |
| I - 531 | 3-Cl-pyridin-2yl | 2-F-4-(2-Cl-4-$CF_3$-Ph-O)-Ph | |
| I - 532 | 3-Cl-pyridin-2yl | 3,5-$Cl_2$-2,4-$F_2$-Ph | |
| I - 533 | 3-Cl-pyridin-2yl | 3,5-$Cl_2$-4-$CF_2HCF_2O$-Ph | |
| I - 534 | 3-Cl-pyridin-2yl | 2,5-$Cl_2$-4-$CF_3CFHCF_2O$-Ph | |
| I - 535 | 3-Cl-pyridin-2yl | 2-F-4-$CFClHCF_2S$-Ph | |
| I - 536 | 3-Cl-pyridin-2yl | 2-F-4-$CFClHCF_2SO$-Ph | |
| I - 537 | 3-Cl-pyridin-2yl | 2-F-4-$CFClHCF_2SO_2$-Ph | |
| I - 538 | 3-Cl-pyridin-2yl | 4-$CFClHCF_2S$-Ph | |
| I - 539 | 3-Cl-pyridin-2yl | 4-$CFClHCF_2SO$-Ph | |
| I - 540 | 3-Cl-pyridin-2yl | 4-$CFClHCF_2SO_2$-Ph | |

(27) $H^1$-NMR ($CDCl_3$)δppm : 5.18 ( 2H, s, $CH_2$), 5.62 ( 2H, s, $CH_2$), 7.16 (2H, m, Ph), 7.25 ( 1H, dd J=8.2,4.7Hz, Py), 7.33 ( 2H, m, Ph), 7.70 ( 1H, dd J=8.2,1.3Hz, Py), 8.42 ( 1H, dd J=4.7,1.3Hz, Py) .

| compound No. | $Q_1$ | $Q_2$ | m. p. (°C) |
|---|---|---|---|
| I - 541 | 3-Cl-pyridin-2yl | 2-Cl-4-CFClHCF$_2$S-Ph | |
| I - 542 | 3-Cl-pyridin-2yl | 2-Cl-4-CFClHCF$_2$SO-Ph | |
| I - 543 | 3-Cl-pyridin-2yl | 2-Cl-4-CFClHCF$_2$SO$_2$-Ph | |
| I - 544 | 3-Cl-pyridin-2yl | 2-Cl-4-CF$_2$HCF$_2$S-Ph | |
| I - 545 | 3-Cl-pyridin-2yl | 2-Cl-4-CF$_2$HCF$_2$SO-Ph | |
| I - 546 | 3-Cl-pyridin-2yl | 2-Cl-4-CF$_2$HCF$_2$SO$_2$-Ph | |
| I - 547 | 3-Cl-pyridin-2yl | 4-CF$_2$HCF$_2$S-Ph | |
| I - 548 | 3-Cl-pyridin-2yl | 4-CF$_2$HCF$_2$SO-Ph | |
| I - 549 | 3-Cl-pyridin-2yl | 4-CF$_2$HCF$_2$SO$_2$-Ph | |
| I - 550 | 3-Cl-pyridin-2yl | 2-F-4-CF$_2$HCF$_2$S-Ph | |
| I - 551 | 3-Cl-pyridin-2yl | 2-F-4-CF$_2$HCF$_2$SO-Ph | |
| I - 552 | 3-Cl-pyridin-2yl | 2-F-4-CF$_2$HCF$_2$SO$_2$-Ph | |
| I - 553 | 3-Cl-pyridin-2yl | 4-CF$_3$S-Ph | |
| I - 554 | 3-Cl-pyridin-2yl | 4-CF$_3$SO-Ph | |
| I - 555 | 3-Cl-pyridin-2yl | 4-CF$_3$SO$_2$-Ph | |
| I - 556 | 3-Cl-pyridin-2yl | 2-F-4-MeS-Ph | |
| I - 557 | 3-Cl-pyridin-2yl | 2-F-4-MeSO-Ph | |
| I - 558 | 3-Cl-pyridin-2yl | 2-F-4-MeSO$_2$-Ph | |
| I - 559 | 3-Cl-pyridin-2yl | 2,6-F$_2$-4-MeS-Ph | |
| I - 560 | 3-Cl-pyridin-2yl | 2,6-F$_2$-4-MeSO-Ph | |
| I - 561 | 3-Cl-pyridin-2yl | 2,6-F$_2$-4-MeSO$_2$-Ph | |
| I - 562 | 3-Cl-pyridin-2yl | 2-F-4-CF$_3$CH$_2$S-Ph | |
| I - 563 | 3-Cl-pyridin-2yl | 2-F-4-CF$_3$CH$_2$SO-Ph | |
| I - 564 | 3-Cl-pyridin-2yl | 2-F-4-CF$_3$CH$_2$SO$_2$-Ph | |
| I - 565 | 3-Cl-pyridin-2yl | 2-F-4-CCl$_2$=CHCH$_2$S-Ph | |
| I - 566 | 3-Cl-pyridin-2yl | 2-F-4-CCl$_2$=CHCH$_2$SO-Ph | |
| I - 567 | 3-Cl-pyridin-2yl | 2-F-4-CCl$_2$=CHCH$_2$SO$_2$-Ph | |
| I - 568 | 3-Cl-pyridin-2yl | CF$_3$CH$_2$ | |
| I - 569 | 3-Cl-pyridin-2yl | 2-F-4-CF$_2$HS-Ph | |
| I - 570 | 3-Cl-pyridin-2yl | 2-F-4-CF$_2$HSO-Ph | |
| I - 571 | 3-Cl-pyridin-2yl | 2-F-4-CF$_2$HSO$_2$-Ph | |
| I - 572 | 3-Cl-pyridin-2yl | 2-F-4-CF$_3$S-Ph | amorphous (28) |
| I - 573 | 3-Cl-pyridin-2yl | 2-F-4-CF$_3$SO-Ph | |
| I - 574 | 3-Cl-pyridin-2yl | 2-F-4-CF$_3$SO$_2$-Ph | |
| I - 575 | 3-Cl-pyridin-2yl | 2-F-4-CH$_3$CH$_2$S-Ph | |
| I - 576 | 3-Cl-pyridin-2yl | 2-F-4-CH$_3$CH$_2$SO-Ph | |
| I - 577 | 3-Cl-pyridin-2yl | 2-F-4-CH$_3$CH$_2$SO$_2$-Ph | |
| I - 578 | 3-Cl-pyridin-2yl | 2-F-4-CH$_3$CH$_2$CH$_2$S-Ph | |
| I - 579 | 3-Cl-pyridin-2yl | 2-F-4-CH$_3$CH$_2$CH$_2$SO-Ph | |
| I - 580 | 3-Cl-pyridin-2yl | 2-F-4-CH$_3$CH$_2$CH$_2$SO$_2$-Ph | |

(28) $H^1$-NMR (CDCl$_3$)δppm : 5.17 ( 2H, s, CH$_2$), 5.65 ( 2H, s, CH$_2$), 7.27 ( 1H, dd J=8.2,4.8Hz, Py), 7.37-7.46 ( 3H, m, Ph), 7.71 ( 1H, dd J=8.2,1.3Hz, Py), 8.45 ( 1H, dd J=4.8,1.3Hz, Py) .

| compound No. | $Q_1$ | $Q_2$ | m. p. (°C) |
|---|---|---|---|
| I - 581 | 3-Cl-pyridin-2yl | 2-F-4-i-PrS-Ph | |
| I - 582 | 3-Cl-pyridin-2yl | 2-F-4-i-PrSO-Ph | |
| I - 583 | 3-Cl-pyridin-2yl | 2-F-4-i-PrSO$_2$-Ph | |
| I - 584 | 3-Cl-pyridin-2yl | 2-F-4-t-BuS-Ph | |
| I - 585 | 3-Cl-pyridin-2yl | 2-F-4-t-BuSO-Ph | |
| I - 586 | 3-Cl-pyridin-2yl | 2-F-4-t-BuSO$_2$-Ph | |
| I - 587 | 3-Cl-pyridin-2yl | 2-F-4-CH$_2$=CHCH$_2$S-Ph | |
| I - 588 | 3-Cl-pyridin-2yl | 2-F-4-CH$_2$=CHCH$_2$SO-Ph | |
| I - 589 | 3-Cl-pyridin-2yl | 2-F-4-CH$_2$=CHCH$_2$SO$_2$-Ph | |
| I - 590 | 3-Cl-pyridin-2yl | 2-F-4-CH≡CCH$_2$S-Ph | |
| I - 591 | 3-Cl-pyridin-2yl | 2-F-4-CH≡CCH$_2$SO-Ph | |
| I - 592 | 3-Cl-pyridin-2yl | 2-F-4-CH≡CCH$_2$SO$_2$-Ph | |
| I - 593 | 3-Cl-pyridin-2yl | 2-F-4-(2-Cl-4-CF$_3$-pyridin-2yl-S)-Ph | |
| I - 594 | 3-Cl-pyridin-2yl | 2-F-4-(2-Cl-4-CF$_3$-pyridin-2yl-SO)-Ph | |
| I - 595 | 3-Cl-pyridin-2yl | 2-F-4-(2-Cl-4-CF$_3$-pyridin-2yl-SO$_2$)-Ph | |
| I - 596 | 3-Cl-pyridin-2yl | 2-Me-4-CF$_3$S-Ph | |
| I - 597 | 3-Cl-pyridin-2yl | 2-Me-4-CF$_3$SO-Ph | |
| I - 598 | 3-Cl-pyridin-2yl | 2-Me-4-CF$_3$SO$_2$-Ph | |
| I - 599 | 3-Cl-pyridin-2yl | 3-Me-4-CF$_3$S-Ph | |
| I - 600 | 3-Cl-pyridin-2yl | 3-Me-4-CF$_3$SO-Ph | |
| I - 601 | 3-Cl-pyridin-2yl | 3-Me-4-CF$_3$SO$_2$-Ph | |
| I - 602 | 3-Cl-pyridin-2yl | 2-Cl-4-CF$_3$S-Ph | |
| I - 603 | 3-Cl-pyridin-2yl | 2-Cl-4-CF$_3$SO-Ph | |
| I - 604 | 3-Cl-pyridin-2yl | 2-Cl-4-CF$_3$SO$_2$-Ph | |
| I - 605 | 3-Cl-pyridin-2yl | 3-Cl-4-CF$_3$S-Ph | |
| I - 606 | 3-Cl-pyridin-2yl | 3-Cl-4-CF$_3$SO-Ph | |
| I - 607 | 3-Cl-pyridin-2yl | 3-Cl-4-CF$_3$SO$_2$-Ph | |
| I - 608 | 3-Cl-pyridin-2yl | 3-F-4-CF$_3$S-Ph | |
| I - 609 | 3-Cl-pyridin-2yl | 3-F-4-CF$_3$SO-Ph | |
| I - 610 | 3-Cl-pyridin-2yl | 3-F-4-CF$_3$SO$_2$-Ph | |
| I - 611 | 3-Cl-pyridin-2yl | 2,3-Me$_2$-4-CF$_3$S-Ph | |
| I - 612 | 3-Cl-pyridin-2yl | 2,3-Me$_2$-4-CF$_3$SO-Ph | |
| I - 613 | 3-Cl-pyridin-2yl | 2,3-Me$_2$-4-CF$_3$SO$_2$-Ph | |
| I - 614 | 3-Cl-pyridin-2yl | 2,5-Me$_2$-4-CF$_3$S-Ph | |
| I - 615 | 3-Cl-pyridin-2yl | 2,5-Me$_2$-4-CF$_3$SO-Ph | |
| I - 616 | 3-Cl-pyridin-2yl | 2,5-Me$_2$-4-CF$_3$SO$_2$-Ph | |
| I - 617 | 3-Cl-pyridin-2yl | 5-Cl-2-Me-4-CF$_3$S-Ph | |
| I - 618 | 3-Cl-pyridin-2yl | 5-Cl-2-Me-4-CF$_3$SO-Ph | |
| I - 619 | 3-Cl-pyridin-2yl | 5-Cl-2-Me-4-CF$_3$SO$_2$-Pl | |
| I - 620 | 3-Cl-pyridin-2yl | 2,3-Cl$_2$-4-CF$_3$S-Ph | |

| compound No. | $Q_1$ | $Q_2$ | m. p. (°C) |
|---|---|---|---|
| I - 621 | 3-Cl-pyridin-2yl | 2,3-$Cl_2$-4-$CF_3$SO-Ph | |
| I - 622 | 3-Cl-pyridin-2yl | 2,3-$Cl_2$-4-$CF_3SO_2$-Ph | |
| I - 623 | 3-Cl-pyridin-2yl | 3-Cl-2-F-4-$CF_3$S-Ph | |
| I - 624 | 3-Cl-pyridin-2yl | 3-Cl-2-F-4-$CF_3$SO-Ph | |
| I - 625 | 3-Cl-pyridin-2yl | 3-Cl-2-F-4-$CF_3SO_2$-Ph | |
| I - 626 | 3-Cl-pyridin-2yl | 5-Cl-2-F-4-$CF_3$S-Ph | |
| I - 627 | 3-Cl-pyridin-2yl | 5-Cl-2-F-4-$CF_3$SO-Ph | |
| I - 628 | 3-Cl-pyridin-2yl | 5-Cl-2-F-4-$CF_3SO_2$-Ph | |
| I - 629 | 3-Cl-pyridin-2yl | 2,6-$F_2$-4-$CF_3$S-Ph | |
| I - 630 | 3-Cl-pyridin-2yl | 2,6-$F_2$-4-$CF_3$SO-Ph | |
| I - 631 | 3-Cl-pyridin-2yl | 2,6-$F_2$-4-$CF_3SO_2$-Ph | |
| I - 632 | 3-Cl-pyridin-2yl | 2,3-$F_2$-4-$CF_3$S-Ph | |
| I - 633 | 3-Cl-pyridin-2yl | 2,3-$F_2$-4-$CF_3$SO-Ph | |
| I - 634 | 3-Cl-pyridin-2yl | 2,3-$F_2$-4-$CF_3SO_2$-Ph | |
| I - 635 | 3-Cl-pyridin-2yl | 2,5-$F_2$-4-$CF_3$S-Ph | |
| I - 636 | 3-Cl-pyridin-2yl | 2,5-$F_2$-4-$CF_3$SO-Ph | |
| I - 637 | 3-Cl-pyridin-2yl | 2,5-$F_2$-4-$CF_3SO_2$-Ph | |
| I - 638 | 3-Cl-pyridin-2yl | 3,5-$F_2$-4-$CF_3$S-Ph | |
| I - 639 | 3-Cl-pyridin-2yl | 3,5-$F_2$-4-$CF_3$SO-Ph | |
| I - 640 | 3-Cl-pyridin-2yl | 3,5-$F_2$-4-$CF_3SO_2$-Ph | |
| I - 641 | 3-Cl-pyridin-2yl | 2,3,6-$F_3$-4-$CF_3$S-Ph | |
| I - 642 | 3-Cl-pyridin-2yl | 2,3,6-$F_3$-4-$CF_3$SO-Ph | |
| I - 643 | 3-Cl-pyridin-2yl | 2,3,6-$F_3$-4-$CF_3SO_2$-Ph | |
| I - 644 | 3-Cl-pyridin-2yl | 2,3,5,6-$F_4$-4-$CF_3$S-Ph | |
| I - 645 | 3-Cl-pyridin-2yl | 2,3,5,6-$F_4$-4-$CF_3$SO-Ph | |
| I - 646 | 3-Cl-pyridin-2yl | 2,3,5,6-$F_4$-4-$CF_3SO_2$-Ph | |
| I - 647 | 3-Cl-pyridin-2yl | 3-Cl-2-Me-4-$CF_3$S-Ph | |
| I - 648 | 3-Cl-pyridin-2yl | 3-Cl-2-Me-4-$CF_3$SO-Ph | |
| I - 649 | 3-Cl-pyridin-2yl | 3-Cl-2-Me-4-$CF_3SO_2$-Ph | |
| I - 650 | 3-Cl-pyridin-2yl | 5-F-2-Me-4-$CF_3$S-Ph | |
| I - 651 | 3-Cl-pyridin-2yl | 5-F-2-Me-4-$CF_3$SO-Ph | |
| I - 652 | 3-Cl-pyridin-2yl | 5-F-2-Me-4-$CF_3SO_2$-Ph | |
| I - 653 | 3-Cl-pyridin-2yl | 2-F-4-$CF_3CF_2$S-Ph | |
| I - 654 | 3-Cl-pyridin-2yl | 2-F-4-$CF_3CF_2$SO-Ph | |
| I - 655 | 3-Cl-pyridin-2yl | 2-F-4-$CF_3CF_2SO_2$-Ph | |
| I - 656 | 3-Cl-pyridin-2yl | 2-Cl-4-$CF_3CF_2$S-Ph | |
| I - 657 | 3-Cl-pyridin-2yl | 2-Cl-4-$CF_3CF_2$SO-Ph | |
| I - 658 | 3-Cl-pyridin-2yl | 2-Cl-4-$CF_3CF_2SO_2$-Ph | |
| I - 659 | 3-Cl-pyridin-2yl | 2-Me-4-$CF_3CF_2$S-Ph | |
| I - 660 | 3-Cl-pyridin-2yl | 2-Me-4-$CF_3CF_2$SO-Ph | |

| compound No. | $Q_1$ | $Q_2$ | m. p. (°C) |
|---|---|---|---|
| I - 661 | 3-Cl-pyridin-2yl | 2-Me-4-$CF_3CF_2SO_2$-Ph | |
| I - 662 | 3-Cl-pyridin-2yl | 5-Cl-2-Me-4-$CF_3CF_2S$-Ph | |
| I - 663 | 3-Cl-pyridin-2yl | 5-Cl-2-Me-4-$CF_3CF_2SO$-Ph | |
| I - 664 | 3-Cl-pyridin-2yl | 5-Cl-2-Me-4-$CF_3CF_2SO_2$-Ph | |
| I - 665 | 3-Cl-pyridin-2yl | 2,3-$Me_2$-4-$CF_3CF_2S$-Ph | |
| I - 666 | 3-Cl-pyridin-2yl | 2,3-$Me_2$-4-$CF_3CF_2SO$-Ph | |
| I - 667 | 3-Cl-pyridin-2yl | 2,3-$Me_2$-4-$CF_3CF_2SO_2$-Ph | |
| I - 668 | 3-Cl-pyridin-2yl | 2-F-4-$CF_3CF_2CF_2S$-Ph | |
| I - 669 | 3-Cl-pyridin-2yl | 2-F-4-$CF_3CF_2CF_2SO$-Ph | |
| I - 670 | 3-Cl-pyridin-2yl | 2-F-4-$CF_3CF_2CF_2SO_2$-Ph | |
| I - 671 | 3-Cl-pyridin-2yl | 2-Cl-4-$CF_3CF_2CF_2S$-Ph | |
| I - 672 | 3-Cl-pyridin-2yl | 2-Cl-4-$CF_3CF_2CF_2SO$-Ph | |
| I - 673 | 3-Cl-pyridin-2yl | 2-Cl-4-$CF_3CF_2CF_2SO_2$-Ph | |
| I - 674 | 3-Cl-pyridin-2yl | 2-Me-4-$CF_3CF_2CF_2S$-Ph | |
| I - 675 | 3-Cl-pyridin-2yl | 2-Me-4-$CF_3CF_2CF_2SO$-Ph | |
| I - 676 | 3-Cl-pyridin-2yl | 2-Me-4-$CF_3CF_2CF_2SO_2$-Ph | |
| I - 677 | 3-Cl-pyridin-2yl | 5-Cl-2-Me-4-$CF_3CF_2CF_2S$-Ph | |
| I - 678 | 3-Cl-pyridin-2yl | 5-Cl-2-Me-4-$CF_3CF_2CF_2SO$-Ph | |
| I - 679 | 3-Cl-pyridin-2yl | 5-Cl-2-Me-4-$CF_3CF_2CF_2SO_2$-Ph | |
| I - 680 | 3-Cl-pyridin-2yl | 2,3-$Me_2$-4-$CF_3CF_2CF_2S$-Ph | |
| I - 681 | 3-Cl-pyridin-2yl | 2,3-$Me_2$-4-$CF_3CF_2CF_2SO$-Ph | |
| I - 682 | 3-Cl-pyridin-2yl | 2,3-$Me_2$-4-$CF_3CF_2CF_2SO_2$-Ph | |
| I - 683 | 3-Cl-pyridin-2yl | 2-F-4-$(CF_3)_2CFS$-Ph | |
| I - 684 | 3-Cl-pyridin-2yl | 2-F-4-$(CF_3)_2CFSO$-Ph | |
| I - 685 | 3-Cl-pyridin-2yl | 2-F-4-$(CF_3)_2CFSO_2$-Ph | |
| I - 686 | 3-Cl-pyridin-2yl | 2-Me-4-$(CF_3)_2CFS$-Ph | |
| I - 687 | 3-Cl-pyridin-2yl | 2-Me-4-$(CF_3)_2CFSO$-Ph | |
| I - 688 | 3-Cl-pyridin-2yl | 2-Me-4-$(CF_3)_2CFSO_2$-Ph | |
| I - 689 | 3-Cl-pyridin-2yl | 2-Cl-4-$(CF_3)_2CFS$-Ph | |
| I - 690 | 3-Cl-pyridin-2yl | 2-Cl-4-$(CF_3)_2CFSO$-Ph | |
| I - 691 | 3-Cl-pyridin-2yl | 2-Cl-4-$(CF_3)_2CFSO_2$-Ph | |
| I - 692 | 3-Cl-pyridin-2yl | 5-Cl-2-Me-4-$(CF_3)_2CFS$-Ph | |
| I - 693 | 3-Cl-pyridin-2yl | 5-Cl-2-Me-4-$(CF_3)_2CFSO$-Ph | |
| I - 694 | 3-Cl-pyridin-2yl | 5-Cl-2-Me-4-$(CF_3)_2CFSO_2$-Ph | |
| I - 695 | 3-Cl-pyridin-2yl | 2,3-$Me_2$-4-$(CF_3)_2CFS$-Ph | |
| I - 696 | 3-Cl-pyridin-2yl | 2,3-$Me_2$-4-$(CF_3)_2CFSO$-Ph | |
| I - 697 | 3-Cl-pyridin-2yl | 2,3-$Me_2$-4-$(CF_3)_2CFSO_2$-Ph | |
| I - 698 | 3-Cl-pyridin-2yl | 2-F-4-MeO-Ph | |
| I - 699 | 3-Cl-pyridin-2yl | 2-Me-4-MeO-Ph | |
| I - 700 | 3-Cl-pyridin-2yl | 2-F-4-CN-Ph | |

| compound No. | $Q_1$ | $Q_2$ | m. p. (°C) |
|---|---|---|---|
| I - 701 | 3-Cl-pyridin-2yl | 2-F-4-COOMe-Ph | |
| I - 702 | 3-Cl-pyridin-2yl | 2-F-4-COOEt-Ph | |
| I - 703 | 2,6-$F_2$-Ph | 2-F-4-$CF_3O$-Ph | |
| I - 704 | 2-Cl-6-F-Ph | 2-F-4-$CF_3O$-Ph | |
| I - 705 | 2,6-$Cl_2$-Ph | 2-F-4-$CF_3O$-Ph | |
| I - 706 | 3-Cl-pyridin-2yl | 2-F-4-$CF_3O$-Ph | |
| I - 707 | 2,6-$F_2$-Ph | 2-F-4-$CF_3CFHCF_2S$-Ph | amorphous (29) |
| I - 708 | 2-Cl-6-F-Ph | 2-F-4-$CF_3CFHCF_2S$-Ph | |
| I - 709 | 2,6-$Cl_2$-Ph | 2-F-4-$CF_3CFHCF_2S$-Ph | |
| I - 710 | 3-Cl-pyridin-2yl | 2-F-4-$CF_3CFHCF_2S$-Ph | |
| I - 711 | 2,6-$F_2$-Ph | 2-F-4-$CF_3CFHCF_2SO$-Ph | |
| I - 712 | 2-Cl-6-F-Ph | 2-F-4-$CF_3CFHCF_2SO$-Ph | |
| I - 713 | 2,6-$Cl_2$-Ph | 2-F-4-$CF_3CFHCF_2SO$-Ph | |
| I - 714 | 3-Cl-pyridin-2yl | 2-F-4-$CF_3CFHCF_2SO$-Ph | |
| I - 715 | 2,6-$F_2$-Ph | 2-F-4-$CF_3CFHCF_2SO_2$-Ph | |
| I - 716 | 2-Cl-6-F-Ph | 2-F-4-$CF_3CFHCF_2SO_2$-Ph | |
| I - 717 | 2,6-$Cl_2$-Ph | 2-F-4-$CF_3CFHCF_2SO_2$-Ph | |
| I - 718 | 3-Cl-pyridin-2yl | 2-F-4-$CF_3CFHCF_2SO_2$-Ph | |
| I - 719 | 2,6-$F_2$-Ph | 2-F-4-COOBu-t-Ph | 142-144 |

(29) $H^1$-NMR ($CDCl_3$)δppm : 4.71-4.90 (1H, m, CFH), 5.15 ( 2H, s, $CH_2$), 5.60 ( 2H, s, $CH_2$), 6.88-6.92 ( 2H, m, Ph), 7.30-7.34 ( 1H, m, Ph), 7.39-7.48 ( 3H, m, Ph).

Table 2

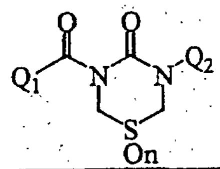

(Ib)

| compound No. | $Q_1$ | $Q_2$ | n | m. p. (°C) |
|---|---|---|---|---|
| II - 1 | 2,6-$F_2$-Ph | 4-Cl-Ph | 0 | 128.5-130.5 |
| II - 2 | 2,6-$F_2$-Ph | 4-Cl-Ph | 1 | 220.0-220.5 |
| II - 3 | 2,6-$F_2$-Ph | 4-Cl-Ph | 2 | 274-275 |
| II - 4 | 2,6-$F_2$-Ph | 3,5-$Cl_2$-4-(2-Cl-4-$CF_3$-pyridin-2yl-O)-Ph | 0 | |
| II - 5 | 2,6-$F_2$-Ph | 3,5-$Cl_2$-4-(2-Cl-4-$CF_3$-pyridin-2yl-O)-Ph | 1 | |
| II - 6 | 2,6-$F_2$-Ph | 3,5-$Cl_2$-4-(2-Cl-4-$CF_3$-pyridin-2yl-O)-Ph | 2 | |
| II - 7 | 2,6-$F_2$-Ph | 2-F-4-(2-Cl-4-$CF_3$-Ph-O)-Ph | 0 | 120.5-123 |
| II - 8 | 2,6-$F_2$-Ph | 2-F-4-(2-Cl-4-$CF_3$-Ph-O)-Ph | 1 | 146-149 |
| II - 9 | 2,6-$F_2$-Ph | 2-F-4-(2-Cl-4-$CF_3$-Ph-O)-Ph | 2 | 220.5-221.5 |
| II - 10 | 2,6-$F_2$-Ph | 3,5-$Cl_2$-2,4-$F_2$-Ph | 0 | 136-137 |
| II - 11 | 2,6-$F_2$-Ph | 3,5-$Cl_2$-2,4-$F_2$-Ph | 1 | 168-171 |
| II - 12 | 2,6-$F_2$-Ph | 3,5-$Cl_2$-2,4-$F_2$-Ph | 2 | 173.5-175 |
| II - 13 | 2,6-$F_2$-Ph | 3,5-$Cl_2$-4-$CF_2HCF_2O$-Ph | 0 | |
| II - 14 | 2,6-$F_2$-Ph | 3,5-$Cl_2$-4-$CF_2HCF_2O$-Ph | 1 | |
| II - 15 | 2,6-$F_2$-Ph | 3,5-$Cl_2$-4-$CF_2HCF_2O$-Ph | 2 | |
| II - 16 | 2,6-$F_2$-Ph | 2,5-$Cl_2$-4-$CF_3CFHCF_2O$-Ph | 0 | 157.5-159 |
| II - 17 | 2,6-$F_2$-Ph | 2,5-$Cl_2$-4-$CF_3CFHCF_2O$-Ph | 1 | 170.5-172.5 |
| II - 18 | 2,6-$F_2$-Ph | 2,5-$Cl_2$-4-$CF_3CFHCF_2O$-Ph | 2 | 196-198 |
| II - 19 | 2,6-$F_2$-Ph | 2-F-4-$CFClHCF_2S$-Ph | 0 | 117-119.5 |
| II - 20 | 2,6-$F_2$-Ph | 2-F-4-$CFClHCF_2S$-Ph | 1 | 203-204.5 |
| II - 21 | 2,6-$F_2$-Ph | 2-F-4-$CFClHCF_2S$-Ph | 2 | 234.5-235.5 |
| II - 22 | 2,6-$F_2$-Ph | 2-F-4-$CFClHCF_2SO$-Ph | 0 | white amorphous (1) |
| II - 23 | 2,6-$F_2$-Ph | 2-F-4-$CFClHCF_2SO$-Ph | 1 | 88-100 |
| II - 24 | 2,6-$F_2$-Ph | 2-F-4-$CFClHCF_2SO$-Ph | 2 | 229-231 |
| II - 25 | 2,6-$F_2$-Ph | 2-F-4-$CFClHCF_2SO_2$-Ph | 0 | |
| II - 26 | 2,6-$F_2$-Ph | 2-F-4-$CFClHCF_2SO_2$-Ph | 1 | |
| II - 27 | 2,6-$F_2$-Ph | 2-F-4-$CFClHCF_2SO_2$-Ph | 2 | |
| II - 28 | 2,6-$F_2$-Ph | 4-$CF_3S$-Ph | 0 | |
| II - 29 | 2,6-$F_2$-Ph | 4-$CF_3S$-Ph | 1 | |
| II - 30 | 2,6-$F_2$-Ph | 4-$CF_3S$-Ph | 2 | |
| II - 31 | 2,6-$F_2$-Ph | 4-$CF_3SO$-Ph | 0 | |
| II - 32 | 2,6-$F_2$-Ph | 4-$CF_3SO$-Ph | 1 | |
| II - 33 | 2,6-$F_2$-Ph | 4-$CF_3SO$-Ph | 2 | |
| II - 34 | 2,6-$F_2$-Ph | 4-$CF_3SO_2$-Ph | 0 | |
| II - 35 | 2,6-$F_2$-Ph | 4-$CF_3SO_2$-Ph | 1 | |
| II - 36 | 2,6-$F_2$-Ph | 4-$CF_3SO_2$-Ph | 2 | |
| II - 37 | 2,6-$F_2$-Ph | 2-F-4-MeS-Ph | 0 | |
| II - 38 | 2,6-$F_2$-Ph | 2-F-4-MeS-Ph | 1 | |
| II - 39 | 2,6-$F_2$-Ph | 2-F-4-MeS-Ph | 2 | |

(1) $H^1$-NMR ($CDCl_3$)δppm : 4.73 ( 2H, s, $SCH_2N$), 5.19 (2H, s, SCH2N), 6.35-6.75 ( 1H, m, CHClF), 6.90 (2H, t J=8.1Hz, Ph), 7.28-7.40 (1H, m, Ph), 7.50-7.62 ( 3H, m, Ph).

| compound No. | $Q_1$ | $Q_2$ | n | m. p. (°C) |
|---|---|---|---|---|
| II - 40 | 2,6-$F_2$-Ph | 2-F-4-MeSO-Ph | 0 | |
| II - 41 | 2,6-$F_2$-Ph | 2-F-4-MeSO-Ph | 1 | |
| II - 42 | 2,6-$F_2$-Ph | 2-F-4-MeSO-Ph | 2 | |
| II - 43 | 2,6-$F_2$-Ph | 2-F-4-$MeSO_2$-Ph | 0 | |
| II - 44 | 2,6-$F_2$-Ph | 2-F-4-$MeSO_2$-Ph | 1 | |
| II - 45 | 2,6-$F_2$-Ph | 2-F-4-$MeSO_2$-Ph | 2 | |
| II - 46 | 2,6-$F_2$-Ph | 2,6-$F_2$-4-MeS-Ph | 0 | |
| II - 47 | 2,6-$F_2$-Ph | 2,6-$F_2$-4-MeS-Ph | 1 | |
| II - 48 | 2,6-$F_2$-Ph | 2,6-$F_2$-4-MeS-Ph | 2 | |
| II - 49 | 2,6-$F_2$-Ph | 2,6-$F_2$-4-MeSO-Ph | 0 | |
| II - 50 | 2,6-$F_2$-Ph | 2,6-$F_2$-4-MeSO-Ph | 1 | |
| II - 51 | 2,6-$F_2$-Ph | 2,6-$F_2$-4-MeSO-Ph | 2 | |
| II - 52 | 2,6-$F_2$-Ph | 2,6-$F_2$-4-$MeSO_2$-Ph | 0 | |
| II - 53 | 2,6-$F_2$-Ph | 2,6-$F_2$-4-$MeSO_2$-Ph | 1 | |
| II - 54 | 2,6-$F_2$-Ph | 2,6-$F_2$-4-$MeSO_2$-Ph | 2 | |
| II - 55 | 2,6-$F_2$-Ph | 2-F-4-$CF_3CH_2S$-Ph | 0 | |
| II - 56 | 2,6-$F_2$-Ph | 2-F-4-$CF_3CH_2S$-Ph | 1 | |
| II - 57 | 2,6-$F_2$-Ph | 2-F-4-$CF_3CH_2S$-Ph | 2 | |
| II - 58 | 2,6-$F_2$-Ph | 2-F-4-$CF_3CH_2SO$-Ph | 0 | |
| II - 59 | 2,6-$F_2$-Ph | 2-F-4-$CF_3CH_2SO$-Ph | 1 | |
| II - 60 | 2,6-$F_2$-Ph | 2-F-4-$CF_3CH_2SO$-Ph | 2 | |
| II - 61 | 2,6-$F_2$-Ph | 2-F-4-$CF_3CH_2SO_2$-Ph | 0 | |
| II - 62 | 2,6-$F_2$-Ph | 2-F-4-$CF_3CH_2SO_2$-Ph | 1 | |
| II - 63 | 2,6-$F_2$-Ph | 2-F-4-$CF_3CH_2SO_2$-Ph | 2 | |
| II - 64 | 2,6-$F_2$-Ph | $CF_3CH_2$ | 0 | oil (2) |
| II - 65 | 2,6-$F_2$-Ph | $CF_3CH_2$ | 1 | |
| II - 66 | 2,6-$F_2$-Ph | $CF_3CH_2$ | 2 | |
| II - 67 | 2,6-$F_2$-Ph | 2-F-4-$CF_2HS$-Ph | 0 | |
| II - 68 | 2,6-$F_2$-Ph | 2-F-4-$CF_2HS$-Ph | 1 | |
| II - 69 | 2,6-$F_2$-Ph | 2-F-4-$CF_2HS$-Ph | 2 | |
| II - 70 | 2,6-$F_2$-Ph | 2-F-4-$CF_2HSO$-Ph | 0 | |
| II - 71 | 2,6-$F_2$-Ph | 2-F-4-$CF_2HSO$-Ph | 1 | |
| II - 72 | 2,6-$F_2$-Ph | 2-F-4-$CF_2HSO$-Ph | 2 | |
| II - 73 | 2,6-$F_2$-Ph | 2-F-4-$CF_2HSO_2$-Ph | 0 | |
| II - 74 | 2,6-$F_2$-Ph | 2-F-4-$CF_2HSO_2$-Ph | 1 | |
| II - 75 | 2,6-$F_2$-Ph | 2-F-4-$CF_2HSO_2$-Ph | 2 | |
| II - 76 | 2,6-$F_2$-Ph | 2-F-4-$CF_3S$-Ph | 0 | |
| II - 77 | 2,6-$F_2$-Ph | 2-F-4-$CF_3S$-Ph | 1 | |
| II - 78 | 2,6-$F_2$-Ph | 2-F-4-$CF_3S$-Ph | 2 | |
| II - 79 | 2,6-$F_2$-Ph | 2-F-4-$CF_3SO$-Ph | 0 | |
| II - 80 | 2,6-$F_2$-Ph | 2-F-4-$CF_3SO$-Ph | 1 | |

(2) $H^1$-NMR ($CDCl_3$)δppm : 4.06 (2H, q J=8.7Hz, $CF_3CH_2$), 4.53 ( 2H, s, $OCH_2N$), 5.05 (2H, s, $OCH_2N$), 6.90-6.95 ( 2H, m, Ph), 7.33-7.37 ( 1H, m, Ph).

| compound No. | $Q_1$ | $Q_2$ | n | m. p. (℃) |
|---|---|---|---|---|
| II - 81 | 2,6-$F_2$-Ph | 2-F-4-$CF_3SO$-Ph | 2 | |
| II - 82 | 2,6-$F_2$-Ph | 2-F-4-$CF_3SO_2$-Ph | 0 | |
| II - 83 | 2,6-$F_2$-Ph | 2-F-4-$CF_3SO_2$-Ph | 1 | |
| II - 84 | 2,6-$F_2$-Ph | 2-F-4-$CF_3SO_2$-Ph | 2 | |
| II - 85 | 2,6-$F_2$-Ph | 2-F-4-$CH_3CH_2S$-Ph | 0 | |
| II - 86 | 2,6-$F_2$-Ph | 2-F-4-$CH_3CH_2S$-Ph | 1 | |
| II - 87 | 2,6-$F_2$-Ph | 2-F-4-$CH_3CH_2S$-Ph | 2 | |
| II - 88 | 2,6-$F_2$-Ph | 2-F-4-$CH_3CH_2SO$-Ph | 0 | |
| II - 89 | 2,6-$F_2$-Ph | 2-F-4-$CH_3CH_2SO$-Ph | 1 | |
| II - 90 | 2,6-$F_2$-Ph | 2-F-4-$CH_3CH_2SO$-Ph | 2 | |
| II - 91 | 2,6-$F_2$-Ph | 2-F-4-$CH_3CH_2SO_2$-Ph | 0 | |
| II - 92 | 2,6-$F_2$-Ph | 2-F-4-$CH_3CH_2SO_2$-Ph | 1 | |
| II - 93 | 2,6-$F_2$-Ph | 2-F-4-$CH_3CH_2SO_2$-Ph | 2 | |
| II - 94 | 2,6-$F_2$-Ph | 2-F-4-(2-Cl-4-$CF_3$-pyridin-2yl-S)-Ph | 0 | |
| II - 95 | 2,6-$F_2$-Ph | 2-F-4-(2-Cl-4-$CF_3$-pyridin-2yl-S)-Ph | 1 | |
| II - 96 | 2,6-$F_2$-Ph | 2-F-4-(2-Cl-4-$CF_3$-pyridin-2yl-S)-Ph | 2 | |
| II - 97 | 2,6-$F_2$-Ph | 2-F-4-(2-Cl-4-$CF_3$-pyridin-2yl-SO)-Ph | 0 | |
| II - 98 | 2,6-$F_2$-Ph | 2-F-4-(2-Cl-4-$CF_3$-pyridin-2yl-SO)-Ph | 1 | |
| II - 99 | 2,6-$F_2$-Ph | 2-F-4-(2-Cl-4-$CF_3$-pyridin-2yl-SO)-Ph | 2 | |
| II - 100 | 2,6-$F_2$-Ph | 2-F-4-(2-Cl-4-$CF_3$-pyridin-2yl-$SO_2$)-Ph | 0 | |
| II - 101 | 2,6-$F_2$-Ph | 2-F-4-(2-Cl-4-$CF_3$-pyridin-2yl-$SO_2$)-Ph | 1 | |
| II - 102 | 2,6-$F_2$-Ph | 2-F-4-(2-Cl-4-$CF_3$-pyridin-2yl-$SO_2$)-Ph | 2 | |
| II - 103 | 2,6-$F_2$-Ph | 2-Me-4-$CF_3S$-Ph | 0 | |
| II - 104 | 2,6-$F_2$-Ph | 2-Me-4-$CF_3S$-Ph | 1 | |
| II - 105 | 2,6-$F_2$-Ph | 2-Me-4-$CF_3S$-Ph | 2 | |
| II - 106 | 2,6-$F_2$-Ph | 2-Me-4-$CF_3SO$-Ph | 0 | |
| II - 107 | 2,6-$F_2$-Ph | 2-Me-4-$CF_3SO$-Ph | 1 | |
| II - 108 | 2,6-$F_2$-Ph | 2-Me-4-$CF_3SO$-Ph | 2 | |
| II - 109 | 2,6-$F_2$-Ph | 2-Me-4-$CF_3SO_2$-Ph | 0 | |
| II - 110 | 2,6-$F_2$-Ph | 2-Me-4-$CF_3SO_2$-Ph | 1 | |
| II - 111 | 2,6-$F_2$-Ph | 2-Me-4-$CF_3SO_2$-Ph | 2 | |
| II - 112 | 2,6-$F_2$-Ph | 3-F-4-$CF_3S$-Ph | 0 | |
| II - 113 | 2,6-$F_2$-Ph | 3-F-4-$CF_3S$-Ph | 1 | |
| II - 114 | 2,6-$F_2$-Ph | 3-F-4-$CF_3S$-Ph | 2 | |
| II - 115 | 2,6-$F_2$-Ph | 3-F-4-$CF_3SO$-Ph | 0 | |
| II - 116 | 2,6-$F_2$-Ph | 3-F-4-$CF_3SO$-Ph | 1 | |
| II - 117 | 2,6-$F_2$-Ph | 3-F-4-$CF_3SO$-Ph | 2 | |
| II - 118 | 2,6-$F_2$-Ph | 3-F-4-$CF_3SO_2$-Ph | 0 | |
| II - 119 | 2,6-$F_2$-Ph | 3-F-4-$CF_3SO_2$-Ph | 1 | |
| II - 120 | 2,6-$F_2$-Ph | 3-F-4-$CF_3SO_2$-Ph | 2 | |

| compound No. | $Q_1$ | $Q_2$ | n | m. p. (°C) |
|---|---|---|---|---|
| II - 121 | 2,6-$F_2$-Ph | 2,3-$Me_2$-4-$CF_3$S-Ph | 0 | |
| II - 122 | 2,6-$F_2$-Ph | 2,3-$Me_2$-4-$CF_3$S-Ph | 1 | |
| II - 123 | 2,6-$F_2$-Ph | 2,3-$Me_2$-4-$CF_3$S-Ph | 2 | |
| II - 124 | 2,6-$F_2$-Ph | 2,3-$Me_2$-4-$CF_3$SO-Ph | 0 | |
| II - 125 | 2,6-$F_2$-Ph | 2,3-$Me_2$-4-$CF_3$SO-Ph | 1 | |
| II - 126 | 2,6-$F_2$-Ph | 2,3-$Me_2$-4-$CF_3$SO-Ph | 2 | |
| II - 127 | 2,6-$F_2$-Ph | 2,3-$Me_2$-4-$CF_3SO_2$-Ph | 0 | |
| II - 128 | 2,6-$F_2$-Ph | 2,3-$Me_2$-4-$CF_3SO_2$-Ph | 1 | |
| II - 129 | 2,6-$F_2$-Ph | 2,3-$Me_2$-4-$CF_3SO_2$-Ph | 2 | |
| II - 130 | 2,6-$F_2$-Ph | 5-Cl-2-Me-4-$CF_3$S-Ph | 0 | |
| II - 131 | 2,6-$F_2$-Ph | 5-Cl-2-Me-4-$CF_3$S-Ph | 1 | |
| II - 132 | 2,6-$F_2$-Ph | 5-Cl-2-Me-4-$CF_3$S-Ph | 2 | |
| II - 133 | 2,6-$F_2$-Ph | 5-Cl-2-Me-4-$CF_3$SO-Ph | 0 | |
| II - 134 | 2,6-$F_2$-Ph | 5-Cl-2-Me-4-$CF_3$SO-Ph | 1 | |
| II - 135 | 2,6-$F_2$-Ph | 5-Cl-2-Me-4-$CF_3$SO-Ph | 2 | |
| II - 136 | 2,6-$F_2$-Ph | 5-Cl-2-Me-4-$CF_3SO_2$-Ph | 0 | |
| II - 137 | 2,6-$F_2$-Ph | 5-Cl-2-Me-4-$CF_3SO_2$-Ph | 1 | |
| II - 138 | 2,6-$F_2$-Ph | 5-Cl-2-Me-4-$CF_3SO_2$-Ph | 2 | |
| II - 139 | 2,6-$F_2$-Ph | 2,6-$F_2$-4-$CF_3$S-Ph | 0 | |
| II - 140 | 2,6-$F_2$-Ph | 2,6-$F_2$-4-$CF_3$S-Ph | 1 | |
| II - 141 | 2,6-$F_2$-Ph | 2,6-$F_2$-4-$CF_3$S-Ph | 2 | |
| II - 142 | 2,6-$F_2$-Ph | 2,6-$F_2$-4-$CF_3$SO-Ph | 0 | |
| II - 143 | 2,6-$F_2$-Ph | 2,6-$F_2$-4-$CF_3$SO-Ph | 1 | |
| II - 144 | 2,6-$F_2$-Ph | 2,6-$F_2$-4-$CF_3$SO-Ph | 2 | |
| II - 145 | 2,6-$F_2$-Ph | 2,6-$F_2$-4-$CF_3SO_2$-Ph | 0 | |
| II - 146 | 2,6-$F_2$-Ph | 2,6-$F_2$-4-$CF_3SO_2$-Ph | 1 | |
| II - 147 | 2,6-$F_2$-Ph | 2,6-$F_2$-4-$CF_3SO_2$-Ph | 2 | |
| II - 148 | 2,6-$F_2$-Ph | 2-F-4-$CF_3CF_2$S-Ph | 0 | |
| II - 149 | 2,6-$F_2$-Ph | 2-F-4-$CF_3CF_2$S-Ph | 1 | |
| II - 150 | 2,6-$F_2$-Ph | 2-F-4-$CF_3CF_2$S-Ph | 2 | |
| II - 151 | 2,6-$F_2$-Ph | 2-F-4-$CF_3CF_2$SO-Ph | 0 | |
| II - 152 | 2,6-$F_2$-Ph | 2-F-4-$CF_3CF_2$SO-Ph | 1 | |
| II - 153 | 2,6-$F_2$-Ph | 2-F-4-$CF_3CF_2$SO-Ph | 2 | |
| II - 154 | 2,6-$F_2$-Ph | 2-F-4-$CF_3CF_2SO_2$-Ph | 0 | |
| II - 155 | 2,6-$F_2$-Ph | 2-F-4-$CF_3CF_2SO_2$-Ph | 1 | |
| II - 156 | 2,6-$F_2$-Ph | 2-F-4-$CF_3CF_2SO_2$-Ph | 2 | |
| II - 157 | 2,6-$F_2$-Ph | 2-F-4-$CF_3CF_2CF_2$S-Ph | 0 | |
| II - 158 | 2,6-$F_2$-Ph | 2-F-4-$CF_3CF_2CF_2$S-Ph | 1 | |
| II - 159 | 2,6-$F_2$-Ph | 2-F-4-$CF_3CF_2CF_2$S-Ph | 2 | |
| II - 160 | 2,6-$F_2$-Ph | 2-F-4-$CF_3CF_2CF_2$SO-Ph | 0 | |

| compound No. | $Q_1$ | $Q_2$ | n | m. p. (°C) |
|---|---|---|---|---|
| II - 161 | 2,6-$F_2$-Ph | 2-F-4-$CF_3CF_2CF_2SO$-Ph | 1 | |
| II - 162 | 2,6-$F_2$-Ph | 2-F-4-$CF_3CF_2CF_2SO$-Ph | 2 | |
| II - 163 | 2,6-$F_2$-Ph | 2-F-4-$CF_3CF_2CF_2SO_2$-Ph | 0 | |
| II - 164 | 2,6-$F_2$-Ph | 2-F-4-$CF_3CF_2CF_2SO_2$-Ph | 1 | |
| II - 165 | 2,6-$F_2$-Ph | 2-F-4-$CF_3CF_2CF_2SO_2$-Ph | 2 | |
| II - 166 | 2,6-$F_2$-Ph | 2-F-4-$(CF_3)_2CFS$-Ph | 0 | |
| II - 167 | 2,6-$F_2$-Ph | 2-F-4-$(CF_3)_2CFS$-Ph | 1 | |
| II - 168 | 2,6-$F_2$-Ph | 2-F-4-$(CF_3)_2CFS$-Ph | 2 | |
| II - 169 | 2,6-$F_2$-Ph | 2-F-4-$(CF_3)_2CFSO$-Ph | 0 | |
| II - 170 | 2,6-$F_2$-Ph | 2-F-4-$(CF_3)_2CFSO$-Ph | 1 | |
| II - 171 | 2,6-$F_2$-Ph | 2-F-4-$(CF_3)_2CFSO$-Ph | 2 | |
| II - 172 | 2,6-$F_2$-Ph | 2-F-4-$(CF_3)_2CFSO_2$-Ph | 0 | |
| II - 173 | 2,6-$F_2$-Ph | 2-F-4-$(CF_3)_2CFSO_2$-Ph | 1 | |
| II - 174 | 2,6-$F_2$-Ph | 2-F-4-$(CF_3)_2CFSO_2$-Ph | 2 | |
| II - 176 | 2-Cl-6-F-Ph | 4-Cl-Ph | 0 | |
| II - 177 | 2-Cl-6-F-Ph | 4-Cl-Ph | 1 | |
| II - 178 | 2-Cl-6-F-Ph | 4-Cl-Ph | 2 | |
| II - 179 | 2-Cl-6-F-Ph | 3,5-$Cl_2$-4-(2-Cl-4-$CF_3$-pyridin-2yl-O)-Ph | 0 | |
| II - 180 | 2-Cl-6-F-Ph | 3,5-$Cl_2$-4-(2-Cl-4-$CF_3$-pyridin-2yl-O)-Ph | 1 | |
| II - 181 | 2-Cl-6-F-Ph | 3,5-$Cl_2$-4-(2-Cl-4-$CF_3$-pyridin-2yl-O)-Ph | 2 | |
| II - 182 | 2-Cl-6-F-Ph | 2-F-4-(2-Cl-4-$CF_3$-Ph-O)-Ph | 0 | |
| II - 183 | 2-Cl-6-F-Ph | 2-F-4-(2-Cl-4-$CF_3$-Ph-O)-Ph | 1 | |
| II - 184 | 2-Cl-6-F-Ph | 2-F-4-(2-Cl-4-$CF_3$-Ph-O)-Ph | 2 | |
| II - 185 | 2-Cl-6-F-Ph | 3,5-$Cl_2$-2,4-$F_2$-Ph | 0 | |
| II - 186 | 2-Cl-6-F-Ph | 3,5-$Cl_2$-2,4-$F_2$-Ph | 1 | |
| II - 187 | 2-Cl-6-F-Ph | 3,5-$Cl_2$-2,4-$F_2$-Ph | 2 | |
| II - 188 | 2-Cl-6-F-Ph | 3,5-$Cl_2$-4-$CF_2HCF_2O$-Ph | 0 | |
| II - 189 | 2-Cl-6-F-Ph | 3,5-$Cl_2$-4-$CF_2HCF_2O$-Ph | 1 | |
| II - 190 | 2-Cl-6-F-Ph | 3,5-$Cl_2$-4-$CF_2HCF_2O$-Ph | 2 | |
| II - 191 | 2-Cl-6-F-Ph | 2,5-$Cl_2$-4-$CF_3CFHCF_2O$-Ph | 0 | |
| II - 192 | 2-Cl-6-F-Ph | 2,5-$Cl_2$-4-$CF_3CFHCF_2O$-Ph | 1 | |
| II - 193 | 2-Cl-6-F-Ph | 2,5-$Cl_2$-4-$CF_3CFHCF_2O$-Ph | 2 | |
| II - 194 | 2-Cl-6-F-Ph | 2-F-4-$CFClHCF_2S$-Ph | 0 | |
| II - 195 | 2-Cl-6-F-Ph | 2-F-4-$CFClHCF_2S$-Ph | 1 | |
| II - 196 | 2-Cl-6-F-Ph | 2-F-4-$CFClHCF_2S$-Ph | 2 | |
| II - 197 | 2-Cl-6-F-Ph | 2-F-4-$CFClHCF_2SO$-Ph | 0 | |
| II - 198 | 2-Cl-6-F-Ph | 2-F-4-$CFClHCF_2SO$-Ph | 1 | |
| II - 199 | 2-Cl-6-F-Ph | 2-F-4-$CFClHCF_2SO$-Ph | 2 | |
| II - 200 | 2-Cl-6-F-Ph | 2-F-4-$CFClHCF_2SO_2$-Ph | 0 | |

| compound No. | $Q_1$ | $Q_2$ | n | m. p. (°C) |
|---|---|---|---|---|
| II - 201 | 2-Cl-6-F-Ph | 2-F-4-CFClHCF$_2$SO$_2$-Ph | 1 | |
| II - 202 | 2-Cl-6-F-Ph | 2-F-4-CFClHCF$_2$SO$_2$-Ph | 2 | |
| II - 203 | 2-Cl-6-F-Ph | 4-CF$_3$S-Ph | 0 | |
| II - 204 | 2-Cl-6-F-Ph | 4-CF$_3$S-Ph | 1 | |
| II - 205 | 2-Cl-6-F-Ph | 4-CF$_3$S-Ph | 2 | |
| II - 206 | 2-Cl-6-F-Ph | 4-CF$_3$SO-Ph | 0 | |
| II - 207 | 2-Cl-6-F-Ph | 4-CF$_3$SO-Ph | 1 | |
| II - 208 | 2-Cl-6-F-Ph | 4-CF$_3$SO-Ph | 2 | |
| II - 209 | 2-Cl-6-F-Ph | 4-CF$_3$SO$_2$-Ph | 0 | |
| II - 210 | 2-Cl-6-F-Ph | 4-CF$_3$SO$_2$-Ph | 1 | |
| II - 211 | 2-Cl-6-F-Ph | 4-CF$_3$SO$_2$-Ph | 2 | |
| II - 212 | 2-Cl-6-F-Ph | 2-F-4-MeS-Ph | 0 | |
| II - 213 | 2-Cl-6-F-Ph | 2-F-4-MeS-Ph | 1 | |
| II - 214 | 2-Cl-6-F-Ph | 2-F-4-MeS-Ph | 2 | |
| II - 215 | 2-Cl-6-F-Ph | 2-F-4-MeSO-Ph | 0 | |
| II - 216 | 2-Cl-6-F-Ph | 2-F-4-MeSO-Ph | 1 | |
| II - 217 | 2-Cl-6-F-Ph | 2-F-4-MeSO-Ph | 2 | |
| II - 218 | 2-Cl-6-F-Ph | 2-F-4-MeSO$_2$-Ph | 0 | |
| II - 219 | 2-Cl-6-F-Ph | 2-F-4-MeSO$_2$-Ph | 1 | |
| II - 220 | 2-Cl-6-F-Ph | 2-F-4-MeSO$_2$-Ph | 2 | |
| II - 221 | 2-Cl-6-F-Ph | 2,6-F$_2$-4-MeS-Ph | 0 | |
| II - 222 | 2-Cl-6-F-Ph | 2,6-F$_2$-4-MeS-Ph | 1 | |
| II - 223 | 2-Cl-6-F-Ph | 2,6-F$_2$-4-MeS-Ph | 2 | |
| II - 224 | 2-Cl-6-F-Ph | 2,6-F$_2$-4-MeSO-Ph | 0 | |
| II - 225 | 2-Cl-6-F-Ph | 2,6-F$_2$-4-MeSO-Ph | 1 | |
| II - 226 | 2-Cl-6-F-Ph | 2,6-F$_2$-4-MeSO-Ph | 2 | |
| II - 227 | 2-Cl-6-F-Ph | 2,6-F$_2$-4-MeSO$_2$-Ph | 0 | |
| II - 228 | 2-Cl-6-F-Ph | 2,6-F$_2$-4-MeSO$_2$-Ph | 1 | |
| II - 229 | 2-Cl-6-F-Ph | 2,6-F$_2$-4-MeSO$_2$-Ph | 2 | |
| II - 230 | 2-Cl-6-F-Ph | 2-F-4-CF$_3$CH$_2$S-Ph | 0 | |
| II - 231 | 2-Cl-6-F-Ph | 2-F-4-CF$_3$CH$_2$S-Ph | 1 | |
| II - 232 | 2-Cl-6-F-Ph | 2-F-4-CF$_3$CH$_2$S-Ph | 2 | |
| II - 233 | 2-Cl-6-F-Ph | 2-F-4-CF$_3$CH$_2$SO-Ph | 0 | |
| II - 234 | 2-Cl-6-F-Ph | 2-F-4-CF$_3$CH$_2$SO-Ph | 1 | |
| II - 235 | 2-Cl-6-F-Ph | 2-F-4-CF$_3$CH$_2$SO-Ph | 2 | |
| II - 236 | 2-Cl-6-F-Ph | 2-F-4-CF$_3$CH$_2$SO$_2$-Ph | 0 | |
| II - 237 | 2-Cl-6-F-Ph | 2-F-4-CF$_3$CH$_2$SO$_2$-Ph | 1 | |
| II - 238 | 2-Cl-6-F-Ph | 2-F-4-CF$_3$CH$_2$SO$_2$-Ph | 2 | |
| II - 239 | 2-Cl-6-F-Ph | CF$_3$CH$_2$ | 0 | |
| II - 240 | 2-Cl-6-F-Ph | CF$_3$CH$_2$ | 1 | |

| compound No. | $Q_1$ | $Q_2$ | n | m. p. (°C) |
|---|---|---|---|---|
| II - 241 | 2-Cl-6-F-Ph | $CF_3CH_2$ | 2 | |
| II - 242 | 2-Cl-6-F-Ph | 2-F-4-$CF_2$HS-Ph | 0 | |
| II - 243 | 2-Cl-6-F-Ph | 2-F-4-$CF_2$HS-Ph | 1 | |
| II - 244 | 2-Cl-6-F-Ph | 2-F-4-$CF_2$HS-Ph | 2 | |
| II - 245 | 2-Cl-6-F-Ph | 2-F-4-$CF_2$HSO-Ph | 0 | |
| II - 246 | 2-Cl-6-F-Ph | 2-F-4-$CF_2$HSO-Ph | 1 | |
| II - 247 | 2-Cl-6-F-Ph | 2-F-4-$CF_2$HSO-Ph | 2 | |
| II - 248 | 2-Cl-6-F-Ph | 2-F-4-$CF_2HSO_2$-Ph | 0 | |
| II - 249 | 2-Cl-6-F-Ph | 2-F-4-$CF_2HSO_2$-Ph | 1 | |
| II - 250 | 2-Cl-6-F-Ph | 2-F-4-$CF_2HSO_2$-Ph | 2 | |
| II - 251 | 2-Cl-6-F-Ph | 2-F-4-$CF_3$S-Ph | 0 | |
| II - 252 | 2-Cl-6-F-Ph | 2-F-4-$CF_3$S-Ph | 1 | |
| II - 253 | 2-Cl-6-F-Ph | 2-F-4-$CF_3$S-Ph | 2 | |
| II - 254 | 2-Cl-6-F-Ph | 2-F-4-$CF_3$SO-Ph | 0 | |
| II - 255 | 2-Cl-6-F-Ph | 2-F-4-$CF_3$SO-Ph | 1 | |
| II - 256 | 2-Cl-6-F-Ph | 2-F-4-$CF_3$SO-Ph | 2 | |
| II - 257 | 2-Cl-6-F-Ph | 2-F-4-$CF_3SO_2$-Ph | 0 | |
| II - 258 | 2-Cl-6-F-Ph | 2-F-4-$CF_3SO_2$-Ph | 1 | |
| II - 259 | 2-Cl-6-F-Ph | 2-F-4-$CF_3SO_2$-Ph | 2 | |
| II - 260 | 2-Cl-6-F-Ph | 2-F-4-$CH_3CH_2$S-Ph | 0 | |
| II - 261 | 2-Cl-6-F-Ph | 2-F-4-$CH_3CH_2$S-Ph | 1 | |
| II - 262 | 2-Cl-6-F-Ph | 2-F-4-$CH_3CH_2$S-Ph | 2 | |
| II - 263 | 2-Cl-6-F-Ph | 2-F-4-$CH_3CH_2$SO-Ph | 0 | |
| II - 264 | 2-Cl-6-F-Ph | 2-F-4-$CH_3CH_2$SO-Ph | 1 | |
| II - 265 | 2-Cl-6-F-Ph | 2-F-4-$CH_3CH_2$SO-Ph | 2 | |
| II - 266 | 2-Cl-6-F-Ph | 2-F-4-$CH_3CH_2SO_2$-Ph | 0 | |
| II - 267 | 2-Cl-6-F-Ph | 2-F-4-$CH_3CH_2SO_2$-Ph | 1 | |
| II - 268 | 2-Cl-6-F-Ph | 2-F-4-$CH_3CH_2SO_2$-Ph | 2 | |
| II - 269 | 2-Cl-6-F-Ph | 2-F-4-(2-Cl-4-$CF_3$-pyridin-2yl-S)-Ph | 0 | |
| II - 270 | 2-Cl-6-F-Ph | 2-F-4-(2-Cl-4-$CF_3$-pyridin-2yl-S)-Ph | 1 | |
| II - 271 | 2-Cl-6-F-Ph | 2-F-4-(2-Cl-4-$CF_3$-pyridin-2yl-S)-Ph | 2 | |
| II - 272 | 2-Cl-6-F-Ph | 2-F-4-(2-Cl-4-$CF_3$-pyridin-2yl-SO)-Ph | 0 | |
| II - 273 | 2-Cl-6-F-Ph | 2-F-4-(2-Cl-4-$CF_3$-pyridin-2yl-SO)-Ph | 1 | |
| II - 274 | 2-Cl-6-F-Ph | 2-F-4-(2-Cl-4-$CF_3$-pyridin-2yl-SO)-Ph | 2 | |
| II - 275 | 2-Cl-6-F-Ph | 2-F-4-(2-Cl-4-$CF_3$-pyridin-2yl-$SO_2$)-Ph | 0 | |
| II - 276 | 2-Cl-6-F-Ph | 2-F-4-(2-Cl-4-$CF_3$-pyridin-2yl-$SO_2$)-Ph | 1 | |
| II - 277 | 2-Cl-6-F-Ph | 2-F-4-(2-Cl-4-$CF_3$-pyridin-2yl-$SO_2$)-Ph | 2 | |
| II - 278 | 2-Cl-6-F-Ph | 2-Me-4-$CF_3$S-Ph | 0 | |
| II - 279 | 2-Cl-6-F-Ph | 2-Me-4-$CF_3$S-Ph | 1 | |
| II - 280 | 2-Cl-6-F-Ph | 2-Me-4-$CF_3$S-Ph | 2 | |

| compound No. | $Q_1$ | $Q_2$ | n | m. p. (°C) |
|---|---|---|---|---|
| II - 281 | 2-Cl-6-F-Ph | 2-Me-4-$CF_3SO$-Ph | 0 | |
| II - 282 | 2-Cl-6-F-Ph | 2-Me-4-$CF_3SO$-Ph | 1 | |
| II - 283 | 2-Cl-6-F-Ph | 2-Me-4-$CF_3SO$-Ph | 2 | |
| II - 284 | 2-Cl-6-F-Ph | 2-Me-4-$CF_3SO_2$-Ph | 0 | |
| II - 285 | 2-Cl-6-F-Ph | 2-Me-4-$CF_3SO_2$-Ph | 1 | |
| II - 286 | 2-Cl-6-F-Ph | 2-Me-4-$CF_3SO_2$-Ph | 2 | |
| II - 287 | 2-Cl-6-F-Ph | 3-F-4-$CF_3S$-Ph | 0 | |
| II - 288 | 2-Cl-6-F-Ph | 3-F-4-$CF_3S$-Ph | 1 | |
| II - 289 | 2-Cl-6-F-Ph | 3-F-4-$CF_3S$-Ph | 2 | |
| II - 290 | 2-Cl-6-F-Ph | 3-F-4-$CF_3SO$-Ph | 0 | |
| II - 291 | 2-Cl-6-F-Ph | 3-F-4-$CF_3SO$-Ph | 1 | |
| II - 292 | 2-Cl-6-F-Ph | 3-F-4-$CF_3SO$-Ph | 2 | |
| II - 293 | 2-Cl-6-F-Ph | 3-F-4-$CF_3SO_2$-Ph | 0 | |
| II - 294 | 2-Cl-6-F-Ph | 3-F-4-$CF_3SO_2$-Ph | 1 | |
| II - 295 | 2-Cl-6-F-Ph | 3-F-4-$CF_3SO_2$-Ph | 2 | |
| II - 296 | 2-Cl-6-F-Ph | 2,3-$Me_2$-4-$CF_3S$-Ph | 0 | |
| II - 297 | 2-Cl-6-F-Ph | 2,3-$Me_2$-4-$CF_3S$-Ph | 1 | |
| II - 298 | 2-Cl-6-F-Ph | 2,3-$Me_2$-4-$CF_3S$-Ph | 2 | |
| II - 299 | 2-Cl-6-F-Ph | 2,3-$Me_2$-4-$CF_3SO$-Ph | 0 | |
| II - 300 | 2-Cl-6-F-Ph | 2,3-$Me_2$-4-$CF_3SO$-Ph | 1 | |
| II - 301 | 2-Cl-6-F-Ph | 2,3-$Me_2$-4-$CF_3SO$-Ph | 2 | |
| II - 302 | 2-Cl-6-F-Ph | 2,3-$Me_2$-4-$CF_3SO_2$-Ph | 0 | |
| II - 303 | 2-Cl-6-F-Ph | 2,3-$Me_2$-4-$CF_3SO_2$-Ph | 1 | |
| II - 304 | 2-Cl-6-F-Ph | 2,3-$Me_2$-4-$CF_3SO_2$-Ph | 2 | |
| II - 305 | 2-Cl-6-F-Ph | 5-Cl-2-Me-4-$CF_3S$-Ph | 0 | |
| II - 306 | 2-Cl-6-F-Ph | 5-Cl-2-Me-4-$CF_3S$-Ph | 1 | |
| II - 307 | 2-Cl-6-F-Ph | 5-Cl-2-Me-4-$CF_3S$-Ph | 2 | |
| II - 308 | 2-Cl-6-F-Ph | 5-Cl-2-Me-4-$CF_3SO$-Ph | 0 | |
| II - 309 | 2-Cl-6-F-Ph | 5-Cl-2-Me-4-$CF_3SO$-Ph | 1 | |
| II - 310 | 2-Cl-6-F-Ph | 5-Cl-2-Me-4-$CF_3SO$-Ph | 2 | |
| II - 311 | 2-Cl-6-F-Ph | 5-Cl-2-Me-4-$CF_3SO_2$-Ph | 0 | |
| II - 312 | 2-Cl-6-F-Ph | 5-Cl-2-Me-4-$CF_3SO_2$-Ph | 1 | |
| II - 313 | 2-Cl-6-F-Ph | 5-Cl-2-Me-4-$CF_3SO_2$-Ph | 2 | |
| II - 314 | 2-Cl-6-F-Ph | 2,6-$F_2$-4-$CF_3S$-Ph | 0 | |
| II - 315 | 2-Cl-6-F-Ph | 2,6-$F_2$-4-$CF_3S$-Ph | 1 | |
| II - 316 | 2-Cl-6-F-Ph | 2,6-$F_2$-4-$CF_3S$-Ph | 2 | |
| II - 317 | 2-Cl-6-F-Ph | 2,6-$F_2$-4-$CF_3SO$-Ph | 0 | |
| II - 318 | 2-Cl-6-F-Ph | 2,6-$F_2$-4-$CF_3SO$-Ph | 1 | |
| II - 319 | 2-Cl-6-F-Ph | 2,6-$F_2$-4-$CF_3SO$-Ph | 2 | |
| II - 320 | 2-Cl-6-F-Ph | 2,6-$F_2$-4-$CF_3SO_2$-Ph | 0 | |

| compound No. | $Q_1$ | $Q_2$ | n | m. p. (°C) |
|---|---|---|---|---|
| II - 321 | 2-Cl-6-F-Ph | 2,6-$F_2$-4-$CF_3SO_2$-Ph | 1 | |
| II - 322 | 2-Cl-6-F-Ph | 2,6-$F_2$-4-$CF_3SO_2$-Ph | 2 | |
| II - 323 | 2-Cl-6-F-Ph | 2-F-4-$CF_3CF_2$S-Ph | 0 | |
| II - 324 | 2-Cl-6-F-Ph | 2-F-4-$CF_3CF_2$S-Ph | 1 | |
| II - 325 | 2-Cl-6-F-Ph | 2-F-4-$CF_3CF_2$S-Ph | 2 | |
| II - 326 | 2-Cl-6-F-Ph | 2-F-4-$CF_3CF_2$SO-Ph | 0 | |
| II - 327 | 2-Cl-6-F-Ph | 2-F-4-$CF_3CF_2$SO-Ph | 1 | |
| II - 328 | 2-Cl-6-F-Ph | 2-F-4-$CF_3CF_2$SO-Ph | 2 | |
| II - 329 | 2-Cl-6-F-Ph | 2-F-4-$CF_3CF_2SO_2$-Ph | 0 | |
| II - 330 | 2-Cl-6-F-Ph | 2-F-4-$CF_3CF_2SO_2$-Ph | 1 | |
| II - 331 | 2-Cl-6-F-Ph | 2-F-4-$CF_3CF_2SO_2$-Ph | 2 | |
| II - 332 | 2-Cl-6-F-Ph | 2-F-4-$CF_3CF_2CF_2$S-Ph | 0 | |
| II - 333 | 2-Cl-6-F-Ph | 2-F-4-$CF_3CF_2CF_2$S-Ph | 1 | |
| II - 334 | 2-Cl-6-F-Ph | 2-F-4-$CF_3CF_2CF_2$S-Ph | 2 | |
| II - 335 | 2-Cl-6-F-Ph | 2-F-4-$CF_3CF_2CF_2$SO-Ph | 0 | |
| II - 336 | 2-Cl-6-F-Ph | 2-F-4-$CF_3CF_2CF_2$SO-Ph | 1 | |
| II - 337 | 2-Cl-6-F-Ph | 2-F-4-$CF_3CF_2CF_2$SO-Ph | 2 | |
| II - 338 | 2-Cl-6-F-Ph | 2-F-4-$CF_3CF_2CF_2SO_2$-Ph | 0 | |
| II - 339 | 2-Cl-6-F-Ph | 2-F-4-$CF_3CF_2CF_2SO_2$-Ph | 1 | |
| II - 340 | 2-Cl-6-F-Ph | 2-F-4-$CF_3CF_2CF_2SO_2$-Ph | 2 | |
| II - 341 | 2-Cl-6-F-Ph | 2-F-4-$(CF_3)_2$CFS-Ph | 0 | |
| II - 342 | 2-Cl-6-F-Ph | 2-F-4-$(CF_3)_2$CFS-Ph | 1 | |
| II - 343 | 2-Cl-6-F-Ph | 2-F-4-$(CF_3)_2$CFS-Ph | 2 | |
| II - 344 | 2-Cl-6-F-Ph | 2-F-4-$(CF_3)_2$CFSO-Ph | 0 | |
| II - 345 | 2-Cl-6-F-Ph | 2-F-4-$(CF_3)_2$CFSO-Ph | 1 | |
| II - 346 | 2-Cl-6-F-Ph | 2-F-4-$(CF_3)_2$CFSO-Ph | 2 | |
| II - 347 | 2-Cl-6-F-Ph | 2-F-4-$(CF_3)_2CFSO_2$-Ph | 0 | |
| II - 348 | 2-Cl-6-F-Ph | 2-F-4-$(CF_3)_2CFSO_2$-Ph | 1 | |
| II - 349 | 2-Cl-6-F-Ph | 2-F-4-$(CF_3)_2CFSO_2$-Ph | 2 | |
| II - 350 | 2,6-$Cl_2$-Ph | 4-Cl-Ph | 0 | |
| II - 351 | 2,6-$Cl_2$-Ph | 4-Cl-Ph | 1 | |
| II - 352 | 2,6-$Cl_2$-Ph | 4-Cl-Ph | 2 | |
| II - 353 | 2,6-$Cl_2$-Ph | 3,5-$Cl_2$-4-(2-Cl-4-$CF_3$-pyridin-2yl-O)-Ph | 0 | |
| II - 354 | 2,6-$Cl_2$-Ph | 3,5-$Cl_2$-4-(2-Cl-4-$CF_3$-pyridin-2yl-O)-Ph | 1 | |
| II - 355 | 2,6-$Cl_2$-Ph | 3,5-$Cl_2$-4-(2-Cl-4-$CF_3$-pyridin-2yl-O)-Ph | 2 | |
| II - 356 | 2,6-$Cl_2$-Ph | 2-F-4-(2-Cl-4-$CF_3$-Ph-O)-Ph | 0 | |
| II - 357 | 2,6-$Cl_2$-Ph | 2-F-4-(2-Cl-4-$CF_3$-Ph-O)-Ph | 1 | |
| II - 358 | 2,6-$Cl_2$-Ph | 2-F-4-(2-Cl-4-$CF_3$-Ph-O)-Ph | 2 | |
| II - 359 | 2,6-$Cl_2$-Ph | 3,5-$Cl_2$-2,4-$F_2$-Ph | 0 | |
| II - 360 | 2,6-$Cl_2$-Ph | 3,5-$Cl_2$-2,4-$F_2$-Ph | 1 | |

| compound No. | $Q_1$ | $Q_2$ | n | m. p. (℃) |
|---|---|---|---|---|
| II - 361 | 2,6-$Cl_2$-Ph | 3,5-$Cl_2$-2,4-$F_2$-Ph | 2 | |
| II - 362 | 2,6-$Cl_2$-Ph | 3,5-$Cl_2$-4-$CF_2HCF_2O$-Ph | 0 | |
| II - 363 | 2,6-$Cl_2$-Ph | 3,5-$Cl_2$-4-$CF_2HCF_2O$-Ph | 1 | |
| II - 364 | 2,6-$Cl_2$-Ph | 3,5-$Cl_2$-4-$CF_2HCF_2O$-Ph | 2 | |
| II - 365 | 2,6-$Cl_2$-Ph | 2,5-$Cl_2$-4-$CF_3CFHCF_2O$-Ph | 0 | |
| II - 366 | 2,6-$Cl_2$-Ph | 2,5-$Cl_2$-4-$CF_3CFHCF_2O$-Ph | 1 | |
| II - 367 | 2,6-$Cl_2$-Ph | 2,5-$Cl_2$-4-$CF_3CFHCF_2O$-Ph | 2 | |
| II - 368 | 2,6-$Cl_2$-Ph | 2-F-4-$CFClHCF_2S$-Ph | 0 | |
| II - 369 | 2,6-$Cl_2$-Ph | 2-F-4-$CFClHCF_2S$-Ph | 1 | |
| II - 370 | 2,6-$Cl_2$-Ph | 2-F-4-$CFClHCF_2S$-Ph | 2 | |
| II - 371 | 2,6-$Cl_2$-Ph | 2-F-4-$CFClHCF_2SO$-Ph | 0 | |
| II - 372 | 2,6-$Cl_2$-Ph | 2-F-4-$CFClHCF_2SO$-Ph | 1 | |
| II - 373 | 2,6-$Cl_2$-Ph | 2-F-4-$CFClHCF_2SO$-Ph | 2 | |
| II - 374 | 2,6-$Cl_2$-Ph | 2-F-4-$CFClHCF_2SO_2$-Ph | 0 | |
| II - 375 | 2,6-$Cl_2$-Ph | 2-F-4-$CFClHCF_2SO_2$-Ph | 1 | |
| II - 376 | 2,6-$Cl_2$-Ph | 2-F-4-$CFClHCF_2SO_2$-Ph | 2 | |
| II - 377 | 2,6-$Cl_2$-Ph | 4-$CF_3S$-Ph | 0 | |
| II - 378 | 2,6-$Cl_2$-Ph | 4-$CF_3S$-Ph | 1 | |
| II - 379 | 2,6-$Cl_2$-Ph | 4-$CF_3S$-Ph | 2 | |
| II - 380 | 2,6-$Cl_2$-Ph | 4-$CF_3SO$-Ph | 0 | |
| II - 381 | 2,6-$Cl_2$-Ph | 4-$CF_3SO$-Ph | 1 | |
| II - 382 | 2,6-$Cl_2$-Ph | 4-$CF_3SO$-Ph | 2 | |
| II - 383 | 2,6-$Cl_2$-Ph | 4-$CF_3SO_2$-Ph | 0 | |
| II - 384 | 2,6-$Cl_2$-Ph | 4-$CF_3SO_2$-Ph | 1 | |
| II - 385 | 2,6-$Cl_2$-Ph | 4-$CF_3SO_2$-Ph | 2 | |
| II - 386 | 2,6-$Cl_2$-Ph | 2-F-4-MeS-Ph | 0 | |
| II - 387 | 2,6-$Cl_2$-Ph | 2-F-4-MeS-Ph | 1 | |
| II - 388 | 2,6-$Cl_2$-Ph | 2-F-4-MeS-Ph | 2 | |
| II - 389 | 2,6-$Cl_2$-Ph | 2-F-4-MeSO-Ph | 0 | |
| II - 390 | 2,6-$Cl_2$-Ph | 2-F-4-MeSO-Ph | 1 | |
| II - 391 | 2,6-$Cl_2$-Ph | 2-F-4-MeSO-Ph | 2 | |
| II - 392 | 2,6-$Cl_2$-Ph | 2-F-4-$MeSO_2$-Ph | 0 | |
| II - 393 | 2,6-$Cl_2$-Ph | 2-F-4-$MeSO_2$-Ph | 1 | |
| II - 394 | 2,6-$Cl_2$-Ph | 2-F-4-$MeSO_2$-Ph | 2 | |
| II - 395 | 2,6-$Cl_2$-Ph | 2,6-$F_2$-4-MeS-Ph | 0 | |
| II - 396 | 2,6-$Cl_2$-Ph | 2,6-$F_2$-4-MeS-Ph | 1 | |
| II - 397 | 2,6-$Cl_2$-Ph | 2,6-$F_2$-4-MeS-Ph | 2 | |
| II - 398 | 2,6-$Cl_2$-Ph | 2,6-$F_2$-4-MeSO-Ph | 0 | |
| II - 399 | 2,6-$Cl_2$-Ph | 2,6-$F_2$-4-MeSO-Ph | 1 | |
| II - 400 | 2,6-$Cl_2$-Ph | 2,6-$F_2$-4-MeSO-Ph | 2 | |

| compound No. | $Q_1$ | $Q_2$ | n | m. p. (°C) |
|---|---|---|---|---|
| II - 401 | 2,6-$Cl_2$-Ph | 2,6-$F_2$-4-$MeSO_2$-Ph | 0 | |
| II - 402 | 2,6-$Cl_2$-Ph | 2,6-$F_2$-4-$MeSO_2$-Ph | 1 | |
| II - 403 | 2,6-$Cl_2$-Ph | 2,6-$F_2$-4-$MeSO_2$-Ph | 2 | |
| II - 404 | 2,6-$Cl_2$-Ph | 2-F-4-$CF_3CH_2S$-Ph | 0 | |
| II - 405 | 2,6-$Cl_2$-Ph | 2-F-4-$CF_3CH_2S$-Ph | 1 | |
| II - 406 | 2,6-$Cl_2$-Ph | 2-F-4-$CF_3CH_2S$-Ph | 2 | |
| II - 407 | 2,6-$Cl_2$-Ph | 2-F-4-$CF_3CH_2SO$-Ph | 0 | |
| II - 408 | 2,6-$Cl_2$-Ph | 2-F-4-$CF_3CH_2SO$-Ph | 1 | |
| II - 409 | 2,6-$Cl_2$-Ph | 2-F-4-$CF_3CH_2SO$-Ph | 2 | |
| II - 410 | 2,6-$Cl_2$-Ph | 2-F-4-$CF_3CH_2SO_2$-Ph | 0 | |
| II - 411 | 2,6-$Cl_2$-Ph | 2-F-4-$CF_3CH_2SO_2$-Ph | 1 | |
| II - 412 | 2,6-$Cl_2$-Ph | 2-F-4-$CF_3CH_2SO_2$-Ph | 2 | |
| II - 413 | 2,6-$Cl_2$-Ph | $CF_3CH_2$ | 0 | |
| II - 414 | 2,6-$Cl_2$-Ph | $CF_3CH_2$ | 1 | |
| II - 415 | 2,6-$Cl_2$-Ph | $CF_3CH_2$ | 2 | |
| II - 416 | 2,6-$Cl_2$-Ph | 2-F-4-$CF_2HS$-Ph | 0 | |
| II - 417 | 2,6-$Cl_2$-Ph | 2-F-4-$CF_2HS$-Ph | 1 | |
| II - 418 | 2,6-$Cl_2$-Ph | 2-F-4-$CF_2HS$-Ph | 2 | |
| II - 419 | 2,6-$Cl_2$-Ph | 2-F-4-$CF_2HSO$-Ph | 0 | |
| II - 420 | 2,6-$Cl_2$-Ph | 2-F-4-$CF_2HSO$-Ph | 1 | |
| II - 421 | 2,6-$Cl_2$-Ph | 2-F-4-$CF_2HSO$-Ph | 2 | |
| II - 422 | 2,6-$Cl_2$-Ph | 2-F-4-$CF_2HSO_2$-Ph | 0 | |
| II - 423 | 2,6-$Cl_2$-Ph | 2-F-4-$CF_2HSO_2$-Ph | 1 | |
| II - 424 | 2,6-$Cl_2$-Ph | 2-F-4-$CF_2HSO_2$-Ph | 2 | |
| II - 425 | 2,6-$Cl_2$-Ph | 2-F-4-$CF_3S$-Ph | 0 | |
| II - 426 | 2,6-$Cl_2$-Ph | 2-F-4-$CF_3S$-Ph | 1 | |
| II - 427 | 2,6-$Cl_2$-Ph | 2-F-4-$CF_3S$-Ph | 2 | |
| II - 428 | 2,6-$Cl_2$-Ph | 2-F-4-$CF_3SO$-Ph | 0 | |
| II - 429 | 2,6-$Cl_2$-Ph | 2-F-4-$CF_3SO$-Ph | 1 | |
| II - 430 | 2,6-$Cl_2$-Ph | 2-F-4-$CF_3SO$-Ph | 2 | |
| II - 431 | 2,6-$Cl_2$-Ph | 2-F-4-$CF_3SO_2$-Ph | 0 | |
| II - 432 | 2,6-$Cl_2$-Ph | 2-F-4-$CF_3SO_2$-Ph | 1 | |
| II - 433 | 2,6-$Cl_2$-Ph | 2-F-4-$CF_3SO_2$-Ph | 2 | |
| II - 434 | 2,6-$Cl_2$-Ph | 2-F-4-$CH_3CH_2S$-Ph | 0 | |
| II - 435 | 2,6-$Cl_2$-Ph | 2-F-4-$CH_3CH_2S$-Ph | 1 | |
| II - 436 | 2,6-$Cl_2$-Ph | 2-F-4-$CH_3CH_2S$-Ph | 2 | |
| II - 437 | 2,6-$Cl_2$-Ph | 2-F-4-$CH_3CH_2SO$-Ph | 0 | |
| II - 438 | 2,6-$Cl_2$-Ph | 2-F-4-$CH_3CH_2SO$-Ph | 1 | |
| II - 439 | 2,6-$Cl_2$-Ph | 2-F-4-$CH_3CH_2SO$-Ph | 2 | |
| II - 440 | 2,6-$Cl_2$-Ph | 2-F-4-$CH_3CH_2SO_2$-Ph | 0 | |

| compound No. | $Q_1$ | $Q_2$ | n | m. p. (℃) |
|---|---|---|---|---|
| Ⅱ-441 | 2,6-$Cl_2$-Ph | 2-F-4-$CH_3CH_2SO_2$-Ph | 1 | |
| Ⅱ-442 | 2,6-$Cl_2$-Ph | 2-F-4-$CH_3CH_2SO_2$-Ph | 2 | |
| Ⅱ-443 | 2,6-$Cl_2$-Ph | 2-F-4-(2-Cl-4-$CF_3$-pyridin-2yl-S)-Ph | 0 | |
| Ⅱ-444 | 2,6-$Cl_2$-Ph | 2-F-4-(2-Cl-4-$CF_3$-pyridin-2yl-S)-Ph | 1 | |
| Ⅱ-445 | 2,6-$Cl_2$-Ph | 2-F-4-(2-Cl-4-$CF_3$-pyridin-2yl-S)-Ph | 2 | |
| Ⅱ-446 | 2,6-$Cl_2$-Ph | 2-F-4-(2-Cl-4-$CF_3$-pyridin-2yl-SO)-Ph | 0 | |
| Ⅱ-447 | 2,6-$Cl_2$-Ph | 2-F-4-(2-Cl-4-$CF_3$-pyridin-2yl-SO)-Ph | 1 | |
| Ⅱ-448 | 2,6-$Cl_2$-Ph | 2-F-4-(2-Cl-4-$CF_3$-pyridin-2yl-SO)-Ph | 2 | |
| Ⅱ-449 | 2,6-$Cl_2$-Ph | 2-F-4-(2-Cl-4-$CF_3$-pyridin-2yl-$SO_2$)-Ph | 0 | |
| Ⅱ-450 | 2,6-$Cl_2$-Ph | 2-F-4-(2-Cl-4-$CF_3$-pyridin-2yl-$SO_2$)-Ph | 1 | |
| Ⅱ-451 | 2,6-$Cl_2$-Ph | 2-F-4-(2-Cl-4-$CF_3$-pyridin-2yl-$SO_2$)-Ph | 2 | |
| Ⅱ-452 | 2,6-$Cl_2$-Ph | 2-Me-4-$CF_3$S-Ph | 0 | |
| Ⅱ-453 | 2,6-$Cl_2$-Ph | 2-Me-4-$CF_3$S-Ph | 1 | |
| Ⅱ-454 | 2,6-$Cl_2$-Ph | 2-Me-4-$CF_3$S-Ph | 2 | |
| Ⅱ-455 | 2,6-$Cl_2$-Ph | 2-Me-4-$CF_3$SO-Ph | 0 | |
| Ⅱ-456 | 2,6-$Cl_2$-Ph | 2-Me-4-$CF_3$SO-Ph | 1 | |
| Ⅱ-457 | 2,6-$Cl_2$-Ph | 2-Me-4-$CF_3$SO-Ph | 2 | |
| Ⅱ-458 | 2,6-$Cl_2$-Ph | 2-Me-4-$CF_3SO_2$-Ph | 0 | |
| Ⅱ-459 | 2,6-$Cl_2$-Ph | 2-Me-4-$CF_3SO_2$-Ph | 1 | |
| Ⅱ-460 | 2,6-$Cl_2$-Ph | 2-Me-4-$CF_3SO_2$-Ph | 2 | |
| Ⅱ-461 | 2,6-$Cl_2$-Ph | 3-F-4-$CF_3$S-Ph | 0 | |
| Ⅱ-462 | 2,6-$Cl_2$-Ph | 3-F-4-$CF_3$S-Ph | 1 | |
| Ⅱ-463 | 2,6-$Cl_2$-Ph | 3-F-4-$CF_3$S-Ph | 2 | |
| Ⅱ-464 | 2,6-$Cl_2$-Ph | 3-F-4-$CF_3$SO-Ph | 0 | |
| Ⅱ-465 | 2,6-$Cl_2$-Ph | 3-F-4-$CF_3$SO-Ph | 1 | |
| Ⅱ-466 | 2,6-$Cl_2$-Ph | 3-F-4-$CF_3$SO-Ph | 2 | |
| Ⅱ-467 | 2,6-$Cl_2$-Ph | 3-F-4-$CF_3SO_2$-Ph | 0 | |
| Ⅱ-468 | 2,6-$Cl_2$-Ph | 3-F-4-$CF_3SO_2$-Ph | 1 | |
| Ⅱ-469 | 2,6-$Cl_2$-Ph | 3-F-4-$CF_3SO_2$-Ph | 2 | |
| Ⅱ-470 | 2,6-$Cl_2$-Ph | 2,3-$Me_2$-4-$CF_3$S-Ph | 0 | |
| Ⅱ-471 | 2,6-$Cl_2$-Ph | 2,3-$Me_2$-4-$CF_3$S-Ph | 1 | |
| Ⅱ-472 | 2,6-$Cl_2$-Ph | 2,3-$Me_2$-4-$CF_3$S-Ph | 2 | |
| Ⅱ-473 | 2,6-$Cl_2$-Ph | 2,3-$Me_2$-4-$CF_3$SO-Ph | 0 | |
| Ⅱ-474 | 2,6-$Cl_2$-Ph | 2,3-$Me_2$-4-$CF_3$SO-Ph | 1 | |
| Ⅱ-475 | 2,6-$Cl_2$-Ph | 2,3-$Me_2$-4-$CF_3$SO-Ph | 2 | |
| Ⅱ-476 | 2,6-$Cl_2$-Ph | 2,3-$Me_2$-4-$CF_3SO_2$-Ph | 0 | |
| Ⅱ-477 | 2,6-$Cl_2$-Ph | 2,3-$Me_2$-4-$CF_3SO_2$-Ph | 1 | |
| Ⅱ-478 | 2,6-$Cl_2$-Ph | 2,3-$Me_2$-4-$CF_3SO_2$-Ph | 2 | |
| Ⅱ-479 | 2,6-$Cl_2$-Ph | 5-Cl-2-Me-4-$CF_3$S-Ph | 0 | |
| Ⅱ-480 | 2,6-$Cl_2$-Ph | 5-Cl-2-Me-4-$CF_3$S-Ph | 1 | |

| compound No. | $Q_1$ | $Q_2$ | n | m. p. (°C) |
|---|---|---|---|---|
| II - 481 | 2,6-$Cl_2$-Ph | 5-Cl-2-Me-4-$CF_3$S-Ph | 2 | |
| II - 482 | 2,6-$Cl_2$-Ph | 5-Cl-2-Me-4-$CF_3$SO-Ph | 0 | |
| II - 483 | 2,6-$Cl_2$-Ph | 5-Cl-2-Me-4-$CF_3$SO-Ph | 1 | |
| II - 484 | 2,6-$Cl_2$-Ph | 5-Cl-2-Me-4-$CF_3$SO-Ph | 2 | |
| II - 485 | 2,6-$Cl_2$-Ph | 5-Cl-2-Me-4-$CF_3SO_2$-Ph | 0 | |
| II - 486 | 2,6-$Cl_2$-Ph | 5-Cl-2-Me-4-$CF_3SO_2$-Ph | 1 | |
| II - 487 | 2,6-$Cl_2$-Ph | 5-Cl-2-Me-4-$CF_3SO_2$-Ph | 2 | |
| II - 488 | 2,6-$Cl_2$-Ph | 2,6-$F_2$-4-$CF_3$S-Ph | 0 | |
| II - 489 | 2,6-$Cl_2$-Ph | 2,6-$F_2$-4-$CF_3$S-Ph | 1 | |
| II - 490 | 2,6-$Cl_2$-Ph | 2,6-$F_2$-4-$CF_3$S-Ph | 2 | |
| II - 491 | 2,6-$Cl_2$-Ph | 2,6-$F_2$-4-$CF_3$SO-Ph | 0 | |
| II - 492 | 2,6-$Cl_2$-Ph | 2,6-$F_2$-4-$CF_3$SO-Ph | 1 | |
| II - 493 | 2,6-$Cl_2$-Ph | 2,6-$F_2$-4-$CF_3$SO-Ph | 2 | |
| II - 494 | 2,6-$Cl_2$-Ph | 2,6-$F_2$-4-$CF_3SO_2$-Ph | 0 | |
| II - 495 | 2,6-$Cl_2$-Ph | 2,6-$F_2$-4-$CF_3SO_2$-Ph | 1 | |
| II - 496 | 2,6-$Cl_2$-Ph | 2,6-$F_2$-4-$CF_3SO_2$-Ph | 2 | |
| II - 497 | 2,6-$Cl_2$-Ph | 2-F-4-$CF_3CF_2$S-Ph | 0 | |
| II - 498 | 2,6-$Cl_2$-Ph | 2-F-4-$CF_3CF_2$S-Ph | 1 | |
| II - 499 | 2,6-$Cl_2$-Ph | 2-F-4-$CF_3CF_2$S-Ph | 2 | |
| II - 500 | 2,6-$Cl_2$-Ph | 2-F-4-$CF_3CF_2$SO-Ph | 0 | |
| II - 501 | 2,6-$Cl_2$-Ph | 2-F-4-$CF_3CF_2$SO-Ph | 1 | |
| II - 502 | 2,6-$Cl_2$-Ph | 2-F-4-$CF_3CF_2$SO-Ph | 2 | |
| II - 503 | 2,6-$Cl_2$-Ph | 2-F-4-$CF_3CF_2SO_2$-Ph | 0 | |
| II - 504 | 2,6-$Cl_2$-Ph | 2-F-4-$CF_3CF_2SO_2$-Ph | 1 | |
| II - 505 | 2,6-$Cl_2$-Ph | 2-F-4-$CF_3CF_2SO_2$-Ph | 2 | |
| II - 506 | 2,6-$Cl_2$-Ph | 2-F-4-$CF_3CF_2CF_2$S-Ph | 0 | |
| II - 507 | 2,6-$Cl_2$-Ph | 2-F-4-$CF_3CF_2CF_2$S-Ph | 1 | |
| II - 508 | 2,6-$Cl_2$-Ph | 2-F-4-$CF_3CF_2CF_2$S-Ph | 2 | |
| II - 509 | 2,6-$Cl_2$-Ph | 2-F-4-$CF_3CF_2CF_2$SO-Ph | 0 | |
| II - 510 | 2,6-$Cl_2$-Ph | 2-F-4-$CF_3CF_2CF_2$SO-Ph | 1 | |
| II - 511 | 2,6-$Cl_2$-Ph | 2-F-4-$CF_3CF_2CF_2$SO-Ph | 2 | |
| II - 512 | 2,6-$Cl_2$-Ph | 2-F-4-$CF_3CF_2CF_2SO_2$-Ph | 0 | |
| II - 513 | 2,6-$Cl_2$-Ph | 2-F-4-$CF_3CF_2CF_2SO_2$-Ph | 1 | |
| II - 514 | 2,6-$Cl_2$-Ph | 2-F-4-$CF_3CF_2CF_2SO_2$-Ph | 2 | |
| II - 515 | 2,6-$Cl_2$-Ph | 2-F-4-$(CF_3)_2$CFS-Ph | 0 | |
| II - 516 | 2,6-$Cl_2$-Ph | 2-F-4-$(CF_3)_2$CFS-Ph | 1 | |
| II - 517 | 2,6-$Cl_2$-Ph | 2-F-4-$(CF_3)_2$CFS-Ph | 2 | |
| II - 518 | 2,6-$Cl_2$-Ph | 2-F-4-$(CF_3)_2$CFSO-Ph | 0 | |
| II - 519 | 2,6-$Cl_2$-Ph | 2-F-4-$(CF_3)_2$CFSO-Ph | 1 | |
| II - 520 | 2,6-$Cl_2$-Ph | 2-F-4-$(CF_3)_2$CFSO-Ph | 2 | |

| compound No. | $Q_1$ | $Q_2$ | n | m. p. (°C) |
|---|---|---|---|---|
| II - 521 | 2,6-$Cl_2$-Ph | 2-F-4-$(CF_3)_2CFSO_2$-Ph | 0 | |
| II - 522 | 2,6-$Cl_2$-Ph | 2-F-4-$(CF_3)_2CFSO_2$-Ph | 1 | |
| II - 523 | 2,6-$Cl_2$-Ph | 2-F-4-$(CF_3)_2CFSO_2$-Ph | 2 | |
| II - 525 | 3-Cl-pyridin-2yl | 4-Cl-Ph | 0 | 48-52 |
| II - 526 | 3-Cl-pyridin-2yl | 4-Cl-Ph | 1 | |
| II - 527 | 3-Cl-pyridin-2yl | 4-Cl-Ph | 2 | |
| II - 528 | 3-Cl-pyridin-2yl | 3,5-$Cl_2$-4-(2-Cl-4-$CF_3$-pyridin-2yl-O)-Ph | 0 | |
| II - 529 | 3-Cl-pyridin-2yl | 3,5-$Cl_2$-4-(2-Cl-4-$CF_3$-pyridin-2yl-O)-Ph | 1 | |
| II - 530 | 3-Cl-pyridin-2yl | 3,5-$Cl_2$-4-(2-Cl-4-$CF_3$-pyridin-2yl-O)-Ph | 2 | |
| II - 531 | 3-Cl-pyridin-2yl | 2-F-4-(2-Cl-4-$CF_3$-Ph-O)-Ph | 0 | |
| II - 532 | 3-Cl-pyridin-2yl | 2-F-4-(2-Cl-4-$CF_3$-Ph-O)-Ph | 1 | |
| II - 533 | 3-Cl-pyridin-2yl | 2-F-4-(2-Cl-4-$CF_3$-Ph-O)-Ph | 2 | |
| II - 534 | 3-Cl-pyridin-2yl | 3,5-$Cl_2$-2,4-$F_2$-Ph | 0 | |
| II - 535 | 3-Cl-pyridin-2yl | 3,5-$Cl_2$-2,4-$F_2$-Ph | 1 | |
| II - 536 | 3-Cl-pyridin-2yl | 3,5-$Cl_2$-2,4-$F_2$-Ph | 2 | |
| II - 537 | 3-Cl-pyridin-2yl | 3,5-$Cl_2$-4-$CF_2HCF_2O$-Ph | 0 | |
| II - 538 | 3-Cl-pyridin-2yl | 3,5-$Cl_2$-4-$CF_2HCF_2O$-Ph | 1 | |
| II - 539 | 3-Cl-pyridin-2yl | 3,5-$Cl_2$-4-$CF_2HCF_2O$-Ph | 2 | |
| II - 540 | 3-Cl-pyridin-2yl | 2,5-$Cl_2$-4-$CF_3CFHCF_2O$-Ph | 0 | |
| II - 541 | 3-Cl-pyridin-2yl | 2,5-$Cl_2$-4-$CF_3CFHCF_2O$-Ph | 1 | |
| II - 542 | 3-Cl-pyridin-2yl | 2,5-$Cl_2$-4-$CF_3CFHCF_2O$-Ph | 2 | |
| II - 543 | 3-Cl-pyridin-2yl | 2-F-4-$CFClHCF_2S$-Ph | 0 | |
| II - 544 | 3-Cl-pyridin-2yl | 2-F-4-$CFClHCF_2S$-Ph | 1 | |
| II - 545 | 3-Cl-pyridin-2yl | 2-F-4-$CFClHCF_2S$-Ph | 2 | |
| II - 546 | 3-Cl-pyridin-2yl | 2-F-4-$CFClHCF_2SO$-Ph | 0 | |
| II - 547 | 3-Cl-pyridin-2yl | 2-F-4-$CFClHCF_2SO$-Ph | 1 | |
| II - 548 | 3-Cl-pyridin-2yl | 2-F-4-$CFClHCF_2SO$-Ph | 2 | |
| II - 549 | 3-Cl-pyridin-2yl | 2-F-4-$CFClHCF_2SO_2$-Ph | 0 | |
| II - 550 | 3-Cl-pyridin-2yl | 2-F-4-$CFClHCF_2SO_2$-Ph | 1 | |
| II - 551 | 3-Cl-pyridin-2yl | 2-F-4-$CFClHCF_2SO_2$-Ph | 2 | |
| II - 552 | 3-Cl-pyridin-2yl | 4-$CF_3S$-Ph | 0 | |
| II - 553 | 3-Cl-pyridin-2yl | 4-$CF_3S$-Ph | 1 | |
| II - 554 | 3-Cl-pyridin-2yl | 4-$CF_3S$-Ph | 2 | |
| II - 555 | 3-Cl-pyridin-2yl | 4-$CF_3SO$-Ph | 0 | |
| II - 556 | 3-Cl-pyridin-2yl | 4-$CF_3SO$-Ph | 1 | |
| II - 557 | 3-Cl-pyridin-2yl | 4-$CF_3SO$-Ph | 2 | |
| II - 558 | 3-Cl-pyridin-2yl | 4-$CF_3SO_2$-Ph | 0 | |
| II - 559 | 3-Cl-pyridin-2yl | 4-$CF_3SO_2$-Ph | 1 | |
| II - 560 | 3-Cl-pyridin-2yl | 4-$CF_3SO_2$-Ph | 2 | |

| compound No. | $Q_1$ | $Q_2$ | n | m. p. (°C) |
|---|---|---|---|---|
| II - 561 | 3-Cl-pyridin-2yl | 2-F-4-MeS-Ph | 0 | |
| II - 562 | 3-Cl-pyridin-2yl | 2-F-4-MeS-Ph | 1 | |
| II - 563 | 3-Cl-pyridin-2yl | 2-F-4-MeS-Ph | 2 | |
| II - 564 | 3-Cl-pyridin-2yl | 2-F-4-MeSO-Ph | 0 | |
| II - 565 | 3-Cl-pyridin-2yl | 2-F-4-MeSO-Ph | 1 | |
| II - 566 | 3-Cl-pyridin-2yl | 2-F-4-MeSO-Ph | 2 | |
| II - 567 | 3-Cl-pyridin-2yl | 2-F-4-$MeSO_2$-Ph | 0 | |
| II - 568 | 3-Cl-pyridin-2yl | 2-F-4-$MeSO_2$-Ph | 1 | |
| II - 569 | 3-Cl-pyridin-2yl | 2-F-4-$MeSO_2$-Ph | 2 | |
| II - 570 | 3-Cl-pyridin-2yl | 2,6-$F_2$-4-MeS-Ph | 0 | |
| II - 571 | 3-Cl-pyridin-2yl | 2,6-$F_2$-4-MeS-Ph | 1 | |
| II - 572 | 3-Cl-pyridin-2yl | 2,6-$F_2$-4-MeS-Ph | 2 | |
| II - 573 | 3-Cl-pyridin-2yl | 2,6-$F_2$-4-MeSO-Ph | 0 | |
| II - 574 | 3-Cl-pyridin-2yl | 2,6-$F_2$-4-MeSO-Ph | 1 | |
| II - 575 | 3-Cl-pyridin-2yl | 2,6-$F_2$-4-MeSO-Ph | 2 | |
| II - 576 | 3-Cl-pyridin-2yl | 2,6-$F_2$-4-$MeSO_2$-Ph | 0 | |
| II - 577 | 3-Cl-pyridin-2yl | 2,6-$F_2$-4-$MeSO_2$-Ph | 1 | |
| II - 578 | 3-Cl-pyridin-2yl | 2,6-$F_2$-4-$MeSO_2$-Ph | 2 | |
| II - 579 | 3-Cl-pyridin-2yl | 2-F-4-$CF_3CH_2S$-Ph | 0 | |
| II - 580 | 3-Cl-pyridin-2yl | 2-F-4-$CF_3CH_2S$-Ph | 1 | |
| II - 581 | 3-Cl-pyridin-2yl | 2-F-4-$CF_3CH_2S$-Ph | 2 | |
| II - 582 | 3-Cl-pyridin-2yl | 2-F-4-$CF_3CH_2SO$-Ph | 0 | |
| II - 583 | 3-Cl-pyridin-2yl | 2-F-4-$CF_3CH_2SO$-Ph | 1 | |
| II - 584 | 3-Cl-pyridin-2yl | 2-F-4-$CF_3CH_2SO$-Ph | 2 | |
| II - 585 | 3-Cl-pyridin-2yl | 2-F-4-$CF_3CH_2SO_2$-Ph | 0 | |
| II - 586 | 3-Cl-pyridin-2yl | 2-F-4-$CF_3CH_2SO_2$-Ph | 1 | |
| II - 587 | 3-Cl-pyridin-2yl | 2-F-4-$CF_3CH_2SO_2$-Ph | 2 | |
| II - 588 | 3-Cl-pyridin-2yl | $CF_3CH_2$ | 0 | |
| II - 589 | 3-Cl-pyridin-2yl | $CF_3CH_2$ | 1 | |
| II - 590 | 3-Cl-pyridin-2yl | $CF_3CH_2$ | 2 | |
| II - 591 | 3-Cl-pyridin-2yl | 2-F-4-$CF_2HS$-Ph | 0 | |
| II - 592 | 3-Cl-pyridin-2yl | 2-F-4-$CF_2HS$-Ph | 1 | |
| II - 593 | 3-Cl-pyridin-2yl | 2-F-4-$CF_2HS$-Ph | 2 | |
| II - 594 | 3-Cl-pyridin-2yl | 2-F-4-$CF_2HSO$-Ph | 0 | |
| II - 595 | 3-Cl-pyridin-2yl | 2-F-4-$CF_2HSO$-Ph | 1 | |
| II - 596 | 3-Cl-pyridin-2yl | 2-F-4-$CF_2HSO$-Ph | 2 | |
| II - 597 | 3-Cl-pyridin-2yl | 2-F-4-$CF_2HSO_2$-Ph | 0 | |
| II - 598 | 3-Cl-pyridin-2yl | 2-F-4-$CF_2HSO_2$-Ph | 1 | |
| II - 599 | 3-Cl-pyridin-2yl | 2-F-4-$CF_2HSO_2$-Ph | 2 | |
| II - 600 | 3-Cl-pyridin-2yl | 2-F-4-$CF_3S$-Ph | 0 | |

| compound No. | $Q_1$ | $Q_2$ | n | m. p. (°C) |
|---|---|---|---|---|
| II - 601 | 3-Cl-pyridin-2yl | 2-F-4-$CF_3$S-Ph | 1 | |
| II - 602 | 3-Cl-pyridin-2yl | 2-F-4-$CF_3$S-Ph | 2 | |
| II - 603 | 3-Cl-pyridin-2yl | 2-F-4-$CF_3$SO-Ph | 0 | |
| II - 604 | 3-Cl-pyridin-2yl | 2-F-4-$CF_3$SO-Ph | 1 | |
| II - 605 | 3-Cl-pyridin-2yl | 2-F-4-$CF_3$SO-Ph | 2 | |
| II - 606 | 3-Cl-pyridin-2yl | 2-F-4-$CF_3SO_2$-Ph | 0 | |
| II - 607 | 3-Cl-pyridin-2yl | 2-F-4-$CF_3SO_2$-Ph | 1 | |
| II - 608 | 3-Cl-pyridin-2yl | 2-F-4-$CF_3SO_2$-Ph | 2 | |
| II - 609 | 3-Cl-pyridin-2yl | 2-F-4-$CH_3CH_2$S-Ph | 0 | |
| II - 610 | 3-Cl-pyridin-2yl | 2-F-4-$CH_3CH_2$S-Ph | 1 | |
| II - 611 | 3-Cl-pyridin-2yl | 2-F-4-$CH_3CH_2$S-Ph | 2 | |
| II - 612 | 3-Cl-pyridin-2yl | 2-F-4-$CH_3CH_2$SO-Ph | 0 | |
| II - 613 | 3-Cl-pyridin-2yl | 2-F-4-$CH_3CH_2$SO-Ph | 1 | |
| II - 614 | 3-Cl-pyridin-2yl | 2-F-4-$CH_3CH_2$SO-Ph | 2 | |
| II - 615 | 3-Cl-pyridin-2yl | 2-F-4-$CH_3CH_2SO_2$-Ph | 0 | |
| II - 616 | 3-Cl-pyridin-2yl | 2-F-4-$CH_3CH_2SO_2$-Ph | 1 | |
| II - 617 | 3-Cl-pyridin-2yl | 2-F-4-$CH_3CH_2SO_2$-Ph | 2 | |
| II - 618 | 3-Cl-pyridin-2yl | 2-F-4-(2-Cl-4-$CF_3$-pyridin-2yl-S)-Ph | 0 | |
| II - 619 | 3-Cl-pyridin-2yl | 2-F-4-(2-Cl-4-$CF_3$-pyridin-2yl-S)-Ph | 1 | |
| II - 620 | 3-Cl-pyridin-2yl | 2-F-4-(2-Cl-4-$CF_3$-pyridin-2yl-S)-Ph | 2 | |
| II - 621 | 3-Cl-pyridin-2yl | 2-F-4-(2-Cl-4-$CF_3$-pyridin-2yl-SO)-Ph | 0 | |
| II - 622 | 3-Cl-pyridin-2yl | 2-F-4-(2-Cl-4-$CF_3$-pyridin-2yl-SO)-Ph | 1 | |
| II - 623 | 3-Cl-pyridin-2yl | 2-F-4-(2-Cl-4-$CF_3$-pyridin-2yl-SO)-Ph | 2 | |
| II - 624 | 3-Cl-pyridin-2yl | 2-F-4-(2-Cl-4-$CF_3$-pyridin-2yl-$SO_2$)-Ph | 0 | |
| II - 625 | 3-Cl-pyridin-2yl | 2-F-4-(2-Cl-4-$CF_3$-pyridin-2yl-$SO_2$)-Ph | 1 | |
| II - 626 | 3-Cl-pyridin-2yl | 2-F-4-(2-Cl-4-$CF_3$-pyridin-2yl-$SO_2$)-Ph | 2 | |
| II - 627 | 3-Cl-pyridin-2yl | 2-Me-4-$CF_3$S-Ph | 0 | |
| II - 628 | 3-Cl-pyridin-2yl | 2-Me-4-$CF_3$S-Ph | 1 | |
| II - 629 | 3-Cl-pyridin-2yl | 2-Me-4-$CF_3$S-Ph | 2 | |
| II - 630 | 3-Cl-pyridin-2yl | 2-Me-4-$CF_3$SO-Ph | 0 | |
| II - 631 | 3-Cl-pyridin-2yl | 2-Me-4-$CF_3$SO-Ph | 1 | |
| II - 632 | 3-Cl-pyridin-2yl | 2-Me-4-$CF_3$SO-Ph | 2 | |
| II - 633 | 3-Cl-pyridin-2yl | 2-Me-4-$CF_3SO_2$-Ph | 0 | |
| II - 634 | 3-Cl-pyridin-2yl | 2-Me-4-$CF_3SO_2$-Ph | 1 | |
| II - 635 | 3-Cl-pyridin-2yl | 2-Me-4-$CF_3SO_2$-Ph | 2 | |
| II - 636 | 3-Cl-pyridin-2yl | 3-F-4-$CF_3$S-Ph | 0 | |
| II - 637 | 3-Cl-pyridin-2yl | 3-F-4-$CF_3$S-Ph | 1 | |
| II - 638 | 3-Cl-pyridin-2yl | 3-F-4-$CF_3$S-Ph | 2 | |
| II - 639 | 3-Cl-pyridin-2yl | 3-F-4-$CF_3$SO-Ph | 0 | |
| II - 640 | 3-Cl-pyridin-2yl | 3-F-4-$CF_3$SO-Ph | 1 | |

| compound No. | $Q_1$ | $Q_2$ | n | m. p. (°C) |
|---|---|---|---|---|
| II-641 | 3-Cl-pyridin-2yl | 3-F-4-$CF_3$SO-Ph | 2 | |
| II-642 | 3-Cl-pyridin-2yl | 3-F-4-$CF_3SO_2$-Ph | 0 | |
| II-643 | 3-Cl-pyridin-2yl | 3-F-4-$CF_3SO_2$-Ph | 1 | |
| II-644 | 3-Cl-pyridin-2yl | 3-F-4-$CF_3SO_2$-Ph | 2 | |
| II-645 | 3-Cl-pyridin-2yl | 2,3-$Me_2$-4-$CF_3$S-Ph | 0 | |
| II-646 | 3-Cl-pyridin-2yl | 2,3-$Me_2$-4-$CF_3$S-Ph | 1 | |
| II-647 | 3-Cl-pyridin-2yl | 2,3-$Me_2$-4-$CF_3$S-Ph | 2 | |
| II-648 | 3-Cl-pyridin-2yl | 2,3-$Me_2$-4-$CF_3$SO-Ph | 0 | |
| II-649 | 3-Cl-pyridin-2yl | 2,3-$Me_2$-4-$CF_3$SO-Ph | 1 | |
| II-650 | 3-Cl-pyridin-2yl | 2,3-$Me_2$-4-$CF_3$SO-Ph | 2 | |
| II-651 | 3-Cl-pyridin-2yl | 2,3-$Me_2$-4-$CF_3SO_2$-Ph | 0 | |
| II-652 | 3-Cl-pyridin-2yl | 2,3-$Me_2$-4-$CF_3SO_2$-Ph | 1 | |
| II-653 | 3-Cl-pyridin-2yl | 2,3-$Me_2$-4-$CF_3SO_2$-Ph | 2 | |
| II-654 | 3-Cl-pyridin-2yl | 5-Cl-2-Me-4-$CF_3$S-Ph | 0 | |
| II-655 | 3-Cl-pyridin-2yl | 5-Cl-2-Me-4-$CF_3$S-Ph | 1 | |
| II-656 | 3-Cl-pyridin-2yl | 5-Cl-2-Me-4-$CF_3$S-Ph | 2 | |
| II-657 | 3-Cl-pyridin-2yl | 5-Cl-2-Me-4-$CF_3$SO-Ph | 0 | |
| II-658 | 3-Cl-pyridin-2yl | 5-Cl-2-Me-4-$CF_3$SO-Ph | 1 | |
| II-659 | 3-Cl-pyridin-2yl | 5-Cl-2-Me-4-$CF_3$SO-Ph | 2 | |
| II-660 | 3-Cl-pyridin-2yl | 5-Cl-2-Me-4-$CF_3SO_2$-Ph | 0 | |
| II-661 | 3-Cl-pyridin-2yl | 5-Cl-2-Me-4-$CF_3SO_2$-Ph | 1 | |
| II-662 | 3-Cl-pyridin-2yl | 5-Cl-2-Me-4-$CF_3SO_2$-Ph | 2 | |
| II-663 | 3-Cl-pyridin-2yl | 2,6-$F_2$-4-$CF_3$S-Ph | 0 | |
| II-664 | 3-Cl-pyridin-2yl | 2,6-$F_2$-4-$CF_3$S-Ph | 1 | |
| II-665 | 3-Cl-pyridin-2yl | 2,6-$F_2$-4-$CF_3$S-Ph | 2 | |
| II-666 | 3-Cl-pyridin-2yl | 2,6-$F_2$-4-$CF_3$SO-Ph | 0 | |
| II-667 | 3-Cl-pyridin-2yl | 2,6-$F_2$-4-$CF_3$SO-Ph | 1 | |
| II-668 | 3-Cl-pyridin-2yl | 2,6-$F_2$-4-$CF_3$SO-Ph | 2 | |
| II-669 | 3-Cl-pyridin-2yl | 2,6-$F_2$-4-$CF_3SO_2$-Ph | 0 | |
| II-670 | 3-Cl-pyridin-2yl | 2,6-$F_2$-4-$CF_3SO_2$-Ph | 1 | |
| II-671 | 3-Cl-pyridin-2yl | 2,6-$F_2$-4-$CF_3SO_2$-Ph | 2 | |
| II-672 | 3-Cl-pyridin-2yl | 2-F-4-$CF_3CF_2$S-Ph | 0 | |
| II-673 | 3-Cl-pyridin-2yl | 2-F-4-$CF_3CF_2$S-Ph | 1 | |
| II-674 | 3-Cl-pyridin-2yl | 2-F-4-$CF_3CF_2$S-Ph | 2 | |
| II-675 | 3-Cl-pyridin-2yl | 2-F-4-$CF_3CF_2$SO-Ph | 0 | |
| II-676 | 3-Cl-pyridin-2yl | 2-F-4-$CF_3CF_2$SO-Ph | 1 | |
| II-677 | 3-Cl-pyridin-2yl | 2-F-4-$CF_3CF_2$SO-Ph | 2 | |
| II-678 | 3-Cl-pyridin-2yl | 2-F-4-$CF_3CF_2SO_2$-Ph | 0 | |
| II-679 | 3-Cl-pyridin-2yl | 2-F-4-$CF_3CF_2SO_2$-Ph | 1 | |
| II-680 | 3-Cl-pyridin-2yl | 2-F-4-$CF_3CF_2SO_2$-Ph | 2 | |

| compound No. | $Q_1$ | $Q_2$ | n | m.p. (°C) |
|---|---|---|---|---|
| II-681 | 3-Cl-pyridin-2yl | 2-F-4-$CF_3CF_2CF_2$S-Ph | 0 | |
| II-682 | 3-Cl-pyridin-2yl | 2-F-4-$CF_3CF_2CF_2$S-Ph | 1 | |
| II-683 | 3-Cl-pyridin-2yl | 2-F-4-$CF_3CF_2CF_2$S-Ph | 2 | |
| II-684 | 3-Cl-pyridin-2yl | 2-F-4-$CF_3CF_2CF_2$SO-Ph | 0 | |
| II-685 | 3-Cl-pyridin-2yl | 2-F-4-$CF_3CF_2CF_2$SO-Ph | 1 | |
| II-686 | 3-Cl-pyridin-2yl | 2-F-4-$CF_3CF_2CF_2$SO-Ph | 2 | |
| II-687 | 3-Cl-pyridin-2yl | 2-F-4-$CF_3CF_2CF_2SO_2$-Ph | 0 | |
| II-688 | 3-Cl-pyridin-2yl | 2-F-4-$CF_3CF_2CF_2SO_2$-Ph | 1 | |
| II-689 | 3-Cl-pyridin-2yl | 2-F-4-$CF_3CF_2CF_2SO_2$-Ph | 2 | |
| II-690 | 3-Cl-pyridin-2yl | 2-F-4-$(CF_3)_2$CFS-Ph | 0 | |
| II-691 | 3-Cl-pyridin-2yl | 2-F-4-$(CF_3)_2$CFS-Ph | 1 | |
| II-692 | 3-Cl-pyridin-2yl | 2-F-4-$(CF_3)_2$CFS-Ph | 2 | |
| II-693 | 3-Cl-pyridin-2yl | 2-F-4-$(CF_3)_2$CFSO-Ph | 0 | |
| II-694 | 3-Cl-pyridin-2yl | 2-F-4-$(CF_3)_2$CFSO-Ph | 1 | |
| II-695 | 3-Cl-pyridin-2yl | 2-F-4-$(CF_3)_2$CFSO-Ph | 2 | |
| II-696 | 3-Cl-pyridin-2yl | 2-F-4-$(CF_3)_2CFSO_2$-Ph | 0 | |
| II-697 | 3-Cl-pyridin-2yl | 2-F-4-$(CF_3)_2CFSO_2$-Ph | 1 | |
| II-698 | 3-Cl-pyridin-2yl | 2-F-4-$(CF_3)_2CFSO_2$-Ph | 2 | |

Table 3

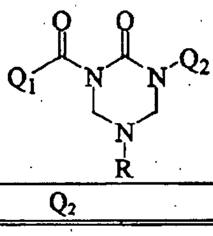

(Ic)

| compound No. | $Q_1$ | $Q_2$ | R | m. p. (℃) |
|---|---|---|---|---|
| Ⅲ-1 | 2,6-$F_2$-Ph | 4-Cl-Ph | Me | 140.0-140.5 |
| Ⅲ-2 | 2,6-$F_2$-Ph | 4-Cl-Ph | Ph | 166.5-167.5 |
| Ⅲ-3 | 2,6-$F_2$-Ph | 4-Cl-Ph | i-Pr | 130-131 |
| Ⅲ-4 | 2,6-$F_2$-Ph | 4-Cl-Ph | $CH_2Ph$ | amorphous (1) |
| Ⅲ-5 | 2,6-$F_2$-Ph | 4-Cl-Ph | $CH_2CH{=}CH_2$ | 87-88 |
| Ⅲ-6 | 2,6-$F_2$-Ph | 4-Cl-Ph | H | 167-168 |
| Ⅲ-7 | 2,6-$F_2$-Ph | 4-Cl-Ph | $COOCH_2Ph$ | oil (2) |
| Ⅲ-8 | 2,6-$F_2$-Ph | 4-Cl-Ph | $CH_2C{\equiv}CH$ | resinoid (3) |
| Ⅲ-9 | 2,6-$F_2$-Ph | 4-Cl-Ph | COMe | 171.0-171.5 |
| Ⅲ-10 | 2,6-$F_2$-Ph | 4-Cl-Ph | $CONMe_2$ | 155.5-157.0 |
| Ⅲ-11 | 2,6-$F_2$-Ph | 4-Cl-Ph | $SO_2CF_3$ | |
| Ⅲ-12 | 2,6-$F_2$-Ph | 4-Cl-Ph | $SO_2Me$ | 178.0-178.5 |
| Ⅲ-13 | 2,6-$F_2$-Ph | 4-Cl-Ph | COOMe | amorphous (4) |
| Ⅲ-14 | 2,6-$F_2$-Ph | 4-Cl-Ph | CHMePh | oil (5) |
| Ⅲ-15 | 2,6-$F_2$-Ph | 4-Cl-Ph | COCOOMe | white amorphous (6) |
| Ⅲ-16 | 2,6-$F_2$-Ph | 4-Cl-Ph | NO | oil (7) |
| Ⅲ-17 | 2,6-$F_2$-Ph | 3,5-$Cl_2$-4-(2-Cl-4-$CF_3$-pyridin-2yl-O)-Ph | Me | 145.0-145.5 |
| Ⅲ-18 | 2,6-$F_2$-Ph | 3,5-$Cl_2$-4-(2-Cl-4-$CF_3$-pyridin-2yl-O)-Ph | COMe | |
| Ⅲ-19 | 2,6-$F_2$-Ph | 3,5-$Cl_2$-4-(2-Cl-4-$CF_3$-pyridin-2yl-O)-Ph | $SO_2Me$ | |
| Ⅲ-20 | 2,6-$F_2$-Ph | 3,5-$Cl_2$-4-(2-Cl-4-$CF_3$-pyridin-2yl-O)-Ph | $SO_2CF_3$ | |
| Ⅲ-21 | 2,6-$F_2$-Ph | 2-F-4-(2-Cl-4-$CF_3$-Ph-O)-Ph | Me | amorphous (8) |
| Ⅲ-22 | 2,6-$F_2$-Ph | 2-F-4-(2-Cl-4-$CF_3$-Ph-O)-Ph | $COOCH_2Ph$ | resinoid (9) |
| Ⅲ-23 | 2,6-$F_2$-Ph | 2-F-4-(2-Cl-4-$CF_3$-Ph-O)-Ph | $CH_2C{\equiv}CH$ | amorphous (10) |
| Ⅲ-24 | 2,6-$F_2$-Ph | 2-F-4-(2-Cl-4-$CF_3$-Ph-O)-Ph | $CH_2CH{=}CH_2$ | resinoid (11) |
| Ⅲ-25 | 2,6-$F_2$-Ph | 2-F-4-(2-Cl-4-$CF_3$-Ph-O)-Ph | i-Pr | 133-134 |

(1) $H^1$-NMR (CDCl$_3$)δppm : 4.12 ( 2H, s, $NCH_2N$), 4.5 7( 2H ,s, $NCH_2N$), 5.08 ( 2H, s, $NCH_2N$), 6.86-6.94 ( 2H, m, Ph), 7.10-7.38 ( 10H, m, Ph).
(2) $H^1$-NMR (CDCl$_3$)δppm : 5.17 ( 2H, s, $NCH_2N$), 5.27 ( 2H, s, $COOCH_2$), 5.57 ( 2H, s, $NCH_2N$), 6.84-6.92 ( 2H, m, Ph), 7.20-7.37 ( 10H, m, Ph).
(3) $H^1$-NMR (CDCl$_3$)δppm : 2.41 ( 1H, t J=2.4Hz, CCH), 3.81 ( 2H, d J=2.4Hz, $NCH_2N$), 4.75 ( 2H, s ,$NCH_2N$), 5.14 ( 2H ,s, $NCH_2N$), 6.83-6.91 ( 2H, m, Ph), 7.16-7.36 ( 5H, m, Ph).
(4) $H^1$-NMR (CDCl$_3$)δppm : 3.87 ( 3H, s, OMe), 5.15 ( 2H, s, $NCH_2N$), 5.53 ( 2H, s, $NCH_2N$), 6.84-6.92 ( 2H, m, Ph), 7.20-7.35 ( 5H, m, Ph).
(5) $H^1$-NMR (CDCl$_3$)δppm : 1.49 ( 3H, d J=6.5Hz, NCHMe), 4.29 ( 1H, q J=6.7Hz, NCH) ,4.36 ( 1H, d J=10.4Hz, NCHN), 4.68 ( 1H, d J=12.2Hz, NCHN) ,4.89 ( 1H, d J=13.3Hz, NCHN), 5.50 ( 1H, d J=13.4Hz, NCHN), 6.85-7.37 ( 12H, m, Ph).
(6) $H^1$-NMR (CDCl$_3$)δppm : (major ) 3.98 ( 3H ,s, OMe), 5.30 ( 2H, s, $NCH_2N$), 5.68 ( 2H, s, $NCH_2N$), 6.91 ( 2H, t J=8.1Hz, Ph), 7.22-7.39 ( 5H, m, Ph) + (minor) 3.89 ( 3H, s, OMe), 5.39 ( 2H, s, $NCH_2N$), 5.63 ( 2H, s, $NCH_2N$), 6.91 ( 2H, t J=8.1Hz, Ph), 7.22-7.39 ( 5H, m, Ph).(rotational isomer mixture)
(7) $H^1$-NMR (CDCl$_3$)δppm : 5.28 ( 1H, s, NCHN), 5.63 ( 1H, s, NCHN), 6.05 ( 1H, s, NCHN), 6.42 ( 1H, s, NCHN), 6.87-6.98 ( 2H, m, Ph), 7.13-7.41 ( 5H, m, Ph).
(8) $H^1$-NMR (CDCl$_3$)δppm : 2.83 ( 3H, s, NMe), 4.42 ( 2H, s, $NCH_2N$), 5.05 ( 2H, s, $NCH_2N$), 6.71-6.79 ( 2H, m, Ph), 6.88 ( 2H, t J=8.3Hz, Ph), 7.70 ( 1H, d J=8.5Hz, Ph), 7.19-7.35 ( 2H, m, Ph), 7.49 ( 1H, dd J=8.5,2.2Hz, Ph), 7.75 ( 1H, d J=2.0Hz, Ph).
(9) $H^1$-NMR (CDCl$_3$)δppm : 5.12 ( 2H, s, $NCH_2N$), 5.28 ( 2H, s, $NCOOCH_2$), 5.59 ( 2H, s, $NCH_2N$), 6.71-6.91 ( 4H, m, Ph), 7.07 ( 1H, d J=8.5Hz, Ph), 7.23-7.36 ( 7H, m, Ph), 7.50 ( 1H, d J=8.6Hz, Ph), 7.75 ( 1H, s, Ph).
(10) $H^1$-NMR (CDCl$_3$)δppm : 2.39 ( 1H, t J=2.5Hz, CCH), 3.84 ( 2H, d J=2.5Hz, $NCH_2$), 4.69 ( 2H, s, $NCH_2N$), 5.17 ( 2H, s, $NCH_2N$), 6.73-6.92 ( 4H, m, Ph), 7.08 ( 1H, d J=8.5Hz, Ph), 7.21-7.33 ( 2H, m, Ph), 7.50 ( 1H, d J=8.1Hz, Ph), 7.75 ( 1H, s, Ph).
(11) $H^1$-NMR (CDCl$_3$)δppm : 3.63 ( 2H, d J=6.5Hz ,$NCH_2$), 4.56 ( 2H, s, $NCH_2N$), 5.10 ( 2H, s, $NCH_2N$), 5.27-5.38 ( 2H, m, $CHCH_2$), 5.81-6.01 ( 1H, m, $CHCH_2$), 6.71-6.92 ( 4H, m, Ph), 7.07 ( 1H, d J=8.6Hz, Ph), 7.16-7.35 ( 2H, m, Ph) ,7.49 ( 1H, dd J=8.6,1.5Hz, Ph), 7.75 ( 1H, d J=1.6Hz, Ph).

| compound No. | $Q_1$ | $Q_2$ | R | m. p. (°C) |
|---|---|---|---|---|
| Ⅲ-26 | 2,6-$F_2$-Ph | 2-F-4-(2-Cl-4-$CF_3$-Ph-O)-Ph | $SO_2Me$ | 165.0-165.5 |
| Ⅲ-27 | 2,6-$F_2$-Ph | 2-F-4-(2-Cl-4-$CF_3$-Ph-O)-Ph | H | 135-137 |
| Ⅲ-28 | 2,6-$F_2$-Ph | 2-F-4-(2-Cl-4-$CF_3$-Ph-O)-Ph | COOMe | amorphous (12) |
| Ⅲ-29 | 2,6-$F_2$-Ph | 2-F-4-(2-Cl-4-$CF_3$-Ph-O)-Ph | $CH_2Ph$ | 129-130 |
| Ⅲ-30 | 2,6-$F_2$-Ph | 2-F-4-(2-Cl-4-$CF_3$-Ph-O)-Ph | Ph | 155.0-155.5 |
| Ⅲ-31 | 2,6-$F_2$-Ph | 2-F-4-(2-Cl-4-$CF_3$-Ph-O)-Ph | SCOOMe | 137-139 |
| Ⅲ-32 | 2,6-$F_2$-Ph | 2-F-4-(2-Cl-4-$CF_3$-Ph-O)-Ph | $COCF_3$ | amorphous (13) |
| Ⅲ-33 | 2,6-$F_2$-Ph | 2-F-4-(2-Cl-4-$CF_3$-Ph-O)-Ph | COMe | |
| Ⅲ-34 | 2,6-$F_2$-Ph | 2-F-4-(2-Cl-4-$CF_3$-Ph-O)-Ph | $SO_2$Ph-4-Me | oil (14) |
| Ⅲ-35 | 2,6-$F_2$-Ph | 2-F-4-(2-Cl-4-$CF_3$-Ph-O)-Ph | $SO_2CF_3$ | oil (15) |
| Ⅲ-36 | 2,6-$F_2$-Ph | 2-F-4-(2-Cl-4-$CF_3$-Ph-O)-Ph | cyclo-Pr | 145.5-155.5 |
| Ⅲ-37 | 2,6-$F_2$-Ph | 2-F-4-(2-Cl-4-$CF_3$-Ph-O)-Ph | COCOOMe | amorphous (16) |
| Ⅲ-38 | 2,6-$F_2$-Ph | 2-F-4-(2-Cl-4-$CF_3$-Ph-O)-Ph | COCOOH | 67-72 |
| Ⅲ-39 | 2,6-$F_2$-Ph | 2-F-4-(2-Cl-4-$CF_3$-Ph-O)-Ph | $COCOOCH_2Ph$ | amorphous (17) |
| Ⅲ-40 | 2,6-$F_2$-Ph | 2-F-4-(2-Cl-4-$CF_3$-Ph-O)-Ph | $COCOOHNH_3$ | 126 (dec.) |
| Ⅲ-41 | 2,6-$F_2$-Ph | 2-F-4-(2-Cl-4-$CF_3$-Ph-O)-Ph | $COCONH_2$ | 78-83 |
| Ⅲ-42 | 2,6-$F_2$-Ph | 2-F-4-(2-Cl-4-$CF_3$-Ph-O)-Ph | COCONHMe | 104-108 |
| Ⅲ-43 | 2,6-$F_2$-Ph | 2-F-4-(2-Cl-4-$CF_3$-Ph-O)-Ph | $COCONMe_2$ | 98-100 |
| Ⅲ-44 | 2,6-$F_2$-Ph | 2-F-4-(2-Cl-4-$CF_3$-Ph-O)-Ph | $COCH_2Br$ | white amorphous (18) |
| Ⅲ-45 | 2,6-$F_2$-Ph | 2-F-4-(2-Cl-4-$CF_3$-Ph-O)-Ph | $COCH_2SMe$ | white amorphous (19) |
| Ⅲ-46 | 2,6-$F_2$-Ph | 2-F-4-(2-Cl-4-$CF_3$-Ph-O)-Ph | $COCH_2SOMe$ | white amorphous (20) |
| Ⅲ-47 | 2,6-$F_2$-Ph | 2-F-4-(2-Cl-4-$CF_3$-Ph-O)-Ph | $COCH_2SO_2Me$ | white amorphous (21) |
| Ⅲ-48 | 2,6-$F_2$-Ph | 2-F-4-(2-Cl-4-$CF_3$-Ph-O)-Ph | COCONMeOMe | oil (22) |
| Ⅲ-49 | 2,6-$F_2$-Ph | 2-F-4-(2-Cl-4-$CF_3$-Ph-O)-Ph | COCONHOMe | 179.5-180 |

(12) $H^1$-NMR (CDCl$_3$)δppm : 3.88 ( 3H, s, COOMe), 5.12 ( 2H, s, $NCH_2N$), 5.57 ( 2H, s, $NCH_2N$), 6.74-6.92 ( 4H, m, Ph), 7.09 ( 1H, d J=8.5Hz, Ph), 7.26-7.34 ( 2H, m, Ph), 7.50 ( 1H, d J=6.7Hz, Ph), 7.75 ( 1H, s, Ph).

(13) $H^1$-NMR (CDCl$_3$)δppm : 5.25 ( 2H, s, $NCH_2N$), 5.72 ( 2H, s, $NCH_2N$), 6.78 ( 2H, d J=8.8Hz, Ph), 6.88-6.97 ( 2H, m, Ph), 7.12 ( 1H, d J=7.3Hz, Ph), 7.26-7.42 ( 2H ,m, Ph), 7.55 ( 1H, d J=7.9Hz, Ph), 7.76 ( 1H, s, Ph).

(14) $H^1$-NMR (CDCl$_3$)δppm : 2.44 ( 3H, s, Me), 5.14 ( 2H, s, $NCH_2N$), 5.61 ( 2H, s, $NCH_2N$), 6.69-6.86 ( 4H, m, Ph), 6.97-7.10 ( 1H, m, Ph), 7.22-7.29 ( 2H, m, Ph), 7.37 ( 2H, d J=8.0Hz, Ph), 7.50 ( 1H, d J=7.9Hz, Ph), 7.75 ( 1H, s, Ph), 7.82 ( 2H, d J=8.1Hz, Ph).

(15) $H^1$-NMR (CDCl$_3$)δppm : 5.11 ( 2H ,s, $NCH_2N$), 5.55 ( 2H, s, $NCH_2N$), 6.77 ( 2H, d J=8.7Hz, Ph), 6.86-6.94 ( 2H, m, Ph), 7.11 ( 1H, d J=8.5Hz, Ph), 7.30-7.38 ( 2H, m, Ph), 7.52 ( 1H, d J=7.3Hz ,Ph), 7.75 ( 1H, s, Ph).

(16) $H^1$-NMR (CDCl$_3$)δppm : (major) 3.97 ( 3H, s, COOMe), 5.24 ( 2H, s, $NCH_2N$), 5.70 ( 2H, s, $NCH_2N$), 6.77 ( 2H, d J=8.9Hz, Ph), 6.87-6.95 ( 2H, m, Ph), 7.10 ( 1H, d J=8.7Hz, Ph), 7.31-7.40 ( 2H, m, Ph), 7.52 ( 1H, d J=8.4Hz, Ph), 7.76 ( 1H, s, Ph) + (minor) 3.91 ( 3H, s, COOMe), 5.33 ( 2H, s, $NCH_2N$), 5.67 ( 2H, s, $NCH_2N$), 6.77 ( 2H, d J=8.9Hz, Ph), 6.87-6.95 ( 2H, m, Ph), 7.10 ( 1H, d J=8.7Hz, Ph), 7.31-7.40 ( 2H, m, Ph), 7.52 ( 1H, d J=8.4Hz, Ph), 7.76 ( 1H, s ,Ph). (rotational isomer mixture)

(17) $H^1$-NMR (CDC$_{13}$)δppm : (major ) 5.21 ( 2H, s, $NCH_2N$), 5.36 ( 2H, s, $COOCH_2Ph$), 5.66 ( 2H, s, $NCH_2N$),6.67-6.91 ( 4H, m, Ph), 7.09 ( 1H, d J=8.5Hz, Ph), 7.25-7.55 ( 8H, m, Ph), 7.75 ( 1H, s, Ph) + (minor)5.21 ( 2H, s, $NCH_2N$), 5.31 ( 2H, s, $COOCH_2Ph$), 5.66 ( 2H, s, $NCH_2N$), 6.67-6.91 ( 4H, m, Ph), 7.09 ( 1H, d J=8.5Hz, Ph), 7.25-7.55 ( 8H, m, Ph), 7.75 ( 1H, s, Ph). (rotational isomer mixture)

(18) $H^1$-NMR (CDCl$_3$)δppm : 4.04 ( 2H, def.s, $BrCH_2$), 5.21 ( 2H, s, $NCH_2N$), 5.67 ( 2H, s, $NCH_2N$), 6.77 ( 2H, d J=8.7Hz, Ph), 6.90 ( 2H, t J=8.1Hz, Ph), 7.10 ( 1H, d J=8.3Hz ,Ph), 7.31-7.36 ( 2H, m, Ph), 7.52 ( 1H, d J=8.3Hz, Ph), 7.76 ( 1H, s, Ph).

(19) $H^1$-NMR (CDCl$_3$)δppm : 2.23 ( 3H, s, SMe), 3.46 ( 2H, def.s, $SCH_2$), 5.21 ( 2H, s, $NCH_2N$), 5.66 ( 2H, s, $NCH_2N$), 6.77 ( 2H, d J=8.7Hz, Ph), 6.89 ( 2H, t J=8.1Hz, Ph), 7.10 ( 1H, d J=8.6Hz, Ph), 7.32 ( 2H, t J=8.4Hz, Ph), 7.51 ( 1H, d J=8.2Hz, Ph), 7.76 ( 1H, s, Ph).

(20) $H^1$-NMR (CDCl$_3$)δppm : 2.79 ( 3H, s, SOMe), 3.58-3.82 ( 2H, br, $SOCH_2$), 5.25 ( 2H, q J=10.1Hz, $NCH_2N$), 5.59-5.84 ( 2H, br, $NCH_2N$), 6.77 ( 2H, d J=8.7Hz, Ph), 6.90 ( 2H, t J=8.5Hz, Ph), 7.10 ( 1H, d J=8.5Hz, Ph), 7.34 ( 2H, br, Ph), 7.52 ( 1H, d J=8.7Hz, Ph), 7.75 ( 1H, s, Ph).

(21) $H^1$-NMR (CDCl$_3$)δppm : 3.16 ( 3H, s, $SO_2Me$), 4.26 ( 2H, def.d J=21Hz, $SO_2CH_2$), 5.25 ( 2H, s, $NCH_2N$), 5.71 ( 2H, def.d J=11.7Hz, $NCH_2N$), 6.77 ( 2H, d J=8.7Hz, Ph), 6.90 ( 2H, t J=8.2Hz, Ph), 7.11 ( 1H, d J=8.5Hz, Ph), 7.30-7.38 ( 2H, m, Ph), 7.52 ( 1H, d J=8.6Hz ,Ph), 7.76 ( 1H,d J=1.8Hz, Ph).

(22) $H^1$-NMR (CDCl$_3$)δppm : (major ) 3.75 ( 3H, s, NMe), 3.96 ( 3H, s, OMe), 5.24 ( 2H, s, $NCH_2N$), 5.70 ( 2H, s, $NCH_2N$), 6.77 ( 2H, d J=8.9Hz, Ph), 6.85-6.94 ( 2H, m, Ph), 7.11 ( 1H, d J=8.6Hz, Ph), 7.27-7.39 ( 2H, m, Ph), 7.52 ( 1H ,d J=8.6Hz, Ph), 7.76 ( 1H d J=1.7Hz, Ph) + (minor) 3.22 ( 3H, s, NMe), 3.88 ( 3H, s, OMe), 5.33 ( 2H, s, $NCH_2N$), 5.66 ( 2H, s, $NCH_2N$), 6.77 ( 2H, d J=8.9Hz, Ph), 6.85-6.94 ( 2H, m, Ph), 7.11 ( 1H, d J=8.6Hz, Ph), 7.27-7.39 ( 2H, m, Ph), 7.52 ( 1H, d J=8.6Hz ,Ph), 7.76 ( 1H, d J=1.7Hz, Ph). (rotational isomer mixture)

| compound No. | $Q_1$ | $Q_2$ | R | m. p. (°C) |
|---|---|---|---|---|
| III-50 | 2,6-$F_2$-Ph | 2-F-4-(2-Cl-4-$CF_3$-Ph-O)-Ph | $COCONHOCH_2Ph$ | 213.5-217 |
| III-51 | 2,6-$F_2$-Ph | 3,5-$Cl_2$-2,4-$F_2$-Ph | Me | 159-160 |
| III-52 | 2,6-$F_2$-Ph | 3,5-$Cl_2$-2,4-$F_2$-Ph | i-Pr | 202-205 |
| III-53 | 2,6-$F_2$-Ph | 3,5-$Cl_2$-2,4-$F_2$-Ph | cyclo-Pr | 216-218 |
| III-54 | 2,6-$F_2$-Ph | 3,5-$Cl_2$-2,4-$F_2$-Ph | Ph | 196-199 |
| III-55 | 2,6-$F_2$-Ph | 3,5-$Cl_2$-2,4-$F_2$-Ph | $COOCH_2Ph$ | amorphous (23) |
| III-56 | 2,6-$F_2$-Ph | 3,5-$Cl_2$-2,4-$F_2$-Ph | CH2CH=CH2 | 178.5-180.0 |
| III-57 | 2,6-$F_2$-Ph | 3,5-$Cl_2$-2,4-$F_2$-Ph | CH2C≡CH | 158-159 |
| III-58 | 2,6-$F_2$-Ph | 3,5-$Cl_2$-2,4-$F_2$-Ph | $SO_2Me$ | 229-230 |
| III-59 | 2,6-$F_2$-Ph | 3,5-$Cl_2$-2,4-$F_2$-Ph | $SO_2CF_3$ | |
| III-60 | 2,6-$F_2$-Ph | 3,5-$Cl_2$-2,4-$F_2$-Ph | $CH_2Ph$ | 118-119 |
| III-61 | 2,6-$F_2$-Ph | 3,5-$Cl_2$-2,4-$F_2$-Ph | COOMe | amorphous (24) |
| III-62 | 2,6-$F_2$-Ph | 3,5-$Cl_2$-2,4-$F_2$-Ph | SCOOMe | 183.5-186.0 |
| III-63 | 2,6-$F_2$-Ph | 3,5-$Cl_2$-2,4-$F_2$-Ph | $SO_2Ph$-4-Me | 150.5-152.5 |
| III-64 | 2,6-$F_2$-Ph | 3,5-$Cl_2$-2,4-$F_2$-Ph | COCOOMe | amorphous (25) |
| III-65 | 2,6-$F_2$-Ph | 3,5-$Cl_2$-2,4-$F_2$-Ph | COMe | |
| III-66 | 2,6-$F_2$-Ph | 3,5-$Cl_2$-4-$CF_2HCF_2O$-Ph | Me | |
| III-67 | 2,6-$F_2$-Ph | 3,5-$Cl_2$-4-$CF_2HCF_2O$-Ph | COMe | |
| III-68 | 2,6-$F_2$-Ph | 3,5-$Cl_2$-4-$CF_2HCF_2O$-Ph | $SO_2Me$ | |
| III-69 | 2,6-$F_2$-Ph | 2,5-$Cl_2$-4-$CF_3CFHCF_2O$-Ph | Me | 159-160 |
| III-70 | 2,6-$F_2$-Ph | 2,5-$Cl_2$-4-$CF_3CFHCF_2O$-Ph | $COOCH_2Ph$ | oil (26) |
| III-71 | 2,6-$F_2$-Ph | 2,5-$Cl_2$-4-$CF_3CFHCF_2O$-Ph | H | 124.5-126.0 |
| III-72 | 2,6-$F_2$-Ph | 2,5-$Cl_2$-4-$CF_3CFHCF_2O$-Ph | COCOOMe | white amorphous (27) |
| III-73 | 2,6-$F_2$-Ph | 2,5-$Cl_2$-4-$CF_3CFHCF_2O$-Ph | COMe | |
| III-74 | 2,6-$F_2$-Ph | 2,5-$Cl_2$-4-$CF_3CFHCF_2O$-Ph | $SO_2Me$ | |
| III-75 | 2,6-$F_2$-Ph | 2,5-$Cl_2$-4-$CF_3CFHCF_2O$-Ph | $SO_2CF_3$ | |
| III-76 | 2,6-$F_2$-Ph | 2-F-4-$CFClHCF_2S$-Ph | Me | 132-134 |
| III-77 | 2,6-$F_2$-Ph | 2-F-4-$CFClHCF_2S$-Ph | $COOCH_2Ph$ | oil (28) |
| III-78 | 2,6-$F_2$-Ph | 2-F-4-$CFClHCF_2S$-Ph | H | 144-147.5 |
| III-79 | 2,6-$F_2$-Ph | 2-F-4-$CFClHCF_2S$-Ph | COCOOMe | white amorphous (29) |
| III-80 | 2,6-$F_2$-Ph | 2-F-4-$CFClHCF_2S$-Ph | Ph | |
| III-81 | 2,6-$F_2$-Ph | 2-F-4-$CFClHCF_2S$-Ph | COMe | |

(23) $H^1$-NMR ($CDCl_3$)δppm: 5.11 ( 2H, s, $NCH_2N$), 5.28 ( 2H, s, $NCOOCH_2$), 5.59 ( 2H, s, $NCH_2N$), 6.85-6.93 ( 2H, m, Ph), 7.26-7.37 ( 7H, m, Ph).
(24) $H^1$-NMR ($CDCl_3$)δppm: 3.89 ( 3H ,s, COOMe), 5.11 ( 2H, s, $NCH_2N$), 5.57 ( 2H, s, $NCH_2N$), 6.86-6.94 ( 2H, m, Ph), 7.26-7.39 ( 2H, m ,Ph).
(25) H1-NMR ($CDCl_3$)δppm : (major ) 3.98 ( 3H, s, COOMe), 5.23 ( 2H, s, $NCH_2N$), 5.71 ( 2H, s, $NCH_2N$), 6.92 ( 2H, t J=8.1Hz, Ph), 7.36 (2H, t J=7.3Hz, Ph) + (minor) 3.94 ( 3H, s, COOMe), 5.34 ( 2H, s, $NCH_2N$), 5.67 ( 2H, s, $NCH_2N$), 6.92 ( 2H, t J=8.1Hz, Ph), 7.36 ( 2H, t J=7.3Hz, Ph), 7.27-7.39 ( 2H, m, Ph), 7.52 ( 1H, d J=8.6Hz ,Ph), 7.76 ( 1H, d J=1.7Hz, Ph). (rotational isomer mixture)
(26) $H^1$-NMR ($CDCl_3$)δppm:4.90-5.30 ( 6H, m, $NCH_2N$+ $NCH_2N$+$COOCH_2$), 5.87 ( 1H, br, $CHFCF_3$), 6.88 ( 2H, t J=8.5Hz, Ph), 7.24-7.45 ( 8H, m, Ph).
(27) $H^1$-NMR ($CDCl_3$)δppm : (major) 3.98 ( 3H, s, COOMe), 4.90-5.92 ( 5H, m, $NCH_2N$+ $NCH_2N$+ $CHFCF_3$), 6.91 ( 2H, t J=8.4Hz, Ph), 7.28-7.40 ( 1H, m, Ph), 7.50 ( 2H, s, Ph) + (minor) 3.92 ( 3H, s, COOMe),4 .90-5.92 ( 5H, m, $NCH_2N$+$NCH_2N$+$CHFCF_3$), 6.91 ( 2H, t J=8.4Hz, Ph), 7.28-7.40 ( 1H, m, Ph), 7.50 ( 2H, s, Ph). (rotational isomer mixture)
(28) $H^1$-NMR ($CDCl_3$)δppm : 5.16 ( 2H, s, $COOCH_2$), 5.27 ( 2H, s, $NCH_2N$), 5.60 ( 2H, s, $NCH_2N$), 6.14 ( 1H, ddd J=48.4,6.9,4.2Hz, CHClF), 6.88 ( 2H, t J=8.1Hz, Ph), 7.27-7.45 (9H, m, Ph).
(29) $H^1$-NMR ($CDCl_3$)δppm : (major ) 3.98 ( 3H, s, COOMe), 4.90-5.92 ( 5H, m, $NCH_2N$+ $NCH_2N$+ CHClF), 6.91 ( 2H, t J=8.4Hz ,Ph), 7.28-7.40 ( 1H, m, Ph), 7.50 ( 1H, s, Ph) + (minor) 3.92 ( 3H, s, COOMe), 4.90-5.92 ( 5H, m, $NCH_2N$+$NCH_2N$+CHClF), 6.91 ( 2H, t J=8.4Hz, Ph), 7.28-7.40 ( 1H, m, Ph), 7.50 ( 1H, s, Ph). (rotational isomer mixture)

| compound No. | $Q_1$ | $Q_2$ | R | m. p. (°C) |
|---|---|---|---|---|
| III-82 | 2,6-$F_2$-Ph | 2-F-4-CFClHCF$_2$S-Ph | $SO_2Me$ | |
| III-83 | 2,6-$F_2$-Ph | 2-F-4-CFClHCF$_2$S-Ph | $SO_2CF_3$ | |
| III-84 | 2,6-$F_2$-Ph | 2-F-4-CFClHCF$_2$SO-Ph | Me | 129-131.5 |
| III-85 | 2,6-$F_2$-Ph | 2-F-4-CFClHCF$_2$SO-Ph | $COOCH_2Ph$ | oil (30) |
| III-86 | 2,6-$F_2$-Ph | 2-F-4-CFClHCF$_2$SO-Ph | COMe | |
| III-87 | 2,6-$F_2$-Ph | 2-F-4-CFClHCF$_2$SO-Ph | $SO_2Me$ | |
| III-88 | 2,6-$F_2$-Ph | 2-F-4-CFClHCF$_2$SO-Ph | $SO_2CF_3$ | |
| III-89 | 2,6-$F_2$-Ph | 2-F-4-CFClHCF$_2$SO-Ph | $CH_2C{\equiv}CH$ | 160-163.5 |
| III-90 | 2,6-$F_2$-Ph | 2-F-4-CFClHCF$_2$SO-Ph | $CH_2CH{=}CH_2$ | |
| III-91 | 2,6-$F_2$-Ph | 2-F-4-CFClHCF$_2$SO-Ph | H | 144-147 |
| III-92 | 2,6-$F_2$-Ph | 2-F-4-CFClHCF$_2SO_2$-Ph | Me | |
| III-93 | 2,6-$F_2$-Ph | 2-F-4-CFClHCF$_2SO_2$-Ph | COMe | |
| III-94 | 2,6-$F_2$-Ph | 2-F-4-CFClHCF$_2SO_2$-Ph | $SO_2Me$ | |
| III-95 | 2,6-$F_2$-Ph | 2-F-4-CFClHCF$_2SO_2$-Ph | $SO_2CF_3$ | |
| III-96 | 2,6-$F_2$-Ph | 4-$CF_3$S-Ph | Me | |
| III-97 | 2,6-$F_2$-Ph | 4-$CF_3$S-Ph | COMe | |
| III-98 | 2,6-$F_2$-Ph | 4-$CF_3$S-Ph | $SO_2Me$ | |
| III-99 | 2,6-$F_2$-Ph | 4-$CF_3$S-Ph | $SO_2CF_3$ | |
| III-100 | 2,6-$F_2$-Ph | 4-$CF_3$SO-Ph | Me | |
| III-101 | 2,6-$F_2$-Ph | 4-$CF_3$SO-Ph | COMe | |
| III-102 | 2,6-$F_2$-Ph | 4-$CF_3$SO-Ph | $SO_2Me$ | |
| III-103 | 2,6-$F_2$-Ph | 4-$CF_3$SO-Ph | $SO_2CF_3$ | |
| III-104 | 2,6-$F_2$-Ph | 4-$CF_3SO_2$-Ph | Me | |
| III-105 | 2,6-$F_2$-Ph | 4-$CF_3SO_2$-Ph | COMe | |
| III-106 | 2,6-$F_2$-Ph | 4-$CF_3SO_2$-Ph | $SO_2Me$ | |
| III-107 | 2,6-$F_2$-Ph | 4-$CF_3SO_2$-Ph | $SO_2CF_3$ | |
| III-108 | 2,6-$F_2$-Ph | 2-F-4-MeS-Ph | Me | |
| III-109 | 2,6-$F_2$-Ph | 2-F-4-MeS-Ph | COMe | |
| III-110 | 2,6-$F_2$-Ph | 2-F-4-MeS-Ph | $SO_2Me$ | |
| III-111 | 2,6-$F_2$-Ph | 2-F-4-MeS-Ph | $SO_2CF_3$ | |
| III-112 | 2,6-$F_2$-Ph | 2-F-4-MeSO-Ph | Me | |
| III-113 | 2,6-$F_2$-Ph | 2-F-4-MeSO-Ph | COMe | |
| III-114 | 2,6-$F_2$-Ph | 2-F-4-MeSO-Ph | $SO_2Me$ | |
| III-115 | 2,6-$F_2$-Ph | 2-F-4-MeSO-Ph | $SO_2CF_3$ | |
| III-116 | 2,6-$F_2$-Ph | 2-F-4-$MeSO_2$-Ph | Me | |
| III-117 | 2,6-$F_2$-Ph | 2-F-4-$MeSO_2$-Ph | COMe | |
| III-118 | 2,6-$F_2$-Ph | 2-F-4-$MeSO_2$-Ph | $SO_2Me$ | |
| III-119 | 2,6-$F_2$-Ph | 2-F-4-$MeSO_2$-Ph | $SO_2CF_3$ | |
| III-120 | 2,6-$F_2$-Ph | 2,6-$F_2$-4-MeS-Ph | Me | |

(30) $H^1$-NMR ($CDCl_3$)δppm : 5.19 ( 2H, s, $COOCH_2$), 5.28 ( 2H, s, $NCH_2N$), 5.61 ( 2H, s, $NCH_2N$), 6.44-6.67 ( 1H, m, CHClF), 6.8 9( 2H, t J=8.3Hz, Ph), 7.29-7.83 ( 9H, m, Ph).

| compound No. | $Q_1$ | $Q_2$ | R | m. p. (°C) |
|---|---|---|---|---|
| III-121 | 2,6-$F_2$-Ph | 2,6-$F_2$-4-MeS-Ph | COMe | |
| III-122 | 2,6-$F_2$-Ph | 2,6-$F_2$-4-MeS-Ph | $SO_2Me$ | |
| III-123 | 2,6-$F_2$-Ph | 2,6-$F_2$-4-MeS-Ph | $SO_2CF_3$ | |
| III-124 | 2,6-$F_2$-Ph | 2,6-$F_2$-4-MeSO-Ph | Me | |
| III-125 | 2,6-$F_2$-Ph | 2,6-$F_2$-4-MeSO-Ph | COMe | |
| III-126 | 2,6-$F_2$-Ph | 2,6-$F_2$-4-MeSO-Ph | $SO_2Me$ | |
| III-127 | 2,6-$F_2$-Ph | 2,6-$F_2$-4-MeSO-Ph | $SO_2CF_3$ | |
| III-128 | 2,6-$F_2$-Ph | 2,6-$F_2$-4-$MeSO_2$-Ph | Me | |
| III-129 | 2,6-$F_2$-Ph | 2,6-$F_2$-4-$MeSO_2$-Ph | COMe | |
| III-130 | 2,6-$F_2$-Ph | 2,6-$F_2$-4-$MeSO_2$-Ph | $SO_2Me$ | |
| III-131 | 2,6-$F_2$-Ph | 2,6-$F_2$-4-$MeSO_2$-Ph | $SO_2CF_3$ | |
| III-132 | 2,6-$F_2$-Ph | 2-F-4-$CF_3CH_2S$-Ph | Me | |
| III-133 | 2,6-$F_2$-Ph | 2-F-4-$CF_3CH_2S$-Ph | COMe | |
| III-134 | 2,6-$F_2$-Ph | 2-F-4-$CF_3CH_2S$-Ph | $SO_2Me$ | |
| III-135 | 2,6-$F_2$-Ph | 2-F-4-$CF_3CH_2S$-Ph | $SO_2CF_3$ | |
| III-136 | 2,6-$F_2$-Ph | 2-F-4-$CF_3CH_2SO$-Ph | Me | |
| III-137 | 2,6-$F_2$-Ph | 2-F-4-$CF_3CH_2SO$-Ph | COMe | |
| III-138 | 2,6-$F_2$-Ph | 2-F-4-$CF_3CH_2SO$-Ph | SO2Me | |
| III-139 | 2,6-$F_2$-Ph | 2-F-4-$CF_3CH_2SO$-Ph | $SO_2CF_3$ | |
| III-140 | 2,6-$F_2$-Ph | 2-F-4-$CF_3CH_2SO_2$-Ph | Me | |
| III-141 | 2,6-$F_2$-Ph | 2-F-4-$CF_3CH_2SO_2$-Ph | COMe | |
| III-142 | 2,6-$F_2$-Ph | 2-F-4-$CF_3CH_2SO_2$-Ph | $SO_2Me$ | |
| III-143 | 2,6-$F_2$-Ph | 2-F-4-$CF_3CH_2SO_2$-Ph | $SO_2CF_3$ | |
| III-144 | 2,6-$F_2$-Ph | $CF_3CH_2$ | Me | 97-99 |
| III-145 | 2,6-$F_2$-Ph | $CF_3CH_2$ | COMe | |
| III-146 | 2,6-$F_2$-Ph | $CF_3CH_2$ | $SO_2Me$ | |
| III-147 | 2,6-$F_2$-Ph | $CF_3CH_2$ | $SO_2CF_3$ | |
| III-148 | 2,6-$F_2$-Ph | 2-F-4-$CF_2HS$-Ph | Me | 135-137 |
| III-149 | 2,6-$F_2$-Ph | 2-F-4-$CF_2HS$-Ph | COMe | amorphous (31) |
| III-150 | 2,6-$F_2$-Ph | 2-F-4-$CF_2HS$-Ph | $SO_2Me$ | amorphous (32) |
| III-151 | 2,6-$F_2$-Ph | 2-F-4-$CF_2HS$-Ph | $SO_2CF_3$ | |
| III-152 | 2,6-$F_2$-Ph | 2-F-4-$CF_2HSO$-Ph | Me | 50-55 |
| III-153 | 2,6-$F_2$-Ph | 2-F-4-$CF_2HSO$-Ph | COMe | oil (33) |
| III-154 | 2,6-$F_2$-Ph | 2-F-4-$CF_2HSO$-Ph | $SO_2Me$ | oil (34) |
| III-155 | 2,6-$F_2$-Ph | 2-F-4-$CF_2HSO$-Ph | $SO_2CF_3$ | |
| III-156 | 2,6-$F_2$-Ph | 2-F-4-$CF_2HSO_2$-Ph | Me | 203-205 |
| III-157 | 2,6-$F_2$-Ph | 2-F-4-$CF_2HSO_2$-Ph | COMe | |
| III-158 | 2,6-$F_2$-Ph | 2-F-4-$CF_2HSO_2$-Ph | $SO_2Me$ | |
| III-159 | 2,6-$F_2$-Ph | 2-F-4-$CF_2HSO_2$-Ph | $SO_2CF_3$ | |
| III-160 | 2,6-$F_2$-Ph | 2-F-4-$CF_3S$-Ph | Me | 92-94 |

(31) $H^1$-NMR ($CDCl_3$)δppm : 2.34 ( 3H, s, $COCH_3$), 5.22 ( 2H, s, $NCH_2N$), 5.63 ( 2H, s, $NCH_2N$), 6.68-6.96 ( 3H, m, Ph+$CF_2H$), 7.30-7.39 ( 4H, m, Ph).
(32) $H^1$-NMR ($CDCl_3$)δppm : 3.21 ( 3H, s, $SO_2$ $CH_3$), 5.14 ( 2H, s, $NCH_2N$), 5.62 ( 2H, s, $NCH_2N$), 6.68-6.96 ( 3H, m, Ph+$CF_2H$), 7.31-7.43 ( 4H, m, Ph).
(33) $H^1$-NMR ($CDCl_3$)δppm : 2.35 ( 3H, s, $COCH_3$), 5.25 ( 2H, s, $NCH_2N$), 5.65 ( 2H, s, $NCH_2N$), 6.04 ( 1H, t J=55.3Hz, $CF_2H$), 6.89-6.93 ( 2H, m, Ph), 7.32-7.62 ( 4H, m, Ph).
(34) $H^1$-NMR ($CDCl_3$)δppm : 3.22 ( 3H, s, $SO_2$ $CH_3$), 5.18 ( 2H, s, $NCH_2N$), 5.63 ( 2H, s, $NCH_2N$), 6.05 ( 1H, t J=55.1Hz, $CF_2H$), 6.88-6.92 ( 2H, m, Ph), 7.26-7.60 ( 4H, m, Ph).

| compound No. | $Q_1$ | $Q_2$ | R | m. p. (°C) |
|---|---|---|---|---|
| Ⅲ-161 | 2,6-$F_2$-Ph | 2-F-4-$CF_3$S-Ph | COMe | amorphous (35) |
| Ⅲ-162 | 2,6-$F_2$-Ph | 2-F-4-$CF_3$S-Ph | $SO_2$Me | oil (36) |
| Ⅲ-163 | 2,6-$F_2$-Ph | 2-F-4-$CF_3$S-Ph | $SO_2CF_3$ | |
| Ⅲ-164 | 2,6-$F_2$-Ph | 2-F-4-$CF_3$SO-Ph | Me | amorphous (37) |
| Ⅲ-165 | 2,6-$F_2$-Ph | 2-F-4-$CF_3$SO-Ph | COMe | amorphous (38) |
| Ⅲ-166 | 2,6-$F_2$-Ph | 2-F-4-$CF_3$SO-Ph | $SO_2$Me | amorphous (39) |
| Ⅲ-167 | 2,6-$F_2$-Ph | 2-F-4-$CF_3$SO-Ph | $SO_2CF_3$ | |
| Ⅲ-168 | 2,6-$F_2$-Ph | 2-F-4-$CF_3SO_2$-Ph | Me | |
| Ⅲ-169 | 2,6-$F_2$-Ph | 2-F-4-$CF_3SO_2$-Ph | COMe | |
| Ⅲ-170 | 2,6-$F_2$-Ph | 2-F-4-$CF_3SO_2$-Ph | $SO_2$Me | |
| Ⅲ-171 | 2,6-$F_2$-Ph | 2-F-4-$CF_3SO_2$-Ph | $SO_2CF_3$ | |
| Ⅲ-172 | 2,6-$F_2$-Ph | 2-F-4-$CH_3CH_2$S-Ph | Me | |
| Ⅲ-173 | 2,6-$F_2$-Ph | 2-F-4-$CH_3CH_2$S-Ph | COMe | |
| Ⅲ-174 | 2,6-$F_2$-Ph | 2-F-4-$CH_3CH_2$S-Ph | $SO_2$Me | |
| Ⅲ-175 | 2,6-$F_2$-Ph | 2-F-4-$CH_3CH_2$S-Ph | $SO_2CF_3$ | |
| Ⅲ-176 | 2,6-$F_2$-Ph | 2-F-4-$CH_3CH_2$SO-Ph | Me | |
| Ⅲ-177 | 2,6-$F_2$-Ph | 2-F-4-$CH_3CH_2$SO-Ph | COMe | |
| Ⅲ-178 | 2,6-$F_2$-Ph | 2-F-4-$CH_3CH_2$SO-Ph | $SO_2$Me | |
| Ⅲ-179 | 2,6-$F_2$-Ph | 2-F-4-$CH_3CH_2$SO-Ph | $SO_2CF_3$ | |
| Ⅲ-180 | 2,6-$F_2$-Ph | 2-F-4-$CH_3CH_2SO_2$-Ph | Me | |
| Ⅲ-181 | 2,6-$F_2$-Ph | 2-F-4-$CH_3CH_2SO_2$-Ph | COMe | |
| Ⅲ-182 | 2,6-$F_2$-Ph | 2-F-4-$CH_3CH_2SO_2$-Ph | $SO_2$Me | |
| Ⅲ-183 | 2,6-$F_2$-Ph | 2-F-4-$CH_3CH_2SO_2$-Ph | $SO_2CF_3$ | |
| Ⅲ-184 | 2,6-$F_2$-Ph | 2-F-4-(2-Cl-4-$CF_3$-pyridin-2yl-S)-Ph | Me | 150-152 |
| Ⅲ-185 | 2,6-$F_2$-Ph | 2-F-4-(2-Cl-4-$CF_3$-pyridin-2yl-S)-Ph | COMe | |
| Ⅲ-186 | 2,6-$F_2$-Ph | 2-F-4-(2-Cl-4-$CF_3$-pyridin-2yl-S)-Ph | $SO_2$Me | |
| Ⅲ-187 | 2,6-$F_2$-Ph | 2-F-4-(2-Cl-4-$CF_3$-pyridin-2yl-S)-Ph | $SO_2CF_3$ | |
| Ⅲ-188 | 2,6-$F_2$-Ph | 2-F-4-(2-Cl-4-$CF_3$-pyridin-2yl-SO)-Ph | Me | white amorphous (40) |
| Ⅲ-189 | 2,6-$F_2$-Ph | 2-F-4-(2-Cl-4-$CF_3$-pyridin-2yl-SO)-Ph | COMe | |
| Ⅲ-190 | 2,6-$F_2$-Ph | 2-F-4-(2-Cl-4-$CF_3$-pyridin-2yl-SO)-Ph | $SO_2$Me | |
| Ⅲ-191 | 2,6-$F_2$-Ph | 2-F-4-(2-Cl-4-$CF_3$-pyridin-2yl-SO)-Ph | $SO_2CF_3$ | |
| Ⅲ-192 | 2,6-$F_2$-Ph | 2-F-4-(2-Cl-4-$CF_3$-pyridin-2yl-$SO_2$)-Ph | Me | 170-172 |
| Ⅲ-193 | 2,6-$F_2$-Ph | 2-F-4-(2-Cl-4-$CF_3$-pyridin-2yl-$SO_2$)-Ph | COMe | |
| Ⅲ-194 | 2,6-$F_2$-Ph | 2-F-4-(2-Cl-4-$CF_3$-pyridin-2yl-$SO_2$)-Ph | $SO_2$Me | |
| Ⅲ-195 | 2,6-$F_2$-Ph | 2-F-4-(2-Cl-4-$CF_3$-pyridin-2yl-$SO_2$)-Ph | $SO_2CF_3$ | |
| Ⅲ-196 | 2,6-$F_2$-Ph | 2-Me-4-$CF_3$S-Ph | Me | |
| Ⅲ-197 | 2,6-$F_2$-Ph | 2-Me-4-$CF_3$S-Ph | COMe | |
| Ⅲ-198 | 2,6-$F_2$-Ph | 2-Me-4-$CF_3$S-Ph | $SO_2$Me | |
| Ⅲ-199 | 2,6-$F_2$-Ph | 2-Me-4-$CF_3$S-Ph | $SO_2CF_3$ | |
| Ⅲ-200 | 2,6-$F_2$-Ph | 2-Me-4-$CF_3$SO-Ph | Me | |

(35) $H^1$-NMR ($CDCl_3$)δppm : 2.34 ( 3H, s, $COCH_3$), 5.23 ( 2H, s, $NCH_2N$), 5.63 ( 2H, s, $NCH_2N$), 6.88-6.92 ( 2H, m, Ph), 7.26-7.47 ( 4H, m, Ph).
(36) $H^1$-NMR ($CDCl_3$)δppm : 3.21 ( 3H, s, $SO_2$ $CH_3$), 5.16 ( 2H, s, $NCH_2N$), 5.62 ( 2H, s, $NCH_2N$), 6.88-6.92 ( 2H, m, Ph), 7.33-7.50 ( 4H, m, Ph).
(37) $H^1$-NMR ($CDCl_3$)δppm : 2.84 ( 3H, s, NMe), 4.60 (2H, s, $NCH_2N$), 5.06 ( 2H, s, $NCH_2N$), 6.87-6.91 ( 2H, m, Ph), 7.26-7.32 ( 1H, m, Ph), 7.53-7.54 ( 2H, m, Ph), 7.60-7.63 ( 1H, m, Ph) .
(38) $H^1$-NMR ($CDCl_3$)δppm : 2.35 ( 3H, s, $COCH_3$), 5.26 ( 2H, s, $NCH_2N$), 5.65 ( 2H, s, $NCH_2N$), 6.89-6.93 ( 2H, m, Ph), 7.27-7.37 ( 1H, m, Ph), 7.58-7.64 ( 3H, m, Ph).
(39) $H^1$-NMR ($CDCl_3$)δppm : 3.21 ( 3H, s, $SO_2$ $CH_3$), 5.19 ( 2H, s, $NCH_2N$), 5.62 ( 2H, s, $NCH_2N$), 6.88-6.93 ( 2H, m, Ph), 7.26-7.35 ( 1H, m, Ph), 7.58-7.68 ( 3H, m, Ph).
(40) $H^1$-NMR ($CDCl_3$)δppm : 2.80 ( 3H, s, NMe), 4.53 (2H, s, $CH_2$), 5.03 ( 2H, s, $CH_2$), 6.83-6.87 ( 2H, m, Ph), 7.24-7.40 ( 2H, m, Ph), 7.64-7.69 ( 2H, m, Ph), 7.94 ( 1H, m, Py.), 8.90 ( 1H, m, Py,) .

| compound No. | $Q_1$ | $Q_2$ | R | m. p. (°C) |
|---|---|---|---|---|
| Ⅲ-201 | 2,6-$F_2$-Ph | 2-Me-4-$CF_3$SO-Ph | COMe | |
| Ⅲ-202 | 2,6-$F_2$-Ph | 2-Me-4-$CF_3$SO-Ph | $SO_2$Me | |
| Ⅲ-203 | 2,6-$F_2$-Ph | 2-Me-4-$CF_3$SO-Ph | $SO_2CF_3$ | |
| Ⅲ-204 | 2,6-$F_2$-Ph | 2-Me-4-$CF_3SO_2$-Ph | Me | |
| Ⅲ-205 | 2,6-$F_2$-Ph | 2-Me-4-$CF_3SO_2$-Ph | COMe | |
| Ⅲ-206 | 2,6-$F_2$-Ph | 2-Me-4-$CF_3SO_2$-Ph | $SO_2$Me | |
| Ⅲ-207 | 2,6-$F_2$-Ph | 2-Me-4-$CF_3SO_2$-Ph | $SO_2CF_3$ | |
| Ⅲ-208 | 2,6-$F_2$-Ph | 3-F-4-$CF_3$S-Ph | Me | |
| Ⅲ-209 | 2,6-$F_2$-Ph | 3-F-4-$CF_3$S-Ph | COMe | |
| Ⅲ-210 | 2,6-$F_2$-Ph | 3-F-4-$CF_3$S-Ph | $SO_2$Me | |
| Ⅲ-211 | 2,6-$F_2$-Ph | 3-F-4-$CF_3$S-Ph | $SO_2CF_3$ | |
| Ⅲ-212 | 2,6-$F_2$-Ph | 3-F-4-$CF_3$SO-Ph | Me | |
| Ⅲ-213 | 2,6-$F_2$-Ph | 3-F-4-$CF_3$SO-Ph | COMe | |
| Ⅲ-214 | 2,6-$F_2$-Ph | 3-F-4-$CF_3$SO-Ph | $SO_2$Me | |
| Ⅲ-215 | 2,6-$F_2$-Ph | 3-F-4-$CF_3$SO-Ph | $SO_2CF_3$ | |
| Ⅲ-216 | 2,6-$F_2$-Ph | 3-F-4-$CF_3SO_2$-Ph | Me | |
| Ⅲ-217 | 2,6-$F_2$-Ph | 3-F-4-$CF_3SO_2$-Ph | COMe | |
| Ⅲ-218 | 2,6-$F_2$-Ph | 3-F-4-$CF_3SO_2$-Ph | $SO_2$Me | |
| Ⅲ-219 | 2,6-$F_2$-Ph | 3-F-4-$CF_3SO_2$-Ph | $SO_2CF_3$ | |
| Ⅲ-220 | 2,6-$F_2$-Ph | 2,3-$Me_2$-4-$CF_3$S-Ph | Me | |
| Ⅲ-221 | 2,6-$F_2$-Ph | 2,3-$Me_2$-4-$CF_3$S-Ph | COMe | 132-140 |
| Ⅲ-222 | 2,6-$F_2$-Ph | 2,3-$Me_2$-4-$CF_3$S-Ph | $SO_2$Me | 125-130 |
| Ⅲ-223 | 2,6-$F_2$-Ph | 2,3-$Me_2$-4-$CF_3$S-Ph | $SO_2CF_3$ | |
| Ⅲ-224 | 2,6-$F_2$-Ph | 2,3-$Me_2$-4-$CF_3$SO-Ph | Me | |
| Ⅲ-225 | 2,6-$F_2$-Ph | 2,3-$Me_2$-4-$CF_3$SO-Ph | COMe | 134-139 |
| Ⅲ-226 | 2,6-$F_2$-Ph | 2,3-$Me_2$-4-$CF_3$SO-Ph | $SO_2$Me | |
| Ⅲ-227 | 2,6-$F_2$-Ph | 2,3-$Me_2$-4-$CF_3$SO-Ph | $SO_2CF_3$ | |
| Ⅲ-228 | 2,6-$F_2$-Ph | 2,3-$Me_2$-4-$CF_3SO_2$-Ph | Me | |
| Ⅲ-229 | 2,6-$F_2$-Ph | 2,3-$Me_2$-4-$CF_3SO_2$-Ph | COMe | |
| Ⅲ-230 | 2,6-$F_2$-Ph | 2,3-$Me_2$-4-$CF_3SO_2$-Ph | $SO_2$Me | |
| Ⅲ-231 | 2,6-$F_2$-Ph | 2,3-$Me_2$-4-$CF_3SO_2$-Ph | $SO_2CF_3$ | |
| Ⅲ-232 | 2,6-$F_2$-Ph | 5-Cl-2-Me-4-$CF_3$S-Ph | Me | |
| Ⅲ-233 | 2,6-$F_2$-Ph | 5-Cl-2-Me-4-$CF_3$S-Ph | COMe | |
| Ⅲ-234 | 2,6-$F_2$-Ph | 5-Cl-2-Me-4-$CF_3$S-Ph | $SO_2$Me | |
| Ⅲ-235 | 2,6-$F_2$-Ph | 5-Cl-2-Me-4-$CF_3$S-Ph | $SO_2CF_3$ | |
| Ⅲ-236 | 2,6-$F_2$-Ph | 5-Cl-2-Me-4-$CF_3$SO-Ph | Me | |
| Ⅲ-237 | 2,6-$F_2$-Ph | 5-Cl-2-Me-4-$CF_3$SO-Ph | COMe | |
| Ⅲ-238 | 2,6-$F_2$-Ph | 5-Cl-2-Me-4-$CF_3$SO-Ph | $SO_2$Me | |
| Ⅲ-239 | 2,6-$F_2$-Ph | 5-Cl-2-Me-4-$CF_3$SO-Ph | $SO_2CF_3$ | |
| Ⅲ-240 | 2,6-$F_2$-Ph | 5-Cl-2-Me-4-$CF_3SO_2$-Ph | Me | |

| compound No. | $Q_1$ | $Q_2$ | R | m. p. (℃) |
|---|---|---|---|---|
| Ⅲ-241 | 2,6-$F_2$-Ph | 5-Cl-2-Me-4-$CF_3SO_2$-Ph | COMe | |
| Ⅲ-242 | 2,6-$F_2$-Ph | 5-Cl-2-Me-4-$CF_3SO_2$-Ph | $SO_2Me$ | |
| Ⅲ-243 | 2,6-$F_2$-Ph | 5-Cl-2-Me-4-$CF_3SO_2$-Ph | $SO_2CF_3$ | |
| Ⅲ-244 | 2,6-$F_2$-Ph | 2,6-$F_2$-4-$CF_3S$-Ph | Me | |
| Ⅲ-245 | 2,6-$F_2$-Ph | 2,6-$F_2$-4-$CF_3S$-Ph | COMe | |
| Ⅲ-246 | 2,6-$F_2$-Ph | 2,6-$F_2$-4-$CF_3S$-Ph | $SO_2Me$ | |
| Ⅲ-247 | 2,6-$F_2$-Ph | 2,6-$F_2$-4-$CF_3S$-Ph | $SO_2CF_3$ | |
| Ⅲ-248 | 2,6-$F_2$-Ph | 2,6-$F_2$-4-$CF_3SO$-Ph | Me | |
| Ⅲ-249 | 2,6-$F_2$-Ph | 2,6-$F_2$-4-$CF_3SO$-Ph | COMe | |
| Ⅲ-250 | 2,6-$F_2$-Ph | 2,6-$F_2$-4-$CF_3SO$-Ph | $SO_2Me$ | |
| Ⅲ-251 | 2,6-$F_2$-Ph | 2,6-$F_2$-4-$CF_3SO$-Ph | $SO_2CF_3$ | |
| Ⅲ-252 | 2,6-$F_2$-Ph | 2,6-$F_2$-4-$CF_3SO_2$-Ph | Me | |
| Ⅲ-253 | 2,6-$F_2$-Ph | 2,6-$F_2$-4-$CF_3SO_2$-Ph | COMe | |
| Ⅲ-254 | 2,6-$F_2$-Ph | 2,6-$F_2$-4-$CF_3SO_2$-Ph | $SO_2Me$ | |
| Ⅲ-255 | 2,6-$F_2$-Ph | 2,6-$F_2$-4-$CF_3SO_2$-Ph | $SO_2CF_3$ | |
| Ⅲ-256 | 2,6-$F_2$-Ph | 2-F-4-$CF_3CF_2S$-Ph | Me | |
| Ⅲ-257 | 2,6-$F_2$-Ph | 2-F-4-$CF_3CF_2S$-Ph | COMe | 105-108 |
| Ⅲ-258 | 2,6-$F_2$-Ph | 2-F-4-$CF_3CF_2S$-Ph | $SO_2Me$ | 93-97 |
| Ⅲ-259 | 2,6-$F_2$-Ph | 2-F-4-$CF_3CF_2S$-Ph | $SO_2CF_3$ | |
| Ⅲ-260 | 2,6-$F_2$-Ph | 2-F-4-$CF_3CF_2SO$-Ph | Me | |
| Ⅲ-261 | 2,6-$F_2$-Ph | 2-F-4-$CF_3CF_2SO$-Ph | COMe | white amorphous(41) |
| Ⅲ-262 | 2,6-$F_2$-Ph | 2-F-4-$CF_3CF_2SO$-Ph | $SO_2Me$ | 49-52 |
| Ⅲ-263 | 2,6-$F_2$-Ph | 2-F-4-$CF_3CF_2SO$-Ph | $SO_2CF_3$ | |
| Ⅲ-264 | 2,6-$F_2$-Ph | 2-F-4-$CF_3CF_2SO_2$-Ph | Me | |
| Ⅲ-265 | 2,6-$F_2$-Ph | 2-F-4-$CF_3CF_2SO_2$-Ph | COMe | 182-196 |
| Ⅲ-266 | 2,6-$F_2$-Ph | 2-F-4-$CF_3CF_2SO_2$-Ph | $SO_2Me$ | |
| Ⅲ-267 | 2,6-$F_2$-Ph | 2-F-4-$CF_3CF_2SO_2$-Ph | $SO_2CF_3$ | |
| Ⅲ-268 | 2,6-$F_2$-Ph | 2-F-4-$CF_3CF_2CF_2S$-Ph | Me | |
| Ⅲ-269 | 2,6-$F_2$-Ph | 2-F-4-$CF_3CF_2CF_2S$-Ph | COMe | 81-83 |
| Ⅲ-270 | 2,6-$F_2$-Ph | 2-F-4-$CF_3CF_2CF_2S$-Ph | $SO_2Me$ | 173-180 |
| Ⅲ-271 | 2,6-$F_2$-Ph | 2-F-4-$CF_3CF_2CF_2S$-Ph | $SO_2CF_3$ | |
| Ⅲ-272 | 2,6-$F_2$-Ph | 2-F-4-$CF_3CF_2CF_2SO$-Ph | Me | |
| Ⅲ-273 | 2,6-$F_2$-Ph | 2-F-4-$CF_3CF_2CF_2SO$-Ph | COMe | white amorphous(42) |
| Ⅲ-274 | 2,6-$F_2$-Ph | 2-F-4-$CF_3CF_2CF_2SO$-Ph | $SO_2Me$ | 65-68 |
| Ⅲ-275 | 2,6-$F_2$-Ph | 2-F-4-$CF_3CF_2CF_2SO$-Ph | $SO_2CF_3$ | |
| Ⅲ-276 | 2,6-$F_2$-Ph | 2-F-4-$CF_3CF_2CF_2SO_2$-Ph | Me | |
| Ⅲ-277 | 2,6-$F_2$-Ph | 2-F-4-$CF_3CF_2CF_2SO_2$-Ph | COMe | 80-83 |
| Ⅲ-278 | 2,6-$F_2$-Ph | 2-F-4-$CF_3CF_2CF_2SO_2$-Ph | $SO_2Me$ | |
| Ⅲ-279 | 2,6-$F_2$-Ph | 2-F-4-$CF_3CF_2CF_2SO_2$-Ph | $SO_2CF_3$ | |
| Ⅲ-280 | 2,6-$F_2$-Ph | 2-F-4-$(CF_3)_2CFS$-Ph | Me | |

(41) $H^1$-NMR ($CDCl_3$)δppm : 2.35 ( 3H, s, $COCH_3$), 5.28 ( 2H, s, $NCH_2N$), 5.66 ( 2H, s, $NCH_2N$), 6.90-6.94 ( 2H, m, Ph), 7.34-7.37 ( 1H, m, Ph), 7.56-7.64 ( 3H, m, Ph) .

(42) $H^1$-NMR ($CDCl_3$)δppm : 2.35 ( 3H, s, $COCH_3$), 5.27 ( 2H, s, $NCH_2N$), 5.65 ( 2H, s, $NCH_2N$), 6.90-6.94 ( 2H, m, Ph), 7.22-7.39 ( 1H, m, Ph), 7.57 ( 1H, d J=8.2Hz, Ph), 7.62-7.65 ( 2H, m, Ph).

| compound No. | $Q_1$ | $Q_2$ | R | m. p. (°C) |
|---|---|---|---|---|
| III-281 | 2,6-$F_2$-Ph | 2-F-4-$(CF_3)_2$CFS-Ph | COMe | |
| III-282 | 2,6-$F_2$-Ph | 2-F-4-$(CF_3)_2$CFS-Ph | $SO_2Me$ | |
| III-283 | 2,6-$F_2$-Ph | 2-F-4-$(CF_3)_2$CFS-Ph | $SO_2CF_3$ | |
| III-284 | 2,6-$F_2$-Ph | 2-F-4-$(CF_3)_2$CFSO-Ph | Me | |
| III-285 | 2,6-$F_2$-Ph | 2-F-4-$(CF_3)_2$CFSO-Ph | COMe | |
| III-286 | 2,6-$F_2$-Ph | 2-F-4-$(CF_3)_2$CFSO-Ph | $SO_2Me$ | |
| III-287 | 2,6-$F_2$-Ph | 2-F-4-$(CF_3)_2$CFSO-Ph | $SO_2CF_3$ | |
| III-288 | 2,6-$F_2$-Ph | 2-F-4-$(CF_3)_2CFSO_2$-Ph | Me | |
| III-289 | 2,6-$F_2$-Ph | 2-F-4-$(CF_3)_2CFSO_2$-Ph | COMe | |
| III-290 | 2,6-$F_2$-Ph | 2-F-4-$(CF_3)_2CFSO_2$-Ph | $SO_2Me$ | |
| III-291 | 2,6-$F_2$-Ph | 2-F-4-$(CF_3)_2CFSO_2$-Ph | $SO_2CF_3$ | |
| III-292 | 2-Cl-6-F-Ph | 4-Cl-Ph | Me | |
| III-293 | 2-Cl-6-F-Ph | 4-Cl-Ph | Ph | |
| III-294 | 2-Cl-6-F-Ph | 4-Cl-Ph | i-Pr | |
| III-295 | 2-Cl-6-F-Ph | 4-Cl-Ph | $CH_2Ph$ | |
| III-296 | 2-Cl-6-F-Ph | 4-Cl-Ph | $CH_2CH{=}CH_2$ | |
| III-297 | 2-Cl-6-F-Ph | 4-Cl-Ph | H | |
| III-298 | 2-Cl-6-F-Ph | 4-Cl-Ph | $COOCH_2Ph$ | |
| III-299 | 2-Cl-6-F-Ph | 4-Cl-Ph | $CH_2C{\equiv}CH$ | |
| III-300 | 2-Cl-6-F-Ph | 4-Cl-Ph | COMe | |
| III-301 | 2-Cl-6-F-Ph | 4-Cl-Ph | $CONMe_2$ | |
| III-302 | 2-Cl-6-F-Ph | 4-Cl-Ph | $SO_2Me$ | |
| III-303 | 2-Cl-6-F-Ph | 4-Cl-Ph | COOMe | |
| III-304 | 2-Cl-6-F-Ph | 4-Cl-Ph | CHMePh | |
| III-305 | 2-Cl-6-F-Ph | 4-Cl-Ph | COCOOMe | |
| III-306 | 2-Cl-6-F-Ph | 4-Cl-Ph | NO | |
| III-307 | 2-Cl-6-F-Ph | 3,5-$Cl_2$-4-(2-Cl-4-$CF_3$-pyridin-2yl-O)-Ph | Me | |
| III-308 | 2-Cl-6-F-Ph | 3,5-$Cl_2$-4-(2-Cl-4-$CF_3$-pyridin-2yl-O)-Ph | COMe | |
| III-309 | 2-Cl-6-F-Ph | 3,5-$Cl_2$-4-(2-Cl-4-$CF_3$-pyridin-2yl-O)-Ph | $SO_2Me$ | |
| III-310 | 2-Cl-6-F-Ph | 2-F-4-(2-Cl-4-$CF_3$-Ph-O)-Ph | Me | |
| III-311 | 2-Cl-6-F-Ph | 2-F-4-(2-Cl-4-$CF_3$-Ph-O)-Ph | $COOCH_2Ph$ | |
| III-312 | 2-Cl-6-F-Ph | 2-F-4-(2-Cl-4-$CF_3$-Ph-O)-Ph | $CH_2C{\equiv}CH$ | |
| III-313 | 2-Cl-6-F-Ph | 2-F-4-(2-Cl-4-$CF_3$-Ph-O)-Ph | $CH_2CH{=}CH_2$ | |
| III-314 | 2-Cl-6-F-Ph | 2-F-4-(2-Cl-4-$CF_3$-Ph-O)-Ph | i-Pr | |
| III-315 | 2-Cl-6-F-Ph | 2-F-4-(2-Cl-4-$CF_3$-Ph-O)-Ph | $SO_2Me$ | |
| III-316 | 2-Cl-6-F-Ph | 2-F-4-(2-Cl-4-$CF_3$-Ph-O)-Ph | H | |
| III-317 | 2-Cl-6-F-Ph | 2-F-4-(2-Cl-4-$CF_3$-Ph-O)-Ph | COOMe | |
| III-318 | 2-Cl-6-F-Ph | 2-F-4-(2-Cl-4-$CF_3$-Ph-O)-Ph | $CH_2Ph$ | |
| III-319 | 2-Cl-6-F-Ph | 2-F-4-(2-Cl-4-$CF_3$-Ph-O)-Ph | Ph | |
| III-320 | 2-Cl-6-F-Ph | 2-F-4-(2-Cl-4-$CF_3$-Ph-O)-Ph | SCOOMe | |

| compound No. | $Q_1$ | $Q_2$ | R | m. p. (°C) |
|---|---|---|---|---|
| Ⅲ-321 | 2-Cl-6-F-Ph | 2-F-4-(2-Cl-4-$CF_3$-Ph-O)-Ph | $COCF_3$ | |
| Ⅲ-322 | 2-Cl-6-F-Ph | 2-F-4-(2-Cl-4-$CF_3$-Ph-O)-Ph | COMe | |
| Ⅲ-323 | 2-Cl-6-F-Ph | 2-F-4-(2-Cl-4-$CF_3$-Ph-O)-Ph | $SO_2$Ph-4-Me | |
| Ⅲ-324 | 2-Cl-6-F-Ph | 2-F-4-(2-Cl-4-$CF_3$-Ph-O)-Ph | $SO_2CF_3$ | |
| Ⅲ-325 | 2-Cl-6-F-Ph | 2-F-4-(2-Cl-4-$CF_3$-Ph-O)-Ph | cyclo-Pr | |
| Ⅲ-326 | 2-Cl-6-F-Ph | 2-F-4-(2-Cl-4-$CF_3$-Ph-O)-Ph | COCOOMe | |
| Ⅲ-327 | 2-Cl-6-F-Ph | 2-F-4-(2-Cl-4-$CF_3$-Ph-O)-Ph | COCOOH | |
| Ⅲ-328 | 2-Cl-6-F-Ph | 2-F-4-(2-Cl-4-$CF_3$-Ph-O)-Ph | $COCOOCH_2Ph$ | |
| Ⅲ-329 | 2-Cl-6-F-Ph | 2-F-4-(2-Cl-4-$CF_3$-Ph-O)-Ph | $COCOOHNH_3$ | |
| Ⅲ-330 | 2-Cl-6-F-Ph | 2-F-4-(2-Cl-4-$CF_3$-Ph-O)-Ph | $COCONH_2$ | |
| Ⅲ-331 | 2-Cl-6-F-Ph | 2-F-4-(2-Cl-4-$CF_3$-Ph-O)-Ph | COCONHMe | |
| Ⅲ-332 | 2-Cl-6-F-Ph | 2-F-4-(2-Cl-4-$CF_3$-Ph-O)-Ph | $COCONMe_2$ | |
| Ⅲ-333 | 2-Cl-6-F-Ph | 2-F-4-(2-Cl-4-$CF_3$-Ph-O)-Ph | $COCH_2Br$ | |
| Ⅲ-334 | 2-Cl-6-F-Ph | 2-F-4-(2-Cl-4-$CF_3$-Ph-O)-Ph | $COCH_2SMe$ | |
| Ⅲ-335 | 2-Cl-6-F-Ph | 2-F-4-(2-Cl-4-$CF_3$-Ph-O)-Ph | $COCH_2SOMe$ | |
| Ⅲ-336 | 2-Cl-6-F-Ph | 2-F-4-(2-Cl-4-$CF_3$-Ph-O)-Ph | $COCH_2SO_2Me$ | |
| Ⅲ-337 | 2-Cl-6-F-Ph | 2-F-4-(2-Cl-4-$CF_3$-Ph-O)-Ph | COCONMeOMe | |
| Ⅲ-338 | 2-Cl-6-F-Ph | 2-F-4-(2-Cl-4-$CF_3$-Ph-O)-Ph | COCONHOMe | |
| Ⅲ-339 | 2-Cl-6-F-Ph | 2-F-4-(2-Cl-4-$CF_3$-Ph-O)-Ph | $COCONHOCH_2Ph$ | |
| Ⅲ-340 | 2-Cl-6-F-Ph | 3,5-$Cl_2$-2,4-$F_2$-Ph | Me | |
| Ⅲ-341 | 2-Cl-6-F-Ph | 3,5-$Cl_2$-2,4-$F_2$-Ph | i-Pr | |
| Ⅲ-342 | 2-Cl-6-F-Ph | 3,5-$Cl_2$-2,4-$F_2$-Ph | cyclo-Pr | |
| Ⅲ-343 | 2-Cl-6-F-Ph | 3,5-$Cl_2$-2,4-$F_2$-Ph | Ph | |
| Ⅲ-344 | 2-Cl-6-F-Ph | 3,5-$Cl_2$-2,4-$F_2$-Ph | $COOCH_2Ph$ | |
| Ⅲ-345 | 2-Cl-6-F-Ph | 3,5-$Cl_2$-2,4-$F_2$-Ph | CH2CH=CH2 | |
| Ⅲ-346 | 2-Cl-6-F-Ph | 3,5-$Cl_2$-2,4-$F_2$-Ph | CH2C≡CH | |
| Ⅲ-347 | 2-Cl-6-F-Ph | 3,5-$Cl_2$-2,4-$F_2$-Ph | $SO_2Me$ | |
| Ⅲ-348 | 2-Cl-6-F-Ph | 3,5-$Cl_2$-2,4-$F_2$-Ph | $CH_2Ph$ | |
| Ⅲ-349 | 2-Cl-6-F-Ph | 3,5-$Cl_2$-2,4-$F_2$-Ph | COOMe | |
| Ⅲ-350 | 2-Cl-6-F-Ph | 3,5-$Cl_2$-2,4-$F_2$-Ph | SCOOMe | |
| Ⅲ-351 | 2-Cl-6-F-Ph | 3,5-$Cl_2$-2,4-$F_2$-Ph | $SO_2$Ph-4-Me | |
| Ⅲ-352 | 2-Cl-6-F-Ph | 3,5-$Cl_2$-2,4-$F_2$-Ph | COCOOMe | |
| Ⅲ-353 | 2-Cl-6-F-Ph | 3,5-$Cl_2$-2,4-$F_2$-Ph | COMe | |
| Ⅲ-354 | 2-Cl-6-F-Ph | 3,5-$Cl_2$-4-$CF_2HCF_2$O-Ph | Me | |
| Ⅲ-355 | 2-Cl-6-F-Ph | 3,5-$Cl_2$-4-$CF_2HCF_2$O-Ph | COMe | |
| Ⅲ-356 | 2-Cl-6-F-Ph | 3,5-$Cl_2$-4-$CF_2HCF_2$O-Ph | $SO_2Me$ | |
| Ⅲ-357 | 2-Cl-6-F-Ph | 2,5-$Cl_2$-4-$CF_3CFHCF_2$O-Ph | Me | |
| Ⅲ-358 | 2-Cl-6-F-Ph | 2,5-$Cl_2$-4-$CF_3CFHCF_2$O-Ph | $COOCH_2Ph$ | |
| Ⅲ-359 | 2-Cl-6-F-Ph | 2,5-$Cl_2$-4-$CF_3CFHCF_2$O-Ph | H | |
| Ⅲ-360 | 2-Cl-6-F-Ph | 2,5-$Cl_2$-4-$CF_3CFHCF_2$O-Ph | COCOOMe | |

| compound No. | $Q_1$ | $Q_2$ | R | m. p. (°C) |
|---|---|---|---|---|
| Ⅲ-361 | 2-Cl-6-F-Ph | 2,5-$Cl_2$-4-$CF_3CFHCF_2O$-Ph | COMe | |
| Ⅲ-362 | 2-Cl-6-F-Ph | 2,5-$Cl_2$-4-$CF_3CFHCF_2O$-Ph | $SO_2Me$ | |
| Ⅲ-363 | 2-Cl-6-F-Ph | 2-F-4-$CFClHCF_2S$-Ph | Me | |
| Ⅲ-364 | 2-Cl-6-F-Ph | 2-F-4-$CFClHCF_2S$-Ph | $COOCH_2Ph$ | |
| Ⅲ-365 | 2-Cl-6-F-Ph | 2-F-4-$CFClHCF_2S$-Ph | H | |
| Ⅲ-366 | 2-Cl-6-F-Ph | 2-F-4-$CFClHCF_2S$-Ph | COCOOMe | |
| Ⅲ-367 | 2-Cl-6-F-Ph | 2-F-4-$CFClHCF_2S$-Ph | Ph | |
| Ⅲ-368 | 2-Cl-6-F-Ph | 2-F-4-$CFClHCF_2S$-Ph | COMe | |
| Ⅲ-369 | 2-Cl-6-F-Ph | 2-F-4-$CFClHCF_2S$-Ph | $SO_2Me$ | |
| Ⅲ-370 | 2-Cl-6-F-Ph | 2-F-4-$CFClHCF_2SO$-Ph | Me | |
| Ⅲ-371 | 2-Cl-6-F-Ph | 2-F-4-$CFClHCF_2SO$-Ph | $COOCH_2Ph$ | |
| Ⅲ-372 | 2-Cl-6-F-Ph | 2-F-4-$CFClHCF_2SO$-Ph | COMe | |
| Ⅲ-373 | 2-Cl-6-F-Ph | 2-F-4-$CFClHCF_2SO$-Ph | $SO_2Me$ | |
| Ⅲ-374 | 2-Cl-6-F-Ph | 2-F-4-$CFClHCF_2SO$-Ph | $CH_2C{\equiv}CH$ | |
| Ⅲ-375 | 2-Cl-6-F-Ph | 2-F-4-$CFClHCF_2SO$-Ph | $CH_2CH{=}CH_2$ | |
| Ⅲ-376 | 2-Cl-6-F-Ph | 2-F-4-$CFClHCF_2SO$-Ph | H | |
| Ⅲ-377 | 2-Cl-6-F-Ph | 2-F-4-$CFClHCF_2SO_2$-Ph | Me | |
| Ⅲ-378 | 2-Cl-6-F-Ph | 2-F-4-$CFClHCF_2SO_2$-Ph | COMe | |
| Ⅲ-379 | 2-Cl-6-F-Ph | 2-F-4-$CFClHCF_2SO_2$-Ph | $SO_2Me$ | |
| Ⅲ-380 | 2-Cl-6-F-Ph | 4-$CF_3S$-Ph | Me | |
| Ⅲ-381 | 2-Cl-6-F-Ph | 4-$CF_3S$-Ph | COMe | |
| Ⅲ-382 | 2-Cl-6-F-Ph | 4-$CF_3S$-Ph | $SO_2Me$ | |
| Ⅲ-383 | 2-Cl-6-F-Ph | 4-$CF_3SO$-Ph | Me | |
| Ⅲ-384 | 2-Cl-6-F-Ph | 4-$CF_3SO$-Ph | COMe | |
| Ⅲ-385 | 2-Cl-6-F-Ph | 4-$CF_3SO$-Ph | $SO_2Me$ | |
| Ⅲ-386 | 2-Cl-6-F-Ph | 4-$CF_3SO_2$-Ph | Me | |
| Ⅲ-387 | 2-Cl-6-F-Ph | 4-$CF_3SO_2$-Ph | COMe | |
| Ⅲ-388 | 2-Cl-6-F-Ph | 4-$CF_3SO_2$-Ph | $SO_2Me$ | |
| Ⅲ-389 | 2-Cl-6-F-Ph | 2-F-4-MeS-Ph | Me | |
| Ⅲ-390 | 2-Cl-6-F-Ph | 2-F-4-MeS-Ph | COMe | |
| Ⅲ-391 | 2-Cl-6-F-Ph | 2-F-4-MeS-Ph | $SO_2Me$ | |
| Ⅲ-392 | 2-Cl-6-F-Ph | 2-F-4-MeSO-Ph | Me | |
| Ⅲ-393 | 2-Cl-6-F-Ph | 2-F-4-MeSO-Ph | COMe | |
| Ⅲ-394 | 2-Cl-6-F-Ph | 2-F-4-MeSO-Ph | $SO_2Me$ | |
| Ⅲ-395 | 2-Cl-6-F-Ph | 2-F-4-$MeSO_2$-Ph | Me | |
| Ⅲ-396 | 2-Cl-6-F-Ph | 2-F-4-$MeSO_2$-Ph | COMe | |
| Ⅲ-397 | 2-Cl-6-F-Ph | 2-F-4-$MeSO_2$-Ph | $SO_2Me$ | |
| Ⅲ-398 | 2-Cl-6-F-Ph | 2,6-$F_2$-4-MeS-Ph | Me | |
| Ⅲ-399 | 2-Cl-6-F-Ph | 2,6-$F_2$-4-MeS-Ph | COMe | |
| Ⅲ-400 | 2-Cl-6-F-Ph | 2,6-$F_2$-4-MeS-Ph | $SO_2Me$ | |

| compound No. | $Q_1$ | $Q_2$ | R | m. p. (°C) |
|---|---|---|---|---|
| Ⅲ-401 | 2-Cl-6-F-Ph | 2,6-$F_2$-4-MeSO-Ph | Me | |
| Ⅲ-402 | 2-Cl-6-F-Ph | 2,6-$F_2$-4-MeSO-Ph | COMe | |
| Ⅲ-403 | 2-Cl-6-F-Ph | 2,6-$F_2$-4-MeSO-Ph | $SO_2Me$ | |
| Ⅲ-404 | 2-Cl-6-F-Ph | 2,6-$F_2$-4-$MeSO_2$-Ph | Me | |
| Ⅲ-405 | 2-Cl-6-F-Ph | 2,6-$F_2$-4-$MeSO_2$-Ph | COMe | |
| Ⅲ-406 | 2-Cl-6-F-Ph | 2,6-$F_2$-4-$MeSO_2$-Ph | $SO_2Me$ | |
| Ⅲ-407 | 2-Cl-6-F-Ph | 2-F-4-$CF_3CH_2S$-Ph | Me | |
| Ⅲ-408 | 2-Cl-6-F-Ph | 2-F-4-$CF_3CH_2S$-Ph | COMe | |
| Ⅲ-409 | 2-Cl-6-F-Ph | 2-F-4-$CF_3CH_2S$-Ph | $SO_2Me$ | |
| Ⅲ-410 | 2-Cl-6-F-Ph | 2-F-4-$CF_3CH_2SO$-Ph | Me | |
| Ⅲ-411 | 2-Cl-6-F-Ph | 2-F-4-$CF_3CH_2SO$-Ph | COMe | |
| Ⅲ-412 | 2-Cl-6-F-Ph | 2-F-4-$CF_3CH_2SO$-Ph | SO2Me | |
| Ⅲ-413 | 2-Cl-6-F-Ph | 2-F-4-$CF_3CH_2SO_2$-Ph | Me | |
| Ⅲ-414 | 2-Cl-6-F-Ph | 2-F-4-$CF_3CH_2SO_2$-Ph | COMe | |
| Ⅲ-415 | 2-Cl-6-F-Ph | 2-F-4-$CF_3CH_2SO_2$-Ph | $SO_2Me$ | |
| Ⅲ-416 | 2-Cl-6-F-Ph | $CF_3CH_2$ | Me | |
| Ⅲ-417 | 2-Cl-6-F-Ph | $CF_3CH_2$ | COMe | |
| Ⅲ-418 | 2-Cl-6-F-Ph | $CF_3CH_2$ | $SO_2Me$ | |
| Ⅲ-419 | 2-Cl-6-F-Ph | 2-F-4-$CF_2HS$-Ph | Me | |
| Ⅲ-420 | 2-Cl-6-F-Ph | 2-F-4-$CF_2HS$-Ph | COMe | |
| Ⅲ-421 | 2-Cl-6-F-Ph | 2-F-4-$CF_2HS$-Ph | $SO_2Me$ | |
| Ⅲ-422 | 2-Cl-6-F-Ph | 2-F-4-$CF_2HSO$-Ph | Me | |
| Ⅲ-423 | 2-Cl-6-F-Ph | 2-F-4-$CF_2HSO$-Ph | COMe | |
| Ⅲ-424 | 2-Cl-6-F-Ph | 2-F-4-$CF_2HSO$-Ph | $SO_2Me$ | |
| Ⅲ-425 | 2-Cl-6-F-Ph | 2-F-4-$CF_2HSO_2$-Ph | Me | |
| Ⅲ-426 | 2-Cl-6-F-Ph | 2-F-4-$CF_2HSO_2$-Ph | COMe | |
| Ⅲ-427 | 2-Cl-6-F-Ph | 2-F-4-$CF_2HSO_2$-Ph | $SO_2Me$ | |
| Ⅲ-428 | 2-Cl-6-F-Ph | 2-F-4-$CF_3S$-Ph | Me | |
| Ⅲ-429 | 2-Cl-6-F-Ph | 2-F-4-$CF_3S$-Ph | COMe | |
| Ⅲ-430 | 2-Cl-6-F-Pb | 2-F-4-$CF_3S$-Ph | $SO_2Me$ | |
| Ⅲ-431 | 2-Cl-6-F-Ph | 2-F-4-$CF_3SO$-Ph | Me | |
| Ⅲ-432 | 2-Cl-6-F-Ph | 2-F-4-$CF_3SO$-Ph | COMe | |
| Ⅲ-433 | 2-Cl-6-F-Ph | 2-F-4-$CF_3SO$-Ph | $SO_2Me$ | |
| Ⅲ-434 | 2-Cl-6-F-Ph | 2-F-4-$CF_3SO_2$-Ph | Me | |
| Ⅲ-435 | 2-Cl-6-F-Ph | 2-F-4-$CF_3SO_2$-Ph | COMe | |
| Ⅲ-436 | 2-Cl-6-F-Ph | 2-F-4-$CF_3SO_2$-Ph | $SO_2Me$ | |
| Ⅲ-437 | 2-Cl-6-F-Ph | 2-F-4-$CH_3CH_2S$-Ph | Me | |
| Ⅲ-438 | 2-Cl-6-F-Ph | 2-F-4-$CH_3CH_2S$-Ph | COMe | |
| Ⅲ-439 | 2-Cl-6-F-Ph | 2-F-4-$CH_3CH_2S$-Ph | $SO_2Me$ | |
| Ⅲ-440 | 2-Cl-6-F-Ph | 2-F-4-$CH_3CH_2SO$-Ph | Me | |

| compound No. | $Q_1$ | $Q_2$ | R | m. p. (°C) |
|---|---|---|---|---|
| Ⅲ-441 | 2-Cl-6-F-Ph | 2-F-4-$CH_3CH_2$SO-Ph | COMe | |
| Ⅲ-442 | 2-Cl-6-F-Ph | 2-F-4-$CH_3CH_2$SO-Ph | $SO_2$Me | |
| Ⅲ-443 | 2-Cl-6-F-Ph | 2-F-4-$CH_3CH_2SO_2$-Ph | Me | |
| Ⅲ-444 | 2-Cl-6-F-Ph | 2-F-4-$CH_3CH_2SO_2$-Ph | COMe | |
| Ⅲ-445 | 2-Cl-6-F-Ph | 2-F-4-$CH_3CH_2SO_2$-Ph | $SO_2$Me | |
| Ⅲ-446 | 2-Cl-6-F-Ph | 2-F-4-(2-Cl-4-$CF_3$-pyridin-2yl-S)-Ph | Me | |
| Ⅲ-447 | 2-Cl-6-F-Ph | 2-F-4-(2-Cl-4-$CF_3$-pyridin-2yl-S)-Ph | COMe | |
| Ⅲ-448 | 2-Cl-6-F-Ph | 2-F-4-(2-Cl-4-$CF_3$-pyridin-2yl-S)-Ph | $SO_2$Me | |
| Ⅲ-449 | 2-Cl-6-F-Ph | 2-F-4-(2-Cl-4-$CF_3$-pyridin-2yl-SO)-Ph | Me | |
| Ⅲ-450 | 2-Cl-6-F-Ph | 2-F-4-(2-Cl-4-$CF_3$-pyridin-2yl-SO)-Ph | COMe | |
| Ⅲ-451 | 2-Cl-6-F-Ph | 2-F-4-(2-Cl-4-$CF_3$-pyridin-2yl-SO)-Ph | $SO_2$Me | |
| Ⅲ-452 | 2-Cl-6-F-Ph | 2-F-4-(2-Cl-4-$CF_3$-pyridin-2yl-$SO_2$)-Ph | Me | |
| Ⅲ-453 | 2-Cl-6-F-Ph | 2-F-4-(2-Cl-4-$CF_3$-pyridin-2yl-$SO_2$)-Ph | COMe | |
| Ⅲ-454 | 2-Cl-6-F-Ph | 2-F-4-(2-Cl-4-$CF_3$-pyridin-2yl-$SO_2$)-Ph | $SO_2$Me | |
| Ⅲ-455 | 2-Cl-6-F-Ph | 2-Me-4-$CF_3$S-Ph | Me | 145-148 |
| Ⅲ-456 | 2-Cl-6-F-Ph | 2-Me-4-$CF_3$S-Ph | COMe | |
| Ⅲ-457 | 2-Cl-6-F-Ph | 2-Me-4-$CF_3$S-Ph | $SO_2$Me | |
| Ⅲ-458 | 2-Cl-6-F-Ph | 2-Me-4-$CF_3$SO-Ph | Me | |
| Ⅲ-459 | 2-Cl-6-F-Ph | 2-Me-4-$CF_3$SO-Ph | COMe | |
| Ⅲ-460 | 2-Cl-6-F-Ph | 2-Me-4-$CF_3$SO-Ph | $SO_2$Me | |
| Ⅲ-461 | 2-Cl-6-F-Ph | 2-Me-4-$CF_3SO_2$-Ph | Me | |
| Ⅲ-462 | 2-Cl-6-F-Ph | 2-Me-4-$CF_3SO_2$-Ph | COMe | |
| Ⅲ-463 | 2-Cl-6-F-Ph | 2-Me-4-$CF_3SO_2$-Ph | $SO_2$Me | |
| Ⅲ-464 | 2-Cl-6-F-Ph | 3-F-4-$CF_3$S-Ph | Me | |
| Ⅲ-465 | 2-Cl-6-F-Ph | 3-F-4-$CF_3$S-Ph | COMe | |
| Ⅲ-466 | 2-Cl-6-F-Ph | 3-F-4-$CF_3$S-Ph | $SO_2$Me | |
| Ⅲ-467 | 2-Cl-6-F-Ph | 3-F-4-$CF_3$SO-Ph | Me | |
| Ⅲ-468 | 2-Cl-6-F-Ph | 3-F-4-$CF_3$SO-Ph | COMe | |
| Ⅲ-469 | 2-Cl-6-F-Ph | 3-F-4-$CF_3$SO-Ph | $SO_2$Me | |
| Ⅲ-470 | 2-Cl-6-F-Ph | 3-F-4-$CF_3SO_2$-Ph | Me | |
| Ⅲ-471 | 2-Cl-6-F-Ph | 3-F-4-$CF_3SO_2$-Ph | COMe | |
| Ⅲ-472 | 2-Cl-6-F-Ph | 3-F-4-$CF_3SO_2$-Ph | $SO_2$Me | |
| Ⅲ-473 | 2-Cl-6-F-Ph | 2,3-$Me_2$-4-$CF_3$S-Ph | Me | |
| Ⅲ-474 | 2-Cl-6-F-Ph | 2,3-$Me_2$-4-$CF_3$S-Ph | COMe | |
| Ⅲ-475 | 2-Cl-6-F-Ph | 2,3-$Me_2$-4-$CF_3$S-Ph | $SO_2$Me | |
| Ⅲ-476 | 2-Cl-6-F-Ph | 2,3-$Me_2$-4-$CF_3$SO-Ph | Me | |
| Ⅲ-477 | 2-Cl-6-F-Ph | 2,3-$Me_2$-4-$CF_3$SO-Ph | COMe | |
| Ⅲ-478 | 2-Cl-6-F-Ph | 2,3-$Me_2$-4-$CF_3$SO-Ph | $SO_2$Me | |
| Ⅲ-479 | 2-Cl-6-F-Ph | 2,3-$Me_2$-4-$CF_3SO_2$-Ph | Me | |
| Ⅲ-480 | 2-Cl-6-F-Ph | 2,3-$Me_2$-4-$CF_3SO_2$-Ph | COMe | |

| compound No. | $Q_1$ | $Q_2$ | R | m. p. (°C) |
|---|---|---|---|---|
| III-481 | 2-Cl-6-F-Ph | 2,3-$Me_2$-4-$CF_3SO_2$-Ph | $SO_2Me$ | |
| III-482 | 2-Cl-6-F-Ph | 5-Cl-2-Me-4-$CF_3S$-Ph | Me | |
| III-483 | 2-Cl-6-F-Ph | 5-Cl-2-Me-4-$CF_3S$-Ph | COMe | |
| III-484 | 2-Cl-6-F-Ph | 5-Cl-2-Me-4-$CF_3S$-Ph | $SO_2Me$ | |
| III-485 | 2-Cl-6-F-Ph | 5-Cl-2-Me-4-$CF_3SO$-Ph | Me | |
| III-486 | 2-Cl-6-F-Ph | 5-Cl-2-Me-4-$CF_3SO$-Ph | COMe | |
| III-487 | 2-Cl-6-F-Ph | 5-Cl-2-Me-4-$CF_3SO$-Ph | $SO_2Me$ | |
| III-488 | 2-Cl-6-F-Ph | 5-Cl-2-Me-4-$CF_3SO_2$-Ph | Me | |
| III-489 | 2-Cl-6-F-Ph | 5-Cl-2-Me-4-$CF_3SO_2$-Ph | COMe | |
| III-490 | 2-Cl-6-F-Ph | 5-Cl-2-Me-4-$CF_3SO_2$-Ph | $SO_2Me$ | |
| III-491 | 2-Cl-6-F-Ph | 2,6-$F_2$-4-$CF_3S$-Ph | Me | |
| III-492 | 2-Cl-6-F-Ph | 2,6-$F_2$-4-$CF_3S$-Ph | COMe | |
| III-493 | 2-Cl-6-F-Ph | 2,6-$F_2$-4-$CF_3S$-Ph | $SO_2Me$ | |
| III-494 | 2-Cl-6-F-Ph | 2,6-$F_2$-4-$CF_3SO$-Ph | Me | |
| III-495 | 2-Cl-6-F-Ph | 2,6-$F_2$-4-$CF_3SO$-Ph | COMe | |
| III-496 | 2-Cl-6-F-Ph | 2,6-$F_2$-4-$CF_3SO$-Ph | $SO_2Me$ | |
| III-497 | 2-Cl-6-F-Ph | 2,6-$F_2$-4-$CF_3SO_2$-Ph | Me | |
| III-498 | 2-Cl-6-F-Ph | 2,6-$F_2$-4-$CF_3SO_2$-Ph | COMe | |
| III-499 | 2-Cl-6-F-Ph | 2,6-$F_2$-4-$CF_3SO_2$-Ph | $SO_2Me$ | |
| III-500 | 2-Cl-6-F-Ph | 2-F-4-$CF_3CF_2S$-Ph | Me | |
| III-501 | 2-Cl-6-F-Ph | 2-F-4-$CF_3CF_2S$-Ph | COMe | |
| III-502 | 2-Cl-6-F-Ph | 2-F-4-$CF_3CF_2S$-Ph | $SO_2Me$ | |
| III-503 | 2-Cl-6-F-Ph | 2-F-4-$CF_3CF_2SO$-Ph | Me | |
| III-504 | 2-Cl-6-F-Ph | 2-F-4-$CF_3CF_2SO$-Ph | COMe | |
| III-505 | 2-Cl-6-F-Ph | 2-F-4-$CF_3CF_2SO$-Ph | $SO_2Me$ | |
| III-506 | 2-Cl-6-F-Ph | 2-F-4-$CF_3CF_2SO_2$-Ph | Me | |
| III-507 | 2-Cl-6-F-Ph | 2-F-4-$CF_3CF_2SO_2$-Ph | COMe | |
| III-508 | 2-Cl-6-F-Ph | 2-F-4-$CF_3CF_2SO_2$-Ph | $SO_2Me$ | |
| III-509 | 2-Cl-6-F-Ph | 2-F-4-$CF_3CF_2CF_2S$-Ph | Me | |
| III-510 | 2-Cl-6-F-Ph | 2-F-4-$CF_3CF_2CF_2S$-Ph | COMe | |
| III-511 | 2-Cl-6-F-Ph | 2-F-4-$CF_3CF_2CF_2S$-Ph | $SO_2Me$ | |
| III-512 | 2-Cl-6-F-Ph | 2-F-4-$CF_3CF_2CF_2SO$-Ph | Me | |
| III-513 | 2-Cl-6-F-Ph | 2-F-4-$CF_3CF_2CF_2SO$-Ph | COMe | |
| III-514 | 2-Cl-6-F-Ph | 2-F-4-$CF_3CF_2CF_2SO$-Ph | $SO_2Me$ | |
| III-515 | 2-Cl-6-F-Ph | 2-F-4-$CF_3CF_2CF_2SO_2$-Ph | Me | |
| III-516 | 2-Cl-6-F-Ph | 2-F-4-$CF_3CF_2CF_2SO_2$-Ph | COMe | |
| III-517 | 2-Cl-6-F-Ph | 2-F-4-$CF_3CF_2CF_2SO_2$-Ph | $SO_2Me$ | |
| III-518 | 2-Cl-6-F-Ph | 2-F-4-$(CF_3)_2CFS$-Ph | Me | |
| III-519 | 2-Cl-6-F-Ph | 2-F-4-$(CF_3)_2CFS$-Ph | COMe | |
| III-520 | 2-Cl-6-F-Ph | 2-F-4-$(CF_3)_2CFS$-Ph | $SO_2Me$ | |

| compound No. | $Q_1$ | $Q_2$ | R | m. p. (°C) |
|---|---|---|---|---|
| Ⅲ-521 | 2-Cl-6-F-Ph | 2-F-4-$(CF_3)_2$CFSO-Ph | Me | |
| Ⅲ-522 | 2-Cl-6-F-Ph | 2-F-4-$(CF_3)_2$CFSO-Ph | COMe | |
| Ⅲ-523 | 2-Cl-6-F-Ph | 2-F-4-$(CF_3)_2$CFSO-Ph | $SO_2$Me | |
| Ⅲ-524 | 2-Cl-6-F-Ph | 2-F-4-$(CF_3)_2CFSO_2$-Ph | Me | |
| Ⅲ-525 | 2-Cl-6-F-Ph | 2-F-4-$(CF_3)_2CFSO_2$-Ph | COMe | |
| Ⅲ-526 | 2-Cl-6-F-Ph | 2-F-4-$(CF_3)_2CFSO_2$-Ph | $SO_2$Me | |
| Ⅲ-527 | 2,6-$Cl_2$-Ph | 4-Cl-Ph | Me | |
| Ⅲ-528 | 2,6-$Cl_2$-Ph | 4-Cl-Ph | Ph | |
| Ⅲ-529 | 2,6-$Cl_2$-Ph | 4-Cl-Ph | i-Pr | |
| Ⅲ-530 | 2,6-$Cl_2$-Ph | 4-Cl-Ph | $CH_2$Ph | |
| Ⅲ-531 | 2,6-$Cl_2$-Ph | 4-Cl-Ph | $CH_2CH=CH_2$ | |
| Ⅲ-532 | 2,6-$Cl_2$-Ph | 4-Cl-Ph | H | |
| Ⅲ-533 | 2,6-$Cl_2$-Ph | 4-Cl-Ph | $COOCH_2$Ph | |
| Ⅲ-534 | 2,6-$Cl_2$-Ph | 4-Cl-Ph | $CH_2C\equiv CH$ | |
| Ⅲ-535 | 2,6-$Cl_2$-Ph | 4-Cl-Ph | COMe | |
| Ⅲ-536 | 2,6-$Cl_2$-Ph | 4-Cl-Ph | $CONMe_2$ | |
| Ⅲ-537 | 2,6-$Cl_2$-Ph | 4-Cl-Ph | $SO_2$Me | |
| Ⅲ-538 | 2,6-$Cl_2$-Ph | 4-Cl-Ph | COOMe | |
| Ⅲ-539 | 2,6-$Cl_2$-Ph | 4-Cl-Ph | CHMePh | |
| Ⅲ-540 | 2,6-$Cl_2$-Ph | 4-Cl-Ph | COCOOMe | |
| Ⅲ-541 | 2,6-$Cl_2$-Ph | 4-Cl-Ph | NO | |
| Ⅲ-542 | 2,6-$Cl_2$-Ph | 3,5-$Cl_2$-4-(2-Cl-4-$CF_3$-pyridin-2yl-O)-Ph | Me | |
| Ⅲ-543 | 2,6-$Cl_2$-Ph | 3,5-$Cl_2$-4-(2-Cl-4-$CF_3$-pyridin-2yl-O)-Ph | COMe | |
| Ⅲ-544 | 2,6-$Cl_2$-Ph | 3,5-$Cl_2$-4-(2-Cl-4-$CF_3$-pyridin-2yl-O)-Ph | $SO_2$Me | |
| Ⅲ-545 | 2,6-$Cl_2$-Ph | 2-F-4-(2-Cl-4-$CF_3$-Ph-O)-Ph | Me | |
| Ⅲ-546 | 2,6-$Cl_2$-Ph | 2-F-4-(2-Cl-4-$CF_3$-Ph-O)-Ph | $COOCH_2$Ph | |
| Ⅲ-547 | 2,6-$Cl_2$-Ph | 2-F-4-(2-Cl-4-$CF_3$-Ph-O)-Ph | $CH_2C\equiv CH$ | |
| Ⅲ-548 | 2,6-$Cl_2$-Ph | 2-F-4-(2-Cl-4-$CF_3$-Ph-O)-Ph | $CH_2CH=CH_2$ | |
| Ⅲ-549 | 2,6-$Cl_2$-Ph | 2-F-4-(2-Cl-4-$CF_3$-Ph-O)-Ph | i-Pr | |
| Ⅲ-550 | 2,6-$Cl_2$-Ph | 2-F-4-(2-Cl-4-$CF_3$-Ph-O)-Ph | $SO_2$Me | |
| Ⅲ-551 | 2,6-$Cl_2$-Ph | 2-F-4-(2-Cl-4-$CF_3$-Ph-O)-Ph | H | |
| Ⅲ-552 | 2,6-$Cl_2$-Ph | 2-F-4-(2-Cl-4-$CF_3$-Ph-O)-Ph | COOMe | |
| Ⅲ-553 | 2,6-$Cl_2$-Ph | 2-F-4-(2-Cl-4-$CF_3$-Ph-O)-Ph | $CH_2$Ph | |
| Ⅲ-554 | 2,6-$Cl_2$-Ph | 2-F-4-(2-Cl-4-$CF_3$-Ph-O)-Ph | Ph | |
| Ⅲ-555 | 2,6-$Cl_2$-Ph | 2-F-4-(2-Cl-4-$CF_3$-Ph-O)-Ph | SCOOMe | |
| Ⅲ-556 | 2,6-$Cl_2$-Ph | 2-F-4-(2-Cl-4-$CF_3$-Ph-O)-Ph | $COCF_3$ | |
| Ⅲ-557 | 2,6-$Cl_2$-Ph | 2-F-4-(2-Cl-4-$CF_3$-Ph-O)-Ph | COMe | |
| Ⅲ-558 | 2,6-$Cl_2$-Ph | 2-F-4-(2-Cl-4-$CF_3$-Ph-O)-Ph | $SO_2$Ph-4-Me | |
| Ⅲ-559 | 2,6-$Cl_2$-Ph | 2-F-4-(2-Cl-4-$CF_3$-Ph-O)-Ph | $SO_2CF_3$ | |
| Ⅲ-560 | 2,6-$Cl_2$-Ph | 2-F-4-(2-Cl-4-$CF_3$-Ph-O)-Ph | cyclo-Pr | |

| compound No. | $Q_1$ | $Q_2$ | R | m. p. (°C) |
|---|---|---|---|---|
| Ⅲ-561 | 2,6-$Cl_2$-Ph | 2-F-4-(2-Cl-4-$CF_3$-Ph-O)-Ph | COCOOMe | |
| Ⅲ-562 | 2,6-$Cl_2$-Ph | 2-F-4-(2-Cl-4-$CF_3$-Ph-O)-Ph | COCOOH | |
| Ⅲ-563 | 2,6-$Cl_2$-Ph | 2-F-4-(2-Cl-4-$CF_3$-Ph-O)-Ph | $COCOOCH_2Ph$ | |
| Ⅲ-564 | 2,6-$Cl_2$-Ph | 2-F-4-(2-Cl-4-$CF_3$-Ph-O)-Ph | $COCOOHNH_3$ | |
| Ⅲ-565 | 2,6-$Cl_2$-Ph | 2-F-4-(2-Cl-4-$CF_3$-Ph-O)-Ph | $COCONH_2$ | |
| Ⅲ-566 | 2,6-$Cl_2$-Ph | 2-F-4-(2-Cl-4-$CF_3$-Ph-O)-Ph | COCONHMe | |
| Ⅲ-567 | 2,6-$Cl_2$-Ph | 2-F-4-(2-Cl-4-$CF_3$-Ph-O)-Ph | $COCONMe_2$ | |
| Ⅲ-568 | 2,6-$Cl_2$-Ph | 2-F-4-(2-Cl-4-$CF_3$-Ph-O)-Ph | $COCH_2Br$ | |
| Ⅲ-569 | 2,6-$Cl_2$-Ph | 2-F-4-(2-Cl-4-$CF_3$-Ph-O)-Ph | $COCH_2SMe$ | |
| Ⅲ-570 | 2,6-$Cl_2$-Ph | 2-F-4-(2-Cl-4-$CF_3$-Ph-O)-Ph | $COCH_2SOMe$ | |
| Ⅲ-571 | 2,6-$Cl_2$-Ph | 2-F-4-(2-Cl-4-$CF_3$-Ph-O)-Ph | $COCH_2SO_2Me$ | |
| Ⅲ-572 | 2,6-$Cl_2$-Ph | 2-F-4-(2-Cl-4-$CF_3$-Ph-O)-Ph | COCONMeOMe | |
| Ⅲ-573 | 2,6-$Cl_2$-Ph | 2-F-4-(2-Cl-4-$CF_3$-Ph-O)-Ph | COCONHOMe | |
| Ⅲ-574 | 2,6-$Cl_2$-Ph | 2-F-4-(2-Cl-4-$CF_3$-Ph-O)-Ph | $COCONHOCH_2Ph$ | |
| Ⅲ-575 | 2,6-$Cl_2$-Ph | 3,5-$Cl_2$-2,4-$F_2$-Ph | Me | |
| Ⅲ-576 | 2,6-$Cl_2$-Ph | 3,5-$Cl_2$-2,4-$F_2$-Ph | i-Pr | |
| Ⅲ-577 | 2,6-$Cl_2$-Ph | 3,5-$Cl_2$-2,4-$F_2$-Ph | cyclo-Pr | |
| Ⅲ-578 | 2,6-$Cl_2$-Ph | 3,5-$Cl_2$-2,4-$F_2$-Ph | Ph | |
| Ⅲ-579 | 2,6-$Cl_2$-Ph | 3,5-$Cl_2$-2,4-$F_2$-Ph | $COOCH_2Ph$ | |
| Ⅲ-580 | 2,6-$Cl_2$-Ph | 3,5-$Cl_2$-2,4-$F_2$-Ph | CH2CH=CH2 | |
| Ⅲ-581 | 2,6-$Cl_2$-Ph | 3,5-$Cl_2$-2,4-$F_2$-Ph | CH2C≡CH | |
| Ⅲ-582 | 2,6-$Cl_2$-Ph | 3,5-$Cl_2$-2,4-$F_2$-Ph | $SO_2Me$ | |
| Ⅲ-583 | 2,6-$Cl_2$-Ph | 3,5-$Cl_2$-2,4-$F_2$-Ph | $CH_2Ph$ | |
| Ⅲ-584 | 2,6-$Cl_2$-Ph | 3,5-$Cl_2$-2,4-$F_2$-Ph | COOMe | |
| Ⅲ-585 | 2,6-$Cl_2$-Ph | 3,5-$Cl_2$-2,4-$F_2$-Ph | SCOOMe | |
| Ⅲ-586 | 2,6-$Cl_2$-Ph | 3,5-$Cl_2$-2,4-$F_2$-Ph | $SO_2Ph$-4-Me | |
| Ⅲ-587 | 2,6-$Cl_2$-Ph | 3,5-$Cl_2$-2,4-$F_2$-Ph | COCOOMe | |
| Ⅲ-588 | 2,6-$Cl_2$-Ph | 3,5-$Cl_2$-2,4-$F_2$-Ph | COMe | |
| Ⅲ-589 | 2,6-$Cl_2$-Ph | 3,5-$Cl_2$-4-$CF_2HCF_2O$-Ph | Me | |
| Ⅲ-590 | 2,6-$Cl_2$-Ph | 3,5-$Cl_2$-4-$CF_2HCF_2O$-Ph | COMe | |
| Ⅲ-591 | 2,6-$Cl_2$-Ph | 3,5-$Cl_2$-4-$CF_2HCF_2O$-Ph | $SO_2Me$ | |
| Ⅲ-592 | 2,6-$Cl_2$-Ph | 2,5-$Cl_2$-4-$CF_3CFHCF_2O$-Ph | Me | |
| Ⅲ-593 | 2,6-$Cl_2$-Ph | 2,5-$Cl_2$-4-$CF_3CFHCF_2O$-Ph | $COOCH_2Ph$ | |
| Ⅲ-594 | 2,6-$Cl_2$-Ph | 2,5-$Cl_2$-4-$CF_3CFHCF_2O$-Ph | H | |
| Ⅲ-595 | 2,6-$Cl_2$-Ph | 2,5-$Cl_2$-4-$CF_3CFHCF_2O$-Ph | COCOOMe | |
| Ⅲ-596 | 2,6-$Cl_2$-Ph | 2,5-$Cl_2$-4-$CF_3CFHCF_2O$-Ph | COMe | |
| Ⅲ-597 | 2,6-$Cl_2$-Ph | 2,5-$Cl_2$-4-$CF_3CFHCF_2O$-Ph | $SO_2Me$ | |
| Ⅲ-598 | 2,6-$Cl_2$-Ph | 2-F-4-$CFClHCF_2S$-Ph | Me | |
| Ⅲ-599 | 2,6-$Cl_2$-Ph | 2-F-4-$CFClHCF_2S$-Ph | $COOCH_2Ph$ | |
| Ⅲ-600 | 2,6-$Cl_2$-Ph | 2-F-4-$CFClHCF_2S$-Ph | H | |

| compound No. | $Q_1$ | $Q_2$ | R | m. p. (°C) |
|---|---|---|---|---|
| Ⅲ-601 | 2,6-$Cl_2$-Ph | 2-F-4-CFClHCF$_2$S-Ph | COCOOMe | |
| Ⅲ-602 | 2,6-$Cl_2$-Ph | 2-F-4-CFClHCF$_2$S-Ph | Ph | |
| Ⅲ-603 | 2,6-$Cl_2$-Ph | 2-F-4-CFClHCF$_2$S-Ph | COMe | |
| Ⅲ-604 | 2,6-$Cl_2$-Ph | 2-F-4-CFClHCF$_2$S-Ph | $SO_2$Me | |
| Ⅲ-605 | 2,6-$Cl_2$-Ph | 2-F-4-CFClHCF$_2$SO-Ph | Me | |
| Ⅲ-606 | 2,6-$Cl_2$-Ph | 2-F-4-CFClHCF$_2$SO-Ph | COOCH$_2$Ph | |
| Ⅲ-607 | 2,6-$Cl_2$-Ph | 2-F-4-CFClHCF$_2$SO-Ph | COMe | |
| Ⅲ-608 | 2,6-$Cl_2$-Ph | 2-F-4-CFClHCF$_2$SO-Ph | $SO_2$Me | |
| Ⅲ-609 | 2,6-$Cl_2$-Ph | 2-F-4-CFClHCF$_2$SO-Ph | $CH_2C\equiv CH$ | |
| Ⅲ-610 | 2,6-$Cl_2$-Ph | 2-F-4-CFClHCF$_2$SO-Ph | $CH_2CH=CH_2$ | |
| Ⅲ-611 | 2,6-$Cl_2$-Ph | 2-F-4-CFClHCF$_2$SO-Ph | H | |
| Ⅲ-612 | 2,6-$Cl_2$-Ph | 2-F-4-CFClHCF$_2$SO$_2$-Ph | Me | |
| Ⅲ-613 | 2,6-$Cl_2$-Ph | 2-F-4-CFClHCF$_2$SO$_2$-Ph | COMe | |
| Ⅲ-614 | 2,6-$Cl_2$-Ph | 2-F-4-CFClHCF$_2$SO$_2$-Ph | $SO_2$Me | |
| Ⅲ-615 | 2,6-$Cl_2$-Ph | 4-$CF_3$S-Ph | Me | |
| Ⅲ-616 | 2,6-$Cl_2$-Ph | 4-$CF_3$S-Ph | COMe | |
| Ⅲ-617 | 2,6-$Cl_2$-Ph | 4-$CF_3$S-Ph | $SO_2$Me | |
| Ⅲ-618 | 2,6-$Cl_2$-Ph | 4-$CF_3$SO-Ph | Me | |
| Ⅲ-619 | 2,6-$Cl_2$-Ph | 4-$CF_3$SO-Ph | COMe | |
| Ⅲ-620 | 2,6-$Cl_2$-Ph | 4-$CF_3$SO-Ph | $SO_2$Me | |
| Ⅲ-621 | 2,6-$Cl_2$-Ph | 4-$CF_3SO_2$-Ph | Me | |
| Ⅲ-622 | 2,6-$Cl_2$-Ph | 4-$CF_3SO_2$-Ph | COMe | |
| Ⅲ-623 | 2,6-$Cl_2$-Ph | 4-$CF_3SO_2$-Ph | $SO_2$Me | |
| Ⅲ-624 | 2,6-$Cl_2$-Ph | 2-F-4-MeS-Ph | Me | |
| Ⅲ-625 | 2,6-$Cl_2$-Ph | 2-F-4-MeS-Ph | COMe | |
| Ⅲ-626 | 2,6-$Cl_2$-Ph | 2-F-4-MeS-Ph | $SO_2$Me | |
| Ⅲ-627 | 2,6-$Cl_2$-Ph | 2-F-4-MeSO-Ph | Me | |
| Ⅲ-628 | 2,6-$Cl_2$-Ph | 2-F-4-MeSO-Ph | COMe | |
| Ⅲ-629 | 2,6-$Cl_2$-Ph | 2-F-4-MeSO-Ph | $SO_2$Me | |
| Ⅲ-630 | 2,6-$Cl_2$-Ph | 2-F-4-MeSO$_2$-Ph | Me | |
| Ⅲ-631 | 2,6-$Cl_2$-Ph | 2-F-4-MeSO$_2$-Ph | COMe | |
| Ⅲ-632 | 2,6-$Cl_2$-Ph | 2-F-4-MeSO$_2$-Ph | $SO_2$Me | |
| Ⅲ-633 | 2,6-$Cl_2$-Ph | 2,6-$F_2$-4-MeS-Ph | Me | |
| Ⅲ-634 | 2,6-$Cl_2$-Ph | 2,6-$F_2$-4-MeS-Ph | COMe | |
| Ⅲ-635 | 2,6-$Cl_2$-Ph | 2,6-$F_2$-4-MeS-Ph | $SO_2$Me | |
| Ⅲ-636 | 2,6-$Cl_2$-Ph | 2,6-$F_2$-4-MeSO-Ph | Me | |
| Ⅲ-637 | 2,6-$Cl_2$-Ph | 2,6-$F_2$-4-MeSO-Ph | COMe | |
| Ⅲ-638 | 2,6-$Cl_2$-Ph | 2,6-$F_2$-4-MeSO-Ph | $SO_2$Me | |
| Ⅲ-639 | 2,6-$Cl_2$-Ph | 2,6-$F_2$-4-MeSO$_2$-Ph | Me | |
| Ⅲ-640 | 2,6-$Cl_2$-Ph | 2,6-$F_2$-4-MeSO$_2$-Ph | COMe | |

| compound No. | $Q_1$ | $Q_2$ | R | m. p. (℃) |
|---|---|---|---|---|
| Ⅲ-641 | 2,6-$Cl_2$-Ph | 2,6-$F_2$-4-$MeSO_2$-Ph | $SO_2Me$ | |
| Ⅲ-642 | 2,6-$Cl_2$-Ph | 2-F-4-$CF_3CH_2S$-Ph | Me | |
| Ⅲ-643 | 2,6-$Cl_2$-Ph | 2-F-4-$CF_3CH_2S$-Ph | COMe | |
| Ⅲ-644 | 2,6-$Cl_2$-Ph | 2-F-4-$CF_3CH_2S$-Ph | $SO_2Me$ | |
| Ⅲ-645 | 2,6-$Cl_2$-Ph | 2-F-4-$CF_3CH_2SO$-Ph | Me | |
| Ⅲ-646 | 2,6-$Cl_2$-Ph | 2-F-4-$CF_3CH_2SO$-Ph | COMe | |
| Ⅲ-647 | 2,6-$Cl_2$-Ph | 2-F-4-$CF_3CH_2SO$-Ph | SO2Me | |
| Ⅲ-648 | 2,6-$Cl_2$-Ph | 2-F-4-$CF_3CH_2SO_2$-Ph | Me | |
| Ⅲ-649 | 2,6-$Cl_2$-Ph | 2-F-4-$CF_3CH_2SO_2$-Ph | COMe | |
| Ⅲ-650 | 2,6-$Cl_2$-Ph | 2-F-4-$CF_3CH_2SO_2$-Ph | $SO_2Me$ | |
| Ⅲ-651 | 2,6-$Cl_2$-Ph | $CF_3CH_2$ | Me | |
| Ⅲ-652 | 2,6-$Cl_2$-Ph | $CF_3CH_2$ | COMe | |
| Ⅲ-653 | 2,6-$Cl_2$-Ph | $CF_3CH_2$ | $SO_2Me$ | |
| Ⅲ-654 | 2,6-$Cl_2$-Ph | 2-F-4-$CF_2HS$-Ph | Me | |
| Ⅲ-655 | 2,6-$Cl_2$-Ph | 2-F-4-$CF_2HS$-Ph | COMe | |
| Ⅲ-656 | 2,6-$Cl_2$-Ph | 2-F-4-$CF_2HS$-Ph | $SO_2Me$ | |
| Ⅲ-657 | 2,6-$Cl_2$-Ph | 2-F-4-$CF_2HSO$-Ph | Me | |
| Ⅲ-658 | 2,6-$Cl_2$-Ph | 2-F-4-$CF_2HSO$-Ph | COMe | |
| Ⅲ-659 | 2,6-$Cl_2$-Ph | 2-F-4-$CF_2HSO$-Ph | $SO_2Me$ | |
| Ⅲ-660 | 2,6-$Cl_2$-Ph | 2-F-4-$CF_2HSO_2$-Ph | Me | |
| Ⅲ-661 | 2,6-$Cl_2$-Ph | 2-F-4-$CF_2HSO_2$-Ph | COMe | |
| Ⅲ-662 | 2,6-$Cl_2$-Ph | 2-F-4-$CF_2HSO_2$-Ph | $SO_2Me$ | |
| Ⅲ-663 | 2,6-$Cl_2$-Ph | 2-F-4-$CF_3S$-Ph | Me | |
| Ⅲ-664 | 2,6-$Cl_2$-Ph | 2-F-4-$CF_3S$-Ph | COMe | |
| Ⅲ-665 | 2,6-$Cl_2$-Ph | 2-F-4-$CF_3S$-Ph | $SO_2Me$ | |
| Ⅲ-666 | 2,6-$Cl_2$-Ph | 2-F-4-$CF_3SO$-Ph | Me | |
| Ⅲ-667 | 2,6-$Cl_2$-Ph | 2-F-4-$CF_3SO$-Ph | COMe | |
| Ⅲ-668 | 2,6-$Cl_2$-Ph | 2-F-4-$CF_3SO$-Ph | $SO_2Me$ | |
| Ⅲ-669 | 2,6-$Cl_2$-Ph | 2-F-4-$CF_3SO_2$-Ph | Me | |
| Ⅲ-670 | 2,6-$Cl_2$-Ph | 2-F-4-$CF_3SO_2$-Ph | COMe | |
| Ⅲ-671 | 2,6-$Cl_2$-Ph | 2-F-4-$CF_3SO_2$-Ph | $SO_2Me$ | |
| Ⅲ-672 | 2,6-$Cl_2$-Ph | 2-F-4-$CH_3CH_2S$-Ph | Me | |
| Ⅲ-673 | 2,6-$Cl_2$-Ph | 2-F-4-$CH_3CH_2S$-Ph | COMe | |
| Ⅲ-674 | 2,6-$Cl_2$-Ph | 2-F-4-$CH_3CH_2S$-Ph | $SO_2Me$ | |
| Ⅲ-675 | 2,6-$Cl_2$-Ph | 2-F-4-$CH_3CH_2SO$-Ph | Me | |
| Ⅲ-676 | 2,6-$Cl_2$-Ph | 2-F-4-$CH_3CH_2SO$-Ph | COMe | |
| Ⅲ-677 | 2,6-$Cl_2$-Ph | 2-F-4-$CH_3CH_2SO$-Ph | $SO_2Me$ | |
| Ⅲ-678 | 2,6-$Cl_2$-Ph | 2-F-4-$CH_3CH_2SO_2$-Ph | Me | |
| Ⅲ-679 | 2,6-$Cl_2$-Ph | 2-F-4-$CH_3CH_2SO_2$-Ph | COMe | |
| Ⅲ-680 | 2,6-$Cl_2$-Ph | 2-F-4-$CH_3CH_2SO_2$-Ph | $SO_2Me$ | |

| compound No. | $Q_1$ | $Q_2$ | R | m. p. (°C) |
|---|---|---|---|---|
| Ⅲ-681 | 2,6-$Cl_2$-Ph | 2-F-4-(2-Cl-4-$CF_3$-pyridin-2yl-S)-Ph | Me | |
| Ⅲ-682 | 2,6-$Cl_2$-Ph | 2-F-4-(2-Cl-4-$CF_3$-pyridin-2yl-S)-Ph | COMe | |
| Ⅲ-683 | 2,6-$Cl_2$-Ph | 2-F-4-(2-Cl-4-$CF_3$-pyridin-2yl-S)-Ph | $SO_2$Me | |
| Ⅲ-684 | 2,6-$Cl_2$-Ph | 2-F-4-(2-Cl-4-$CF_3$-pyridin-2yl-SO)-Ph | Me | |
| Ⅲ-685 | 2,6-$Cl_2$-Ph | 2-F-4-(2-Cl-4-$CF_3$-pyridin-2yl-SO)-Ph | COMe | |
| Ⅲ-686 | 2,6-$Cl_2$-Ph | 2-F-4-(2-Cl-4-$CF_3$-pyridin-2yl-SO)-Ph | $SO_2$Me | |
| Ⅲ-687 | 2,6-$Cl_2$-Ph | 2-F-4-(2-Cl-4-$CF_3$-pyridin-2yl-$SO_2$)-Ph | Me | |
| Ⅲ-688 | 2,6-$Cl_2$-Ph | 2-F-4-(2-Cl-4-$CF_3$-pyridin-2yl-$SO_2$)-Ph | COMe | |
| Ⅲ-689 | 2,6-$Cl_2$-Ph | 2-F-4-(2-Cl-4-$CF_3$-pyridin-2yl-$SO_2$)-Ph | $SO_2$Me | |
| Ⅲ-690 | 2,6-$Cl_2$-Ph | 2-Me-4-$CF_3$S-Ph | Me | |
| Ⅲ-691 | 2,6-$Cl_2$-Ph | 2-Me-4-$CF_3$S-Ph | COMe | |
| Ⅲ-692 | 2,6-$Cl_2$-Ph | 2-Me-4-$CF_3$S-Ph | $SO_2$Me | |
| Ⅲ-693 | 2,6-$Cl_2$-Ph | 2-Me-4-$CF_3$SO-Ph | Me | |
| Ⅲ-694 | 2,6-$Cl_2$-Ph | 2-Me-4-$CF_3$SO-Ph | COMe | |
| Ⅲ-695 | 2,6-$Cl_2$-Ph | 2-Me-4-$CF_3$SO-Ph | $SO_2$Me | |
| Ⅲ-696 | 2,6-$Cl_2$-Ph | 2-Me-4-$CF_3SO_2$-Ph | Me | |
| Ⅲ-697 | 2,6-$Cl_2$-Ph | 2-Me-4-$CF_3SO_2$-Ph | COMe | |
| Ⅲ-698 | 2,6-$Cl_2$-Ph | 2-Me-4-$CF_3SO_2$-Ph | $SO_2$Me | |
| Ⅲ-699 | 2,6-$Cl_2$-Ph | 3-F-4-$CF_3$S-Ph | Me | |
| Ⅲ-700 | 2,6-$Cl_2$-Ph | 3-F-4-$CF_3$S-Ph | COMe | |
| Ⅲ-701 | 2,6-$Cl_2$-Ph | 3-F-4-$CF_3$S-Ph | $SO_2$Me | |
| Ⅲ-702 | 2,6-$Cl_2$-Ph | 3-F-4-$CF_3$SO-Ph | Me | |
| Ⅲ-703 | 2,6-$Cl_2$-Ph | 3-F-4-$CF_3$SO-Ph | COMe | |
| Ⅲ-704 | 2,6-$Cl_2$-Ph | 3-F-4-$CF_3$SO-Ph | $SO_2$Me | |
| Ⅲ-705 | 2,6-$Cl_2$-Ph | 3-F-4-$CF_3SO_2$-Ph | Me | |
| Ⅲ-706 | 2,6-$Cl_2$-Ph | 3-F-4-$CF_3SO_2$-Ph | COMe | |
| Ⅲ-707 | 2,6-$Cl_2$-Ph | 3-F-4-$CF_3SO_2$-Ph | $SO_2$Me | |
| Ⅲ-708 | 2,6-$Cl_2$-Ph | 2,3-$Me_2$-4-$CF_3$S-Ph | Me | |
| Ⅲ-709 | 2,6-$Cl_2$-Ph | 2,3-$Me_2$-4-$CF_3$S-Ph | COMe | |
| Ⅲ-710 | 2,6-$Cl_2$-Ph | 2,3-$Me_2$-4-$CF_3$S-Ph | $SO_2$Me | |
| Ⅲ-711 | 2,6-$Cl_2$-Ph | 2,3-$Me_2$-4-$CF_3$SO-Ph | Me | |
| Ⅲ-712 | 2,6-$Cl_2$-Ph | 2,3-$Me_2$-4-$CF_3$SO-Ph | COMe | |
| Ⅲ-713 | 2,6-$Cl_2$-Ph | 2,3-$Me_2$-4-$CF_3$SO-Ph | $SO_2$Me | |
| Ⅲ-714 | 2,6-$Cl_2$-Ph | 2,3-$Me_2$-4-$CF_3SO_2$-Ph | Me | |
| Ⅲ-715 | 2,6-$Cl_2$-Ph | 2,3-$Me_2$-4-$CF_3SO_2$-Ph | COMe | |
| Ⅲ-716 | 2,6-$Cl_2$-Ph | 2,3-$Me_2$-4-$CF_3SO_2$-Ph | $SO_2$Me | |
| Ⅲ-717 | 2,6-$Cl_2$-Ph | 5-Cl-2-Me-4-$CF_3$S-Ph | Me | |
| Ⅲ-718 | 2,6-$Cl_2$-Ph | 5-Cl-2-Me-4-$CF_3$S-Ph | COMe | |
| Ⅲ-719 | 2,6-$Cl_2$-Ph | 5-Cl-2-Me-4-$CF_3$S-Ph | $SO_2$Me | |
| Ⅲ-720 | 2,6-$Cl_2$-Ph | 5-Cl-2-Me-4-$CF_3$SO-Ph | Me | |

| compound No. | $Q_1$ | $Q_2$ | R | m. p. (°C) |
|---|---|---|---|---|
| Ⅲ-721 | 2,6-$Cl_2$-Ph | 5-Cl-2-Me-4-$CF_3$SO-Ph | COMe | |
| Ⅲ-722 | 2,6-$Cl_2$-Ph | 5-Cl-2-Me-4-$CF_3$SO-Ph | $SO_2$Me | |
| Ⅲ-723 | 2,6-$Cl_2$-Ph | 5-Cl-2-Me-4-$CF_3SO_2$-Ph | Me | |
| Ⅲ-724 | 2,6-$Cl_2$-Ph | 5-Cl-2-Me-4-$CF_3SO_2$-Ph | COMe | |
| Ⅲ-725 | 2,6-$Cl_2$-Ph | 5-Cl-2-Me-4-$CF_3SO_2$-Ph | $SO_2$Me | |
| Ⅲ-726 | 2,6-$Cl_2$-Ph | 2,6-$F_2$-4-$CF_3$S-Ph | Me | |
| Ⅲ-727 | 2,6-$Cl_2$-Ph | 2,6-$F_2$-4-$CF_3$S-Ph | COMe | |
| Ⅲ-728 | 2,6-$Cl_2$-Ph | 2,6-$F_2$-4-$CF_3$S-Ph | $SO_2$Me | |
| Ⅲ-729 | 2,6-$Cl_2$-Ph | 2,6-$F_2$-4-$CF_3$SO-Ph | Me | |
| Ⅲ-730 | 2,6-$Cl_2$-Ph | 2,6-$F_2$-4-$CF_3$SO-Ph | COMe | |
| Ⅲ-731 | 2,6-$Cl_2$-Ph | 2,6-$F_2$-4-$CF_3$SO-Ph | $SO_2$Me | |
| Ⅲ-732 | 2,6-$Cl_2$-Ph | 2,6-$F_2$-4-$CF_3SO_2$-Ph | Me | |
| Ⅲ-733 | 2,6-$Cl_2$-Ph | 2,6-$F_2$-4-$CF_3SO_2$-Ph | COMe | |
| Ⅲ-734 | 2,6-$Cl_2$-Ph | 2,6-$F_2$-4-$CF_3SO_2$-Ph | $SO_2$Me | |
| Ⅲ-735 | 2,6-$Cl_2$-Ph | 2-F-4-$CF_3CF_2$S-Ph | Me | |
| Ⅲ-736 | 2,6-$Cl_2$-Ph | 2-F-4-$CF_3CF_2$S-Ph | COMe | |
| Ⅲ-737 | 2,6-$Cl_2$-Ph | 2-F-4-$CF_3CF_2$S-Ph | $SO_2$Me | |
| Ⅲ-738 | 2,6-$Cl_2$-Ph | 2-F-4-$CF_3CF_2$SO-Ph | Me | |
| Ⅲ-739 | 2,6-$Cl_2$-Ph | 2-F-4-$CF_3CF_2$SO-Ph | COMe | |
| Ⅲ-740 | 2,6-$Cl_2$-Ph | 2-F-4-$CF_3CF_2$SO-Ph | $SO_2$Me | |
| Ⅲ-741 | 2,6-$Cl_2$-Ph | 2-F-4-$CF_3CF_2SO_2$-Ph | Me | |
| Ⅲ-742 | 2,6-$Cl_2$-Ph | 2-F-4-$CF_3CF_2SO_2$-Ph | COMe | |
| Ⅲ-743 | 2,6-$Cl_2$-Ph | 2-F-4-$CF_3CF_2SO_2$-Ph | $SO_2$Me | |
| Ⅲ-744 | 2,6-$Cl_2$-Ph | 2-F-4-$CF_3CF_2CF_2$S-Ph | Me | |
| Ⅲ-745 | 2,6-$Cl_2$-Ph | 2-F-4-$CF_3CF_2CF_2$S-Ph | COMe | |
| Ⅲ-746 | 2,6-$Cl_2$-Ph | 2-F-4-$CF_3CF_2CF_2$S-Ph | $SO_2$Me | |
| Ⅲ-747 | 2,6-$Cl_2$-Ph | 2-F-4-$CF_3CF_2CF_2$SO-Ph | Me | |
| Ⅲ-748 | 2,6-$Cl_2$-Ph | 2-F-4-$CF_3CF_2CF_2$SO-Ph | COMe | |
| Ⅲ-749 | 2,6-$Cl_2$-Ph | 2-F-4-$CF_3CF_2CF_2$SO-Ph | $SO_2$Me | |
| Ⅲ-750 | 2,6-$Cl_2$-Ph | 2-F-4-$CF_3CF_2CF_2SO_2$-Ph | Me | |
| Ⅲ-751 | 2,6-$Cl_2$-Ph | 2-F-4-$CF_3CF_2CF_2SO_2$-Ph | COMe | |
| Ⅲ-752 | 2,6-$Cl_2$-Ph | 2-F-4-$CF_3CF_2CF_2SO_2$-Ph | $SO_2$Me | |
| Ⅲ-753 | 2,6-$Cl_2$-Ph | 2-F-4-$(CF_3)_2$CFS-Ph | Me | |
| Ⅲ-754 | 2,6-$Cl_2$-Ph | 2-F-4-$(CF_3)_2$CFS-Ph | COMe | |
| Ⅲ-755 | 2,6-$Cl_2$-Ph | 2-F-4-$(CF_3)_2$CFS-Ph | $SO_2$Me | |
| Ⅲ-756 | 2,6-$Cl_2$-Ph | 2-F-4-$(CF_3)_2$CFSO-Ph | Me | |
| Ⅲ-757 | 2,6-$Cl_2$-Ph | 2-F-4-$(CF_3)_2$CFSO-Ph | COMe | |
| Ⅲ-758 | 2,6-$Cl_2$-Ph | 2-F-4-$(CF_3)_2$CFSO-Ph | $SO_2$Me | |
| Ⅲ-759 | 2,6-$Cl_2$-Ph | 2-F-4-$(CF_3)_2CFSO_2$-Ph | Me | |
| Ⅲ-760 | 2,6-$Cl_2$-Ph | 2-F-4-$(CF_3)_2CFSO_2$-Ph | COMe | |

| compound No. | $Q_1$ | $Q_2$ | R | m. p. (°C) |
|---|---|---|---|---|
| Ⅲ-761 | 2,6-$Cl_2$-Ph | 2-F-4-$(CF_3)_2CFSO_2$-Ph | $SO_2Me$ | |
| Ⅲ-762 | 3-Cl-pyridin-2yl | 4-Cl-Ph | Me | 165-167 |
| Ⅲ-763 | 3-Cl-pyridin-2yl | 4-Cl-Ph | Ph | |
| Ⅲ-764 | 3-Cl-pyridin-2yl | 4-Cl-Ph | i-Pr | |
| Ⅲ-765 | 3-Cl-pyridin-2yl | 4-Cl-Ph | $CH_2Ph$ | |
| Ⅲ-766 | 3-Cl-pyridin-2yl | 4-Cl-Ph | $CH_2CH=CH_2$ | |
| Ⅲ-767 | 3-Cl-pyridin-2yl | 4-Cl-Ph | H | |
| Ⅲ-768 | 3-Cl-pyridin-2yl | 4-Cl-Ph | $COOCH_2Ph$ | |
| Ⅲ-769 | 3-Cl-pyridin-2yl | 4-Cl-Ph | $CH_2C\equiv CH$ | |
| Ⅲ-770 | 3-Cl-pyridin-2yl | 4-Cl-Ph | COMe | |
| Ⅲ-771 | 3-Cl-pyridin-2yl | 4-Cl-Ph | $CONMe_2$ | |
| Ⅲ-772 | 3-Cl-pyridin-2yl | 4-Cl-Ph | $SO_2Me$ | |
| Ⅲ-773 | 3-Cl-pyridin-2yl | 4-Cl-Ph | COOMe | |
| Ⅲ-774 | 3-Cl-pyridin-2yl | 4-Cl-Ph | CHMePh | |
| Ⅲ-775 | 3-Cl-pyridin-2yl | 4-Cl-Ph | COCOOMe | |
| Ⅲ-776 | 3-Cl-pyridin-2yl | 4-Cl-Ph | NO | |
| Ⅲ-777 | 3-Cl-pyridin-2yl | 3,5-$Cl_2$-4-(2-Cl-4-$CF_3$-pyridin-2yl-O)-Ph | Me | |
| Ⅲ-778 | 3-Cl-pyridin-2yl | 3,5-$Cl_2$-4-(2-Cl-4-$CF_3$-pyridin-2yl-O)-Ph | COMe | |
| Ⅲ-779 | 3-Cl-pyridin-2yl | 3,5-$Cl_2$-4-(2-Cl-4-$CF_3$-pyridin-2yl-O)-Ph | $SO_2Me$ | |
| Ⅲ-780 | 3-Cl-pyridin-2yl | 2-F-4-(2-Cl-4-$CF_3$-Ph-O)-Ph | Me | |
| Ⅲ-781 | 3-Cl-pyridin-2yl | 2-F-4-(2-Cl-4-$CF_3$-Ph-O)-Ph | $COOCH_2Ph$ | |
| Ⅲ-782 | 3-Cl-pyridin-2yl | 2-F-4-(2-Cl-4-$CF_3$-Ph-O)-Ph | $CH_2C\equiv CH$ | |
| Ⅲ-783 | 3-Cl-pyridin-2yl | 2-F-4-(2-Cl-4-$CF_3$-Ph-O)-Ph | $CH_2CH=CH_2$ | |
| Ⅲ-784 | 3-Cl-pyridin-2yl | 2-F-4-(2-Cl-4-$CF_3$-Ph-O)-Ph | i-Pr | |
| Ⅲ-785 | 3-Cl-pyridin-2yl | 2-F-4-(2-Cl-4-$CF_3$-Ph-O)-Ph | $SO_2Me$ | |
| Ⅲ-786 | 3-Cl-pyridin-2yl | 2-F-4-(2-Cl-4-$CF_3$-Ph-O)-Ph | H | |
| Ⅲ-787 | 3-Cl-pyridin-2yl | 2-F-4-(2-Cl-4-$CF_3$-Ph-O)-Ph | COOMe | |
| Ⅲ-788 | 3-Cl-pyridin-2yl | 2-F-4-(2-Cl-4-$CF_3$-Ph-O)-Ph | $CH_2Ph$ | |
| Ⅲ-789 | 3-Cl-pyridin-2yl | 2-F-4-(2-Cl-4-$CF_3$-Ph-O)-Ph | Ph | |
| Ⅲ-790 | 3-Cl-pyridin-2yl | 2-F-4-(2-Cl-4-$CF_3$-Ph-O)-Ph | SCOOMe | |
| Ⅲ-791 | 3-Cl-pyridin-2yl | 2-F-4-(2-Cl-4-$CF_3$-Ph-O)-Ph | $COCF_3$ | |
| Ⅲ-792 | 3-Cl-pyridin-2yl | 2-F-4-(2-Cl-4-$CF_3$-Ph-O)-Ph | COMe | |
| Ⅲ-793 | 3-Cl-pyridin-2yl | 2-F-4-(2-Cl-4-$CF_3$-Ph-O)-Ph | $SO_2Ph$-4-Me | |
| Ⅲ-794 | 3-Cl-pyridin-2yl | 2-F-4-(2-Cl-4-$CF_3$-Ph-O)-Ph | $SO_2CF_3$ | |
| Ⅲ-795 | 3-Cl-pyridin-2yl | 2-F-4-(2-Cl-4-$CF_3$-Ph-O)-Ph | cyclo-Pr | |
| Ⅲ-796 | 3-Cl-pyridin-2yl | 2-F-4-(2-Cl-4-$CF_3$-Ph-O)-Ph | COCOOMe | |
| Ⅲ-797 | 3-Cl-pyridin-2yl | 2-F-4-(2-Cl-4-$CF_3$-Ph-O)-Ph | COCOOH | |
| Ⅲ-798 | 3-Cl-pyridin-2yl | 2-F-4-(2-Cl-4-$CF_3$-Ph-O)-Ph | $COCOOCH_2Ph$ | |
| Ⅲ-799 | 3-Cl-pyridin-2yl | 2-F-4-(2-Cl-4-$CF_3$-Ph-O)-Ph | $COCOOHNH_3$ | |
| Ⅲ-800 | 3-Cl-pyridin-2yl | 2-F-4-(2-Cl-4-$CF_3$-Ph-O)-Ph | $CONH_2$ | |

| compound No. | $Q_1$ | $Q_2$ | R | m. p. (°C) |
|---|---|---|---|---|
| III-801 | 3-Cl-pyridin-2yl | 2-F-4-(2-Cl-4-$CF_3$-Ph-O)-Ph | COCONHMe | |
| III-802 | 3-Cl-pyridin-2yl | 2-F-4-(2-Cl-4-$CF_3$-Ph-O)-Ph | $COCONMe_2$ | |
| III-803 | 3-Cl-pyridin-2yl | 2-F-4-(2-Cl-4-$CF_3$-Ph-O)-Ph | $COCH_2Br$ | |
| III-804 | 3-Cl-pyridin-2yl | 2-F-4-(2-Cl-4-$CF_3$-Ph-O)-Ph | $COCH_2SMe$ | |
| III-805 | 3-Cl-pyridin-2yl | 2-F-4-(2-Cl-4-$CF_3$-Ph-O)-Ph | $COCH_2SOMe$ | |
| III-806 | 3-Cl-pyridin-2yl | 2-F-4-(2-Cl-4-$CF_3$-Ph-O)-Ph | $COCH_2SO_2Me$ | |
| III-807 | 3-Cl-pyridin-2yl | 2-F-4-(2-Cl-4-$CF_3$-Ph-O)-Ph | COCONMeOMe | |
| III-808 | 3-Cl-pyridin-2yl | 2-F-4-(2-Cl-4-$CF_3$-Ph-O)-Ph | COCONHOMe | |
| III-809 | 3-Cl-pyridin-2yl | 2-F-4-(2-Cl-4-$CF_3$-Ph-O)-Ph | $COCONHOCH_2Ph$ | |
| III-810 | 3-Cl-pyridin-2yl | 3,5-$Cl_2$-2,4-$F_2$-Ph | Me | |
| III-811 | 3-Cl-pyridin-2yl | 3,5-$Cl_2$-2,4-$F_2$-Ph | i-Pr | |
| III-812 | 3-Cl-pyridin-2yl | 3,5-$Cl_2$-2,4-$F_2$-Ph | cyclo-Pr | |
| III-813 | 3-Cl-pyridin-2yl | 3,5-$Cl_2$-2,4-$F_2$-Ph | Ph | |
| III-814 | 3-Cl-pyridin-2yl | 3,5-$Cl_2$-2,4-$F_2$-Ph | $COOCH_2Ph$ | |
| III-815 | 3-Cl-pyridin-2yl | 3,5-$Cl_2$-2,4-$F_2$-Ph | CH2CH=CH2 | |
| III-816 | 3-Cl-pyridin-2yl | 3,5-$Cl_2$-2,4-$F_2$-Ph | CH2C≡CH | |
| III-817 | 3-Cl-pyridin-2yl | 3,5-$Cl_2$-2,4-$F_2$-Ph | $SO_2Me$ | |
| III-818 | 3-Cl-pyridin-2yl | 3,5-$Cl_2$-2,4-$F_2$-Ph | $CH_2Ph$ | |
| III-819 | 3-Cl-pyridin-2yl | 3,5-$Cl_2$-2,4-$F_2$-Ph | COOMe | |
| III-820 | 3-Cl-pyridin-2yl | 3,5-$Cl_2$-2,4-$F_2$-Ph | SCOOMe | |
| III-821 | 3-Cl-pyridin-2yl | 3,5-$Cl_2$-2,4-$F_2$-Ph | $SO_2Ph$-4-Me | |
| III-822 | 3-Cl-pyridin-2yl | 3,5-$Cl_2$-2,4-$F_2$-Ph | COCOOMe | |
| III-823 | 3-Cl-pyridin-2yl | 3,5-$Cl_2$-2,4-$F_2$-Ph | COMe | |
| III-824 | 3-Cl-pyridin-2yl | 3,5-$Cl_2$-4-$CF_2HCF_2O$-Ph | Me | |
| III-825 | 3-Cl-pyridin-2yl | 3,5-$Cl_2$-4-$CF_2HCF_2O$-Ph | COMe | |
| III-826 | 3-Cl-pyridin-2yl | 3,5-$Cl_2$-4-$CF_2HCF_2O$-Ph | $SO_2Me$ | |
| III-827 | 3-Cl-pyridin-2yl | 2,5-$Cl_2$-4-$CF_3CFHCF_2O$-Ph | Me | |
| III-828 | 3-Cl-pyridin-2yl | 2,5-$Cl_2$-4-$CF_3CFHCF_2O$-Ph | $COOCH_2Ph$ | |
| III-829 | 3-Cl-pyridin-2yl | 2,5-$Cl_2$-4-$CF_3CFHCF_2O$-Ph | H | |
| III-830 | 3-Cl-pyridin-2yl | 2,5-$Cl_2$-4-$CF_3CFHCF_2O$-Ph | COCOOMe | |
| III-831 | 3-Cl-pyridin-2yl | 2,5-$Cl_2$-4-$CF_3CFHCF_2O$-Ph | COMe | |
| III-832 | 3-Cl-pyridin-2yl | 2,5-$Cl_2$-4-$CF_3CFHCF_2O$-Ph | $SO_2Me$ | |
| III-833 | 3-Cl-pyridin-2yl | 2-F-4-$CFClHCF_2S$-Ph | Me | |
| III-834 | 3-Cl-pyridin-2yl | 2-F-4-$CFClHCF_2S$-Ph | $COOCH_2Ph$ | |
| III-835 | 3-Cl-pyridin-2yl | 2-F-4-$CFClHCF_2S$-Ph | H | |
| III-836 | 3-Cl-pyridin-2yl | 2-F-4-$CFClHCF_2S$-Ph | COCOOMe | |
| III-837 | 3-Cl-pyridin-2yl | 2-F-4-$CFClHCF_2S$-Ph | Ph | |
| III-838 | 3-Cl-pyridin-2yl | 2-F-4-$CFClHCF_2S$-Ph | COMe | |
| III-839 | 3-Cl-pyridin-2yl | 2-F-4-$CFClHCF_2S$-Ph | $SO_2Me$ | |
| III-840 | 3-Cl-pyridin-2yl | 2-F-4-$CFClHCF_2SO$-Ph | Me | |

| compound No. | $Q_1$ | $Q_2$ | R | m. p. (°C) |
|---|---|---|---|---|
| Ⅲ-841 | 3-Cl-pyridin-2yl | 2-F-4-$CFClHCF_2SO$-Ph | $COOCH_2Ph$ | |
| Ⅲ-842 | 3-Cl-pyridin-2yl | 2-F-4-$CFClHCF_2SO$-Ph | COMe | |
| Ⅲ-843 | 3-Cl-pyridin-2yl | 2-F-4-$CFClHCF_2SO$-Ph | $SO_2Me$ | |
| Ⅲ-844 | 3-Cl-pyridin-2yl | 2-F-4-$CFClHCF_2SO$-Ph | $CH_2C\equiv CH$ | |
| Ⅲ-845 | 3-Cl-pyridin-2yl | 2-F-4-$CFClHCF_2SO$-Ph | $CH_2CH=CH_2$ | |
| Ⅲ-846 | 3-Cl-pyridin-2yl | 2-F-4-$CFClHCF_2SO$-Ph | H | |
| Ⅲ-847 | 3-Cl-pyridin-2yl | 2-F-4-$CFClHCF_2SO_2$-Ph | Me | |
| Ⅲ-848 | 3-Cl-pyridin-2yl | 2-F-4-$CFClHCF_2SO_2$-Ph | COMe | |
| Ⅲ-849 | 3-Cl-pyridin-2yl | 2-F-4-$CFClHCF_2SO_2$-Ph | $SO_2Me$ | |
| Ⅲ-850 | 3-Cl-pyridin-2yl | 4-$CF_3S$-Ph | Me | |
| Ⅲ-851 | 3-Cl-pyridin-2yl | 4-$CF_3S$-Ph | COMe | |
| Ⅲ-852 | 3-Cl-pyridin-2yl | 4-$CF_3S$-Ph | $SO_2Me$ | |
| Ⅲ-853 | 3-Cl-pyridin-2yl | 4-$CF_3SO$-Ph | Me | |
| Ⅲ-854 | 3-Cl-pyridin-2yl | 4-$CF_3SO$-Ph | COMe | |
| Ⅲ-855 | 3-Cl-pyridin-2yl | 4-$CF_3SO$-Ph | $SO_2Me$ | |
| Ⅲ-856 | 3-Cl-pyridin-2yl | 4-$CF_3SO_2$-Ph | Me | |
| Ⅲ-857 | 3-Cl-pyridin-2yl | 4-$CF_3SO_2$-Ph | COMe | |
| Ⅲ-858 | 3-Cl-pyridin-2yl | 4-$CF_3SO_2$-Ph | $SO_2Me$ | |
| Ⅲ-859 | 3-Cl-pyridin-2yl | 2-F-4-MeS-Ph | Me | |
| Ⅲ-860 | 3-Cl-pyridin-2yl | 2-F-4-MeS-Ph | COMe | |
| Ⅲ-861 | 3-Cl-pyridin-2yl | 2-F-4-MeS-Ph | $SO_2Me$ | |
| Ⅲ-862 | 3-Cl-pyridin-2yl | 2-F-4-MeSO-Ph | Me | |
| Ⅲ-863 | 3-Cl-pyridin-2yl | 2-F-4-MeSO-Ph | COMe | |
| Ⅲ-864 | 3-Cl-pyridin-2yl | 2-F-4-MeSO-Ph | $SO_2Me$ | |
| Ⅲ-865 | 3-Cl-pyridin-2yl | 2-F-4-$MeSO_2$-Ph | Me | |
| Ⅲ-866 | 3-Cl-pyridin-2yl | 2-F-4-$MeSO_2$-Ph | COMe | |
| Ⅲ-867 | 3-Cl-pyridin-2yl | 2-F-4-$MeSO_2$-Ph | $SO_2Me$ | |
| Ⅲ-868 | 3-Cl-pyridin-2yl | 2,6-$F_2$-4-MeS-Ph | Me | |
| Ⅲ-869 | 3-Cl-pyridin-2yl | 2,6-$F_2$-4-MeS-Ph | COMe | |
| Ⅲ-870 | 3-Cl-pyridin-2yl | 2,6-$F_2$-4-MeS-Ph | $SO_2Me$ | |
| Ⅲ-871 | 3-Cl-pyridin-2yl | 2,6-$F_2$-4-MeSO-Ph | Me | |
| Ⅲ-872 | 3-Cl-pyridin-2yl | 2,6-$F_2$-4-MeSO-Ph | COMe | |
| Ⅲ-873 | 3-Cl-pyridin-2yl | 2,6-$F_2$-4-MeSO-Ph | $SO_2Me$ | |
| Ⅲ-874 | 3-Cl-pyridin-2yl | 2,6-$F_2$-4-$MeSO_2$-Ph | Me | |
| Ⅲ-875 | 3-Cl-pyridin-2yl | 2,6-$F_2$-4-$MeSO_2$-Ph | COMe | |
| Ⅲ-876 | 3-Cl-pyridin-2yl | 2,6-$F_2$-4-$MeSO_2$-Ph | $SO_2Me$ | |
| Ⅲ-877 | 3-Cl-pyridin-2yl | 2-F-4-$CF_3CH_2S$-Ph | Me | |
| Ⅲ-878 | 3-Cl-pyridin-2yl | 2-F-4-$CF_3CH_2S$-Ph | COMe | |
| Ⅲ-879 | 3-Cl-pyridin-2yl | 2-F-4-$CF_3CH_2S$-Ph | $SO_2Me$ | |
| Ⅲ-880 | 3-Cl-pyridin-2yl | 2-F-4-$CF_3CH_2SO$-Ph | Me | |

| compound No. | $Q_1$ | $Q_2$ | R | m. p. (°C) |
|---|---|---|---|---|
| III-881 | 3-Cl-pyridin-2yl | 2-F-4-$CF_3CH_2SO$-Ph | COMe | |
| III-882 | 3-Cl-pyridin-2yl | 2-F-4-$CF_3CH_2SO$-Ph | SO2Me | |
| III-883 | 3-Cl-pyridin-2yl | 2-F-4-$CF_3CH_2SO_2$-Ph | Me | |
| III-884 | 3-Cl-pyridin-2yl | 2-F-4-$CF_3CH_2SO_2$-Ph | COMe | |
| III-885 | 3-Cl-pyridin-2yl | 2-F-4-$CF_3CH_2SO_2$-Ph | $SO_2Me$ | |
| III-886 | 3-Cl-pyridin-2yl | $CF_3CH_2$ | Me | |
| III-887 | 3-Cl-pyridin-2yl | $CF_3CH_2$ | COMe | |
| III-888 | 3-Cl-pyridin-2yl | $CF_3CH_2$ | $SO_2Me$ | |
| III-889 | 3-Cl-pyridin-2yl | 2-F-4-$CF_2HS$-Ph | Me | |
| III-890 | 3-Cl-pyridin-2yl | 2-F-4-$CF_2HS$-Ph | COMe | |
| III-891 | 3-Cl-pyridin-2yl | 2-F-4-$CF_2HS$-Ph | $SO_2Me$ | |
| III-892 | 3-Cl-pyridin-2yl | 2-F-4-$CF_2HSO$-Ph | Me | |
| III-893 | 3-Cl-pyridin-2yl | 2-F-4-$CF_2HSO$-Ph | COMe | |
| III-894 | 3-Cl-pyridin-2yl | 2-F-4-$CF_2HSO$-Ph | $SO_2Me$ | |
| III-895 | 3-Cl-pyridin-2yl | 2-F-4-$CF_2HSO_2$-Ph | Me | |
| III-896 | 3-Cl-pyridin-2yl | 2-F-4-$CF_2HSO_2$-Ph | COMe | |
| III-897 | 3-Cl-pyridin-2yl | 2-F-4-$CF_2HSO_2$-Ph | $SO_2Me$ | |
| III-898 | 3-Cl-pyridin-2yl | 2-F-4-$CF_3S$-Ph | Me | |
| III-899 | 3-Cl-pyridin-2yl | 2-F-4-$CF_3S$-Ph | COMe | |
| III-900 | 3-Cl-pyridin-2yl | 2-F-4-$CF_3S$-Ph | $SO_2Me$ | |
| III-901 | 3-Cl-pyridin-2yl | 2-F-4-$CF_3SO$-Ph | Me | |
| III-902 | 3-Cl-pyridin-2yl | 2-F-4-$CF_3SO$-Ph | COMe | |
| III-903 | 3-Cl-pyridin-2yl | 2-F-4-$CF_3SO$-Ph | $SO_2Me$ | |
| III-904 | 3-Cl-pyridin-2yl | 2-F-4-$CF_3SO_2$-Ph | Me | |
| III-905 | 3-Cl-pyridin-2yl | 2-F-4-$CF_3SO_2$-Ph | COMe | |
| III-906 | 3-Cl-pyridin-2yl | 2-F-4-$CF_3SO_2$-Ph | $SO_2Me$ | |
| III-907 | 3-Cl-pyridin-2yl | 2-F-4-$CH_3CH_2S$-Ph | Me | |
| III-908 | 3-Cl-pyridin-2yl | 2-F-4-$CH_3CH_2S$-Ph | COMe | |
| III-909 | 3-Cl-pyridin-2yl | 2-F-4-$CH_3CH_2S$-Ph | $SO_2Me$ | |
| III-910 | 3-Cl-pyridin-2yl | 2-F-4-$CH_3CH_2SO$-Ph | Me | |
| III-911 | 3-Cl-pyridin-2yl | 2-F-4-$CH_3CH_2SO$-Ph | COMe | |
| III-912 | 3-Cl-pyridin-2yl | 2-F-4-$CH_3CH_2SO$-Ph | $SO_2Me$ | |
| III-913 | 3-Cl-pyridin-2yl | 2-F-4-$CH_3CH_2SO_2$-Ph | Me | |
| III-914 | 3-Cl-pyridin-2yl | 2-F-4-$CH_3CH_2SO_2$-Ph | COMe | |
| III-915 | 3-Cl-pyridin-2yl | 2-F-4-$CH_3CH_2SO_2$-Ph | $SO_2Me$ | |
| III-916 | 3-Cl-pyridin-2yl | 2-F-4-(2-Cl-4-$CF_3$-pyridin-2yl-S)-Ph | Me | |
| III-917 | 3-Cl-pyridin-2yl | 2-F-4-(2-Cl-4-$CF_3$-pyridin-2yl-S)-Ph | COMe | |
| III-918 | 3-Cl-pyridin-2yl | 2-F-4-(2-Cl-4-$CF_3$-pyridin-2yl-S)-Ph | $SO_2Me$ | |
| III-919 | 3-Cl-pyridin-2yl | 2-F-4-(2-Cl-4-$CF_3$-pyridin-2yl-SO)-Ph | Me | |
| III-920 | 3-Cl-pyridin-2yl | 2-F-4-(2-Cl-4-$CF_3$-pyridin-2yl-SO)-Ph | COMe | |

| compound No. | $Q_1$ | $Q_2$ | R | m. p. (°C) |
|---|---|---|---|---|
| III-921 | 3-Cl-pyridin-2yl | 2-F-4-(2-Cl-4-$CF_3$-pyridin-2yl-SO)-Ph | $SO_2Me$ | |
| III-922 | 3-Cl-pyridin-2yl | 2-F-4-(2-Cl-4-$CF_3$-pyridin-2yl-$SO_2$)-Ph | Me | |
| III-923 | 3-Cl-pyridin-2yl | 2-F-4-(2-Cl-4-$CF_3$-pyridin-2yl-$SO_2$)-Ph | COMe | |
| III-924 | 3-Cl-pyridin-2yl | 2-F-4-(2-Cl-4-$CF_3$-pyridin-2yl-$SO_2$)-Ph | $SO_2Me$ | |
| III-925 | 3-Cl-pyridin-2yl | 2-Me-4-$CF_3$S-Ph | Me | |
| III-926 | 3-Cl-pyridin-2yl | 2-Me-4-$CF_3$S-Ph | COMe | |
| III-927 | 3-Cl-pyridin-2yl | 2-Me-4-$CF_3$S-Ph | $SO_2Me$ | |
| III-928 | 3-Cl-pyridin-2yl | 2-Me-4-$CF_3$SO-Ph | Me | |
| III-929 | 3-Cl-pyridin-2yl | 2-Me-4-$CF_3$SO-Ph | COMe | |
| III-930 | 3-Cl-pyridin-2yl | 2-Me-4-$CF_3$SO-Ph | $SO_2Me$ | |
| III-931 | 3-Cl-pyridin-2yl | 2-Me-4-$CF_3SO_2$-Ph | Me | |
| III-932 | 3-Cl-pyridin-2yl | 2-Me-4-$CF_3SO_2$-Ph | COMe | |
| III-933 | 3-Cl-pyridin-2yl | 2-Me-4-$CF_3SO_2$-Ph | $SO_2Me$ | |
| III-934 | 3-Cl-pyridin-2yl | 3-F-4-$CF_3$S-Ph | Me | |
| III-935 | 3-Cl-pyridin-2yl | 3-F-4-$CF_3$S-Ph | COMe | |
| III-936 | 3-Cl-pyridin-2yl | 3-F-4-$CF_3$S-Ph | $SO_2Me$ | |
| III-937 | 3-Cl-pyridin-2yl | 3-F-4-$CF_3$SO-Ph | Me | |
| III-938 | 3-Cl-pyridin-2yl | 3-F-4-$CF_3$SO-Ph | COMe | |
| III-939 | 3-Cl-pyridin-2yl | 3-F-4-$CF_3$SO-Ph | $SO_2Me$ | |
| III-940 | 3-Cl-pyridin-2yl | 3-F-4-$CF_3SO_2$-Ph | Me | |
| III-941 | 3-Cl-pyridin-2yl | 3-F-4-$CF_3SO_2$-Ph | COMe | |
| III-942 | 3-Cl-pyridin-2yl | 3-F-4-$CF_3SO_2$-Ph | $SO_2Me$ | |
| III-943 | 3-Cl-pyridin-2yl | 2,3-$Me_2$-4-$CF_3$S-Ph | Me | |
| III-944 | 3-Cl-pyridin-2yl | 2,3-$Me_2$-4-$CF_3$S-Ph | COMe | |
| III-945 | 3-Cl-pyridin-2yl | 2,3-$Me_2$-4-$CF_3$S-Ph | $SO_2Me$ | |
| III-946 | 3-Cl-pyridin-2yl | 2,3-$Me_2$-4-$CF_3$SO-Ph | Me | |
| III-947 | 3-Cl-pyridin-2yl | 2,3-$Me_2$-4-$CF_3$SO-Ph | COMe | |
| III-948 | 3-Cl-pyridin-2yl | 2,3-$Me_2$-4-$CF_3$SO-Ph | $SO_2Me$ | |
| III-949 | 3-Cl-pyridin-2yl | 2,3-$Me_2$-4-$CF_3SO_2$-Ph | Me | |
| III-950 | 3-Cl-pyridin-2yl | 2,3-$Me_2$-4-$CF_3SO_2$-Ph | COMe | |
| III-951 | 3-Cl-pyridin-2yl | 2,3-$Me_2$-4-$CF_3SO_2$-Ph | $SO_2Me$ | |
| III-952 | 3-Cl-pyridin-2yl | 5-Cl-2-Me-4-$CF_3$S-Ph | Me | |
| III-953 | 3-Cl-pyridin-2yl | 5-Cl-2-Me-4-$CF_3$S-Ph | COMe | |
| III-954 | 3-Cl-pyridin-2yl | 5-Cl-2-Me-4-$CF_3$S-Ph | $SO_2Me$ | |
| III-955 | 3-Cl-pyridin-2yl | 5-Cl-2-Me-4-$CF_3$SO-Ph | Me | |
| III-956 | 3-Cl-pyridin-2yl | 5-Cl-2-Me-4-$CF_3$SO-Ph | COMe | |
| III-957 | 3-Cl-pyridin-2yl | 5-Cl-2-Me-4-$CF_3$SO-Ph | $SO_2Me$ | |
| III-958 | 3-Cl-pyridin-2yl | 5-Cl-2-Me-4-$CF_3SO_2$-Ph | Me | |
| III-959 | 3-Cl-pyridin-2yl | 5-Cl-2-Me-4-$CF_3SO_2$-Ph | COMe | |
| III-960 | 3-Cl-pyridin-2yl | 5-Cl-2-Me-4-$CF_3SO_2$-Ph | $SO_2Me$ | |

| compound No. | $Q_1$ | $Q_2$ | R | m. p. (°C) |
|---|---|---|---|---|
| Ⅲ-961 | 3-Cl-pyridin-2yl | 2,6-$F_2$-4-$CF_3$S-Ph | Me | |
| Ⅲ-962 | 3-Cl-pyridin-2yl | 2,6-$F_2$-4-$CF_3$S-Ph | COMe | |
| Ⅲ-963 | 3-Cl-pyridin-2yl | 2,6-$F_2$-4-$CF_3$S-Ph | $SO_2$Me | |
| Ⅲ-964 | 3-Cl-pyridin-2yl | 2,6-$F_2$-4-$CF_3$SO-Ph | Me | |
| Ⅲ-965 | 3-Cl-pyridin-2yl | 2,6-$F_2$-4-$CF_3$SO-Ph | COMe | |
| Ⅲ-966 | 3-Cl-pyridin-2yl | 2,6-$F_2$-4-$CF_3$SO-Ph | $SO_2$Me | |
| Ⅲ-967 | 3-Cl-pyridin-2yl | 2,6-$F_2$-4-$CF_3SO_2$-Ph | Me | |
| Ⅲ-968 | 3-Cl-pyridin-2yl | 2,6-$F_2$-4-$CF_3SO_2$-Ph | COMe | |
| Ⅲ-969 | 3-Cl-pyridin-2yl | 2,6-$F_2$-4-$CF_3SO_2$-Ph | $SO_2$Me | |
| Ⅲ-970 | 3-Cl-pyridin-2yl | 2-F-4-$CF_3CF_2$S-Ph | Me | |
| Ⅲ-971 | 3-Cl-pyridin-2yl | 2-F-4-$CF_3CF_2$S-Ph | COMe | |
| Ⅲ-972 | 3-Cl-pyridin-2yl | 2-F-4-$CF_3CF_2$S-Ph | $SO_2$Me | |
| Ⅲ-973 | 3-Cl-pyridin-2yl | 2-F-4-$CF_3CF_2$SO-Ph | Me | |
| Ⅲ-974 | 3-Cl-pyridin-2yl | 2-F-4-$CF_3CF_2$SO-Ph | COMe | |
| Ⅲ-975 | 3-Cl-pyridin-2yl | 2-F-4-$CF_3CF_2$SO-Ph | $SO_2$Me | |
| Ⅲ-976 | 3-Cl-pyridin-2yl | 2-F-4-$CF_3CF_2SO_2$-Ph | Me | |
| Ⅲ-977 | 3-Cl-pyridin-2yl | 2-F-4-$CF_3CF_2SO_2$-Ph | COMe | |
| Ⅲ-978 | 3-Cl-pyridin-2yl | 2-F-4-$CF_3CF_2SO_2$-Ph | $SO_2$Me | |
| Ⅲ-979 | 3-Cl-pyridin-2yl | 2-F-4-$CF_3CF_2CF_2$S-Ph | Me | |
| Ⅲ-980 | 3-Cl-pyridin-2yl | 2-F-4-$CF_3CF_2CF_2$S-Ph | COMe | |
| Ⅲ-981 | 3-Cl-pyridin-2yl | 2-F-4-$CF_3CF_2CF_2$S-Ph | $SO_2$Me | |
| Ⅲ-982 | 3-Cl-pyridin-2yl | 2-F-4-$CF_3CF_2CF_2$SO-Ph | Me | |
| Ⅲ-983 | 3-Cl-pyridin-2yl | 2-F-4-$CF_3CF_2CF_2$SO-Ph | COMe | |
| Ⅲ-984 | 3-Cl-pyridin-2yl | 2-F-4-$CF_3CF_2CF_2$SO-Ph | $SO_2$Me | |
| Ⅲ-985 | 3-Cl-pyridin-2yl | 2-F-4-$CF_3CF_2CF_2SO_2$-Ph | Me | |
| Ⅲ-986 | 3-Cl-pyridin-2yl | 2-F-4-$CF_3CF_2CF_2SO_2$-Ph | COMe | |
| Ⅲ-987 | 3-Cl-pyridin-2yl | 2-F-4-$CF_3CF_2CF_2SO_2$-Ph | $SO_2$Me | |
| Ⅲ-988 | 3-Cl-pyridin-2yl | 2-F-4-$(CF_3)_2$CFS-Ph | Me | |
| Ⅲ-989 | 3-Cl-pyridin-2yl | 2-F-4-$(CF_3)_2$CFS-Ph | COMe | |
| Ⅲ-990 | 3-Cl-pyridin-2yl | 2-F-4-$(CF_3)_2$CFS-Ph | $SO_2$Me | |
| Ⅲ-991 | 3-Cl-pyridin-2yl | 2-F-4-$(CF_3)_2$CFSO-Ph | Me | |
| Ⅲ-992 | 3-Cl-pyridin-2yl | 2-F-4-$(CF_3)_2$CFSO-Ph | COMe | |
| Ⅲ-993 | 3-Cl-pyridin-2yl | 2-F-4-$(CF_3)_2$CFSO-Ph | $SO_2$Me | |
| Ⅲ-994 | 3-Cl-pyridin-2yl | 2-F-4-$(CF_3)_2CFSO_2$-Ph | Me | |
| Ⅲ-995 | 3-Cl-pyridin-2yl | 2-F-4-$(CF_3)_2CFSO_2$-Ph | COMe | |
| Ⅲ-996 | 3-Cl-pyridin-2yl | 2-F-4-$(CF_3)_2CFSO_2$-Ph | $SO_2$Me | |

Table 4

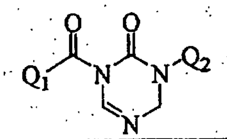

(Id)

| compound No. | $Q_1$ | $Q_2$ | m. p. (°C) |
|---|---|---|---|
| IV-1 | 2,6-$F_2$-Ph | 4-Cl-Ph | |
| IV-2 | 2,6-$F_2$-Ph | 3,5-$Cl_2$-4-(2-Cl-4-$CF_3$-pyridin-2yl-O)-Ph | |
| IV-3 | 2,6-$F_2$-Ph | 2-F-4-(2-Cl-4-$CF_3$-Ph-O)-Ph | |
| IV-4 | 2,6-$F_2$-Ph | 3,5-$Cl_2$-2,4-$F_2$-Ph | amorphous (1) |
| IV-5 | 2,6-$F_2$-Ph | 3,5-$Cl_2$-4-$CF_2HCF_2O$-Ph | |
| IV-6 | 2,6-$F_2$-Ph | 2,5-$Cl_2$-4-$CF_3CFHCF_2O$-Ph | |
| IV-7 | 2,6-$F_2$-Ph | 2-F-4-$CFClHCF_2S$-Ph | |
| IV-8 | 2,6-$F_2$-Ph | 2-F-4-$CFClHCF_2SO$-Ph | |
| IV-9 | 2,6-$F_2$-Ph | 2-F-4-$CFClHCF_2SO_2$-Ph | |
| IV-10 | 2,6-$F_2$-Ph | 4-$CFClHCF_2S$-Ph | |
| IV-11 | 2,6-$F_2$-Ph | 4-$CFClHCF_2SO$-Ph | |
| IV-12 | 2,6-$F_2$-Ph | 4-$CFClHCF_2SO_2$-Ph | |
| IV-13 | 2,6-$F_2$-Ph | 2-Cl-4-$CFClHCF_2S$-Ph | |
| IV-14 | 2,6-$F_2$-Ph | 2-Cl-4-$CFClHCF_2SO$-Ph | |
| IV-15 | 2,6-$F_2$-Ph | 2-Cl-4-$CFClHCF_2SO_2$-Ph | |
| IV-16 | 2,6-$F_2$-Ph | 2-Cl-4-$CF_2HCF_2S$-Ph | |
| IV-17 | 2,6-$F_2$-Ph | 2-Cl-4-$CF_2HCF_2SO$-Ph | |
| IV-18 | 2,6-$F_2$-Ph | 2-Cl-4-$CF_2HCF_2SO_2$-Ph | |
| IV-19 | 2,6-$F_2$-Ph | 4-$CF_2HCF_2S$-Ph | |
| IV-20 | 2,6-$F_2$-Ph | 4-$CF_2HCF_2SO$-Ph | |
| IV-21 | 2,6-$F_2$-Ph | 4-$CF_2HCF_2SO_2$-Ph | |
| IV-22 | 2,6-$F_2$-Ph | 2-F-4-$CF_2HCF_2S$-Ph | |
| IV-23 | 2,6-$F_2$-Ph | 2-F-4-$CF_2HCF_2SO$-Ph | |
| IV-24 | 2,6-$F_2$-Ph | 2-F-4-$CF_2HCF_2SO_2$-Ph | |
| IV-25 | 2,6-$F_2$-Ph | 4-$CF_3S$-Ph | |
| IV-26 | 2,6-$F_2$-Ph | 4-$CF_3SO$-Ph | |
| IV-27 | 2,6-$F_2$-Ph | 4-$CF_3SO_2$-Ph | |
| IV-28 | 2,6-$F_2$-Ph | 2-F-4-MeS-Ph | |
| IV-29 | 2,6-$F_2$-Ph | 2-F-4-MeSO-Ph | |
| IV-30 | 2,6-$F_2$-Ph | 2-F-4-$MeSO_2$-Ph | |
| IV-31 | 2,6-$F_2$-Ph | 2,6-$F_2$-4-MeS-Ph | |
| IV-32 | 2,6-$F_2$-Ph | 2,6-$F_2$-4-MeSO-Ph | |
| IV-33 | 2,6-$F_2$-Ph | 2,6-$F_2$-4-$MeSO_2$-Ph | |
| IV-34 | 2,6-$F_2$-Ph | 2-F-4-$CF_3CH_2S$-Ph | |
| IV-35 | 2,6-$F_2$-Ph | 2-F-4-$CF_3CH_2SO$-Ph | |
| IV-36 | 2,6-$F_2$-Ph | 2-F-4-$CF_3CH_2SO_2$-Ph | |
| IV-37 | 2,6-$F_2$-Ph | 2-F-4-$CCl_2$=$CHCH_2S$-Ph | |
| IV-38 | 2,6-$F_2$-Ph | 2-F-4-$CCl_2$=$CHCH_2SO$-Ph | |
| IV-39 | 2,6-$F_2$-Ph | 2-F-4-$CCl_2$=$CHCH_2SO_2$-Ph | |
| IV-40 | 2,6-$F_2$-Ph | $CF_3CH_2$ | |

(1) $H^1$-NMR ($CDCl_3$)δppm : 5.20 ( 2H, d J=2.1Hz, $NCH_2N$), 6.89-7.00 ( 2H, m, Ph), 7.26-7.48 ( 2H, m, Ph), 8.38 ( 1H, t J=2.1Hz, NCHN).

| compound No. | $Q_1$ | $Q_2$ | m. p. (°C) |
|---|---|---|---|
| IV-41 | 2,6-$F_2$-Ph | 2-F-4-$CF_2HS$-Ph | |
| IV-42 | 2,6-$F_2$-Ph | 2-F-4-$CF_2HSO$-Ph | |
| IV-43 | 2,6-$F_2$-Ph | 2-F-4-$CF_2HSO_2$-Ph | |
| IV-44 | 2,6-$F_2$-Ph | 2-F-4-$CF_3S$-Ph | |
| IV-45 | 2,6-$F_2$-Ph | 2-F-4-$CF_3SO$-Ph | |
| IV-46 | 2,6-$F_2$-Ph | 2-F-4-$CF_3SO_2$-Ph | |
| IV-47 | 2,6-$F_2$-Ph | 2-F-4-$CH_3CH_2S$-Ph | |
| IV-48 | 2,6-$F_2$-Ph | 2-F-4-$CH_3CH_2SO$-Ph | |
| IV-49 | 2,6-$F_2$-Ph | 2-F-4-$CH_3CH_2SO_2$-Ph | |
| IV-50 | 2,6-$F_2$-Ph | 2-F-4-$CH_3CH_2CH_2S$-Ph | |
| IV-51 | 2,6-$F_2$-Ph | 2-F-4-$CH_3CH_2CH_2SO$-Ph | |
| IV-52 | 2,6-$F_2$-Ph | 2-F-4-$CH_3CH_2CH_2SO_2$-Ph | |
| IV-53 | 2,6-$F_2$-Ph | 2-F-4-i-PrS-Ph | |
| IV-54 | 2,6-$F_2$-Ph | 2-F-4-i-PrSO-Ph | |
| IV-55 | 2,6-$F_2$-Ph | 2-F-4-i-$PrSO_2$-Ph | |
| IV-56 | 2,6-$F_2$-Ph | 2-F-4-t-BuS-Ph | |
| IV-57 | 2,6-$F_2$-Ph | 2-F-4-t-BuSO-Ph | |
| IV-58 | 2,6-$F_2$-Ph | 2-F-4-t-$BuSO_2$-Ph | |
| IV-59 | 2,6-$F_2$-Ph | 2-F-4-$CH_2$=$CHCH_2S$-Ph | |
| IV-60 | 2,6-$F_2$-Ph | 2-F-4-$CH_2$=$CHCH_2SO$-Ph | |
| IV-61 | 2,6-$F_2$-Ph | 2-F-4-$CH_2$=$CHCH_2SO_2$-Ph | |
| IV-62 | 2,6-$F_2$-Ph | 2-F-4-CH≡$CCH_2S$-Ph | |
| IV-63 | 2,6-$F_2$-Ph | 2-F-4-CH≡$CCH_2SO$-Ph | |
| IV-64 | 2,6-$F_2$-Ph | 2-F-4-CH≡$CCH_2SO_2$-Ph | |
| IV-65 | 2,6-$F_2$-Ph | 2-F-4-(2-Cl-4-$CF_3$-pyridin-2yl-S)-Ph | |
| IV-66 | 2,6-$F_2$-Ph | 2-F-4-(2-Cl-4-$CF_3$-pyridin-2yl-SO)-Ph | |
| IV-67 | 2,6-$F_2$-Ph | 2-F-4-(2-Cl-4-$CF_3$-pyridin-2yl-$SO_2$)-Ph | |
| IV-68 | 2,6-$F_2$-Ph | 2-Me-4-$CF_3S$-Ph | |
| IV-69 | 2,6-$F_2$-Ph | 2-Me-4-$CF_3SO$-Ph | |
| IV-70 | 2,6-$F_2$-Ph | 2-Me-4-$CF_3SO_2$-Ph | |
| IV-71 | 2,6-$F_2$-Ph | 3-Me-4-$CF_3S$-Ph | |
| IV-72 | 2,6-$F_2$-Ph | 3-Me-4-$CF_3SO$-Ph | |
| IV-73 | 2,6-$F_2$-Ph | 3-Me-4-$CF_3SO_2$-Ph | |
| IV-74 | 2,6-$F_2$-Ph | 2-Cl-4-$CF_3S$-Ph | |
| IV-75 | 2,6-$F_2$-Ph | 2-Cl-4-$CF_3SO$-Ph | |
| IV-76 | 2,6-$F_2$-Ph | 2-Cl-4-$CF_3SO_2$-Ph | |
| IV-77 | 2,6-$F_2$-Ph | 3-Cl-4-$CF_3S$-Ph | |
| IV-78 | 2,6-$F_2$-Ph | 3-Cl-4-$CF_3SO$-Ph | |
| IV-79 | 2,6-$F_2$-Ph | 3-Cl-4-$CF_3SO_2$-Ph | |
| IV-80 | 2,6-$F_2$-Ph | 3-F-4-$CF_3S$-Ph | |

| compound No. | $Q_1$ | $Q_2$ | m. p. (℃) |
|---|---|---|---|
| IV-81 | 2,6-$F_2$-Ph | 3-F-4-$CF_3SO$-Ph | |
| IV-82 | 2,6-$F_2$-Ph | 3-F-4-$CF_3SO_2$-Ph | |
| IV-83 | 2,6-$F_2$-Ph | 2,3-$Me_2$-4-$CF_3S$-Ph | |
| IV-84 | 2,6-$F_2$-Ph | 2,3-$Me_2$-4-$CF_3SO$-Ph | |
| IV-85 | 2,6-$F_2$-Ph | 2,3-$Me_2$-4-$CF_3SO_2$-Ph | |
| IV-86 | 2,6-$F_2$-Ph | 2,5-$Me_2$-4-$CF_3S$-Ph | |
| IV-87 | 2,6-$F_2$-Ph | 2,5-$Me_2$-4-$CF_3SO$-Ph | |
| IV-88 | 2,6-$F_2$-Ph | 2,5-$Me_2$-4-$CF_3SO_2$-Ph | |
| IV-89 | 2,6-$F_2$-Ph | 5-Cl-2-Me-4-$CF_3S$-Ph | |
| IV-90 | 2,6-$F_2$-Ph | 5-Cl-2-Me-4-$CF_3SO$-Ph | |
| IV-91 | 2,6-$F_2$-Ph | 5-Cl-2-Me-4-$CF_3SO_2$-Ph | |
| IV-92 | 2,6-$F_2$-Ph | 2,3-$Cl_2$-4-$CF_3S$-Ph | |
| IV-93 | 2,6-$F_2$-Ph | 2,3-$Cl_2$-4-$CF_3SO$-Ph | |
| IV-94 | 2,6-$F_2$-Ph | 2,3-$Cl_2$-4-$CF_3SO_2$-Ph | |
| IV-95 | 2,6-$F_2$-Ph | 3-Cl-2-F-4-$CF_3S$-Ph | |
| IV-96 | 2,6-$F_2$-Ph | 3-Cl-2-F-4-$CF_3SO$-Ph | |
| IV-97 | 2,6-$F_2$-Ph | 3-Cl-2-F-4-$CF_3SO_2$-Ph | |
| IV-98 | 2,6-$F_2$-Ph | 5-Cl-2-F-4-$CF_3S$-Ph | |
| IV-99 | 2,6-$F_2$-Ph | 5-Cl-2-F-4-$CF_3SO$-Ph | |
| IV-100 | 2,6-$F_2$-Ph | 5-Cl-2-F-4-$CF_3SO_2$-Ph | |
| IV-101 | 2,6-$F_2$-Ph | 2,6-$F_2$-4-$CF_3S$-Ph | |
| IV-102 | 2,6-$F_2$-Ph | 2,6-$F_2$-4-$CF_3SO$-Ph | |
| IV-103 | 2,6-$F_2$-Ph | 2,6-$F_2$-4-$CF_3SO_2$-Ph | |
| IV-104 | 2,6-$F_2$-Ph | 2,3-$F_2$-4-$CF_3S$-Ph | |
| IV-105 | 2,6-$F_2$-Ph | 2,3-$F_2$-4-$CF_3SO$-Ph | |
| IV-106 | 2,6-$F_2$-Ph | 2,3-$F_2$-4-$CF_3SO_2$-Ph | |
| IV-107 | 2,6-$F_2$-Ph | 2,5-$F_2$-4-$CF_3S$-Ph | |
| IV-108 | 2,6-$F_2$-Ph | 2,5-$F_2$-4-$CF_3SO$-Ph | |
| IV-109 | 2,6-$F_2$-Ph | 2,5-$F_2$-4-$CF_3SO_2$-Ph | |
| IV-110 | 2,6-$F_2$-Ph | 3,5-$F_2$-4-$CF_3S$-Ph | |
| IV-111 | 2,6-$F_2$-Ph | 3,5-$F_2$-4-$CF_3SO$-Ph | |
| IV-112 | 2,6-$F_2$-Ph | 3,5-$F_2$-4-$CF_3SO_2$-Ph | |
| IV-113 | 2,6-$F_2$-Ph | 2,3,6-$F_3$-4-$CF_3S$-Ph | |
| IV-114 | 2,6-$F_2$-Ph | 2,3,6-$F_3$-4-$CF_3SO$-Ph | |
| IV-115 | 2,6-$F_2$-Ph | 2,3,6-$F_3$-4-$CF_3SO_2$-Ph | |
| IV-116 | 2,6-$F_2$-Ph | 2,3,5,6-$F_4$-4-$CF_3S$-Ph | |
| IV-117 | 2,6-$F_2$-Ph | 2,3,5,6-$F_4$-4-$CF_3SO$-Ph | |
| IV-118 | 2,6-$F_2$-Ph | 2,3,5,6-$F_4$-4-$CF_3SO_2$-Ph | |
| IV-119 | 2,6-$F_2$-Ph | 2-F-4-$CF_3CF_2S$-Ph | |
| IV-120 | 2,6-$F_2$-Ph | 2-F-4-$CF_3CF_2SO$-Ph | |

| compound No. | $Q_1$ | $Q_2$ | m. p. (°C) |
|---|---|---|---|
| IV-121 | 2,6-$F_2$-Ph | 2-F-4-$CF_3CF_2SO_2$-Ph | |
| IV-122 | 2,6-$F_2$-Ph | 2-Cl-4-$CF_3CF_2S$-Ph | |
| IV-123 | 2,6-$F_2$-Ph | 2-Cl-4-$CF_3CF_2SO$-Ph | |
| IV-124 | 2,6-$F_2$-Ph | 2-Cl-4-$CF_3CF_2SO_2$-Ph | |
| IV-125 | 2,6-$F_2$-Ph | 2-Me-4-$CF_3CF_2S$-Ph | |
| IV-126 | 2,6-$F_2$-Ph | 2-Me-4-$CF_3CF_2SO$-Ph | |
| IV-127 | 2,6-$F_2$-Ph | 2-Me-4-$CF_3CF_2SO_2$-Ph | |
| IV-128 | 2,6-$F_2$-Ph | 5-Cl-2-Me-4-$CF_3CF_2S$-Ph | |
| IV-129 | 2,6-$F_2$-Ph | 5-Cl-2-Me-4-$CF_3CF_2SO$-Ph | |
| IV-130 | 2,6-$F_2$-Ph | 5-Cl-2-Me-4-$CF_3CF_2SO_2$-Ph | |
| IV-131 | 2,6-$F_2$-Ph | 2,3-$Me_2$-4-$CF_3CF_2S$-Ph | |
| IV-132 | 2,6-$F_2$-Ph | 2,3-$Me_2$-4-$CF_3CF_2SO$-Ph | |
| IV-133 | 2,6-$F_2$-Ph | 2,3-$Me_2$-4-$CF_3CF_2SO_2$-Ph | |
| IV-134 | 2,6-$F_2$-Ph | 2-F-4-$CF_3CF_2CF_2S$-Ph | |
| IV-135 | 2,6-$F_2$-Ph | 2-F-4-$CF_3CF_2CF_2SO$-Ph | |
| IV-136 | 2,6-$F_2$-Ph | 2-F-4-$CF_3CF_2CF_2SO_2$-Ph | |
| IV-137 | 2,6-$F_2$-Ph | 2-Cl-4-$CF_3CF_2CF_2S$-Ph | |
| IV-138 | 2,6-$F_2$-Ph | 2-Cl-4-$CF_3CF_2CF_2SO$-Ph | |
| IV-139 | 2,6-$F_2$-Ph | 2-Cl-4-$CF_3CF_2CF_2SO_2$-Ph | |
| IV-140 | 2,6-$F_2$-Ph | 2-Me-4-$CF_3CF_2CF_2S$-Ph | |
| IV-141 | 2,6-$F_2$-Ph | 2-Me-4-$CF_3CF_2CF_2SO$-Ph | |
| IV-142 | 2,6-$F_2$-Ph | 2-Me-4-$CF_3CF_2CF_2SO_2$-Ph | |
| IV-143 | 2,6-$F_2$-Ph | 5-Cl-2-Me-4-$CF_3CF_2CF_2S$-Ph | |
| IV-144 | 2,6-$F_2$-Ph | 5-Cl-2-Me-4-$CF_3CF_2CF_2SO$-Ph | |
| IV-145 | 2,6-$F_2$-Ph | 5-Cl-2-Me-4-$CF_3CF_2CF_2SO_2$-Ph | |
| IV-146 | 2,6-$F_2$-Ph | 2,3-$Me_2$-4-$CF_3CF_2CF_2S$-Ph | |
| IV-147 | 2,6-$F_2$-Ph | 2,3-$Me_2$-4-$CF_3CF_2CF_2SO$-Ph | |
| IV-148 | 2,6-$F_2$-Ph | 2,3-$Me_2$-4-$CF_3CF_2CF_2SO_2$-Ph | |
| IV-149 | 2,6-$F_2$-Ph | 2-F-4-$(CF_3)_2CFS$-Ph | |
| IV-150 | 2,6-$F_2$-Ph | 2-F-4-$(CF_3)_2CFSO$-Ph | |
| IV-151 | 2,6-$F_2$-Ph | 2-F-4-$(CF_3)_2CFSO_2$-Ph | |
| IV-152 | 2,6-$F_2$-Ph | 2-Me-4-$(CF_3)_2CFS$-Ph | |
| IV-153 | 2,6-$F_2$-Ph | 2-Me-4-$(CF_3)_2CFSO$-Ph | |
| IV-154 | 2,6-$F_2$-Ph | 2-Me-4-$(CF_3)_2CFSO_2$-Ph | |
| IV-155 | 2,6-$F_2$-Ph | 2-Cl-4-$(CF_3)_2CFS$-Ph | |
| IV-156 | 2,6-$F_2$-Ph | 2-Cl-4-$(CF_3)_2CFSO$-Ph | |
| IV-157 | 2,6-$F_2$-Ph | 2-Cl-4-$(CF_3)_2CFSO_2$-Ph | |
| IV-158 | 2,6-$F_2$-Ph | 5-Cl-2-Me-4-$(CF_3)_2CFS$-Ph | |
| IV-159 | 2,6-$F_2$-Ph | 5-Cl-2-Me-4-$(CF_3)_2CFSO$-Ph | |
| IV-160 | 2,6-$F_2$-Ph | 5-Cl-2-Me-4-$(CF_3)_2CFSO_2$-Ph | |

| compound No. | $Q_1$ | $Q_2$ | m. p. (°C) |
|---|---|---|---|
| IV-161 | 2,6-$F_2$-Ph | 2,3-$Me_2$-4-$(CF_3)_2CFS$-Ph | |
| IV-162 | 2,6-$F_2$-Ph | 2,3-$Me_2$-4-$(CF_3)_2CFSO$-Ph | |
| IV-163 | 2,6-$F_2$-Ph | 2,3-$Me_2$-4-$(CF_3)_2CFSO_2$-Ph | |
| IV-164 | 2,6-$F_2$-Ph | 2-F-4-MeO-Ph | |
| IV-165 | 2,6-$F_2$-Ph | 2-Me-4-MeO-Ph | |
| IV-166 | 2,6-$F_2$-Ph | 2-F-4-CN-Ph | |
| IV-167 | 2,6-$F_2$-Ph | 2-F-4-COOMe-Ph | |
| IV-168 | 2,6-$F_2$-Ph | 2-F-4-COOEt-Ph | |
| IV-169 | 2-Cl-6-F-Ph | 4-Cl-Ph | |
| IV-170 | 2-Cl-6-F-Ph | 3,5-$Cl_2$-4-(2-Cl-4-$CF_3$-pyridin-2yl-O)-Ph | |
| IV-171 | 2-Cl-6-F-Ph | 2-F-4-(2-Cl-4-$CF_3$-Ph-O)-Ph | |
| IV-172 | 2-Cl-6-F-Ph | 3,5-$Cl_2$-2,4-$F_2$-Ph | |
| IV-173 | 2-Cl-6-F-Ph | 3,5-$Cl_2$-4-$CF_2HCF_2O$-Ph | |
| IV-174 | 2-Cl-6-F-Ph | 2,5-$Cl_2$-4-$CF_3CFHCF_2O$-Ph | |
| IV-175 | 2-Cl-6-F-Ph | 2-F-4-$CFClHCF_2S$-Ph | |
| IV-176 | 2-Cl-6-F-Ph | 2-F-4-$CFClHCF_2SO$-Ph | |
| IV-177 | 2-Cl-6-F-Ph | 2-F-4-$CFClHCF_2SO_2$-Ph | |
| IV-178 | 2-Cl-6-F-Ph | 4-$CFClHCF_2S$-Ph | |
| IV-179 | 2-Cl-6-F-Ph | 4-$CFClHCF_2SO$-Ph | |
| IV-180 | 2-Cl-6-F-Ph | 4-$CFClHCF_2SO_2$-Ph | |
| IV-181 | 2-Cl-6-F-Ph | 2-Cl-4-$CFClHCF_2S$-Ph | |
| IV-182 | 2-Cl-6-F-Ph | 2-Cl-4-$CFClHCF_2SO$-Ph | |
| IV-183 | 2-Cl-6-F-Ph | 2-Cl-4-$CFClHCF_2SO_2$-Ph | |
| IV-184 | 2-Cl-6-F-Ph | 2-Cl-4-$CF_2HCF_2S$-Ph | |
| IV-185 | 2-Cl-6-F-Ph | 2-Cl-4-$CF_2HCF_2SO$-Ph | |
| IV-186 | 2-Cl-6-F-Ph | 2-Cl-4-$CF_2HCF_2SO_2$-Ph | |
| IV-187 | 2-Cl-6-F-Ph | 4-$CF_2HCF_2S$-Ph | |
| IV-188 | 2-Cl-6-F-Ph | 4-$CF_2HCF_2SO$-Ph | |
| IV-189 | 2-Cl-6-F-Ph | 4-$CF_2HCF_2SO_2$-Ph | |
| IV-190 | 2-Cl-6-F-Ph | 2-F-4-$CF_2HCF_2S$-Ph | |
| IV-191 | 2-Cl-6-F-Ph | 2-F-4-$CF_2HCF_2SO$-Ph | |
| IV-192 | 2-Cl-6-F-Ph | 2-F-4-$CF_2HCF_2SO_2$-Ph | |
| IV-193 | 2-Cl-6-F-Ph | 4-$CF_3S$-Ph | |
| IV-194 | 2-Cl-6-F-Ph | 4-$CF_3SO$-Ph | |
| IV-195 | 2-Cl-6-F-Ph | 4-$CF_3SO_2$-Ph | |
| IV-196 | 2-Cl-6-F-Ph | 2-F-4-MeS-Ph | |
| IV-197 | 2-Cl-6-F-Ph | 2-F-4-MeSO-Ph | |
| IV-198 | 2-Cl-6-F-Ph | 2-F-4-$MeSO_2$-Ph | |
| IV-199 | 2-Cl-6-F-Ph | 2,6-$F_2$-4-MeS-Ph | |
| IV-200 | 2-Cl-6-F-Ph | 2,6-$F_2$-4-MeSO-Ph | |

| compound No. | $Q_1$ | $Q_2$ | m. p. (°C) |
|---|---|---|---|
| IV-201 | 2-Cl-6-F-Ph | 2,6-$F_2$-4-$MeSO_2$-Ph | |
| IV-202 | 2-Cl-6-F-Ph | 2-F-4-$CF_3CH_2S$-Ph | |
| IV-203 | 2-Cl-6-F-Ph | 2-F-4-$CF_3CH_2SO$-Ph | |
| IV-204 | 2-Cl-6-F-Ph | 2-F-4-$CF_3CH_2SO_2$-Ph | |
| IV-205 | 2-Cl-6-F-Ph | 2-F-4-$CCl_2$=$CHCH_2S$-Ph | |
| IV-206 | 2-Cl-6-F-Ph | 2-F-4-$CCl_2$=$CHCH_2SO$-Ph | |
| IV-207 | 2-Cl-6-F-Ph | 2-F-4-$CCl_2$=$CHCH_2SO_2$-Ph | |
| IV-208 | 2-Cl-6-F-Ph | $CF_3CH_2$ | |
| IV-209 | 2-Cl-6-F-Ph | 2-F-4-$CF_2HS$-Ph | |
| IV-210 | 2-Cl-6-F-Ph | 2-F-4-$CF_2HSO$-Ph | |
| IV-211 | 2-Cl-6-F-Ph | 2-F-4-$CF_2HSO_2$-Ph | |
| IV-212 | 2-Cl-6-F-Ph | 2-F-4-$CF_3S$-Ph | |
| IV-213 | 2-Cl-6-F-Ph | 2-F-4-$CF_3SO$-Ph | |
| IV-214 | 2-Cl-6-F-Ph | 2-F-4-$CF_3SO_2$-Ph | |
| IV-215 | 2-Cl-6-F-Ph | 2-F-4-$CH_3CH_2S$-Ph | |
| IV-216 | 2-Cl-6-F-Ph | 2-F-4-$CH_3CH_2SO$-Ph | |
| IV-217 | 2-Cl-6-F-Ph | 2-F-4-$CH_3CH_2SO_2$-Ph | |
| IV-218 | 2-Cl-6-F-Ph | 2-F-4-$CH_3CH_2CH_2S$-Ph | |
| IV-219 | 2-Cl-6-F-Ph | 2-F-4-$CH_3CH_2CH_2SO$-Ph | |
| IV-220 | 2-Cl-6-F-Ph | 2-F-4-$CH_3CH_2CH_2SO_2$-Ph | |
| IV-221 | 2-Cl-6-F-Ph | 2-F-4-i-PrS-Ph | |
| IV-222 | 2-Cl-6-F-Ph | 2-F-4-i-PrSO-Ph | |
| IV-223 | 2-Cl-6-F-Ph | 2-F-4-i-$PrSO_2$-Ph | |
| IV-224 | 2-Cl-6-F-Ph | 2-F-4-t-BuS-Ph | |
| IV-225 | 2-Cl-6-F-Ph | 2-F-4-t-BuSO-Ph | |
| IV-226 | 2-Cl-6-F-Ph | 2-F-4-t-$BuSO_2$-Ph | |
| IV-227 | 2-Cl-6-F-Ph | 2-F-4-$CH_2$=$CHCH_2S$-Ph | |
| IV-228 | 2-Cl-6-F-Ph | 2-F-4-$CH_2$=$CHCH_2SO$-Ph | |
| IV-229 | 2-Cl-6-F-Ph | 2-F-4-$CH_2$=$CHCH_2SO_2$-Ph | |
| IV-230 | 2-Cl-6-F-Ph | 2-F-4-CH≡$CCH_2S$-Ph | |
| IV-231 | 2-Cl-6-F-Ph | 2-F-4-CH≡$CCH_2SO$-Ph | |
| IV-232 | 2-Cl-6-F-Ph | 2-F-4-CH≡$CCH_2SO_2$-Ph | |
| IV-233 | 2-Cl-6-F-Ph | 2-F-4-(2-Cl-4-$CF_3$-pyridin-2yl-S)-Ph | |
| IV-234 | 2-Cl-6-F-Ph | 2-F-4-(2-Cl-4-$CF_3$-pyridin-2yl-SO)-Ph | |
| IV-235 | 2-Cl-6-F-Ph | 2-F-4-(2-Cl-4-$CF_3$-pyridin-2yl-$SO_2$)-Ph | |
| IV-236 | 2-Cl-6-F-Ph | 2-Me-4-$CF_3S$-Ph | |
| IV-237 | 2-Cl-6-F-Ph | 2-Me-4-$CF_3SO$-Ph | |
| IV-238 | 2-Cl-6-F-Ph | 2-Me-4-$CF_3SO_2$-Ph | |
| IV-239 | 2-Cl-6-F-Ph | 3-Me-4-$CF_3S$-Ph | |
| IV-240 | 2-Cl-6-F-Ph | 3-Me-4-$CF_3SO$-Ph | |

| compound No. | $Q_1$ | $Q_2$ | m. p. (℃) |
|---|---|---|---|
| IV-241 | 2-Cl-6-F-Ph | 3-Me-4-$CF_3SO_2$-Ph | |
| IV-242 | 2-Cl-6-F-Ph | 2-Cl-4-$CF_3S$-Ph | |
| IV-243 | 2-Cl-6-F-Ph | 2-Cl-4-$CF_3SO$-Ph | |
| IV-244 | 2-Cl-6-F-Ph | 2-Cl-4-$CF_3SO_2$-Ph | |
| IV-245 | 2-Cl-6-F-Ph | 3-Cl-4-$CF_3S$-Ph | |
| IV-246 | 2-Cl-6-F-Ph | 3-Cl-4-$CF_3SO$-Ph | |
| IV-247 | 2-Cl-6-F-Ph | 3-Cl-4-$CF_3SO_2$-Ph | |
| IV-248 | 2-Cl-6-F-Ph | 3-F-4-$CF_3S$-Ph | |
| IV-249 | 2-Cl-6-F-Ph | 3-F-4-$CF_3SO$-Ph | |
| IV-250 | 2-Cl-6-F-Ph | 3-F-4-$CF_3SO_2$-Ph | |
| IV-251 | 2-Cl-6-F-Ph | 2,3-$Me_2$-4-$CF_3S$-Ph | |
| IV-252 | 2-Cl-6-F-Ph | 2,3-$Me_2$-4-$CF_3SO$-Ph | |
| IV-253 | 2-Cl-6-F-Ph | 2,3-$Me_2$-4-$CF_3SO_2$-Ph | |
| IV-254 | 2-Cl-6-F-Ph | 2,5-$Me_2$-4-$CF_3S$-Ph | |
| IV-255 | 2-Cl-6-F-Ph | 2,5-$Me_2$-4-$CF_3SO$-Ph | |
| IV-256 | 2-Cl-6-F-Ph | 2,5-$Me_2$-4-$CF_3SO_2$-Ph | |
| IV-257 | 2-Cl-6-F-Ph | 5-Cl-2-Me-4-$CF_3S$-Ph | |
| IV-258 | 2-Cl-6-F-Ph | 5-Cl-2-Me-4-$CF_3SO$-Ph | |
| IV-259 | 2-Cl-6-F-Ph | 5-Cl-2-Me-4-$CF_3SO_2$-Ph | |
| IV-260 | 2-Cl-6-F-Ph | 2,3-$Cl_2$-4-$CF_3S$-Ph | |
| IV-261 | 2-Cl-6-F-Ph | 2,3-$Cl_2$-4-$CF_3SO$-Ph | |
| IV-262 | 2-Cl-6-F-Ph | 2,3-$Cl_2$-4-$CF_3SO_2$-Ph | |
| IV-263 | 2-Cl-6-F-Ph | 3-Cl-2-F-4-$CF_3S$-Ph | |
| IV-264 | 2-Cl-6-F-Ph | 3-Cl-2-F-4-$CF_3SO$-Ph | |
| IV-265 | 2-Cl-6-F-Ph | 3-Cl-2-F-4-$CF_3SO_2$-Ph | |
| IV-266 | 2-Cl-6-F-Ph | 5-Cl-2-F-4-$CF_3S$-Ph | |
| IV-267 | 2-Cl-6-F-Ph | 5-Cl-2-F-4-$CF_3SO$-Ph | |
| IV-268 | 2-Cl-6-F-Ph | 5-Cl-2-F-4-$CF_3SO_2$-Ph | |
| IV-269 | 2-Cl-6-F-Ph | 2,6-$F_2$-4-$CF_3S$-Ph | |
| IV-270 | 2-Cl-6-F-Ph | 2,6-$F_2$-4-$CF_3SO$-Ph | |
| IV-271 | 2-Cl-6-F-Ph | 2,6-$F_2$-4-$CF_3SO_2$-Ph | |
| IV-272 | 2-Cl-6-F-Ph | 2,3-$F_2$-4-$CF_3S$-Ph | |
| IV-273 | 2-Cl-6-F-Ph | 2,3-$F_2$-4-$CF_3SO$-Ph | |
| IV-274 | 2-Cl-6-F-Ph | 2,3-$F_2$-4-$CF_3SO_2$-Ph | |
| IV-275 | 2-Cl-6-F-Ph | 2,5-$F_2$-4-$CF_3S$-Ph | |
| IV-276 | 2-Cl-6-F-Ph | 2,5-$F_2$-4-$CF_3SO$-Ph | |
| IV-277 | 2-Cl-6-F-Ph | 2,5-$F_2$-4-$CF_3SO_2$-Ph | |
| IV-278 | 2-Cl-6-F-Ph | 3,5-$F_2$-4-$CF_3S$-Ph | |
| IV-279 | 2-Cl-6-F-Ph | 3,5-$F_2$-4-$CF_3SO$-Ph | |
| IV-280 | 2-Cl-6-F-Ph | 3,5-$F_2$-4-$CF_3SO_2$-Ph | |

| compound No. | $Q_1$ | $Q_2$ | m. p. (°C) |
|---|---|---|---|
| IV-281 | 2-Cl-6-F-Ph | 2,3,6-$F_3$-4-$CF_3S$-Ph | |
| IV-282 | 2-Cl-6-F-Ph | 2,3,6-$F_3$-4-$CF_3SO$-Ph | |
| IV-283 | 2-Cl-6-F-Ph | 2,3,6-$F_3$-4-$CF_3SO_2$-Ph | |
| IV-284 | 2-Cl-6-F-Ph | 2,3,5,6-$F_4$-4-$CF_3S$-Ph | |
| IV-285 | 2-Cl-6-F-Ph | 2,3,5,6-$F_4$-4-$CF_3SO$-Ph | |
| IV-286 | 2-Cl-6-F-Ph | 2,3,5,6-$F_4$-4-$CF_3SO_2$-Ph | |
| IV-287 | 2-Cl-6-F-Ph | 2-F-4-$CF_3CF_2S$-Ph | |
| IV-288 | 2-Cl-6-F-Ph | 2-F-4-$CF_3CF_2SO$-Ph | |
| IV-289 | 2-Cl-6-F-Ph | 2-F-4-$CF_3CF_2SO_2$-Ph | |
| IV-290 | 2-Cl-6-F-Ph | 2-Cl-4-$CF_3CF_2S$-Ph | |
| IV-291 | 2-Cl-6-F-Ph | 2-Cl-4-$CF_3CF_2SO$-Ph | |
| IV-292 | 2-Cl-6-F-Ph | 2-Cl-4-$CF_3CF_2SO_2$-Ph | |
| IV-293 | 2-Cl-6-F-Ph | 2-Me-4-$CF_3CF_2S$-Ph | |
| IV-294 | 2-Cl-6-F-Ph | 2-Me-4-$CF_3CF_2SO$-Ph | |
| IV-295 | 2-Cl-6-F-Ph | 2-Me-4-$CF_3CF_2SO_2$-Ph | |
| IV-296 | 2-Cl-6-F-Ph | 5-Cl-2-Me-4-$CF_3CF_2S$-Ph | |
| IV-297 | 2-Cl-6-F-Ph | 5-Cl-2-Me-4-$CF_3CF_2SO$-Ph | |
| IV-298 | 2-Cl-6-F-Ph | 5-Cl-2-Me-4-$CF_3CF_2SO_2$-Ph | |
| IV-299 | 2-Cl-6-F-Ph | 2,3-$Me_2$-4-$CF_3CF_2S$-Ph | |
| IV-300 | 2-Cl-6-F-Ph | 2,3-$Me_2$-4-$CF_3CF_2SO$-Ph | |
| IV-301 | 2-Cl-6-F-Ph | 2,3-$Me_2$-4-$CF_3CF_2SO_2$-Ph | |
| IV-302 | 2-Cl-6-F-Ph | 2-F-4-$CF_3CF_2CF_2S$-Ph | |
| IV-303 | 2-Cl-6-F-Ph | 2-F-4-$CF_3CF_2CF_2SO$-Ph | |
| IV-304 | 2-Cl-6-F-Ph | 2-F-4-$CF_3CF_2CF_2SO_2$-Ph | |
| IV-305 | 2-Cl-6-F-Ph | 2-Cl-4-$CF_3CF_2CF_2S$-Ph | |
| IV-306 | 2-Cl-6-F-Ph | 2-Cl-4-$CF_3CF_2CF_2SO$-Ph | |
| IV-307 | 2-Cl-6-F-Ph | 2-Cl-4-$CF_3CF_2CF_2SO_2$-Ph | |
| IV-308 | 2-Cl-6-F-Ph | 2-Me-4-$CF_3CF_2CF_2S$-Ph | |
| IV-309 | 2-Cl-6-F-Ph | 2-Me-4-$CF_3CF_2CF_2SO$-Ph | |
| IV-310 | 2-Cl-6-F-Ph | 2-Me-4-$CF_3CF_2CF_2SO_2$-Ph | |
| IV-311 | 2-Cl-6-F-Ph | 5-Cl-2-Me-4-$CF_3CF_2CF_2S$-Ph | |
| IV-312 | 2-Cl-6-F-Ph | 5-Cl-2-Me-4-$CF_3CF_2CF_2SO$-Ph | |
| IV-313 | 2-Cl-6-F-Ph | 5-Cl-2-Me-4-$CF_3CF_2CF_2SO_2$-Ph | |
| IV-314 | 2-Cl-6-F-Ph | 2,3-$Me_2$-4-$CF_3CF_2CF_2S$-Ph | |
| IV-315 | 2-Cl-6-F-Ph | 2,3-$Me_2$-4-$CF_3CF_2CF_2SO$-Ph | |
| IV-316 | 2-Cl-6-F-Ph | 2,3-$Me_2$-4-$CF_3CF_2CF_2SO_2$-Ph | |
| IV-317 | 2-Cl-6-F-Ph | 2-F-4-$(CF_3)_2CFS$-Ph | |
| IV-318 | 2-Cl-6-F-Ph | 2-F-4-$(CF_3)_2CFSO$-Ph | |
| IV-319 | 2-Cl-6-F-Ph | 2-F-4-$(CF_3)_2CFSO_2$-Ph | |
| IV-320 | 2-Cl-6-F-Ph | 2-Me-4-$(CF_3)_2CFS$-Ph | |

| compound No. | $Q_1$ | $Q_2$ | m. p. (°C) |
|---|---|---|---|
| IV-321 | 2-Cl-6-F-Ph | 2-Me-4-$(CF_3)_2CFSO$-Ph | |
| IV-322 | 2-Cl-6-F-Ph | 2-Me-4-$(CF_3)_2CFSO_2$-Ph | |
| IV-323 | 2-Cl-6-F-Ph | 2-Cl-4-$(CF_3)_2CFS$-Ph | |
| IV-324 | 2-Cl-6-F-Ph | 2-Cl-4-$(CF_3)_2CFSO$-Ph | |
| IV-325 | 2-Cl-6-F-Ph | 2-Cl-4-$(CF_3)_2CFSO_2$-Ph | |
| IV-326 | 2-Cl-6-F-Ph | 5-Cl-2-Me-4-$(CF_3)_2CFS$-Ph | |
| IV-327 | 2-Cl-6-F-Ph | 5-Cl-2-Me-4-$(CF_3)_2CFSO$-Ph | |
| IV-328 | 2-Cl-6-F-Ph | 5-Cl-2-Me-4-$(CF_3)_2CFSO_2$-Ph | |
| IV-329 | 2-Cl-6-F-Ph | 2,3-$Me_2$-4-$(CF_3)_2CFS$-Ph | |
| IV-330 | 2-Cl-6-F-Ph | 2,3-$Me_2$-4-$(CF_3)_2CFSO$-Ph | |
| IV-331 | 2-Cl-6-F-Ph | 2,3-$Me_2$-4-$(CF_3)_2CFSO_2$-Ph | |
| IV-332 | 2-Cl-6-F-Ph | 2-F-4-MeO-Ph | |
| IV-333 | 2-Cl-6-F-Ph | 2-Me-4-MeO-Ph | |
| IV-334 | 2-Cl-6-F-Ph | 2-F-4-CN-Ph | |
| IV-335 | 2-Cl-6-F-Ph | 2-F-4-COOMe-Ph | |
| IV-336 | 2-Cl-6-F-Ph | 2-F-4-COOEt-Ph | |
| IV-337 | 2,6-$Cl_2$-Ph | 4-Cl-Ph | |
| IV-338 | 2,6-$Cl_2$-Ph | 3,5-$Cl_2$-4-(2-Cl-4-$CF_3$-pyridin-2yl-O)-Ph | |
| IV-339 | 2,6-$Cl_2$-Ph | 2-F-4-(2-Cl-4-$CF_3$-Ph-O)-Ph | |
| IV-340 | 2,6-$Cl_2$-Ph | 3,5-$Cl_2$-2,4-$F_2$-Ph | |
| IV-341 | 2,6-$Cl_2$-Ph | 3,5-$Cl_2$-4-$CF_2HCF_2O$-Ph | |
| IV-342 | 2,6-$Cl_2$-Ph | 2,5-$Cl_2$-4-$CF_3CFHCF_2O$-Ph | |
| IV-343 | 2,6-$Cl_2$-Ph | 2-F-4-$CFClHCF_2S$-Ph | |
| IV-344 | 2,6-$Cl_2$-Ph | 2-F-4-$CFClHCF_2SO$-Ph | |
| IV-345 | 2,6-$Cl_2$-Ph | 2-F-4-$CFClHCF_2SO_2$-Ph | |
| IV-346 | 2,6-$Cl_2$-Ph | 4-$CFClHCF_2S$-Ph | |
| IV-347 | 2,6-$Cl_2$-Ph | 4-$CFClHCF_2SO$-Ph | |
| IV-348 | 2,6-$Cl_2$-Ph | 4-$CFClHCF_2SO_2$-Ph | |
| IV-349 | 2,6-$Cl_2$-Ph | 2-Cl-4-$CFClHCF_2S$-Ph | |
| IV-350 | 2,6-$Cl_2$-Ph | 2-Cl-4-$CFClHCF_2SO$-Ph | |
| IV-351 | 2,6-$Cl_2$-Ph | 2-Cl-4-$CFClHCF_2SO_2$-Ph | |
| IV-352 | 2,6-$Cl_2$-Ph | 2-Cl-4-$CF_2HCF_2S$-Ph | |
| IV-353 | 2,6-$Cl_2$-Ph | 2-Cl-4-$CF_2HCF_2SO$-Ph | |
| IV-354 | 2,6-$Cl_2$-Ph | 2-Cl-4-$CF_2HCF_2SO_2$-Ph | |
| IV-355 | 2,6-$Cl_2$-Ph | 4-$CF_2HCF_2S$-Ph | |
| IV-356 | 2,6-$Cl_2$-Ph | 4-$CF_2HCF_2SO$-Ph | |
| IV-357 | 2,6-$Cl_2$-Ph | 4-$CF_2HCF_2SO_2$-Ph | |
| IV-358 | 2,6-$Cl_2$-Ph | 2-F-4-$CF_2HCF_2S$-Ph | |
| IV-359 | 2,6-$Cl_2$-Ph | 2-F-4-$CF_2HCF_2SO$-Ph | |
| IV-360 | 2,6-$Cl_2$-Ph | 2-F-4-$CF_2HCF_2SO_2$-Ph | |

| compound No. | $Q_1$ | $Q_2$ | m. p. (°C) |
|---|---|---|---|
| IV-361 | 2,6-$Cl_2$-Ph | 4-$CF_3$S-Ph | |
| IV-362 | 2,6-$Cl_2$-Ph | 4-$CF_3$SO-Ph | |
| IV-363 | 2,6-$Cl_2$-Ph | 4-$CF_3SO_2$-Ph | |
| IV-364 | 2,6-$Cl_2$-Ph | 2-F-4-MeS-Ph | |
| IV-365 | 2,6-$Cl_2$-Ph | 2-F-4-MeSO-Ph | |
| IV-366 | 2,6-$Cl_2$-Ph | 2-F-4-$MeSO_2$-Ph | |
| IV-367 | 2,6-$Cl_2$-Ph | 2,6-$F_2$-4-MeS-Ph | |
| IV-368 | 2,6-$Cl_2$-Ph | 2,6-$F_2$-4-MeSO-Ph | |
| IV-369 | 2,6-$Cl_2$-Ph | 2,6-$F_2$-4-$MeSO_2$-Ph | |
| IV-370 | 2,6-$Cl_2$-Ph | 2-F-4-$CF_3CH_2$S-Ph | |
| IV-371 | 2,6-$Cl_2$-Ph | 2-F-4-$CF_3CH_2$SO-Ph | |
| IV-372 | 2,6-$Cl_2$-Ph | 2-F-4-$CF_3CH_2SO_2$-Ph | |
| IV-373 | 2,6-$Cl_2$-Ph | 2-F-4-$CCl_2$=$CHCH_2$S-Ph | |
| IV-374 | 2,6-$Cl_2$-Ph | 2-F-4-$CCl_2$=$CHCH_2$SO-Ph | |
| IV-375 | 2,6-$Cl_2$-Ph | 2-F-4-$CCl_2$=$CHCH_2SO_2$-Ph | |
| IV-376 | 2,6-$Cl_2$-Ph | $CF_3CH_2$ | |
| IV-377 | 2,6-$Cl_2$-Ph | 2-F-4-$CF_2$HS-Ph | |
| IV-378 | 2,6-$Cl_2$-Ph | 2-F-4-$CF_2$HSO-Ph | |
| IV-379 | 2,6-$Cl_2$-Ph | 2-F-4-$CF_2HSO_2$-Ph | |
| IV-380 | 2,6-$Cl_2$-Ph | 2-F-4-$CF_3$S-Ph | |
| IV-381 | 2,6-$Cl_2$-Ph | 2-F-4-$CF_3$SO-Ph | |
| IV-382 | 2,6-$Cl_2$-Ph | 2-F-4-$CF_3SO_2$-Ph | |
| IV-383 | 2,6-$Cl_2$-Ph | 2-F-4-$CH_3CH_2$S-Ph | |
| IV-384 | 2,6-$Cl_2$-Ph | 2-F-4-$CH_3CH_2$SO-Ph | |
| IV-385 | 2,6-$Cl_2$-Ph | 2-F-4-$CH_3CH_2SO_2$-Ph | |
| IV-386 | 2,6-$Cl_2$-Ph | 2-F-4-$CH_3CH_2CH_2$S-Ph | |
| IV-387 | 2,6-$Cl_2$-Ph | 2-F-4-$CH_3CH_2CH_2$SO-Ph | |
| IV-388 | 2,6-$Cl_2$-Ph | 2-F-4-$CH_3CH_2CH_2SO_2$-Ph | |
| IV-389 | 2,6-$Cl_2$-Ph | 2-F-4-i-PrS-Ph | |
| IV-390 | 2,6-$Cl_2$-Ph | 2-F-4-i-PrSO-Ph | |
| IV-391 | 2,6-$Cl_2$-Ph | 2-F-4-i-$PrSO_2$-Ph | |
| IV-392 | 2,6-$Cl_2$-Ph | 2-F-4-t-BuS-Ph | |
| IV-393 | 2,6-$Cl_2$-Ph | 2-F-4-t-BuSO-Ph | |
| IV-394 | 2,6-$Cl_2$-Ph | 2-F-4-t-$BuSO_2$-Ph | |
| IV-395 | 2,6-$Cl_2$-Ph | 2-F-4-$CH_2$=$CHCH_2$S-Ph | |
| IV-396 | 2,6-$Cl_2$-Ph | 2-F-4-$CH_2$=$CHCH_2$SO-Ph | |
| IV-397 | 2,6-$Cl_2$-Ph | 2-F-4-$CH_2$=$CHCH_2SO_2$-Ph | |
| IV-398 | 2,6-$Cl_2$-Ph | 2-F-4-CH≡$CCH_2$S-Ph | |
| IV-399 | 2,6-$Cl_2$-Ph | 2-F-4-CH≡$CCH_2$SO-Ph | |
| IV-400 | 2,6-$Cl_2$-Ph | 2-F-4-CH≡$CCH_2SO_2$-Ph | |

| compound No. | $Q_1$ | $Q_2$ | m. p. (°C) |
|---|---|---|---|
| IV-401 | 2,6-$Cl_2$-Ph | 2-F-4-(2-Cl-4-$CF_3$-pyridin-2yl-S)-Ph | |
| IV-402 | 2,6-$Cl_2$-Ph | 2-F-4-(2-Cl-4-$CF_3$-pyridin-2yl-SO)-Ph | |
| IV-403 | 2,6-$Cl_2$-Ph | 2-F-4-(2-Cl-4-$CF_3$-pyridin-2yl-$SO_2$)-Ph | |
| IV-404 | 2,6-$Cl_2$-Ph | 2-Me-4-$CF_3$S-Ph | |
| IV-405 | 2,6-$Cl_2$-Ph | 2-Me-4-$CF_3$SO-Ph | |
| IV-406 | 2,6-$Cl_2$-Ph | 2-Me-4-$CF_3SO_2$-Ph | |
| IV-407 | 2,6-$Cl_2$-Ph | 3-Me-4-$CF_3$S-Ph | |
| IV-408 | 2,6-$Cl_2$-Ph | 3-Me-4-$CF_3$SO-Ph | |
| IV-409 | 2,6-$Cl_2$-Ph | 3-Me-4-$CF_3SO_2$-Ph | |
| IV-410 | 2,6-$Cl_2$-Ph | 2-Cl-4-$CF_3$S-Ph | |
| IV-411 | 2,6-$Cl_2$-Ph | 2-Cl-4-$CF_3$SO-Ph | |
| IV-412 | 2,6-$Cl_2$-Ph | 2-Cl-4-$CF_3SO_2$-Ph | |
| IV-413 | 2,6-$Cl_2$-Ph | 3-Cl-4-$CF_3$S-Ph | |
| IV-414 | 2,6-$Cl_2$-Ph | 3-Cl-4-$CF_3$SO-Ph | |
| IV-415 | 2,6-$Cl_2$-Ph | 3-Cl-4-$CF_3SO_2$-Ph | |
| IV-416 | 2,6-$Cl_2$-Ph | 3-F-4-$CF_3$S-Ph | |
| IV-417 | 2,6-$Cl_2$-Ph | 3-F-4-$CF_3$SO-Ph | |
| IV-418 | 2,6-$Cl_2$-Ph | 3-F-4-$CF_3SO_2$-Ph | |
| IV-419 | 2,6-$Cl_2$-Ph | 2,3-$Me_2$-4-$CF_3$S-Ph | |
| IV-420 | 2,6-$Cl_2$-Ph | 2,3-$Me_2$-4-$CF_3$SO-Ph | |
| IV-421 | 2,6-$Cl_2$-Ph | 2,3-$Me_2$-4-$CF_3SO_2$-Ph | |
| IV-422 | 2,6-$Cl_2$-Ph | 2,5-$Me_2$-4-$CF_3$S-Ph | |
| IV-423 | 2,6-$Cl_2$-Ph | 2,5-$Me_2$-4-$CF_3$SO-Ph | |
| IV-424 | 2,6-$Cl_2$-Ph | 2,5-$Me_2$-4-$CF_3SO_2$-Ph | |
| IV-425 | 2,6-$Cl_2$-Ph | 5-Cl-2-Me-4-$CF_3$S-Ph | |
| IV-426 | 2,6-$Cl_2$-Ph | 5-Cl-2-Me-4-$CF_3$SO-Ph | |
| IV-427 | 2,6-$Cl_2$-Ph | 5-Cl-2-Me-4-$CF_3SO_2$-Ph | |
| IV-428 | 2,6-$Cl_2$-Ph | 2,3-$Cl_2$-4-$CF_3$S-Ph | |
| IV-429 | 2,6-$Cl_2$-Ph | 2,3-$Cl_2$-4-$CF_3$SO-Ph | |
| IV-430 | 2,6-$Cl_2$-Ph | 2,3-$Cl_2$-4-$CF_3SO_2$-Ph | |
| IV-431 | 2,6-$Cl_2$-Ph | 3-Cl-2-F-4-$CF_3$S-Ph | |
| IV-432 | 2,6-$Cl_2$-Ph | 3-Cl-2-F-4-$CF_3$SO-Ph | |
| IV-433 | 2,6-$Cl_2$-Ph | 3-Cl-2-F-4-$CF_3SO_2$-Ph | |
| IV-434 | 2,6-$Cl_2$-Ph | 5-Cl-2-F-4-$CF_3$S-Ph | |
| IV-435 | 2,6-$Cl_2$-Ph | 5-Cl-2-F-4-$CF_3$SO-Ph | |
| IV-436 | 2,6-$Cl_2$-Ph | 5-Cl-2-F-4-$CF_3SO_2$-Ph | |
| IV-437 | 2,6-$Cl_2$-Ph | 2,6-$F_2$-4-$CF_3$S-Ph | |
| IV-438 | 2,6-$Cl_2$-Ph | 2,6-$F_2$-4-$CF_3$SO-Ph | |
| IV-439 | 2,6-$Cl_2$-Ph | 2,6-$F_2$-4-$CF_3SO_2$-Ph | |
| IV-440 | 2,6-$Cl_2$-Ph | 2,3-$F_2$-4-$CF_3$S-Ph | |

| compound No. | $Q_1$ | $Q_2$ | m. p. (°C) |
|---|---|---|---|
| IV-441 | 2,6-$Cl_2$-Ph | 2,3-$F_2$-4-$CF_3$SO-Ph | |
| IV-442 | 2,6-$Cl_2$-Ph | 2,3-$F_2$-4-$CF_3SO_2$-Ph | |
| IV-443 | 2,6-$Cl_2$-Ph | 2,5-$F_2$-4-$CF_3$S-Ph | |
| IV-444 | 2,6-$Cl_2$-Ph | 2,5-$F_2$-4-$CF_3$SO-Ph | |
| IV-445 | 2,6-$Cl_2$-Ph | 2,5-$F_2$-4-$CF_3SO_2$-Ph | |
| IV-446 | 2,6-$Cl_2$-Ph | 3,5-$F_2$-4-$CF_3$S-Ph | |
| IV-447 | 2,6-$Cl_2$-Ph | 3,5-$F_2$-4-$CF_3$SO-Ph | |
| IV-448 | 2,6-$Cl_2$-Ph | 3,5-$F_2$-4-$CF_3SO_2$-Ph | |
| IV-449 | 2,6-$Cl_2$-Ph | 2,3,6-$F_3$-4-$CF_3$S-Ph | |
| IV-450 | 2,6-$Cl_2$-Ph | 2,3,6-$F_3$-4-$CF_3$SO-Ph | |
| IV-451 | 2,6-$Cl_2$-Ph | 2,3,6-$F_3$-4-$CF_3SO_2$-Ph | |
| IV-452 | 2,6-$Cl_2$-Ph | 2,3,5,6-$F_4$-4-$CF_3$S-Ph | |
| IV-453 | 2,6-$Cl_2$-Ph | 2,3,5,6-$F_4$-4-$CF_3$SO-Ph | |
| IV-454 | 2,6-$Cl_2$-Ph | 2,3,5,6-$F_4$-4-$CF_3SO_2$-Ph | |
| IV-455 | 2,6-$Cl_2$-Ph | 2-F-4-$CF_3CF_2$S-Ph | |
| IV-456 | 2,6-$Cl_2$-Ph | 2-F-4-$CF_3CF_2$SO-Ph | |
| IV-457 | 2,6-$Cl_2$-Ph | 2-F-4-$CF_3CF_2SO_2$-Ph | |
| IV-458 | 2,6-$Cl_2$-Ph | 2-Cl-4-$CF_3CF_2$S-Ph | |
| IV-459 | 2,6-$Cl_2$-Ph | 2-Cl-4-$CF_3CF_2$SO-Ph | |
| IV-460 | 2,6-$Cl_2$-Ph | 2-Cl-4-$CF_3CF_2SO_2$-Ph | |
| IV-461 | 2,6-$Cl_2$-Ph | 2-Me-4-$CF_3CF_2$S-Ph | |
| IV-462 | 2,6-$Cl_2$-Ph | 2-Me-4-$CF_3CF_2$SO-Ph | |
| IV-463 | 2,6-$Cl_2$-Ph | 2-Me-4-$CF_3CF_2SO_2$-Ph | |
| IV-464 | 2,6-$Cl_2$-Ph | 5-Cl-2-Me-4-$CF_3CF_2$S-Ph | |
| IV-465 | 2,6-$Cl_2$-Ph | 5-Cl-2-Me-4-$CF_3CF_2$SO-Ph | |
| IV-466 | 2,6-$Cl_2$-Ph | 5-Cl-2-Me-4-$CF_3CF_2SO_2$-Ph | |
| IV-467 | 2,6-$Cl_2$-Ph | 2,3-$Me_2$-4-$CF_3CF_2$S-Ph | |
| IV-468 | 2,6-$Cl_2$-Ph | 2,3-$Me_2$-4-$CF_3CF_2$SO-Ph | |
| IV-469 | 2,6-$Cl_2$-Ph | 2,3-$Me_2$-4-$CF_3CF_2SO_2$-Ph | |
| IV-470 | 2,6-$Cl_2$-Ph | 2-F-4-$CF_3CF_2CF_2$S-Ph | |
| IV-471 | 2,6-$Cl_2$-Ph | 2-F-4-$CF_3CF_2CF_2$SO-Ph | |
| IV-472 | 2,6-$Cl_2$-Ph | 2-F-4-$CF_3CF_2CF_2SO_2$-Ph | |
| IV-473 | 2,6-$Cl_2$-Ph | 2-Cl-4-$CF_3CF_2CF_2$S-Ph | |
| IV-474 | 2,6-$Cl_2$-Ph | 2-Cl-4-$CF_3CF_2CF_2$SO-Ph | |
| IV-475 | 2,6-$Cl_2$-Ph | 2-Cl-4-$CF_3CF_2CF_2SO_2$-Ph | |
| IV-476 | 2,6-$Cl_2$-Ph | 2-Me-4-$CF_3CF_2CF_2$S-Ph | |
| IV-477 | 2,6-$Cl_2$-Ph | 2-Me-4-$CF_3CF_2CF_2$SO-Ph | |
| IV-478 | 2,6-$Cl_2$-Ph | 2-Me-4-$CF_3CF_2CF_2SO_2$-Ph | |
| IV-479 | 2,6-$Cl_2$-Ph | 5-Cl-2-Me-4-$CF_3CF_2CF_2$S-Ph | |
| IV-480 | 2,6-$Cl_2$-Ph | 5-Cl-2-Me-4-$CF_3CF_2CF_2$SO-Ph | |

| compound No. | $Q_1$ | $Q_2$ | m. p. (°C) |
|---|---|---|---|
| IV-481 | 2,6-$Cl_2$-Ph | 5-Cl-2-Me-4-$CF_3CF_2CF_2SO_2$-Ph | |
| IV-482 | 2,6-$Cl_2$-Ph | 2,3-$Me_2$-4-$CF_3CF_2CF_2S$-Ph | |
| IV-483 | 2,6-$Cl_2$-Ph | 2,3-$Me_2$-4-$CF_3CF_2CF_2SO$-Ph | |
| IV-484 | 2,6-$Cl_2$-Ph | 2,3-$Me_2$-4-$CF_3CF_2CF_2SO_2$-Ph | |
| IV-485 | 2,6-$Cl_2$-Ph | 2-F-4-$(CF_3)_2CFS$-Ph | |
| IV-486 | 2,6-$Cl_2$-Ph | 2-F-4-$(CF_3)_2CFSO$-Ph | |
| IV-487 | 2,6-$Cl_2$-Ph | 2-F-4-$(CF_3)_2CFSO_2$-Ph | |
| IV-488 | 2,6-$Cl_2$-Ph | 2-Me-4-$(CF_3)_2CFS$-Ph | |
| IV-489 | 2,6-$Cl_2$-Ph | 2-Me-4-$(CF_3)_2CFSO$-Ph | |
| IV-490 | 2,6-$Cl_2$-Ph | 2-Me-4-$(CF_3)_2CFSO_2$-Ph | |
| IV-491 | 2,6-$Cl_2$-Ph | 2-Cl-4-$(CF_3)_2CFS$-Ph | |
| IV-492 | 2,6-$Cl_2$-Ph | 2-Cl-4-$(CF_3)_2CFSO$-Ph | |
| IV-493 | 2,6-$Cl_2$-Ph | 2-Cl-4-$(CF_3)_2CFSO_2$-Ph | |
| IV-494 | 2,6-$Cl_2$-Ph | 5-Cl-2-Me-4-$(CF_3)_2CFS$-Ph | |
| IV-495 | 2,6-$Cl_2$-Ph | 5-Cl-2-Me-4-$(CF_3)_2CFSO$-Ph | |
| IV-496 | 2,6-$Cl_2$-Ph | 5-Cl-2-Me-4-$(CF_3)_2CFSO_2$-Ph | |
| IV-497 | 2,6-$Cl_2$-Ph | 2,3-$Me_2$-4-$(CF_3)_2CFS$-Ph | |
| IV-498 | 2,6-$Cl_2$-Ph | 2,3-$Me_2$-4-$(CF_3)_2CFSO$-Ph | |
| IV-499 | 2,6-$Cl_2$-Ph | 2,3-$Me_2$-4-$(CF_3)_2CFSO_2$-Ph | |
| IV-500 | 2,6-$Cl_2$-Ph | 2-F-4-MeO-Ph | |
| IV-501 | 2,6-$Cl_2$-Ph | 2-Me-4-MeO-Ph | |
| IV-502 | 2,6-$Cl_2$-Ph | 2-F-4-CN-Ph | |
| IV-503 | 2,6-$Cl_2$-Ph | 2-F-4-COOMe-Ph | |
| IV-504 | 2,6-$Cl_2$-Ph | 2-F-4-COOEt-Ph | |
| IV-505 | 3-Cl-pyridin-2yl | 4-Cl-Ph | |
| IV-506 | 3-Cl-pyridin-2yl | 3,5-$Cl_2$-4-(2-Cl-4-$CF_3$-pyridin-2yl-O)-Ph | |
| IV-507 | 3-Cl-pyridin-2yl | 2-F-4-(2-Cl-4-$CF_3$-Ph-O)-Ph | |
| IV-508 | 3-Cl-pyridin-2yl | 3,5-$Cl_2$-2,4-$F_2$-Ph | |
| IV-509 | 3-Cl-pyridin-2yl | 3,5-$Cl_2$-4-$CF_2HCF_2O$-Ph | |
| IV-510 | 3-Cl-pyridin-2yl | 2,5-$Cl_2$-4-$CF_3CFHCF_2O$-Ph | |
| IV-511 | 3-Cl-pyridin-2yl | 2-F-4-$CFClHCF_2S$-Ph | |
| IV-512 | 3-Cl-pyridin-2yl | 2-F-4-$CFClHCF_2SO$-Ph | |
| IV-513 | 3-Cl-pyridin-2yl | 2-F-4-$CFClHCF_2SO_2$-Ph | |
| IV-514 | 3-Cl-pyridin-2yl | 4-$CFClHCF_2S$-Ph | |
| IV-515 | 3-Cl-pyridin-2yl | 4-$CFClHCF_2SO$-Ph | |
| IV-516 | 3-Cl-pyridin-2yl | 4-$CFClHCF_2SO_2$-Ph | |
| IV-517 | 3-Cl-pyridin-2yl | 2-Cl-4-$CFClHCF_2S$-Ph | |
| IV-518 | 3-Cl-pyridin-2yl | 2-Cl-4-$CFClHCF_2SO$-Ph | |
| IV-519 | 3-Cl-pyridin-2yl | 2-Cl-4-$CFClHCF_2SO_2$-Ph | |
| IV-520 | 3-Cl-pyridin-2yl | 2-Cl-4-$CF_2HCF_2S$-Ph | |

| compound No. | $Q_1$ | $Q_2$ | m. p. (°C) |
|---|---|---|---|
| IV- 521 | 3-Cl-pyridin-2yl | 2-Cl-4-$CF_2HCF_2SO$-Ph | |
| IV- 522 | 3-Cl-pyridin-2yl | 2-Cl-4-$CF_2HCF_2SO_2$-Ph | |
| IV- 523 | 3-Cl-pyridin-2yl | 4-$CF_2HCF_2S$-Ph | |
| IV- 524 | 3-Cl-pyridin-2yl | 4-$CF_2HCF_2SO$-Ph | |
| IV- 525 | 3-Cl-pyridin-2yl | 4-$CF_2HCF_2SO_2$-Ph | |
| IV- 526 | 3-Cl-pyridin-2yl | 2-F-4-$CF_2HCF_2S$-Ph | |
| IV- 527 | 3-Cl-pyridin-2yl | 2-F-4-$CF_2HCF_2SO$-Ph | |
| IV- 528 | 3-Cl-pyridin-2yl | 2-F-4-$CF_2HCF_2SO_2$-Ph | |
| IV- 529 | 3-Cl-pyridin-2yl | 4-$CF_3S$-Ph | |
| IV- 530 | 3-Cl-pyridin-2yl | 4-$CF_3SO$-Ph | |
| IV- 531 | 3-Cl-pyridin-2yl | 4-$CF_3SO_2$-Ph | |
| IV- 532 | 3-Cl-pyridin-2yl | 2-F-4-MeS-Ph | |
| IV- 533 | 3-Cl-pyridin-2yl | 2-F-4-MeSO-Ph | |
| IV- 534 | 3-Cl-pyridin-2yl | 2-F-4-$MeSO_2$-Ph | |
| IV- 535 | 3-Cl-pyridin-2yl | 2,6-$F_2$-4-MeS-Ph | |
| IV- 536 | 3-Cl-pyridin-2yl | 2,6-$F_2$-4-MeSO-Ph | |
| IV- 537 | 3-Cl-pyridin-2yl | 2,6-$F_2$-4-$MeSO_2$-Ph | |
| IV- 538 | 3-Cl-pyridin-2yl | 2-F-4-$CF_3CH_2S$-Ph | |
| IV- 539 | 3-Cl-pyridin-2yl | 2-F-4-$CF_3CH_2SO$-Ph | |
| IV- 540 | 3-Cl-pyridin-2yl | 2-F-4-$CF_3CH_2SO_2$-Ph | |
| IV- 541 | 3-Cl-pyridin-2yl | 2-F-4-$CCl_2$=$CHCH_2S$-Ph | |
| IV- 542 | 3-Cl-pyridin-2yl | 2-F-4-$CCl_2$=$CHCH_2SO$-Ph | |
| IV- 543 | 3-Cl-pyridin-2yl | 2-F-4-$CCl_2$=$CHCH_2SO_2$-Ph | |
| IV- 544 | 3-Cl-pyridin-2yl | $CF_3CH_2$ | |
| IV- 545 | 3-Cl-pyridin-2yl | 2-F-4-$CF_2HS$-Ph | |
| IV- 546 | 3-Cl-pyridin-2yl | 2-F-4-$CF_2HSO$-Ph | |
| IV- 547 | 3-Cl-pyridin-2yl | 2-F-4-$CF_2HSO_2$-Ph | |
| IV- 548 | 3-Cl-pyridin-2yl | 2-F-4-$CF_3S$-Ph | |
| IV- 549 | 3-Cl-pyridin-2yl | 2-F-4-$CF_3SO$-Ph | |
| IV- 550 | 3-Cl-pyridin-2yl | 2-F-4-$CF_3SO_2$-Ph | |
| IV- 551 | 3-Cl-pyridin-2yl | 2-F-4-$CH_3CH_2S$-Ph | |
| IV- 552 | 3-Cl-pyridin-2yl | 2-F-4-$CH_3CH_2SO$-Ph | |
| IV- 553 | 3-Cl-pyridin-2yl | 2-F-4-$CH_3CH_2SO_2$-Ph | |
| IV- 554 | 3-Cl-pyridin-2yl | 2-F-4-$CH_3CH_2CH_2S$-Ph | |
| IV- 555 | 3-Cl-pyridin-2yl | 2-F-4-$CH_3CH_2CH_2SO$-Ph | |
| IV- 556 | 3-Cl-pyridin-2yl | 2-F-4-$CH_3CH_2CH_2SO_2$-Ph | |
| IV- 557 | 3-Cl-pyridin-2yl | 2-F-4-i-PrS-Ph | |
| IV- 558 | 3-Cl-pyridin-2yl | 2-F-4-i-PrSO-Ph | |
| IV- 559 | 3-Cl-pyridin-2yl | 2-F-4-i-$PrSO_2$-Ph | |
| IV- 560 | 3-Cl-pyridin-2yl | 2-F-4-t-BuS-Ph | |

| compound No. | $Q_1$ | $Q_2$ | m. p. (°C) |
|---|---|---|---|
| IV-561 | 3-Cl-pyridin-2yl | 2-F-4-t-BuSO-Ph | |
| IV-562 | 3-Cl-pyridin-2yl | 2-F-4-t-$BuSO_2$-Ph | |
| IV-563 | 3-Cl-pyridin-2yl | 2-F-4-$CH_2$=$CHCH_2S$-Ph | |
| IV-564 | 3-Cl-pyridin-2yl | 2-F-4-$CH_2$=$CHCH_2SO$-Ph | |
| IV-565 | 3-Cl-pyridin-2yl | 2-F-4-$CH_2$=$CHCH_2SO_2$-Ph | |
| IV-566 | 3-Cl-pyridin-2yl | 2-F-4-CH≡$CCH_2S$-Ph | |
| IV-567 | 3-Cl-pyridin-2yl | 2-F-4-CH≡$CCH_2SO$-Ph | |
| IV-568 | 3-Cl-pyridin-2yl | 2-F-4-CH≡$CCH_2SO_2$-Ph | |
| IV-569 | 3-Cl-pyridin-2yl | 2-F-4-(2-Cl-4-$CF_3$-pyridin-2yl-S)-Ph | |
| IV-570 | 3-Cl-pyridin-2yl | 2-F-4-(2-Cl-4-$CF_3$-pyridin-2yl-SO)-Ph | |
| IV-571 | 3-Cl-pyridin-2yl | 2-F-4-(2-Cl-4-$CF_3$-pyridin-2yl-$SO_2$)-Ph | |
| IV-572 | 3-Cl-pyridin-2yl | 2-Me-4-$CF_3S$-Ph | |
| IV-573 | 3-Cl-pyridin-2yl | 2-Me-4-$CF_3SO$-Ph | |
| IV-574 | 3-Cl-pyridin-2yl | 2-Me-4-$CF_3SO_2$-Ph | |
| IV-575 | 3-Cl-pyridin-2yl | 3-Me-4-$CF_3S$-Ph | |
| IV-576 | 3-Cl-pyridin-2yl | 3-Me-4-$CF_3SO$-Ph | |
| IV-577 | 3-Cl-pyridin-2yl | 3-Me-4-$CF_3SO_2$-Ph | |
| IV-578 | 3-Cl-pyridin-2yl | 2-Cl-4-$CF_3S$-Ph | |
| IV-579 | 3-Cl-pyridin-2yl | 2-Cl-4-$CF_3SO$-Ph | |
| IV-580 | 3-Cl-pyridin-2yl | 2-Cl-4-$CF_3SO_2$-Ph | |
| IV-581 | 3-Cl-pyridin-2yl | 3-Cl-4-$CF_3S$-Ph | |
| IV-582 | 3-Cl-pyridin-2yl | 3-Cl-4-$CF_3SO$-Ph | |
| IV-583 | 3-Cl-pyridin-2yl | 3-Cl-4-$CF_3SO_2$-Ph | |
| IV-584 | 3-Cl-pyridin-2yl | 3-F-4-$CF_3S$-Ph | |
| IV-585 | 3-Cl-pyridin-2yl | 3-F-4-$CF_3SO$-Ph | |
| IV-586 | 3-Cl-pyridin-2yl | 3-F-4-$CF_3SO_2$-Ph | |
| IV-587 | 3-Cl-pyridin-2yl | 2,3-$Me_2$-4-$CF_3S$-Ph | |
| IV-588 | 3-Cl-pyridin-2yl | 2,3-$Me_2$-4-$CF_3SO$-Ph | |
| IV-589 | 3-Cl-pyridin-2yl | 2,3-$Me_2$-4-$CF_3SO_2$-Ph | |
| IV-590 | 3-Cl-pyridin-2yl | 2,5-$Me_2$-4-$CF_3S$-Ph | |
| IV-591 | 3-Cl-pyridin-2yl | 2,5-$Me_2$-4-$CF_3SO$-Ph | |
| IV-592 | 3-Cl-pyridin-2yl | 2,5-$Me_2$-4-$CF_3SO_2$-Ph | |
| IV-593 | 3-Cl-pyridin-2yl | 5-Cl-2-Me-4-$CF_3S$-Ph | |
| IV-594 | 3-Cl-pyridin-2yl | 5-Cl-2-Me-4-$CF_3SO$-Ph | |
| IV-595 | 3-Cl-pyridin-2yl | 5-Cl-2-Me-4-$CF_3SO_2$-Ph | |
| IV-596 | 3-Cl-pyridin-2yl | 2,3-$Cl_2$-4-$CF_3S$-Ph | |
| IV-597 | 3-Cl-pyridin-2yl | 2,3-$Cl_2$-4-$CF_3SO$-Ph | |
| IV-598 | 3-Cl-pyridin-2yl | 2,3-$Cl_2$-4-$CF_3SO_2$-Ph | |
| IV-599 | 3-Cl-pyridin-2yl | 3-Cl-2-F-4-$CF_3S$-Ph | |
| IV-600 | 3-Cl-pyridin-2yl | 3-Cl-2-F-4-$CF_3SO$-Ph | |

| compound No. | $Q_1$ | $Q_2$ | m.p. (℃) |
|---|---|---|---|
| IV-601 | 3-Cl-pyridin-2yl | 3-Cl-2-F-4-$CF_3SO_2$-Ph | |
| IV-602 | 3-Cl-pyridin-2yl | 5-Cl-2-F-4-$CF_3S$-Ph | |
| IV-603 | 3-Cl-pyridin-2yl | 5-Cl-2-F-4-$CF_3SO$-Ph | |
| IV-604 | 3-Cl-pyridin-2yl | 5-Cl-2-F-4-$CF_3SO_2$-Ph | |
| IV-605 | 3-Cl-pyridin-2yl | 2,6-$F_2$-4-$CF_3S$-Ph | |
| IV-606 | 3-Cl-pyridin-2yl | 2,6-$F_2$-4-$CF_3SO$-Ph | |
| IV-607 | 3-Cl-pyridin-2yl | 2,6-$F_2$-4-$CF_3SO_2$-Ph | |
| IV-608 | 3-Cl-pyridin-2yl | 2,3-$F_2$-4-$CF_3S$-Ph | |
| IV-609 | 3-Cl-pyridin-2yl | 2,3-$F_2$-4-$CF_3SO$-Ph | |
| IV-610 | 3-Cl-pyridin-2yl | 2,3-$F_2$-4-$CF_3SO_2$-Ph | |
| IV-611 | 3-Cl-pyridin-2yl | 2,5-$F_2$-4-$CF_3S$-Ph | |
| IV-612 | 3-Cl-pyridin-2yl | 2,5-$F_2$-4-$CF_3SO$-Ph | |
| IV-613 | 3-Cl-pyridin-2yl | 2,5-$F_2$-4-$CF_3SO_2$-Ph | |
| IV-614 | 3-Cl-pyridin-2yl | 3,5-$F_2$-4-$CF_3S$-Ph | |
| IV-615 | 3-Cl-pyridin-2yl | 3,5-$F_2$-4-$CF_3SO$-Ph | |
| IV-616 | 3-Cl-pyridin-2yl | 3,5-$F_2$-4-$CF_3SO_2$-Ph | |
| IV-617 | 3-Cl-pyridin-2yl | 2,3,6-$F_3$-4-$CF_3S$-Ph | |
| IV-618 | 3-Cl-pyridin-2yl | 2,3,6-$F_3$-4-$CF_3SO$-Ph | |
| IV-619 | 3-Cl-pyridin-2yl | 2,3,6-$F_3$-4-$CF_3SO_2$-Ph | |
| IV-620 | 3-Cl-pyridin-2yl | 2,3,5,6-$F_4$-4-$CF_3S$-Ph | |
| IV-621 | 3-Cl-pyridin-2yl | 2,3,5,6-$F_4$-4-$CF_3SO$-Ph | |
| IV-622 | 3-Cl-pyridin-2yl | 2,3,5,6-$F_4$-4-$CF_3SO_2$-Ph | |
| IV-623 | 3-Cl-pyridin-2yl | 2-F-4-$CF_3CF_2S$-Ph | |
| IV-624 | 3-Cl-pyridin-2yl | 2-F-4-$CF_3CF_2SO$-Ph | |
| IV-625 | 3-Cl-pyridin-2yl | 2-F-4-$CF_3CF_2SO_2$-Ph | |
| IV-626 | 3-Cl-pyridin-2yl | 2-Cl-4-$CF_3CF_2S$-Ph | |
| IV-627 | 3-Cl-pyridin-2yl | 2-Cl-4-$CF_3CF_2SO$-Ph | |
| IV-628 | 3-Cl-pyridin-2yl | 2-Cl-4-$CF_3CF_2SO_2$-Ph | |
| IV-629 | 3-Cl-pyridin-2yl | 2-Me-4-$CF_3CF_2S$-Ph | |
| IV-630 | 3-Cl-pyridin-2yl | 2-Me-4-$CF_3CF_2SO$-Ph | |
| IV-631 | 3-Cl-pyridin-2yl | 2-Me-4-$CF_3CF_2SO_2$-Ph | |
| IV-632 | 3-Cl-pyridin-2yl | 5-Cl-2-Me-4-$CF_3CF_2S$-Ph | |
| IV-633 | 3-Cl-pyridin-2yl | 5-Cl-2-Me-4-$CF_3CF_2SO$-Ph | |
| IV-634 | 3-Cl-pyridin-2yl | 5-Cl-2-Me-4-$CF_3CF_2SO_2$-Ph | |
| IV-635 | 3-Cl-pyridin-2yl | 2,3-$Me_2$-4-$CF_3CF_2S$-Ph | |
| IV-636 | 3-Cl-pyridin-2yl | 2,3-$Me_2$-4-$CF_3CF_2SO$-Ph | |
| IV-637 | 3-Cl-pyridin-2yl | 2,3-$Me_2$-4-$CF_3CF_2SO_2$-Ph | |
| IV-638 | 3-Cl-pyridin-2yl | 2-F-4-$CF_3CF_2CF_2S$-Ph | |
| IV-639 | 3-Cl-pyridin-2yl | 2-F-4-$CF_3CF_2CF_2SO$-Ph | |
| IV-640 | 3-Cl-pyridin-2yl | 2-F-4-$CF_3CF_2CF_2SO_2$-Ph | |

| compound No. | $Q_1$ | $Q_2$ | m. p. (℃) |
|---|---|---|---|
| Ⅳ- 641 | 3-Cl-pyridin-2yl | 2-Cl-4-$CF_3CF_2CF_2S$-Ph | |
| Ⅳ- 642 | 3-Cl-pyridin-2yl | 2-Cl-4-$CF_3CF_2CF_2SO$-Ph | |
| Ⅳ- 643 | 3-Cl-pyridin-2yl | 2-Cl-4-$CF_3CF_2CF_2SO_2$-Ph | |
| Ⅳ- 644 | 3-Cl-pyridin-2yl | 2-Me-4-$CF_3CF_2CF_2S$-Ph | |
| Ⅳ- 645 | 3-Cl-pyridin-2yl | 2-Me-4-$CF_3CF_2CF_2SO$-Ph | |
| Ⅳ- 646 | 3-Cl-pyridin-2yl | 2-Me-4-$CF_3CF_2CF_2SO_2$-Ph | |
| Ⅳ- 647 | 3-Cl-pyridin-2yl | 5-Cl-2-Me-4-$CF_3CF_2CF_2S$-Ph | |
| Ⅳ- 648 | 3-Cl-pyridin-2yl | 5-Cl-2-Me-4-$CF_3CF_2CF_2SO$-Ph | |
| Ⅳ- 649 | 3-Cl-pyridin-2yl | 5-Cl-2-Me-4-$CF_3CF_2CF_2SO_2$-Ph | |
| Ⅳ- 650 | 3-Cl-pyridin-2yl | 2,3-$Me_2$-4-$CF_3CF_2CF_2S$-Ph | |
| Ⅳ- 651 | 3-Cl-pyridin-2yl | 2,3-$Me_2$-4-$CF_3CF_2CF_2SO$-Ph | |
| Ⅳ- 652 | 3-Cl-pyridin-2yl | 2,3-$Me_2$-4-$CF_3CF_2CF_2SO_2$-Ph | |
| Ⅳ- 653 | 3-Cl-pyridin-2yl | 2-F-4-$(CF_3)_2CFS$-Ph | |
| Ⅳ- 654 | 3-Cl-pyridin-2yl | 2-F-4-$(CF_3)_2CFSO$-Ph | |
| Ⅳ- 655 | 3-Cl-pyridin-2yl | 2-F-4-$(CF_3)_2CFSO_2$-Ph | |
| Ⅳ- 656 | 3-Cl-pyridin-2yl | 2-Me-4-$(CF_3)_2CFS$-Ph | |
| Ⅳ- 657 | 3-Cl-pyridin-2yl | 2-Me-4-$(CF_3)_2CFSO$-Ph | |
| Ⅳ- 658 | 3-Cl-pyridin-2yl | 2-Me-4-$(CF_3)_2CFSO_2$-Ph | |
| Ⅳ- 659 | 3-Cl-pyridin-2yl | 2-Cl-4-$(CF_3)_2CFS$-Ph | |
| Ⅳ- 660 | 3-Cl-pyridin-2yl | 2-Cl-4-$(CF_3)_2CFSO$-Ph | |
| Ⅳ- 661 | 3-Cl-pyridin-2yl | 2-Cl-4-$(CF_3)_2CFSO_2$-Ph | |
| Ⅳ- 662 | 3-Cl-pyridin-2yl | 5-Cl-2-Me-4-$(CF_3)_2CFS$-Ph | |
| Ⅳ- 663 | 3-Cl-pyridin-2yl | 5-Cl-2-Me-4-$(CF_3)_2CFSO$-Ph | |
| Ⅳ- 664 | 3-Cl-pyridin-2yl | 5-Cl-2-Me-4-$(CF_3)_2CFSO_2$-Ph | |
| Ⅳ- 665 | 3-Cl-pyridin-2yl | 2,3-$Me_2$-4-$(CF_3)_2CFS$-Ph | |
| Ⅳ- 666 | 3-Cl-pyridin-2yl | 2,3-$Me_2$-4-$(CF_3)_2CFSO$-Ph | |
| Ⅳ- 667 | 3-Cl-pyridin-2yl | 2,3-$Me_2$-4-$(CF_3)_2CFSO_2$-Ph | |
| Ⅳ- 668 | 3-Cl-pyridin-2yl | 2-F-4-MeO-Ph | |
| Ⅳ- 669 | 3-Cl-pyridin-2yl | 2-Me-4-MeO-Ph | |
| Ⅳ- 670 | 3-Cl-pyridin-2yl | 2-F-4-CN-Ph | |
| Ⅳ- 671 | 3-Cl-pyridin-2yl | 2-F-4-COOMe-Ph | |
| Ⅳ- 672 | 3-Cl-pyridin-2yl | 2-F-4-COOEt-Ph | |

Table 5

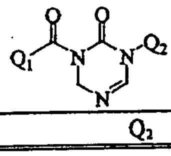
(Ie)

| compound No. | $Q_1$ | $Q_2$ | m. p. (℃) |
|---|---|---|---|
| V-1 | 2,6-$F_2$-Ph | 4-Cl-Ph | 151-154 |
| V-2 | 2,6-$F_2$-Ph | 3,5-$Cl_2$-4-(2-Cl-4-$CF_3$-pyridin-2yl-O)-Ph | |
| V-3 | 2,6-$F_2$-Ph | 2-F-4-(2-Cl-4-$CF_3$-Ph-O)-Ph | resinoid (1) |
| V-4 | 2,6-$F_2$-Ph | 3,5-$Cl_2$-2,4-$F_2$-Ph | oil (2) |
| V-5 | 2,6-$F_2$-Ph | 3,5-$Cl_2$-4-$CF_2HCF_2O$-Ph | |
| V-6 | 2,6-$F_2$-Ph | 2,5-$Cl_2$-4-$CF_3CFHCF_2O$-Ph | |
| V-7 | 2,6-$F_2$-Ph | 2-F-4-$CFClHCF_2S$-Ph | |
| V-8 | 2,6-$F_2$-Ph | 2-F-4-$CFClHCF_2SO$-Ph | |
| V-9 | 2,6-$F_2$-Ph | 2-F-4-$CFClHCF_2SO_2$-Ph | |
| V-10 | 2,6-$F_2$-Ph | 4-$CFClHCF_2S$-Ph | |
| V-11 | 2,6-$F_2$-Ph | 4-$CFClHCF_2SO$-Ph | |
| V-12 | 2,6-$F_2$-Ph | 4-$CFClHCF_2SO_2$-Ph | |
| V-13 | 2,6-$F_2$-Ph | 2-Cl-4-$CFClHCF_2S$-Ph | |
| V-14 | 2,6-$F_2$-Ph | 2-Cl-4-$CFClHCF_2SO$-Ph | |
| V-15 | 2,6-$F_2$-Ph | 2-Cl-4-$CFClHCF_2SO_2$-Ph | |
| V-16 | 2,6-$F_2$-Ph | 2-Cl-4-$CF_2HCF_2S$-Ph | |
| V-17 | 2,6-$F_2$-Ph | 2-Cl-4-$CF_2HCF_2SO$-Ph | |
| V-18 | 2,6-$F_2$-Ph | 2-Cl-4-$CF_2HCF_2SO_2$-Ph | |
| V-19 | 2,6-$F_2$-Ph | 4-$CF_2HCF_2S$-Ph | |
| V-20 | 2,6-$F_2$-Ph | 4-$CF_2HCF_2SO$-Ph | |
| V-21 | 2,6-$F_2$-Ph | 4-$CF_2HCF_2SO_2$-Ph | |
| V-22 | 2,6-$F_2$-Ph | 2-F-4-$CF_2HCF_2S$-Ph | |
| V-23 | 2,6-$F_2$-Ph | 2-F-4-$CF_2HCF_2SO$-Ph | |
| V-24 | 2,6-$F_2$-Ph | 2-F-4-$CF_2HCF_2SO_2$-Ph | |
| V-25 | 2,6-$F_2$-Ph | 4-$CF_3S$-Ph | |
| V-26 | 2,6-$F_2$-Ph | 4-$CF_3SO$-Ph | |
| V-27 | 2,6-$F_2$-Ph | 4-$CF_3SO_2$-Ph | |
| V-28 | 2,6-$F_2$-Ph | 2-F-4-MeS-Ph | |
| V-29 | 2,6-$F_2$-Ph | 2-F-4-MeSO-Ph | |
| V-30 | 2,6-$F_2$-Ph | 2-F-4-$MeSO_2$-Ph | |
| V-31 | 2,6-$F_2$-Ph | 2,6-$F_2$-4-MeS-Ph | |
| V-32 | 2,6-$F_2$-Ph | 2,6-$F_2$-4-MeSO-Ph | |
| V-33 | 2,6-$F_2$-Ph | 2,6-$F_2$-4-$MeSO_2$-Ph | |
| V-34 | 2,6-$F_2$-Ph | 2-F-4-$CF_3CH_2S$-Ph | |
| V-35 | 2,6-$F_2$-Ph | 2-F-4-$CF_3CH_2SO$-Ph | |
| V-36 | 2,6-$F_2$-Ph | 2-F-4-$CF_3CH_2SO_2$-Ph | |
| V-37 | 2,6-$F_2$-Ph | 2-F-4-$CCl_2$=$CHCH_2S$-Ph | |
| V-38 | 2,6-$F_2$-Ph | 2-F-4-$CCl_2$=$CHCH_2SO$-Ph | |
| V-39 | 2,6-$F_2$-Ph | 2-F-4-$CCl_2$=$CHCH_2SO_2$-Ph | |
| V-40 | 2,6-$F_2$-Ph | $CF_3CH_2$ | |

(1) $H^1$-NMR ($CDCl_3$)δppm : 5.60 ( 2H, d J=1.9Hz, $NCH_2N$), 6.77-7.00 ( 4H, m, Ph), 7.14 ( 1H, d J=8.4Hz, Ph), 7.26-7.35 ( 3H, m, NCHN+Ph), 7.54 ( 1H, d J=8.1Hz, Ph), 7.78 ( 1H, s, Ph).
(2) $H^1$-NMR ($CDCl_3$)δppm : 5.60 ( 2H, d J=2.0Hz, $NCH_2N$),6.88-6.97 ( 2H, m, Ph), 7.24-7.42 ( 3H, m, Ph+NCHN) .

| compound No. | $Q_1$ | $Q_2$ | m. p. (°C) |
|---|---|---|---|
| V-41 | 2,6-$F_2$-Ph | 2-F-4-$CF_2HS$-Ph | |
| V-42 | 2,6-$F_2$-Ph | 2-F-4-$CF_2HSO$-Ph | |
| V-43 | 2,6-$F_2$-Ph | 2-F-4-$CF_2HSO_2$-Ph | |
| V-44 | 2,6-$F_2$-Ph | 2-F-4-$CF_3S$-Ph | |
| V-45 | 2,6-$F_2$-Ph | 2-F-4-$CF_3SO$-Ph | |
| V-46 | 2,6-$F_2$-Ph | 2-F-4-$CF_3SO_2$-Ph | |
| V-47 | 2,6-$F_2$-Ph | 2-F-4-$CH_3CH_2S$-Ph | |
| V-48 | 2,6-$F_2$-Ph | 2-F-4-$CH_3CH_2SO$-Ph | |
| V-49 | 2,6-$F_2$-Ph | 2-F-4-$CH_3CH_2SO_2$-Ph | |
| V-50 | 2,6-$F_2$-Ph | 2-F-4-$CH_3CH_2CH_2S$-Ph | |
| V-51 | 2,6-$F_2$-Ph | 2-F-4-$CH_3CH_2CH_2SO$-Ph | |
| V-52 | 2,6-$F_2$-Ph | 2-F-4-$CH_3CH_2CH_2SO_2$-Ph | |
| V-53 | 2,6-$F_2$-Ph | 2-F-4-i-PrS-Ph | |
| V-54 | 2,6-$F_2$-Ph | 2-F-4-i-PrSO-Ph | |
| V-55 | 2,6-$F_2$-Ph | 2-F-4-i-$PrSO_2$-Ph | |
| V-56 | 2,6-$F_2$-Ph | 2-F-4-t-BuS-Ph | |
| V-57 | 2,6-$F_2$-Ph | 2-F-4-t-BuSO-Ph | |
| V-58 | 2,6-$F_2$-Ph | 2-F-4-t-$BuSO_2$-Ph | |
| V-59 | 2,6-$F_2$-Ph | 2-F-4-$CH_2$=$CHCH_2S$-Ph | |
| V-60 | 2,6-$F_2$-Ph | 2-F-4-$CH_2$=$CHCH_2SO$-Ph | |
| V-61 | 2,6-$F_2$-Ph | 2-F-4-$CH_2$=$CHCH_2SO_2$-Ph | |
| V-62 | 2,6-$F_2$-Ph | 2-F-4-CH≡$CCH_2S$-Ph | |
| V-63 | 2,6-$F_2$-Ph | 2-F-4-CH≡$CCH_2SO$-Ph | |
| V-64 | 2,6-$F_2$-Ph | 2-F-4-CH≡$CCH_2SO_2$-Ph | |
| V-65 | 2,6-$F_2$-Ph | 2-F-4-(2-Cl-4-$CF_3$-pyridin-2yl-S)-Ph | |
| V-66 | 2,6-$F_2$-Ph | 2-F-4-(2-Cl-4-$CF_3$-pyridin-2yl-SO)-Ph | |
| V-67 | 2,6-$F_2$-Ph | 2-F-4-(2-Cl-4-$CF_3$-pyridin-2yl-$SO_2$)-Ph | |
| V-68 | 2,6-$F_2$-Ph | 2-Me-4-$CF_3S$-Ph | |
| V-69 | 2,6-$F_2$-Ph | 2-Me-4-$CF_3SO$-Ph | |
| V-70 | 2,6-$F_2$-Ph | 2-Me-4-$CF_3SO_2$-Ph | |
| V-71 | 2,6-$F_2$-Ph | 3-Me-4-$CF_3S$-Ph | |
| V-72 | 2,6-$F_2$-Ph | 3-Me-4-$CF_3SO$-Ph | |
| V-73 | 2,6-$F_2$-Ph | 3-Me-4-$CF_3SO_2$-Ph | |
| V-74 | 2,6-$F_2$-Ph | 2-Cl-4-$CF_3S$-Ph | |
| V-75 | 2,6-$F_2$-Ph | 2-Cl-4-$CF_3SO$-Ph | |
| V-76 | 2,6-$F_2$-Ph | 2-Cl-4-$CF_3SO_2$-Ph | |
| V-77 | 2,6-$F_2$-Ph | 3-Cl-4-$CF_3S$-Ph | |
| V-78 | 2,6-$F_2$-Ph | 3-Cl-4-$CF_3SO$-Ph | |
| V-79 | 2,6-$F_2$-Ph | 3-Cl-4-$CF_3SO_2$-Ph | |
| V-80 | 2,6-$F_2$-Ph | 3-F-4-$CF_3S$-Ph | |

| compound No. | $Q_1$ | $Q_2$ | m. p. (°C) |
|---|---|---|---|
| V-81 | 2,6-$F_2$-Ph | 3-F-4-$CF_3$SO-Ph | |
| V-82 | 2,6-$F_2$-Ph | 3-F-4-$CF_3SO_2$-Ph | |
| V-83 | 2,6-$F_2$-Ph | 2,3-$Me_2$-4-$CF_3$S-Ph | |
| V-84 | 2,6-$F_2$-Ph | 2,3-$Me_2$-4-$CF_3$SO-Ph | |
| V-85 | 2,6-$F_2$-Ph | 2,3-$Me_2$-4-$CF_3SO_2$-Ph | |
| V-86 | 2,6-$F_2$-Ph | 2,5-$Me_2$-4-$CF_3$S-Ph | |
| V-87 | 2,6-$F_2$-Ph | 2,5-$Me_2$-4-$CF_3$SO-Ph | |
| V-88 | 2,6-$F_2$-Ph | 2,5-$Me_2$-4-$CF_3SO_2$-Ph | |
| V-89 | 2,6-$F_2$-Ph | 5-Cl-2-Me-4-$CF_3$S-Ph | |
| V-90 | 2,6-$F_2$-Ph | 5-Cl-2-Me-4-$CF_3$SO-Ph | |
| V-91 | 2,6-$F_2$-Ph | 5-Cl-2-Me-4-$CF_3SO_2$-Ph | |
| V-92 | 2,6-$F_2$-Ph | 2,3-$Cl_2$-4-$CF_3$S-Ph | |
| V-93 | 2,6-$F_2$-Ph | 2,3-$Cl_2$-4-$CF_3$SO-Ph | |
| V-94 | 2,6-$F_2$-Ph | 2,3-$Cl_2$-4-$CF_3SO_2$-Ph | |
| V-95 | 2,6-$F_2$-Ph | 3-Cl-2-F-4-$CF_3$S-Ph | |
| V-96 | 2,6-$F_2$-Ph | 3-Cl-2-F-4-$CF_3$SO-Ph | |
| V-97 | 2,6-$F_2$-Ph | 3-Cl-2-F-4-$CF_3SO_2$-Ph | |
| V-98 | 2,6-$F_2$-Ph | 5-Cl-2-F-4-$CF_3$S-Ph | |
| V-99 | 2,6-$F_2$-Ph | 5-Cl-2-F-4-$CF_3$SO-Ph | |
| V-100 | 2,6-$F_2$-Ph | 5-Cl-2-F-4-$CF_3SO_2$-Ph | |
| V-101 | 2,6-$F_2$-Ph | 2,6-$F_2$-4-$CF_3$S-Ph | |
| V-102 | 2,6-$F_2$-Ph | 2,6-$F_2$-4-$CF_3$SO-Ph | |
| V-103 | 2,6-$F_2$-Ph | 2,6-$F_2$-4-$CF_3SO_2$-Ph | |
| V-104 | 2,6-$F_2$-Ph | 2,3-$F_2$-4-$CF_3$S-Ph | |
| V-105 | 2,6-$F_2$-Ph | 2,3-$F_2$-4-$CF_3$SO-Ph | |
| V-106 | 2,6-$F_2$-Ph | 2,3-$F_2$-4-$CF_3SO_2$-Ph | |
| V-107 | 2,6-$F_2$-Ph | 2,5-$F_2$-4-$CF_3$S-Ph | |
| V-108 | 2,6-$F_2$-Ph | 2,5-$F_2$-4-$CF_3$SO-Ph | |
| V-109 | 2,6-$F_2$-Ph | 2,5-$F_2$-4-$CF_3SO_2$-Ph | |
| V-110 | 2,6-$F_2$-Ph | 3,5-$F_2$-4-$CF_3$S-Ph | |
| V-111 | 2,6-$F_2$-Ph | 3,5-$F_2$-4-$CF_3$SO-Ph | |
| V-112 | 2,6-$F_2$-Ph | 3,5-$F_2$-4-$CF_3SO_2$-Ph | |
| V-113 | 2,6-$F_2$-Ph | 2,3,6-$F_3$-4-$CF_3$S-Ph | |
| V-114 | 2,6-$F_2$-Ph | 2,3,6-$F_3$-4-$CF_3$SO-Ph | |
| V-115 | 2,6-$F_2$-Ph | 2,3,6-$F_3$-4-$CF_3SO_2$-Ph | |
| V-116 | 2,6-$F_2$-Ph | 2,3,5,6-$F_4$-4-$CF_3$S-Ph | |
| V-117 | 2,6-$F_2$-Ph | 2,3,5,6-$F_4$-4-$CF_3$SO-Ph | |
| V-118 | 2,6-$F_2$-Ph | 2,3,5,6-$F_4$-4-$CF_3SO_2$-Ph | |
| V-119 | 2,6-$F_2$-Ph | 2-F-4-$CF_3CF_2$S-Ph | |
| V-120 | 2,6-$F_2$-Ph | 2-F-4-$CF_3CF_2$SO-Ph | |

| compound No. | $Q_1$ | $Q_2$ | m. p. (°C) |
|---|---|---|---|
| V - 121 | 2,6-$F_2$-Ph | 2-F-4-$CF_3CF_2SO_2$-Ph | |
| V - 122 | 2,6-$F_2$-Ph | 2-Cl-4-$CF_3CF_2S$-Ph | |
| V - 123 | 2,6-$F_2$-Ph | 2-Cl-4-$CF_3CF_2SO$-Ph | |
| V - 124 | 2,6-$F_2$-Ph | 2-Cl-4-$CF_3CF_2SO_2$-Ph | |
| V - 125 | 2,6-$F_2$-Ph | 2-Me-4-$CF_3CF_2S$-Ph | |
| V - 126 | 2,6-$F_2$-Ph | 2-Me-4-$CF_3CF_2SO$-Ph | |
| V - 127 | 2,6-$F_2$-Ph | 2-Me-4-$CF_3CF_2SO_2$-Ph | |
| V - 128 | 2,6-$F_2$-Ph | 5-Cl-2-Me-4-$CF_3CF_2S$-Ph | |
| V - 129 | 2,6-$F_2$-Ph | 5-Cl-2-Me-4-$CF_3CF_2SO$-Ph | |
| V - 130 | 2,6-$F_2$-Ph | 5-Cl-2-Me-4-$CF_3CF_2SO_2$-Ph | |
| V - 131 | 2,6-$F_2$-Ph | 2,3-$Me_2$-4-$CF_3CF_2S$-Ph | |
| V - 132 | 2,6-$F_2$-Ph | 2,3-$Me_2$-4-$CF_3CF_2SO$-Ph | |
| V - 133 | 2,6-$F_2$-Ph | 2,3-$Me_2$-4-$CF_3CF_2SO_2$-Ph | |
| V - 134 | 2,6-$F_2$-Ph | 2-F-4-$CF_3CF_2CF_2S$-Ph | |
| V - 135 | 2,6-$F_2$-Ph | 2-F-4-$CF_3CF_2CF_2SO$-Ph | |
| V - 136 | 2,6-$F_2$-Ph | 2-F-4-$CF_3CF_2CF_2SO_2$-Ph | |
| V - 137 | 2,6-$F_2$-Ph | 2-Cl-4-$CF_3CF_2CF_2S$-Ph | |
| V - 138 | 2,6-$F_2$-Ph | 2-Cl-4-$CF_3CF_2CF_2SO$-Ph | |
| V - 139 | 2,6-$F_2$-Ph | 2-Cl-4-$CF_3CF_2CF_2SO_2$-Ph | |
| V - 140 | 2,6-$F_2$-Ph | 2-Me-4-$CF_3CF_2CF_2S$-Ph | |
| V - 141 | 2,6-$F_2$-Ph | 2-Me-4-$CF_3CF_2CF_2SO$-Ph | |
| V - 142 | 2,6-$F_2$-Ph | 2-Me-4-$CF_3CF_2CF_2SO_2$-Ph | |
| V - 143 | 2,6-$F_2$-Ph | 5-Cl-2-Me-4-$CF_3CF_2CF_2S$-Ph | |
| V - 144 | 2,6-$F_2$-Ph | 5-Cl-2-Me-4-$CF_3CF_2CF_2SO$-Ph | |
| V - 145 | 2,6-$F_2$-Ph | 5-Cl-2-Me-4-$CF_3CF_2CF_2SO_2$-Ph | |
| V - 146 | 2,6-$F_2$-Ph | 2,3-$Me_2$-4-$CF_3CF_2CF_2S$-Ph | |
| V - 147 | 2,6-$F_2$-Ph | 2,3-$Me_2$-4-$CF_3CF_2CF_2SO$-Ph | |
| V - 148 | 2,6-$F_2$-Ph | 2,3-$Me_2$-4-$CF_3CF_2CF_2SO_2$-Ph | |
| V - 149 | 2,6-$F_2$-Ph | 2-F-4-$(CF_3)_2CFS$-Ph | |
| V - 150 | 2,6-$F_2$-Ph | 2-F-4-$(CF_3)_2CFSO$-Ph | |
| V - 151 | 2,6-$F_2$-Ph | 2-F-4-$(CF_3)_2CFSO_2$-Ph | |
| V - 152 | 2,6-$F_2$-Ph | 2-Me-4-$(CF_3)_2CFS$-Ph | |
| V - 153 | 2,6-$F_2$-Ph | 2-Me-4-$(CF_3)_2CFSO$-Ph | |
| V - 154 | 2,6-$F_2$-Ph | 2-Me-4-$(CF_3)_2CFSO_2$-Ph | |
| V - 155 | 2,6-$F_2$-Ph | 2-Cl-4-$(CF_3)_2CFS$-Ph | |
| V - 156 | 2,6-$F_2$-Ph | 2-Cl-4-$(CF_3)_2CFSO$-Ph | |
| V - 157 | 2,6-$F_2$-Ph | 2-Cl-4-$(CF_3)_2CFSO_2$-Ph | |
| V - 158 | 2,6-$F_2$-Ph | 5-Cl-2-Me-4-$(CF_3)_2CFS$-Ph | |
| V - 159 | 2,6-$F_2$-Ph | 5-Cl-2-Me-4-$(CF_3)_2CFSO$-Ph | |
| V - 160 | 2,6-$F_2$-Ph | 5-Cl-2-Me-4-$(CF_3)_2CFSO_2$-Ph | |

| compound No. | $Q_1$ | $Q_2$ | m. p. (℃) |
|---|---|---|---|
| V-161 | 2,6-$F_2$-Ph | 2,3-$Me_2$-4-$(CF_3)_2$CFS-Ph | |
| V-162 | 2,6-$F_2$-Ph | 2,3-$Me_2$-4-$(CF_3)_2$CFSO-Ph | |
| V-163 | 2,6-$F_2$-Ph | 2,3-$Me_2$-4-$(CF_3)_2$CF$SO_2$-Ph | |
| V-164 | 2,6-$F_2$-Ph | 2-F-4-MeO-Ph | |
| V-165 | 2,6-$F_2$-Ph | 2-Me-4-MeO-Ph | |
| V-166 | 2,6-$F_2$-Ph | 2-F-4-CN-Ph | |
| V-167 | 2,6-$F_2$-Ph | 2-F-4-COOMe-Ph | |
| V-168 | 2,6-$F_2$-Ph | 2-F-4-COOEt-Ph | |
| V-169 | 2-Cl-6-F-Ph | 4-Cl-Ph | |
| V-170 | 2-Cl-6-F-Ph | 3,5-$Cl_2$-4-(2-Cl-4-$CF_3$-pyridin-2yl-O)-Ph | |
| V-171 | 2-Cl-6-F-Ph | 2-F-4-(2-Cl-4-$CF_3$-Ph-O)-Ph | |
| V-172 | 2-Cl-6-F-Ph | 3,5-$Cl_2$-2,4-$F_2$-Ph | |
| V-173 | 2-Cl-6-F-Ph | 3,5-$Cl_2$-4-$CF_2HCF_2O$-Ph | |
| V-174 | 2-Cl-6-F-Ph | 2,5-$Cl_2$-4-$CF_3CFHCF_2O$-Ph | |
| V-175 | 2-Cl-6-F-Ph | 2-F-4-CFClH$CF_2$S-Ph | |
| V-176 | 2-Cl-6-F-Ph | 2-F-4-CFClH$CF_2$SO-Ph | |
| V-177 | 2-Cl-6-F-Ph | 2-F-4-CFClH$CF_2SO_2$-Ph | |
| V-178 | 2-Cl-6-F-Ph | 4-CFClH$CF_2$S-Ph | |
| V-179 | 2-Cl-6-F-Ph | 4-CFClH$CF_2$SO-Ph | |
| V-180 | 2-Cl-6-F-Ph | 4-CFClH$CF_2SO_2$-Ph | |
| V-181 | 2-Cl-6-F-Ph | 2-Cl-4-CFClH$CF_2$S-Ph | |
| V-182 | 2-Cl-6-F-Ph | 2-Cl-4-CFClH$CF_2$SO-Ph | |
| V-183 | 2-Cl-6-F-Ph | 2-Cl-4-CFClH$CF_2SO_2$-Ph | |
| V-184 | 2-Cl-6-F-Ph | 2-Cl-4-$CF_2HCF_2$S-Ph | |
| V-185 | 2-Cl-6-F-Ph | 2-Cl-4-$CF_2HCF_2$SO-Ph | |
| V-186 | 2-Cl-6-F-Ph | 2-Cl-4-$CF_2HCF_2SO_2$-Ph | |
| V-187 | 2-Cl-6-F-Ph | 4-$CF_2HCF_2$S-Ph | |
| V-188 | 2-Cl-6-F-Ph | 4-$CF_2HCF_2$SO-Ph | |
| V-189 | 2-Cl-6-F-Ph | 4-$CF_2HCF_2SO_2$-Ph | |
| V-190 | 2-Cl-6-F-Ph | 2-F-4-$CF_2HCF_2$S-Ph | |
| V-191 | 2-Cl-6-F-Ph | 2-F-4-$CF_2HCF_2$SO-Ph | |
| V-192 | 2-Cl-6-F-Ph | 2-F-4-$CF_2HCF_2SO_2$-Ph | |
| V-193 | 2-Cl-6-F-Ph | 4-$CF_3$S-Ph | |
| V-194 | 2-Cl-6-F-Ph | 4-$CF_3$SO-Ph | |
| V-195 | 2-Cl-6-F-Ph | 4-$CF_3SO_2$-Ph | |
| V-196 | 2-Cl-6-F-Ph | 2-F-4-MeS-Ph | |
| V-197 | 2-Cl-6-F-Ph | 2-F-4-MeSO-Ph | |
| V-198 | 2-Cl-6-F-Ph | 2-F-4-Me$SO_2$-Ph | |
| V-199 | 2-Cl-6-F-Ph | 2,6-$F_2$-4-MeS-Ph | |
| V-200 | 2-Cl-6-F-Ph | 2,6-$F_2$-4-MeSO-Ph | |

| compound No. | $Q_1$ | $Q_2$ | m.p. (°C) |
|---|---|---|---|
| V-201 | 2-Cl-6-F-Ph | 2,6-$F_2$-4-$MeSO_2$-Ph | |
| V-202 | 2-Cl-6-F-Ph | 2-F-4-$CF_3CH_2S$-Ph | |
| V-203 | 2-Cl-6-F-Ph | 2-F-4-$CF_3CH_2SO$-Ph | |
| V-204 | 2-Cl-6-F-Ph | 2-F-4-$CF_3CH_2SO_2$-Ph | |
| V-205 | 2-Cl-6-F-Ph | 2-F-4-$CCl_2$=$CHCH_2S$-Ph | |
| V-206 | 2-Cl-6-F-Ph | 2-F-4-$CCl_2$=$CHCH_2SO$-Ph | |
| V-207 | 2-Cl-6-F-Ph | 2-F-4-$CCl_2$=$CHCH_2SO_2$-Ph | |
| V-208 | 2-Cl-6-F-Ph | $CF_3CH_2$ | |
| V-209 | 2-Cl-6-F-Ph | 2-F-4-$CF_2HS$-Ph | |
| V-210 | 2-Cl-6-F-Ph | 2-F-4-$CF_2HSO$-Ph | |
| V-211 | 2-Cl-6-F-Ph | 2-F-4-$CF_2HSO_2$-Ph | |
| V-212 | 2-Cl-6-F-Ph | 2-F-4-$CF_3S$-Ph | |
| V-213 | 2-Cl-6-F-Ph | 2-F-4-$CF_3SO$-Ph | |
| V-214 | 2-Cl-6-F-Ph | 2-F-4-$CF_3SO_2$-Ph | |
| V-215 | 2-Cl-6-F-Ph | 2-F-4-$CH_3CH_2S$-Ph | |
| V-216 | 2-Cl-6-F-Ph | 2-F-4-$CH_3CH_2SO$-Ph | |
| V-217 | 2-Cl-6-F-Ph | 2-F-4-$CH_3CH_2SO_2$-Ph | |
| V-218 | 2-Cl-6-F-Ph | 2-F-4-$CH_3CH_2CH_2S$-Ph | |
| V-219 | 2-Cl-6-F-Ph | 2-F-4-$CH_3CH_2CH_2SO$-Ph | |
| V-220 | 2-Cl-6-F-Ph | 2-F-4-$CH_3CH_2CH_2SO_2$-Ph | |
| V-221 | 2-Cl-6-F-Ph | 2-F-4-i-PrS-Ph | |
| V-222 | 2-Cl-6-F-Ph | 2-F-4-i-PrSO-Ph | |
| V-223 | 2-Cl-6-F-Ph | 2-F-4-i-$PrSO_2$-Ph | |
| V-224 | 2-Cl-6-F-Ph | 2-F-4-t-BuS-Ph | |
| V-225 | 2-Cl-6-F-Ph | 2-F-4-t-BuSO-Ph | |
| V-226 | 2-Cl-6-F-Ph | 2-F-4-t-$BuSO_2$-Ph | |
| V-227 | 2-Cl-6-F-Ph | 2-F-4-$CH_2$=$CHCH_2S$-Ph | |
| V-228 | 2-Cl-6-F-Ph | 2-F-4-$CH_2$=$CHCH_2SO$-Ph | |
| V-229 | 2-Cl-6-F-Ph | 2-F-4-$CH_2$=$CHCH_2SO_2$-Ph | |
| V-230 | 2-Cl-6-F-Ph | 2-F-4-$CH\equiv CCH_2S$-Ph | |
| V-231 | 2-Cl-6-F-Ph | 2-F-4-$CH\equiv CCH_2SO$-Ph | |
| V-232 | 2-Cl-6-F-Ph | 2-F-4-$CH\equiv CCH_2SO_2$-Ph | |
| V-233 | 2-Cl-6-F-Ph | 2-F-4-(2-Cl-4-$CF_3$-pyridin-2yl-S)-Ph | |
| V-234 | 2-Cl-6-F-Ph | 2-F-4-(2-Cl-4-$CF_3$-pyridin-2yl-SO)-Ph | |
| V-235 | 2-Cl-6-F-Ph | 2-F-4-(2-Cl-4-$CF_3$-pyridin-2yl-$SO_2$)-Ph | |
| V-236 | 2-Cl-6-F-Ph | 2-Me-4-$CF_3S$-Ph | |
| V-237 | 2-Cl-6-F-Ph | 2-Me-4-$CF_3SO$-Ph | |
| V-238 | 2-Cl-6-F-Ph | 2-Me-4-$CF_3SO_2$-Ph | |
| V-239 | 2-Cl-6-F-Ph | 3-Me-4-$CF_3S$-Ph | |
| V-240 | 2-Cl-6-F-Ph | 3-Me-4-$CF_3SO$-Ph | |

| compound No. | $Q_1$ | $Q_2$ | m. p. (°C) |
|---|---|---|---|
| V-241 | 2-Cl-6-F-Ph | 3-Me-4-$CF_3SO_2$-Ph | |
| V-242 | 2-Cl-6-F-Ph | 2-Cl-4-$CF_3S$-Ph | |
| V-243 | 2-Cl-6-F-Ph | 2-Cl-4-$CF_3SO$-Ph | |
| V-244 | 2-Cl-6-F-Ph | 2-Cl-4-$CF_3SO_2$-Ph | |
| V-245 | 2-Cl-6-F-Ph | 3-Cl-4-$CF_3S$-Ph | |
| V-246 | 2-Cl-6-F-Ph | 3-Cl-4-$CF_3SO$-Ph | |
| V-247 | 2-Cl-6-F-Ph | 3-Cl-4-$CF_3SO_2$-Ph | |
| V-248 | 2-Cl-6-F-Ph | 3-F-4-$CF_3S$-Ph | |
| V-249 | 2-Cl-6-F-Ph | 3-F-4-$CF_3SO$-Ph | |
| V-250 | 2-Cl-6-F-Ph | 3-F-4-$CF_3SO_2$-Ph | |
| V-251 | 2-Cl-6-F-Ph | 2,3-$Me_2$-4-$CF_3S$-Ph | |
| V-252 | 2-Cl-6-F-Ph | 2,3-$Me_2$-4-$CF_3SO$-Ph | |
| V-253 | 2-Cl-6-F-Ph | 2,3-$Me_2$-4-$CF_3SO_2$-Ph | |
| V-254 | 2-Cl-6-F-Ph | 2,5-$Me_2$-4-$CF_3S$-Ph | |
| V-255 | 2-Cl-6-F-Ph | 2,5-$Me_2$-4-$CF_3SO$-Ph | |
| V-256 | 2-Cl-6-F-Ph | 2,5-$Me_2$-4-$CF_3SO_2$-Ph | |
| V-257 | 2-Cl-6-F-Ph | 5-Cl-2-Me-4-$CF_3S$-Ph | |
| V-258 | 2-Cl-6-F-Ph | 5-Cl-2-Me-4-$CF_3SO$-Ph | |
| V-259 | 2-Cl-6-F-Ph | 5-Cl-2-Me-4-$CF_3SO_2$-Ph | |
| V-260 | 2-Cl-6-F-Ph | 2,3-$Cl_2$-4-$CF_3S$-Ph | |
| V-261 | 2-Cl-6-F-Ph | 2,3-$Cl_2$-4-$CF_3SO$-Ph | |
| V-262 | 2-Cl-6-F-Ph | 2,3-$Cl_2$-4-$CF_3SO_2$-Ph | |
| V-263 | 2-Cl-6-F-Ph | 3-Cl-2-F-4-$CF_3S$-Ph | |
| V-264 | 2-Cl-6-F-Ph | 3-Cl-2-F-4-$CF_3SO$-Ph | |
| V-265 | 2-Cl-6-F-Ph | 3-Cl-2-F-4-$CF_3SO_2$-Ph | |
| V-266 | 2-Cl-6-F-Ph | 5-Cl-2-F-4-$CF_3S$-Ph | |
| V-267 | 2-Cl-6-F-Ph | 5-Cl-2-F-4-$CF_3SO$-Ph | |
| V-268 | 2-Cl-6-F-Ph | 5-Cl-2-F-4-$CF_3SO_2$-Ph | |
| V-269 | 2-Cl-6-F-Ph | 2,6-$F_2$-4-$CF_3S$-Ph | |
| V-270 | 2-Cl-6-F-Ph | 2,6-$F_2$-4-$CF_3SO$-Ph | |
| V-271 | 2-Cl-6-F-Ph | 2,6-$F_2$-4-$CF_3SO_2$-Ph | |
| V-272 | 2-Cl-6-F-Ph | 2,3-$F_2$-4-$CF_3S$-Ph | |
| V-273 | 2-Cl-6-F-Ph | 2,3-$F_2$-4-$CF_3SO$-Ph | |
| V-274 | 2-Cl-6-F-Ph | 2,3-$F_2$-4-$CF_3SO_2$-Ph | |
| V-275 | 2-Cl-6-F-Ph | 2,5-$F_2$-4-$CF_3S$-Ph | |
| V-276 | 2-Cl-6-F-Ph | 2,5-$F_2$-4-$CF_3SO$-Ph | |
| V-277 | 2-Cl-6-F-Ph | 2,5-$F_2$-4-$CF_3SO_2$-Ph | |
| V-278 | 2-Cl-6-F-Ph | 3,5-$F_2$-4-$CF_3S$-Ph | |
| V-279 | 2-Cl-6-F-Ph | 3,5-$F_2$-4-$CF_3SO$-Ph | |
| V-280 | 2-Cl-6-F-Ph | 3,5-$F_2$-4-$CF_3SO_2$-Ph | |

| compound No. | $Q_1$ | $Q_2$ | m. p. (°C) |
|---|---|---|---|
| V-281 | 2-Cl-6-F-Ph | 2,3,6-$F_3$-4-$CF_3S$-Ph | |
| V-282 | 2-Cl-6-F-Ph | 2,3,6-$F_3$-4-$CF_3SO$-Ph | |
| V-283 | 2-Cl-6-F-Ph | 2,3,6-$F_3$-4-$CF_3SO_2$-Ph | |
| V-284 | 2-Cl-6-F-Ph | 2,3,5,6-$F_4$-4-$CF_3S$-Ph | |
| V-285 | 2-Cl-6-F-Ph | 2,3,5,6-$F_4$-4-$CF_3SO$-Ph | |
| V-286 | 2-Cl-6-F-Ph | 2,3,5,6-$F_4$-4-$CF_3SO_2$-Ph | |
| V-287 | 2-Cl-6-F-Ph | 2-F-4-$CF_3CF_2S$-Ph | |
| V-288 | 2-Cl-6-F-Ph | 2-F-4-$CF_3CF_2SO$-Ph | |
| V-289 | 2-Cl-6-F-Ph | 2-F-4-$CF_3CF_2SO_2$-Ph | |
| V-290 | 2-Cl-6-F-Ph | 2-Cl-4-$CF_3CF_2S$-Ph | |
| V-291 | 2-Cl-6-F-Ph | 2-Cl-4-$CF_3CF_2SO$-Ph | |
| V-292 | 2-Cl-6-F-Ph | 2-Cl-4-$CF_3CF_2SO_2$-Ph | |
| V-293 | 2-Cl-6-F-Ph | 2-Me-4-$CF_3CF_2S$-Ph | |
| V-294 | 2-Cl-6-F-Ph | 2-Me-4-$CF_3CF_2SO$-Ph | |
| V-295 | 2-Cl-6-F-Ph | 2-Me-4-$CF_3CF_2SO_2$-Ph | |
| V-296 | 2-Cl-6-F-Ph | 5-Cl-2-Me-4-$CF_3CF_2S$-Ph | |
| V-297 | 2-Cl-6-F-Ph | 5-Cl-2-Me-4-$CF_3CF_2SO$-Ph | |
| V-298 | 2-Cl-6-F-Ph | 5-Cl-2-Me-4-$CF_3CF_2SO_2$-Ph | |
| V-299 | 2-Cl-6-F-Ph | 2,3-$Me_2$-4-$CF_3CF_2S$-Ph | |
| V-300 | 2-Cl-6-F-Ph | 2,3-$Me_2$-4-$CF_3CF_2SO$-Ph | |
| V-301 | 2-Cl-6-F-Ph | 2,3-$Me_2$-4-$CF_3CF_2SO_2$-Ph | |
| V-302 | 2-Cl-6-F-Ph | 2-F-4-$CF_3CF_2CF_2S$-Ph | |
| V-303 | 2-Cl-6-F-Ph | 2-F-4-$CF_3CF_2CF_2SO$-Ph | |
| V-304 | 2-Cl-6-F-Ph | 2-F-4-$CF_3CF_2CF_2SO_2$-Ph | |
| V-305 | 2-Cl-6-F-Ph | 2-Cl-4-$CF_3CF_2CF_2S$-Ph | |
| V-306 | 2-Cl-6-F-Ph | 2-Cl-4-$CF_3CF_2CF_2SO$-Ph | |
| V-307 | 2-Cl-6-F-Ph | 2-Cl-4-$CF_3CF_2CF_2SO_2$-Ph | |
| V-308 | 2-Cl-6-F-Ph | 2-Me-4-$CF_3CF_2CF_2S$-Ph | |
| V-309 | 2-Cl-6-F-Ph | 2-Me-4-$CF_3CF_2CF_2SO$-Ph | |
| V-310 | 2-Cl-6-F-Ph | 2-Me-4-$CF_3CF_2CF_2SO_2$-Ph | |
| V-311 | 2-Cl-6-F-Ph | 5-Cl-2-Me-4-$CF_3CF_2CF_2S$-Ph | |
| V-312 | 2-Cl-6-F-Ph | 5-Cl-2-Me-4-$CF_3CF_2CF_2SO$-Ph | |
| V-313 | 2-Cl-6-F-Ph | 5-Cl-2-Me-4-$CF_3CF_2CF_2SO_2$-Ph | |
| V-314 | 2-Cl-6-F-Ph | 2,3-$Me_2$-4-$CF_3CF_2CF_2S$-Ph | |
| V-315 | 2-Cl-6-F-Ph | 2,3-$Me_2$-4-$CF_3CF_2CF_2SO$-Ph | |
| V-316 | 2-Cl-6-F-Ph | 2,3-$Me_2$-4-$CF_3CF_2CF_2SO_2$-Ph | |
| V-317 | 2-Cl-6-F-Ph | 2-F-4-$(CF_3)_2CFS$-Ph | |
| V-318 | 2-Cl-6-F-Ph | 2-F-4-$(CF_3)_2CFSO$-Ph | |
| V-319 | 2-Cl-6-F-Ph | 2-F-4-$(CF_3)_2CFSO_2$-Ph | |
| V-320 | 2-Cl-6-F-Ph | 2-Me-4-$(CF_3)_2CFS$-Ph | |

| compound No. | $Q_1$ | $Q_2$ | m. p. (°C) |
|---|---|---|---|
| V-321 | 2-Cl-6-F-Ph | 2-Me-4-$(CF_3)_2$CFSO-Ph | |
| V-322 | 2-Cl-6-F-Ph | 2-Me-4-$(CF_3)_2CFSO_2$-Ph | |
| V-323 | 2-Cl-6-F-Ph | 2-Cl-4-$(CF_3)_2$CFS-Ph | |
| V-324 | 2-Cl-6-F-Ph | 2-Cl-4-$(CF_3)_2$CFSO-Ph | |
| V-325 | 2-Cl-6-F-Ph | 2-Cl-4-$(CF_3)_2CFSO_2$-Ph | |
| V-326 | 2-Cl-6-F-Ph | 5-Cl-2-Me-4-$(CF_3)_2$CFS-Ph | |
| V-327 | 2-Cl-6-F-Ph | 5-Cl-2-Me-4-$(CF_3)_2$CFSO-Ph | |
| V-328 | 2-Cl-6-F-Ph | 5-Cl-2-Me-4-$(CF_3)_2CFSO_2$-Ph | |
| V-329 | 2-Cl-6-F-Ph | 2,3-$Me_2$-4-$(CF_3)_2$CFS-Ph | |
| V-330 | 2-Cl-6-F-Ph | 2,3-$Me_2$-4-$(CF_3)_2$CFSO-Ph | |
| V-331 | 2-Cl-6-F-Ph | 2,3-$Me_2$-4-$(CF_3)_2CFSO_2$-Ph | |
| V-332 | 2-Cl-6-F-Ph | 2-F-4-MeO-Ph | |
| V-333 | 2-Cl-6-F-Ph | 2-Me-4-MeO-Ph | |
| V-334 | 2-Cl-6-F-Ph | 2-F-4-CN-Ph | |
| V-335 | 2-Cl-6-F-Ph | 2-F-4-COOMe-Ph | |
| V-336 | 2-Cl-6-F-Ph | 2-F-4-COOEt-Ph | |
| V-337 | 2,6-$Cl_2$-Ph | 4-Cl-Ph | |
| V-338 | 2,6-$Cl_2$-Ph | 3,5-$Cl_2$-4-(2-Cl-4-$CF_3$-pyridin-2yl-O)-Ph | |
| V-339 | 2,6-$Cl_2$-Ph | 2-F-4-(2-Cl-4-$CF_3$-Ph-O)-Ph | |
| V-340 | 2,6-$Cl_2$-Ph | 3,5-$Cl_2$-2,4-$F_2$-Ph | |
| V-341 | 2,6-$Cl_2$-Ph | 3,5-$Cl_2$-4-$CF_2HCF_2$O-Ph | |
| V-342 | 2,6-$Cl_2$-Ph | 2,5-$Cl_2$-4-$CF_3CFHCF_2$O-Ph | |
| V-343 | 2,6-$Cl_2$-Ph | 2-F-4-$CFClHCF_2$S-Ph | |
| V-344 | 2,6-$Cl_2$-Ph | 2-F-4-$CFClHCF_2$SO-Ph | |
| V-345 | 2,6-$Cl_2$-Ph | 2-F-4-$CFClHCF_2SO_2$-Ph | |
| V-346 | 2,6-$Cl_2$-Ph | 4-$CFClHCF_2$S-Ph | |
| V-347 | 2,6-$Cl_2$-Ph | 4-$CFClHCF_2$SO-Ph | |
| V-348 | 2,6-$Cl_2$-Ph | 4-$CFClHCF_2SO_2$-Ph | |
| V-349 | 2,6-$Cl_2$-Ph | 2-Cl-4-$CFClHCF_2$S-Ph | |
| V-350 | 2,6-$Cl_2$-Ph | 2-Cl-4-$CFClHCF_2$SO-Ph | |
| V-351 | 2,6-$Cl_2$-Ph | 2-Cl-4-$CFClHCF_2SO_2$-Ph | |
| V-352 | 2,6-$Cl_2$-Ph | 2-Cl-4-$CF_2HCF_2$S-Ph | |
| V-353 | 2,6-$Cl_2$-Ph | 2-Cl-4-$CF_2HCF_2$SO-Ph | |
| V-354 | 2,6-$Cl_2$-Ph | 2-Cl-4-$CF_2HCF_2SO_2$-Ph | |
| V-355 | 2,6-$Cl_2$-Ph | 4-$CF_2HCF_2$S-Ph | |
| V-356 | 2,6-$Cl_2$-Ph | 4-$CF_2HCF_2$SO-Ph | |
| V-357 | 2,6-$Cl_2$-Ph | 4-$CF_2HCF_2SO_2$-Ph | |
| V-358 | 2,6-$Cl_2$-Ph | 2-F-4-$CF_2HCF_2$S-Ph | |
| V-359 | 2,6-$Cl_2$-Ph | 2-F-4-$CF_2HCF_2$SO-Ph | |
| V-360 | 2,6-$Cl_2$-Ph | 2-F-4-$CF_2HCF_2SO_2$-Ph | |

| compound No. | $Q_1$ | $Q_2$ | m. p. (°C) |
|---|---|---|---|
| V-361 | 2,6-$Cl_2$-Ph | 4-$CF_3$S-Ph | |
| V-362 | 2,6-$Cl_2$-Ph | 4-$CF_3$SO-Ph | |
| V-363 | 2,6-$Cl_2$-Ph | 4-$CF_3SO_2$-Ph | |
| V-364 | 2,6-$Cl_2$-Ph | 2-F-4-MeS-Ph | |
| V-365 | 2,6-$Cl_2$-Ph | 2-F-4-MeSO-Ph | |
| V-366 | 2,6-$Cl_2$-Ph | 2-F-4-$MeSO_2$-Ph | |
| V-367 | 2,6-$Cl_2$-Ph | 2,6-$F_2$-4-MeS-Ph | |
| V-368 | 2,6-$Cl_2$-Ph | 2,6-$F_2$-4-MeSO-Ph | |
| V-369 | 2,6-$Cl_2$-Ph | 2,6-$F_2$-4-$MeSO_2$-Ph | |
| V-370 | 2,6-$Cl_2$-Ph | 2-F-4-$CF_3CH_2$S-Ph | |
| V-371 | 2,6-$Cl_2$-Ph | 2-F-4-$CF_3CH_2$SO-Ph | |
| V-372 | 2,6-$Cl_2$-Ph | 2-F-4-$CF_3CH_2SO_2$-Ph | |
| V-373 | 2,6-$Cl_2$-Ph | 2-F-4-$CCl_2$=$CHCH_2$S-Ph | |
| V-374 | 2,6-$Cl_2$-Ph | 2-F-4-$CCl_2$=$CHCH_2$SO-Ph | |
| V-375 | 2,6-$Cl_2$-Ph | 2-F-4-$CCl_2$=$CHCH_2SO_2$-Ph | |
| V-376 | 2,6-$Cl_2$-Ph | $CF_3CH_2$ | |
| V-377 | 2,6-$Cl_2$-Ph | 2-F-4-$CF_2$HS-Ph | |
| V-378 | 2,6-$Cl_2$-Ph | 2-F-4-$CF_2$HSO-Ph | |
| V-379 | 2,6-$Cl_2$-Ph | 2-F-4-$CF_2HSO_2$-Ph | |
| V-380 | 2,6-$Cl_2$-Ph | 2-F-4-$CF_3$S-Ph | |
| V-381 | 2,6-$Cl_2$-Ph | 2-F-4-$CF_3$SO-Ph | |
| V-382 | 2,6-$Cl_2$-Ph | 2-F-4-$CF_3SO_2$-Ph | |
| V-383 | 2,6-$Cl_2$-Ph | 2-F-4-$CH_3CH_2$S-Ph | |
| V-384 | 2,6-$Cl_2$-Ph | 2-F-4-$CH_3CH_2$SO-Ph | |
| V-385 | 2,6-$Cl_2$-Ph | 2-F-4-$CH_3CH_2SO_2$-Ph | |
| V-386 | 2,6-$Cl_2$-Ph | 2-F-4-$CH_3CH_2CH_2$S-Ph | |
| V-387 | 2,6-$Cl_2$-Ph | 2-F-4-$CH_3CH_2CH_2$SO-Ph | |
| V-388 | 2,6-$Cl_2$-Ph | 2-F-4-$CH_3CH_2CH_2SO_2$-Ph | |
| V-389 | 2,6-$Cl_2$-Ph | 2-F-4-i-PrS-Ph | |
| V-390 | 2,6-$Cl_2$-Ph | 2-F-4-i-PrSO-Ph | |
| V-391 | 2,6-$Cl_2$-Ph | 2-F-4-i-$PrSO_2$-Ph | |
| V-392 | 2,6-$Cl_2$-Ph | 2-F-4-t-BuS-Ph | |
| V-393 | 2,6-$Cl_2$-Ph | 2-F-4-t-BuSO-Ph | |
| V-394 | 2,6-$Cl_2$-Ph | 2-F-4-t-$BuSO_2$-Ph | |
| V-395 | 2,6-$Cl_2$-Ph | 2-F-4-$CH_2$=$CHCH_2$S-Ph | |
| V-396 | 2,6-$Cl_2$-Ph | 2-F-4-$CH_2$=$CHCH_2$SO-Ph | |
| V-397 | 2,6-$Cl_2$-Ph | 2-F-4-$CH_2$=$CHCH_2SO_2$-Ph | |
| V-398 | 2,6-$Cl_2$-Ph | 2-F-4-CH≡$CCH_2$S-Ph | |
| V-399 | 2,6-$Cl_2$-Ph | 2-F-4-CH≡$CCH_2$SO-Ph | |
| V-400 | 2,6-$Cl_2$-Ph | 2-F-4-CH≡$CCH_2SO_2$-Ph | |

| compound No. | $Q_1$ | $Q_2$ | m. p. (°C) |
|---|---|---|---|
| V-401 | 2,6-$Cl_2$-Ph | 2-F-4-(2-Cl-4-$CF_3$-pyridin-2yl-S)-Ph | |
| V-402 | 2,6-$Cl_2$-Ph | 2-F-4-(2-Cl-4-$CF_3$-pyridin-2yl-SO)-Ph | |
| V-403 | 2,6-$Cl_2$-Ph | 2-F-4-(2-Cl-4-$CF_3$-pyridin-2yl-$SO_2$)-Ph | |
| V-404 | 2,6-$Cl_2$-Ph | 2-Me-4-$CF_3$S-Ph | |
| V-405 | 2,6-$Cl_2$-Ph | 2-Me-4-$CF_3$SO-Ph | |
| V-406 | 2,6-$Cl_2$-Ph | 2-Me-4-$CF_3SO_2$-Ph | |
| V-407 | 2,6-$Cl_2$-Ph | 3-Me-4-$CF_3$S-Ph | |
| V-408 | 2,6-$Cl_2$-Ph | 3-Me-4-$CF_3$SO-Ph | |
| V-409 | 2,6-$Cl_2$-Ph | 3-Me-4-$CF_3SO_2$-Ph | |
| V-410 | 2,6-$Cl_2$-Ph | 2-Cl-4-$CF_3$S-Ph | |
| V-411 | 2,6-$Cl_2$-Ph | 2-Cl-4-$CF_3$SO-Ph | |
| V-412 | 2,6-$Cl_2$-Ph | 2-Cl-4-$CF_3SO_2$-Ph | |
| V-413 | 2,6-$Cl_2$-Ph | 3-Cl-4-$CF_3$S-Ph | |
| V-414 | 2,6-$Cl_2$-Ph | 3-Cl-4-$CF_3$SO-Ph | |
| V-415 | 2,6-$Cl_2$-Ph | 3-Cl-4-$CF_3SO_2$-Ph | |
| V-416 | 2,6-$Cl_2$-Ph | 3-F-4-$CF_3$S-Ph | |
| V-417 | 2,6-$Cl_2$-Ph | 3-F-4-$CF_3$SO-Ph | |
| V-418 | 2,6-$Cl_2$-Ph | 3-F-4-$CF_3SO_2$-Ph | |
| V-419 | 2,6-$Cl_2$-Ph | 2,3-$Me_2$-4-$CF_3$S-Ph | |
| V-420 | 2,6-$Cl_2$-Ph | 2,3-$Me_2$-4-$CF_3$SO-Ph | |
| V-421 | 2,6-$Cl_2$-Ph | 2,3-$Me_2$-4-$CF_3SO_2$-Ph | |
| V-422 | 2,6-$Cl_2$-Ph | 2,5-$Me_2$-4-$CF_3$S-Ph | |
| V-423 | 2,6-$Cl_2$-Ph | 2,5-$Me_2$-4-$CF_3$SO-Ph | |
| V-424 | 2,6-$Cl_2$-Ph | 2,5-$Me_2$-4-$CF_3SO_2$-Ph | |
| V-425 | 2,6-$Cl_2$-Ph | 5-Cl-2-Me-4-$CF_3$S-Ph | |
| V-426 | 2,6-$Cl_2$-Ph | 5-Cl-2-Me-4-$CF_3$SO-Ph | |
| V-427 | 2,6-$Cl_2$-Ph | 5-Cl-2-Me-4-$CF_3SO_2$-Ph | |
| V-428 | 2,6-$Cl_2$-Ph | 2,3-$Cl_2$-4-$CF_3$S-Ph | |
| V-429 | 2,6-$Cl_2$-Ph | 2,3-$Cl_2$-4-$CF_3$SO-Ph | |
| V-430 | 2,6-$Cl_2$-Ph | 2,3-$Cl_2$-4-$CF_3SO_2$-Ph | |
| V-431 | 2,6-$Cl_2$-Ph | 3-Cl-2-F-4-$CF_3$S-Ph | |
| V-432 | 2,6-$Cl_2$-Ph | 3-Cl-2-F-4-$CF_3$SO-Ph | |
| V-433 | 2,6-$Cl_2$-Ph | 3-Cl-2-F-4-$CF_3SO_2$-Ph | |
| V-434 | 2,6-$Cl_2$-Ph | 5-Cl-2-F-4-$CF_3$S-Ph | |
| V-435 | 2,6-$Cl_2$-Ph | 5-Cl-2-F-4-$CF_3$SO-Ph | |
| V-436 | 2,6-$Cl_2$-Ph | 5-Cl-2-F-4-$CF_3SO_2$-Ph | |
| V-437 | 2,6-$Cl_2$-Ph | 2,6-$F_2$-4-$CF_3$S-Ph | |
| V-438 | 2,6-$Cl_2$-Ph | 2,6-$F_2$-4-$CF_3$SO-Ph | |
| V-439 | 2,6-$Cl_2$-Ph | 2,6-$F_2$-4-$CF_3SO_2$-Ph | |
| V-440 | 2,6-$Cl_2$-Ph | 2,3-$F_2$-4-$CF_3$S-Ph | |

| compound No. | $Q_1$ | $Q_2$ | m. p. (°C) |
|---|---|---|---|
| V-441 | 2,6-$Cl_2$-Ph | 2,3-$F_2$-4-$CF_3SO$-Ph | |
| V-442 | 2,6-$Cl_2$-Ph | 2,3-$F_2$-4-$CF_3SO_2$-Ph | |
| V-443 | 2,6-$Cl_2$-Ph | 2,5-$F_2$-4-$CF_3S$-Ph | |
| V-444 | 2,6-$Cl_2$-Ph | 2,5-$F_2$-4-$CF_3SO$-Ph | |
| V-445 | 2,6-$Cl_2$-Ph | 2,5-$F_2$-4-$CF_3SO_2$-Ph | |
| V-446 | 2,6-$Cl_2$-Ph | 3,5-$F_2$-4-$CF_3S$-Ph | |
| V-447 | 2,6-$Cl_2$-Ph | 3,5-$F_2$-4-$CF_3SO$-Ph | |
| V-448 | 2,6-$Cl_2$-Ph | 3,5-$F_2$-4-$CF_3SO_2$-Ph | |
| V-449 | 2,6-$Cl_2$-Ph | 2,3,6-$F_3$-4-$CF_3S$-Ph | |
| V-450 | 2,6-$Cl_2$-Ph | 2,3,6-$F_3$-4-$CF_3SO$-Ph | |
| V-451 | 2,6-$Cl_2$-Ph | 2,3,6-$F_3$-4-$CF_3SO_2$-Ph | |
| V-452 | 2,6-$Cl_2$-Ph | 2,3,5,6-$F_4$-4-$CF_3S$-Ph | |
| V-453 | 2,6-$Cl_2$-Ph | 2,3,5,6-$F_4$-4-$CF_3SO$-Ph | |
| V-454 | 2,6-$Cl_2$-Ph | 2,3,5,6-$F_4$-4-$CF_3SO_2$-Ph | |
| V-455 | 2,6-$Cl_2$-Ph | 2-F-4-$CF_3CF_2S$-Ph | |
| V-456 | 2,6-$Cl_2$-Ph | 2-F-4-$CF_3CF_2SO$-Ph | |
| V-457 | 2,6-$Cl_2$-Ph | 2-F-4-$CF_3CF_2SO_2$-Ph | |
| V-458 | 2,6-$Cl_2$-Ph | 2-Cl-4-$CF_3CF_2S$-Ph | |
| V-459 | 2,6-$Cl_2$-Ph | 2-Cl-4-$CF_3CF_2SO$-Ph | |
| V-460 | 2,6-$Cl_2$-Ph | 2-Cl-4-$CF_3CF_2SO_2$-Ph | |
| V-461 | 2,6-$Cl_2$-Ph | 2-Me-4-$CF_3CF_2S$-Ph | |
| V-462 | 2,6-$Cl_2$-Ph | 2-Me-4-$CF_3CF_2SO$-Ph | |
| V-463 | 2,6-$Cl_2$-Ph | 2-Me-4-$CF_3CF_2SO_2$-Ph | |
| V-464 | 2,6-$Cl_2$-Ph | 5-Cl-2-Me-4-$CF_3CF_2S$-Ph | |
| V-465 | 2,6-$Cl_2$-Ph | 5-Cl-2-Me-4-$CF_3CF_2SO$-Ph | |
| V-466 | 2,6-$Cl_2$-Ph | 5-Cl-2-Me-4-$CF_3CF_2SO_2$-Ph | |
| V-467 | 2,6-$Cl_2$-Ph | 2,3-$Me_2$-4-$CF_3CF_2S$-Ph | |
| V-468 | 2,6-$Cl_2$-Ph | 2,3-$Me_2$-4-$CF_3CF_2SO$-Ph | |
| V-469 | 2,6-$Cl_2$-Ph | 2,3-$Me_2$-4-$CF_3CF_2SO_2$-Ph | |
| V-470 | 2,6-$Cl_2$-Ph | 2-F-4-$CF_3CF_2CF_2S$-Ph | |
| V-471 | 2,6-$Cl_2$-Ph | 2-F-4-$CF_3CF_2CF_2SO$-Ph | |
| V-472 | 2,6-$Cl_2$-Ph | 2-F-4-$CF_3CF_2CF_2SO_2$-Ph | |
| V-473 | 2,6-$Cl_2$-Ph | 2-Cl-4-$CF_3CF_2CF_2S$-Ph | |
| V-474 | 2,6-$Cl_2$-Ph | 2-Cl-4-$CF_3CF_2CF_2SO$-Ph | |
| V-475 | 2,6-$Cl_2$-Ph | 2-Cl-4-$CF_3CF_2CF_2SO_2$-Ph | |
| V-476 | 2,6-$Cl_2$-Ph | 2-Me-4-$CF_3CF_2CF_2S$-Ph | |
| V-477 | 2,6-$Cl_2$-Ph | 2-Me-4-$CF_3CF_2CF_2SO$-Ph | |
| V-478 | 2,6-$Cl_2$-Ph | 2-Me-4-$CF_3CF_2CF_2SO_2$-Ph | |
| V-479 | 2,6-$Cl_2$-Ph | 5-Cl-2-Me-4-$CF_3CF_2CF_2S$-Ph | |
| V-480 | 2,6-$Cl_2$-Ph | 5-Cl-2-Me-4-$CF_3CF_2CF_2SO$-Ph | |

| compound No. | $Q_1$ | $Q_2$ | m. p. (°C) |
|---|---|---|---|
| V-481 | 2,6-$Cl_2$-Ph | 5-Cl-2-Me-4-$CF_3CF_2CF_2SO_2$-Ph | |
| V-482 | 2,6-$Cl_2$-Ph | 2,3-$Me_2$-4-$CF_3CF_2CF_2S$-Ph | |
| V-483 | 2,6-$Cl_2$-Ph | 2,3-$Me_2$-4-$CF_3CF_2CF_2SO$-Ph | |
| V-484 | 2,6-$Cl_2$-Ph | 2,3-$Me_2$-4-$CF_3CF_2CF_2SO_2$-Ph | |
| V-485 | 2,6-$Cl_2$-Ph | 2-F-4-$(CF_3)_2CFS$-Ph | |
| V-486 | 2,6-$Cl_2$-Ph | 2-F-4-$(CF_3)_2CFSO$-Ph | |
| V-487 | 2,6-$Cl_2$-Ph | 2-F-4-$(CF_3)_2CFSO_2$-Ph | |
| V-488 | 2,6-$Cl_2$-Ph | 2-Me-4-$(CF_3)_2CFS$-Ph | |
| V-489 | 2,6-$Cl_2$-Ph | 2-Me-4-$(CF_3)_2CFSO$-Ph | |
| V-490 | 2,6-$Cl_2$-Ph | 2-Me-4-$(CF_3)_2CFSO_2$-Ph | |
| V-491 | 2,6-$Cl_2$-Ph | 2-Cl-4-$(CF_3)_2CFS$-Ph | |
| V-492 | 2,6-$Cl_2$-Ph | 2-Cl-4-$(CF_3)_2CFSO$-Ph | |
| V-493 | 2,6-$Cl_2$-Ph | 2-Cl-4-$(CF_3)_2CFSO_2$-Ph | |
| V-494 | 2,6-$Cl_2$-Ph | 5-Cl-2-Me-4-$(CF_3)_2CFS$-Ph | |
| V-495 | 2,6-$Cl_2$-Ph | 5-Cl-2-Me-4-$(CF_3)_2CFSO$-Ph | |
| V-496 | 2,6-$Cl_2$-Ph | 5-Cl-2-Me-4-$(CF_3)_2CFSO_2$-Ph | |
| V-497 | 2,6-$Cl_2$-Ph | 2,3-$Me_2$-4-$(CF_3)_2CFS$-Ph | |
| V-498 | 2,6-$Cl_2$-Ph | 2,3-$Me_2$-4-$(CF_3)_2CFSO$-Ph | |
| V-499 | 2,6-$Cl_2$-Ph | 2,3-$Me_2$-4-$(CF_3)_2CFSO_2$-Ph | |
| V-500 | 2,6-$Cl_2$-Ph | 2-F-4-MeO-Ph | |
| V-501 | 2,6-$Cl_2$-Ph | 2-Me-4-MeO-Ph | |
| V-502 | 2,6-$Cl_2$-Ph | 2-F-4-CN-Ph | |
| V-503 | 2,6-$Cl_2$-Ph | 2-F-4-COOMe-Ph | |
| V-504 | 2,6-$Cl_2$-Ph | 2-F-4-COOEt-Ph | |
| V-505 | 3-Cl-pyridin-2yl | 4-Cl-Ph | |
| V-506 | 3-Cl-pyridin-2yl | 3,5-$Cl_2$-4-(2-Cl-4-$CF_3$-pyridin-2yl-O)-Ph | |
| V-507 | 3-Cl-pyridin-2yl | 2-F-4-(2-Cl-4-$CF_3$-Ph-O)-Ph | |
| V-508 | 3-Cl-pyridin-2yl | 3,5-$Cl_2$-2,4-$F_2$-Ph | |
| V-509 | 3-Cl-pyridin-2yl | 3,5-$Cl_2$-4-$CF_2HCF_2O$-Ph | |
| V-510 | 3-Cl-pyridin-2yl | 2,5-$Cl_2$-4-$CF_3CFHCF_2O$-Ph | |
| V-511 | 3-Cl-pyridin-2yl | 2-F-4-$CFClHCF_2S$-Ph | |
| V-512 | 3-Cl-pyridin-2yl | 2-F-4-$CFClHCF_2SO$-Ph | |
| V-513 | 3-Cl-pyridin-2yl | 2-F-4-$CFClHCF_2SO_2$-Ph | |
| V-514 | 3-Cl-pyridin-2yl | 4-$CFClHCF_2S$-Ph | |
| V-515 | 3-Cl-pyridin-2yl | 4-$CFClHCF_2SO$-Ph | |
| V-516 | 3-Cl-pyridin-2yl | 4-$CFClHCF_2SO_2$-Ph | |
| V-517 | 3-Cl-pyridin-2yl | 2-Cl-4-$CFClHCF_2S$-Ph | |
| V-518 | 3-Cl-pyridin-2yl | 2-Cl-4-$CFClHCF_2SO$-Ph | |
| V-519 | 3-Cl-pyridin-2yl | 2-Cl-4-$CFClHCF_2SO_2$-Ph | |
| V-520 | 3-Cl-pyridin-2yl | 2-Cl-4-$CF_2HCF_2S$-Ph | |

| compound No. | $Q_1$ | $Q_2$ | m.p. (°C) |
|---|---|---|---|
| V-521 | 3-Cl-pyridin-2yl | 2-Cl-4-$CF_2HCF_2SO$-Ph | |
| V-522 | 3-Cl-pyridin-2yl | 2-Cl-4-$CF_2HCF_2SO_2$-Ph | |
| V-523 | 3-Cl-pyridin-2yl | 4-$CF_2HCF_2S$-Ph | |
| V-524 | 3-Cl-pyridin-2yl | 4-$CF_2HCF_2SO$-Ph | |
| V-525 | 3-Cl-pyridin-2yl | 4-$CF_2HCF_2SO_2$-Ph | |
| V-526 | 3-Cl-pyridin-2yl | 2-F-4-$CF_2HCF_2S$-Ph | |
| V-527 | 3-Cl-pyridin-2yl | 2-F-4-$CF_2HCF_2SO$-Ph | |
| V-528 | 3-Cl-pyridin-2yl | 2-F-4-$CF_2HCF_2SO_2$-Ph | |
| V-529 | 3-Cl-pyridin-2yl | 4-$CF_3S$-Ph | |
| V-530 | 3-Cl-pyridin-2yl | 4-$CF_3SO$-Ph | |
| V-531 | 3-Cl-pyridin-2yl | 4-$CF_3SO_2$-Ph | |
| V-532 | 3-Cl-pyridin-2yl | 2-F-4-MeS-Ph | |
| V-533 | 3-Cl-pyridin-2yl | 2-F-4-MeSO-Ph | |
| V-534 | 3-Cl-pyridin-2yl | 2-F-4-$MeSO_2$-Ph | |
| V-535 | 3-Cl-pyridin-2yl | 2,6-$F_2$-4-MeS-Ph | |
| V-536 | 3-Cl-pyridin-2yl | 2,6-$F_2$-4-MeSO-Ph | |
| V-537 | 3-Cl-pyridin-2yl | 2,6-$F_2$-4-$MeSO_2$-Ph | |
| V-538 | 3-Cl-pyridin-2yl | 2-F-4-$CF_3CH_2S$-Ph | |
| V-539 | 3-Cl-pyridin-2yl | 2-F-4-$CF_3CH_2SO$-Ph | |
| V-540 | 3-Cl-pyridin-2yl | 2-F-4-$CF_3CH_2SO_2$-Ph | |
| V-541 | 3-Cl-pyridin-2yl | 2-F-4-$CCl_2$=$CHCH_2S$-Ph | |
| V-542 | 3-Cl-pyridin-2yl | 2-F-4-$CCl_2$=$CHCH_2SO$-Ph | |
| V-543 | 3-Cl-pyridin-2yl | 2-F-4-$CCl_2$=$CHCH_2SO_2$-Ph | |
| V-544 | 3-Cl-pyridin-2yl | $CF_3CH_2$ | |
| V-545 | 3-Cl-pyridin-2yl | 2-F-4-$CF_2HS$-Ph | |
| V-546 | 3-Cl-pyridin-2yl | 2-F-4-$CF_2HSO$-Ph | |
| V-547 | 3-Cl-pyridin-2yl | 2-F-4-$CF_2HSO_2$-Ph | |
| V-548 | 3-Cl-pyridin-2yl | 2-F-4-$CF_3S$-Ph | |
| V-549 | 3-Cl-pyridin-2yl | 2-F-4-$CF_3SO$-Ph | |
| V-550 | 3-Cl-pyridin-2yl | 2-F-4-$CF_3SO_2$-Ph | |
| V-551 | 3-Cl-pyridin-2yl | 2-F-4-$CH_3CH_2S$-Ph | |
| V-552 | 3-Cl-pyridin-2yl | 2-F-4-$CH_3CH_2SO$-Ph | |
| V-553 | 3-Cl-pyridin-2yl | 2-F-4-$CH_3CH_2SO_2$-Ph | |
| V-554 | 3-Cl-pyridin-2yl | 2-F-4-$CH_3CH_2CH_2S$-Ph | |
| V-555 | 3-Cl-pyridin-2yl | 2-F-4-$CH_3CH_2CH_2SO$-Ph | |
| V-556 | 3-Cl-pyridin-2yl | 2-F-4-$CH_3CH_2CH_2SO_2$-Ph | |
| V-557 | 3-Cl-pyridin-2yl | 2-F-4-i-PrS-Ph | |
| V-558 | 3-Cl-pyridin-2yl | 2-F-4-i-PrSO-Ph | |
| V-559 | 3-Cl-pyridin-2yl | 2-F-4-i-$PrSO_2$-Ph | |
| V-560 | 3-Cl-pyridin-2yl | 2-F-4-t-BuS-Ph | |

| compound No. | $Q_1$ | $Q_2$ | m. p. (°C) |
|---|---|---|---|
| V-561 | 3-Cl-pyridin-2yl | 2-F-4-t-BuSO-Ph | |
| V-562 | 3-Cl-pyridin-2yl | 2-F-4-t-$BuSO_2$-Ph | |
| V-563 | 3-Cl-pyridin-2yl | 2-F-4-$CH_2$=$CHCH_2S$-Ph | |
| V-564 | 3-Cl-pyridin-2yl | 2-F-4-$CH_2$=$CHCH_2SO$-Ph | |
| V-565 | 3-Cl-pyridin-2yl | 2-F-4-$CH_2$=$CHCH_2SO_2$-Ph | |
| V-566 | 3-Cl-pyridin-2yl | 2-F-4-$CH{\equiv}CCH_2S$-Ph | |
| V-567 | 3-Cl-pyridin-2yl | 2-F-4-$CH{\equiv}CCH_2SO$-Ph | |
| V-568 | 3-Cl-pyridin-2yl | 2-F-4-$CH{\equiv}CCH_2SO_2$-Ph | |
| V-569 | 3-Cl-pyridin-2yl | 2-F-4-(2-Cl-4-$CF_3$-pyridin-2yl-S)-Ph | |
| V-570 | 3-Cl-pyridin-2yl | 2-F-4-(2-Cl-4-$CF_3$-pyridin-2yl-SO)-Ph | |
| V-571 | 3-Cl-pyridin-2yl | 2-F-4-(2-Cl-4-$CF_3$-pyridin-2yl-$SO_2$)-Ph | |
| V-572 | 3-Cl-pyridin-2yl | 2-Me-4-$CF_3S$-Ph | |
| V-573 | 3-Cl-pyridin-2yl | 2-Me-4-$CF_3SO$-Ph | |
| V-574 | 3-Cl-pyridin-2yl | 2-Me-4-$CF_3SO_2$-Ph | |
| V-575 | 3-Cl-pyridin-2yl | 3-Me-4-$CF_3S$-Ph | |
| V-576 | 3-Cl-pyridin-2yl | 3-Me-4-$CF_3SO$-Ph | |
| V-577 | 3-Cl-pyridin-2yl | 3-Me-4-$CF_3SO_2$-Ph | |
| V-578 | 3-Cl-pyridin-2yl | 2-Cl-4-$CF_3S$-Ph | |
| V-579 | 3-Cl-pyridin-2yl | 2-Cl-4-$CF_3SO$-Ph | |
| V-580 | 3-Cl-pyridin-2yl | 2-Cl-4-$CF_3SO_2$-Ph | |
| V-581 | 3-Cl-pyridin-2yl | 3-Cl-4-$CF_3S$-Ph | |
| V-582 | 3-Cl-pyridin-2yl | 3-Cl-4-$CF_3SO$-Ph | |
| V-583 | 3-Cl-pyridin-2yl | 3-Cl-4-$CF_3SO_2$-Ph | |
| V-584 | 3-Cl-pyridin-2yl | 3-F-4-$CF_3S$-Ph | |
| V-585 | 3-Cl-pyridin-2yl | 3-F-4-$CF_3SO$-Ph | |
| V-586 | 3-Cl-pyridin-2yl | 3-F-4-$CF_3SO_2$-Ph | |
| V-587 | 3-Cl-pyridin-2yl | 2,3-$Me_2$-4-$CF_3S$-Ph | |
| V-588 | 3-Cl-pyridin-2yl | 2,3-$Me_2$-4-$CF_3SO$-Ph | |
| V-589 | 3-Cl-pyridin-2yl | 2,3-$Me_2$-4-$CF_3SO_2$-Ph | |
| V-590 | 3-Cl-pyridin-2yl | 2,5-$Me_2$-4-$CF_3S$-Ph | |
| V-591 | 3-Cl-pyridin-2yl | 2,5-$Me_2$-4-$CF_3SO$-Ph | |
| V-592 | 3-Cl-pyridin-2yl | 2,5-$Me_2$-4-$CF_3SO_2$-Ph | |
| V-593 | 3-Cl-pyridin-2yl | 5-Cl-2-Me-4-$CF_3S$-Ph | |
| V-594 | 3-Cl-pyridin-2yl | 5-Cl-2-Me-4-$CF_3SO$-Ph | |
| V-595 | 3-Cl-pyridin-2yl | 5-Cl-2-Me-4-$CF_3SO_2$-Ph | |
| V-596 | 3-Cl-pyridin-2yl | 2,3-$Cl_2$-4-$CF_3S$-Ph | |
| V-597 | 3-Cl-pyridin-2yl | 2,3-$Cl_2$-4-$CF_3SO$-Ph | |
| V-598 | 3-Cl-pyridin-2yl | 2,3-$Cl_2$-4-$CF_3SO_2$-Ph | |
| V-599 | 3-Cl-pyridin-2yl | 3-Cl-2-F-4-$CF_3S$-Ph | |
| V-600 | 3-Cl-pyridin-2yl | 3-Cl-2-F-4-$CF_3SO$-Ph | |

| compound No. | $Q_1$ | $Q_2$ | m. p. (°C) |
|---|---|---|---|
| V-601 | 3-Cl-pyridin-2yl | 3-Cl-2-F-4-$CF_3SO_2$-Ph | |
| V-602 | 3-Cl-pyridin-2yl | 5-Cl-2-F-4-$CF_3S$-Ph | |
| V-603 | 3-Cl-pyridin-2yl | 5-Cl-2-F-4-$CF_3SO$-Ph | |
| V-604 | 3-Cl-pyridin-2yl | 5-Cl-2-F-4-$CF_3SO_2$-Ph | |
| V-605 | 3-Cl-pyridin-2yl | 2,6-$F_2$-4-$CF_3S$-Ph | |
| V-606 | 3-Cl-pyridin-2yl | 2,6-$F_2$-4-$CF_3SO$-Ph | |
| V-607 | 3-Cl-pyridin-2yl | 2,6-$F_2$-4-$CF_3SO_2$-Ph | |
| V-608 | 3-Cl-pyridin-2yl | 2,3-$F_2$-4-$CF_3S$-Ph | |
| V-609 | 3-Cl-pyridin-2yl | 2,3-$F_2$-4-$CF_3SO$-Ph | |
| V-610 | 3-Cl-pyridin-2yl | 2,3-$F_2$-4-$CF_3SO_2$-Ph | |
| V-611 | 3-Cl-pyridin-2yl | 2,5-$F_2$-4-$CF_3S$-Ph | |
| V-612 | 3-Cl-pyridin-2yl | 2,5-$F_2$-4-$CF_3SO$-Ph | |
| V-613 | 3-Cl-pyridin-2yl | 2,5-$F_2$-4-$CF_3SO_2$-Ph | |
| V-614 | 3-Cl-pyridin-2yl | 3,5-$F_2$-4-$CF_3S$-Ph | |
| V-615 | 3-Cl-pyridin-2yl | 3,5-$F_2$-4-$CF_3SO$-Ph | |
| V-616 | 3-Cl-pyridin-2yl | 3,5-$F_2$-4-$CF_3SO_2$-Ph | |
| V-617 | 3-Cl-pyridin-2yl | 2,3,6-$F_3$-4-$CF_3S$-Ph | |
| V-618 | 3-Cl-pyridin-2yl | 2,3,6-$F_3$-4-$CF_3SO$-Ph | |
| V-619 | 3-Cl-pyridin-2yl | 2,3,6-$F_3$-4-$CF_3SO_2$-Ph | |
| V-620 | 3-Cl-pyridin-2yl | 2,3,5,6-$F_4$-4-$CF_3S$-Ph | |
| V-621 | 3-Cl-pyridin-2yl | 2,3,5,6-$F_4$-4-$CF_3SO$-Ph | |
| V-622 | 3-Cl-pyridin-2yl | 2,3,5,6-$F_4$-4-$CF_3SO_2$-Ph | |
| V-623 | 3-Cl-pyridin-2yl | 2-F-4-$CF_3CF_2S$-Ph | |
| V-624 | 3-Cl-pyridin-2yl | 2-F-4-$CF_3CF_2SO$-Ph | |
| V-625 | 3-Cl-pyridin-2yl | 2-F-4-$CF_3CF_2SO_2$-Ph | |
| V-626 | 3-Cl-pyridin-2yl | 2-Cl-4-$CF_3CF_2S$-Ph | |
| V-627 | 3-Cl-pyridin-2yl | 2-Cl-4-$CF_3CF_2SO$-Ph | |
| V-628 | 3-Cl-pyridin-2yl | 2-Cl-4-$CF_3CF_2SO_2$-Ph | |
| V-629 | 3-Cl-pyridin-2yl | 2-Me-4-$CF_3CF_2S$-Ph | |
| V-630 | 3-Cl-pyridin-2yl | 2-Me-4-$CF_3CF_2SO$-Ph | |
| V-631 | 3-Cl-pyridin-2yl | 2-Me-4-$CF_3CF_2SO_2$-Ph | |
| V-632 | 3-Cl-pyridin-2yl | 5-Cl-2-Me-4-$CF_3CF_2S$-Ph | |
| V-633 | 3-Cl-pyridin-2yl | 5-Cl-2-Me-4-$CF_3CF_2SO$-Ph | |
| V-634 | 3-Cl-pyridin-2yl | 5-Cl-2-Me-4-$CF_3CF_2SO_2$-Ph | |
| V-635 | 3-Cl-pyridin-2yl | 2,3-$Me_2$-4-$CF_3CF_2S$-Ph | |
| V-636 | 3-Cl-pyridin-2yl | 2,3-$Me_2$-4-$CF_3CF_2SO$-Ph | |
| V-637 | 3-Cl-pyridin-2yl | 2,3-$Me_2$-4-$CF_3CF_2SO_2$-Ph | |
| V-638 | 3-Cl-pyridin-2yl | 2-F-4-$CF_3CF_2CF_2S$-Ph | |
| V-639 | 3-Cl-pyridin-2yl | 2-F-4-$CF_3CF_2CF_2SO$-Ph | |
| V-640 | 3-Cl-pyridin-2yl | 2-F-4-$CF_3CF_2CF_2SO_2$-Ph | |

| compound No. | $Q_1$ | $Q_2$ | m. p. (°C) |
|---|---|---|---|
| V-641 | 3-Cl-pyridin-2yl | 2-Cl-4-$CF_3CF_2CF_2$S-Ph | |
| V-642 | 3-Cl-pyridin-2yl | 2-Cl-4-$CF_3CF_2CF_2$SO-Ph | |
| V-643 | 3-Cl-pyridin-2yl | 2-Cl-4-$CF_3CF_2CF_2SO_2$-Ph | |
| V-644 | 3-Cl-pyridin-2yl | 2-Me-4-$CF_3CF_2CF_2$S-Ph | |
| V-645 | 3-Cl-pyridin-2yl | 2-Me-4-$CF_3CF_2CF_2$SO-Ph | |
| V-646 | 3-Cl-pyridin-2yl | 2-Me-4-$CF_3CF_2CF_2SO_2$-Ph | |
| V-647 | 3-Cl-pyridin-2yl | 5-Cl-2-Me-4-$CF_3CF_2CF_2$S-Ph | |
| V-648 | 3-Cl-pyridin-2yl | 5-Cl-2-Me-4-$CF_3CF_2CF_2$SO-Ph | |
| V-649 | 3-Cl-pyridin-2yl | 5-Cl-2-Me-4-$CF_3CF_2CF_2SO_2$-Ph | |
| V-650 | 3-Cl-pyridin-2yl | 2,3-$Me_2$-4-$CF_3CF_2CF_2$S-Ph | |
| V-651 | 3-Cl-pyridin-2yl | 2,3-$Me_2$-4-$CF_3CF_2CF_2$SO-Ph | |
| V-652 | 3-Cl-pyridin-2yl | 2,3-$Me_2$-4-$CF_3CF_2CF_2SO_2$-Ph | |
| V-653 | 3-Cl-pyridin-2yl | 2-F-4-$(CF_3)_2$CFS-Ph | |
| V-654 | 3-Cl-pyridin-2yl | 2-F-4-$(CF_3)_2$CFSO-Ph | |
| V-655 | 3-Cl-pyridin-2yl | 2-F-4-$(CF_3)_2CFSO_2$-Ph | |
| V-656 | 3-Cl-pyridin-2yl | 2-Me-4-$(CF_3)_2$CFS-Ph | |
| V-657 | 3-Cl-pyridin-2yl | 2-Me-4-$(CF_3)_2$CFSO-Ph | |
| V-658 | 3-Cl-pyridin-2yl | 2-Me-4-$(CF_3)_2CFSO_2$-Ph | |
| V-659 | 3-Cl-pyridin-2yl | 2-Cl-4-$(CF_3)_2$CFS-Ph | |
| V-660 | 3-Cl-pyridin-2yl | 2-Cl-4-$(CF_3)_2$CFSO-Ph | |
| V-661 | 3-Cl-pyridin-2yl | 2-Cl-4-$(CF_3)_2CFSO_2$-Ph | |
| V-662 | 3-Cl-pyridin-2yl | 5-Cl-2-Me-4-$(CF_3)_2$CFS-Ph | |
| V-663 | 3-Cl-pyridin-2yl | 5-Cl-2-Me-4-$(CF_3)_2$CFSO-Ph | |
| V-664 | 3-Cl-pyridin-2yl | 5-Cl-2-Me-4-$(CF_3)_2CFSO_2$-Ph | |
| V-665 | 3-Cl-pyridin-2yl | 2,3-$Me_2$-4-$(CF_3)_2$CFS-Ph | |
| V-666 | 3-Cl-pyridin-2yl | 2,3-$Me_2$-4-$(CF_3)_2$CFSO-Ph | |
| V-667 | 3-Cl-pyridin-2yl | 2,3-$Me_2$-4-$(CF_3)_2CFSO_2$-Ph | |
| V-668 | 3-Cl-pyridin-2yl | 2-F-4-MeO-Ph | |
| V-669 | 3-Cl-pyridin-2yl | 2-Me-4-MeO-Ph | |
| V-670 | 3-Cl-pyridin-2yl | 2-F-4-CN-Ph | |
| V-671 | 3-Cl-pyridin-2yl | 2-F-4-COOMe-Ph | |
| V-672 | 3-Cl-pyridin-2yl | 2-F-4-COOEt-Ph | |

Reference Example 5

Synthesis of 1-(2,6-difluorobenzoyl)-3-[2-fluoro-4-(trifluoromethylthio)phenyl]urea

To a solution of 2.14 g (10.1 mmol) of 2-fluoro-4-trifluoromethylthioaniline in 20 ml of diethyl ether was added dropwise a solution of 1.90 g (10.4 mmol) of 2,6-difluorobenzoylisocyanate in 4 ml of diethyl ether under ice-cooling. The reaction solution was stirred for 2.5 hrs, and the. precipitated crystals was collected by filtration to give 2.87 g (7.28 mmol) of the target 1-(2,6-difluorobenzoyl)-3-[2-fluoro-4-(trifluoromethylthio)phenyl]urea as a white solid (m.p. 173-175°C, yield 72.1%).
$^1$HNMR (DMSO-$d_6$)δppm : 7.24-7.29 ( 2H, m, Ph), 7.54-7.79 ( 3H, m, Ph), 8.25-8.34 ( 1H, m, Ph), 10.65 ( 1H, br, NH), 11.72 ( 1H, br, NH) .
IR (nujol)ν$cm^{-1}$ : 3121, 1706, 1599, 1545, 1500, 1469, 1377, 1284, 1266, 1244, 1155, 1130, 1112, 1017, 795.

The following raw material compounds were synthesized by a similar method.

1-(2-chloro-6-fluorobenzoyl)-3-[2-fluoro-4-(trifluoromethylthio)phenyl]urea : m.p. 190-193°C
1-(2-chloro-6-fluorobenzoyl)-3-[3-fluoro-4-(trifluoromethylthio)phenyl]urea : m.p. 177-178°C
1-(2,6-difluorobenzoyl)-3-[3-fluoro-4-(trifluoromethylthio)phenyl]urea : m.p. 147-148°C
1-(2-chloro-6-fluorobenzoyl)-3-[2-chloro-4-(trifluoromethylthio)phenyl]urea : m.p. 174-176°C
1-(2,6-difluorobenzoyl)-3-[2-chloro-4-(trifluoromethylthio)phenyl]urea : m.p. 147-150°C
1-(2-chloro-6-fluorobenzoyl)-3-[2-methyl-4-(trifluoromethylthio)phenyl]urea : m.p. 165-167°C
1-(2,6-difluorobenzoyl)-3-[2-methyl-4-(trifluoromethylthio)phenyl]urea : m.p. 150-153°C
1-(2-chloro-6-fluorobenzoyl)-3-[3-methyl-4-(trifluoromethylthio)phenyl]urea : m.p. 176-177°C
1-(2,6-difluorobenzoyl)-3-[3-methyl-4-(trifluoromethylthio)phenyl]urea : m.p. 198°C
1-(2-chloro-6-fluorobenzoyl)-3-[2,3-difluoro-4-(trifluoromethylthio)phenyl]urea : m.p. 182°C
1-(2,6-difluorobenzoyl)-3-[2,3-difluoro-4-(trifluoromethylthio)phenyl]urea : m.p. 171-173°C
1-(2-chloro-6-fluorobenzoyl)-3-[2,3-difluoro-4-(trifluoromethylthio)phenyl]urea : m.p. 199-202°C
1-(2,6-difluorobenzoyl)-3-[2,6-difluoro-4-(trifluoromethylthio)phenyl]urea : m.p. 197-199°C
1-(2-chloro-6-fluorobenzoyl)-3-[2,5-difluoro-4-(trifluoromethylthio)phenyl]urea : m.p. 173-175°C
1-(2,6-difluorobenzoyl)-3-[2,5-difluoro-4-(trifluoromethylthio)phenyl]urea : m.p. 158-160°C
1-(2,6-difluorobenzoyl)-3-[3,5-difluoro-4-(trifluoromethylthio)phenyl]urea : m.p. 208°C
1-(2,6-difluorobenzoyl)-3-[2,3-dimethyl-4-(trifluoromethylthio)phenyl]urea : m.p. 183-185°C
1-(2,6-difluorobenzoyl)-3-[2,5-dimethyl-4-(trifluoromethylthio)phenyl]urea : m.p. 158-160°C
1-(2-chloro-6-fluorobenzoyl)-3-[2,3-dichloro-4-(trifluoromethylthio)phenyl]urea : m.p. 185°C
1-(2,6-difluorobenzoyl)-3-[2,3-dichloro-4-(trifluoromethylthio)phenyl]urea : m.p. 186-187°C
1-(2-chloro-6-fluorobenzoyl)-3-[5-chloro-2-fluoro-4-(trifluoromethylthio)phenyl]urea : m.p. 195-196°C
1-(2,6-difluorobenzoyl)-3-[5-chloro-2-fluoro-4-(trifluoromethylthio)phenyl]urea : m.p. 166°C
1-(2-chloro-6-fluorobenzoyl)-3-[3-chloro-2-fluoro-4-(trifluoromethylthio)phenyl]urea : m.p. 180°C
1-(2,6-difluorobenzoyl)-3-[3-chloro-2-fluoro-4-(trifluoromethylthio)phenyl]urea : m.p. 187-188°C
1-(2-chloro-6-fluorobenzoyl)-3-[2-chloro-6-fluoro-4-(trifluoromethylthio)phenyl]urea : m.p. 191-192°C
1-(2,6-difluorobenzoyl)-3-[2-chloro-6-fluoro-4-(trifluoromethylthio)phenyl]urea : m.p. 177°C
1-(2-chloro-6-fluorobenzoyl)-3-[5-fluoro-2-methyl-4-(trifluoromethylthio)phenyl]urea : m.p. 139-140°C
1-(2,6-difluorobenzoyl)-3-[5-fluoro-2-methyl-4-(trifluoromethylthio)phenyl]urea : m.p. 158°C
1-(2-chloro-6-fluorobenzoyl)-3-[5-chloro-2-methyl-4-(trifluoromethylthio)phenyl]urea : m.p. 181-183°C
1-(2,6-difluorobenzoyl)-3-[5-chloro-2-methyl-4-(trifluoromethylthio)phenyl]urea : m.p. 180-183°C
1-(2-chloro-6-fluorobenzoyl)-3-[3-chloro-2-methyl-4-(trifluoromethylthio)phenyl]urea : m.p. 184°C
1-(2,6-difluorobenzoyl)-3-[3-chloro-2-methyl-4-(trifluoromethylthio)phenyl]urea : m.p. 176-177°C
1-(2-chloro-6-fluorobenzoyl)-3-[2,3,6-trifluoro-4-(trifluoromethylthio)phenyl]urea m.p. 197°C
1-(2,6-difluorobenzoyl)-3-[2,3,6-trifluoro-4-(trifluoromethylthio)phenyl]urea : m.p. 182°C
1-(2-chloro-6-fluorobenzoyl)-3-[2,3,5,6-tetrafluoro-4-(trifluoromethylthio)phenyl]urea : m.p. 195°C
1-(2,6-difluorobenzoyl)-3-[2,3,5,6-tetrafluoro-4-(trifluoromethylthio)phenyl]urea : m.p. 186-188°C.
1-(2,6-difluorobenzoyl)-3-[2-fluoro-4-(1,1,2,2,2-pentafluoroethylthio)phenyl]urea : m.p. 172-174°C
1-(2-chloro-6-fluorobenzoyl)-3-[2-fluoro-4-(1,1,2,2,2-pentafluoroethylthio)phenyl]urea : m.p. 155-157°C
1-(2,6-difluorobenzoyl)-3-[2-fluoro-4-(1,1,2,2,3,3,3-heptafluoro-1-propylthio)phenyl]urea : m.p. 172-175°C
1-(2-chloro-6-fluorobenzoyl)-3-[2-fluoro-4-(1,1,2,2,3,3,3-heptafluoro-1-propylthio)phenyl]urea : m.p. 150-153°C
1-(2,6-difluorobenzoyl)-3-[2-fluoro-4-(1,1,1,2,3,3,3-heptafluoro-2-propylthio)phenyl]urea : m.p. 205-208°C
1-(2-chloro-6-fluorobenzoyl)-3-[2-fluoro-4-(1,1,1,2,3,3,3-heptafluoro-2-propylthio)phenyl]urea : m.p. 193-195°C
1-(2-chloro-6-fluorobenzoyl)-3-[2-chloro-4-(1,1,2,2,3,3,3-heptafluoro-1-propylthio)phenyl]urea : m.p. 151-152°C
1-(2-chloro-6-fluorobenzoyl)-3-[5-chloro-2-methyl-4-(1,1,2,2,3,3,3-heptafluoro-1-propylthio)phenyl]urea : m.p. 167-168°C
1-(2,6-difluorobenzoyl)-3-[4-[(3-chloro-5-trifluoromethyl-2-pyridyl)thio]2-fluorophenyl]urea : m.p. 185-186°C
1-(2,6-difluorobenzoyl)-3-[2-fluoro-4-(trifluoromethylsulfinyl)phenyl]urea : m.p. 214-215°C
1-(2-chloro-6-fluorobenzoyl)-3-[2-fluoro-4-(trifluoromethylsulfinyl)phenyl]urea : m.p. 228-229°C
1-(3-chloropyridin-2-ylcarbonyl)-3-[2-fluoro-4-(trifluoromethylthio)phenyl]urea : m.p. 159-162°C
1-(2,6-difluorobenzoyl)-3-(4-trifluoromethylthiophenyl)urea : m.p. 215-216.5°C

Formulation Example 1

Compound No. I-44 (20% by weight), xylene (75% by weight) and polyoxyethylene alkyl ether (Neugen ET-135 (trade name), 5% by weight) are mixed well to prepare an emulsion.

Formulation Example 2

Compound No. I-44 (30% by weight), sodium lignin sulfonate (5% by weight), polyoxyethylene glycol ether (Neugen ET-135 (trade name), 5% by weight), white carbon (30% by weight) and clay (30% by weight) are mixed well to prepare a wettable powder.

Formulation Example 3

Compound No. I-44 (3% by weight), white carbon (3% by weight) and clay (94% by weight) are mixed well to prepare a powder.

Formulation Example 4

Compound No. I-44 (10% by weight), polyoxyethylene polyoxypropylene block copolymer (Newpol PE-64 (trade name), 1% by weight), sodium lignin sulfonate (5% by weight) and clay (84% by weight) are pulverized and mixed well. To the resulting mixture is added water, followed by well kneading, granulating and drying, to prepare a granule.

Formulation Example 5

Compound No. I-44 (3% by weight), white carbon (3% by weight), liquid paraffin (Doriresu C (trade name), 1% by weight), Hartol fatty acid (0.5% by weight) and clay (92.5% by weight) are mixed well to prepare a powder DL.

Formulation Example 6

Compound No. I-44 (10% by weight), polyoxyalkylene allyl phenyl ether sulfate (New Cargen FS-7 (trade name), 3% by weight), ethylene glycol (8% by weight), colloidal hydrated aluminum silicate (Kunipia F (trade name), 1% by weight), silicone emulsion (Antifoam E-20 (trade name), 0.2% by weight), n-butyl p-hydroxybenzoate (0.1% by weight) and water (77.7% by weight) are mixed and milled in a wet system to prepare a flowable formulation.

Test Example 1

Insecticidal effect for *Plutella xylostella* by immersion treatment of host crop

One true leaf of 7 to 8-leave stage cabbage was cut off and dipped in a pesticidal solution for a few seconds, which was prepared by dissolving 5 mg of test compound (represented by the compound Nos. in the above Examples) in 0.5 ml of acetone containing Tween 20 (trade name) and then diluting it to the prescribed concentration (100 ppm) with Dain (trade name, sticker) water diluted 5000-fold. After drying the pesticidal solution, the leaf was put into an ice cream cup (180 ml), and ten second-instar larvae of *Plutella xylostella* were set free therein. The cup was left in a temperature-controlled breeding room (24°C). On the fifth day after, the number of surviving *Plutella xylostella* was examined. The rate of dead pests was calculated by the following formula.

$$\text{rate of dead pests (\%)} = (\text{number of dead pests/ number of test pests}) \times 100$$

The result was shown in table 6.

Table 6

| Compound No. | Concentration (ppm) | rate of dead pests (%) |
|---|---|---|
| I-8 | 100 | 100 |
| I-11 | 100 | 100 |
| I-17 | 100 | 100 |
| I-20 | 100 | 100 |

Table 6 (continued)

| Compound No. | Concentration (ppm) | rate of dead pests (%) |
|---|---|---|
| I-22 | 100 | 100 |
| I-25 | 100 | 100 |
| I-26 | 100 | 100 |
| I-28 | 100 | 100 |
| I-29 | 100 | 100 |
| I-34 | 100 | 100 |
| I-41 | 100 | 100 |
| I-42 | 100 | 100 |
| I-43 | 100 | 100 |
| I-44 | 100 | 100 |
| I-81 | 100 | 100 |
| I-108 | 100 | 100 |
| I-116 | 100 | 100 |
| I-205 | 100 | 100 |
| I-206 | 100 | 100 |
| I-218 | 100 | 100 |
| I-221 | 100 | 100 |
| I-222 | 100 | 100 |
| I-398 | 100 | 100 |
| I=399 | 100 | 100 |
| II-10 | 100 | 100 |
| II-19 | 100 | 100 |
| II-22 | 100 | 100 |
| III-23 | 100 | 100 |
| III-24 | 100 | 100 |
| III-25 | 100 | 100 |
| III-26 | 100 | 100 |
| III-27 | 100 | 100 |
| III-28 | 100 | 100 |
| III-31 | 100 | 100 |
| III-39 | 100 | 100 |
| III-41 | 100 | 100 |
| III-43 | 100 | 100 |
| III-44 | 100 | 100 |
| III-45 | 100 | 100 |
| III=46 | 100 | 100 |
| III-48 | 100 | 100 |
| III=49 | 100 | 100 |

Table 6 (continued)

| Compound No. | Concentration (ppm) | rate of dead pests (%) |
|---|---|---|
| III-50 | 100 | 100 |
| III-54 | 100 | 100 |
| III-55 | 100 | 100 |
| III-56 | 100 | 100 |
| III-57 | 100 | 100 |
| III-58 | 100 | 100 |
| III-60 | 100 | 100 |
| III-61 | 100 . | 100 |
| III-62 | 100 | 100 |
| III-63 | 100 | 100 |
| III-64 | 100 | 100 |
| III-70 | 100 | 100 |
| III-71 | 100 | 100 |
| III-72 | 100 | 100 |
| III-77 | 100 | 100 |
| III-78 | 100 | 100 |
| III-79 | 100 | 100 |
| III-84 | 100 | 100 |
| III-89 | 100 | 100 |
| III-148 | 100 | 100 |
| III-149 | 100 | 100 |
| III-152 | 100 | 100 |
| III-153 | 100 | 100 |
| III-156 | 100 | 100 |
| III-160 | 100 | 100 |
| III-164 | 100 | 100 |
| III-184 | 100 | 100 |
| III-188 | 100 | 100 |
| V-3 | 100 | 100 |
| V-4 | 100 | 100 |

Test Example 2

Insecticidal effect for *Spodoptera litura* by spraying treatment to host crop

A pesticidal solution, which was prepared by dissolving 5 mg of test compound (represented by the compound Nos. in the above Examples) in 0.5 ml of acetone containing Tween 20 (trade name). and then diluting it to the prescribed concentration (100 ppm) with Dain (trade name, sticker) water diluted 5000-fold, was sprayed on a soybean whose primary leaves had developed completely with spray gun in the amounts wherein the pesticidal solution dripped. After drying the pesticidal solution, two primary leaves were cut off. The. leaves were put into an ice cream cup (180 ml), and ten third-instar larvae of *Spodoptera litura* were set free therein. The cup was left in a temperature-controlled

breeding room (25°C). On the fifth day after, the number of surviving *Spodoptera litura* was examined. The rate of dead pests was calculated by the following formula.

$$\text{rate of dead pests (\%)} = (\text{number of dead pests/ number of test pests}) \times 100$$

The result was shown in table 7.

Table 7

| Compound No. | Concentration (ppm) | rate of dead pests (%) |
|---|---|---|
| I-1 | 100 | 100 |
| I-2 | 100 | 100 |
| I-3 | 100 | 100 |
| I-4 | 100 | 100 |
| I-5 | 100 | 100 |
| I-6 | 100 | 100. |
| I-7 | 100 | 100 |
| I-8 | 100 | 100 |
| I-11 | 100 | 100 |
| I-14 | 100 | 100 |
| I-17 | 100 | 100 |
| I-20 | 100 | 100 |
| I-22 | 100 | 100 |
| I-23 | 100 | 100 |
| I-26 | 100 | 100 |
| I-28 | 100 | 100 |
| I-29 | 100 | 100 |
| I-31 | 100 | 100 |
| I-32 | 100 | 100 |
| I-34 | 100 | 100 |
| I-37 | 100 | 100 |
| I-40 | 100 | 100 |
| I-41 | 100 | 100 |
| I-42 | 100 | 100 |
| I-43 | 100 | 100 |
| I-44 | 100 | 100 |
| I-45 | 100 | 100 |
| I-47 | 100 | 100 |
| I-53 | 100 | 100 |
| I-59 | 100 | 100 |
| I-62 | 100 | 100 |

Table 7 (continued)

| Compound No. | Concentration (ppm) | rate of dead pests (%) |
|---|---|---|
| I-65 | 100 | 100 |
| I-68 | 100 | 100 |
| I-69 | 100 | 100 |
| I-71 | 100 | 100 |
| I-72 | 100 | 100 |
| I-74 | 100 | 100 |
| I-75 | 100 | 100 |
| I-77 | 100 | 100 |
| I-78 | 100 | 100 |
| I-80 | 100 | 100 |
| I-81 | 100 | 100 |
| I-83 | 100 | 100 |
| I-84 | 100 | 100 |
| I-86 | 100 | 100 |
| I-87 | 100 | 100 |
| I-89 | 100 | 100 |
| I-90 | 100 | 100 |
| I-92 | 100 | 100 |
| I-93 | 100 | 100 |
| I-95 | 100 | 100 |
| I-96 | 100 | 100 |
| I-98 | 100 | 100 |
| I-99 | 100 | 100 |
| I-101 | 100 | 100 |
| I-102 | 100 | 100 |
| I-104 | 100 | 100 |
| I-105 | 100 | 100 |
| I-107 | 100 | 100 |
| I-110 | 100 | 100 |
| I-111 | 100 | 100 |
| I-113 | 100 | 100 |
| I-116 | 100 | 100 |
| I-117 | 100 | 100 |
| I-122 | 100 | 100 |
| I-123 | 100 | 100 |
| I-125 | 100 | 100 |
| I-126 | 100 | 100 |
| I-128 | 100 | 100 |

Table 7 (continued)

| Compound No. | Concentration (ppm) | rate of dead pests (%) |
|---|---|---|
| I-129 | 100 | 100 |
| I-143 | 100 | 100 |
| I-158 | 100 | 100 |
| I-205 | 100 | 100 |
| I-208 | 100 | 100 |
| I-218 | 100 | 100 |
| I-219 | 100 | 100 |
| I-221 | 100 | 100 |
| I-222 | 100 | 100 |
| 1-248 | 100 | 100 |
| I-249 | 100 | 100 |
| I-252 | 100 | 100 |
| I-254 | 100 | 100 |
| I-255 | 100 | 100 |
| I-257 | 100 | 100 |
| I-258 | 100 | 100 |
| I-266 | 100 | 100 |
| I-269 | 100 | 100 |
| I-270 | 100 | 100 |
| I-272 | 100 | 100 |
| I-273 | 100 | 100 |
| I-275 | 100 | 100 |
| I-276 | 100 | 100 |
| I-278 | 100 | 100 |
| I-279 | 100. | 100 |
| I-281 | 100 | 100 |
| I-282 | 100 | 100 |
| I-284 | 100 | 100 |
| I-285 | 100 | 100 |
| I-287 | 100 | 100 |
| I-288 | 100 | 100 |
| I-290 | 100 | 100 |
| I-293 | 100 | 100 |
| I-294 | 100 | 100 |
| I-296 | 100 | 100 |
| I-299 | 100 | 100 |
| I-300 | 100 | 100 |
| I-302 | 100 | 100 |

Table 7 (continued)

| Compound No. | Concentration (ppm) | rate of dead pests (%) |
|---|---|---|
| I-303 | 100 | 100 |
| I-305 | 100 | 100 |
| I-306 | 100 | 100 |
| I-307 | 100 | 100 |
| I-320 | 100 | 100 |
| I-321 | 100 | 100 |
| I-323 | 100 | 100 |
| I-324 | 100 | 100 |
| I-329 | 100 | 100 |
| I-330 | 100 | 100 |
| I-335 | 100 | 100 |
| I-395 | 100 | 100 |
| I=396 | 100 | 100 |
| I-398 | 100 | 100 |
| I-399 | 100 | 100 |
| II-10 | 100 | 100 |
| II-11 | 100 | 100 |
| III-1 | 100 | 100 |
| III-2 | 100 | 100 |
| III=3 | 100 | 100 |
| III-4 | 100 | 100 |
| III-5 | 100 | 100 |
| III-6 | 100 | 100 |
| III-7 | 100 | 100 |
| III-8 | 100 | 100 |
| III-12 | 100 | 100 |
| III-13 | 100 | 100 |
| III-14 | 100 | 100 |
| III-17 | 100 | 100 |
| III-21 | 100 | 100 |
| III-22 | 100 | 100 |
| III-23 | 100 | 100 |
| III-24 | 100 | 100 |
| III-25 | 100 | 100 |
| III-26 | 100 | 100 |
| III-27 | 100 | 100 |
| III-28 | 100 | 100 |
| III-29 | 100 | 100 |

Table 7 (continued)

| Compound No. | Concentration (ppm) | rate of dead pests (%) |
|---|---|---|
| III-30 | 100 | 100 |
| III-31 | 100 | 100 |
| III-32 | 100 | 100 |
| III-35 | 100 | 100 |
| III-36 | 100 | 100 |
| III-37 | 100 | 100 |
| III-38 | 100 | 100 |
| III-39 | 100 | 100 |
| III-41 | 100 | 100 |
| III-42 | 100 | 100 |
| III-43 | 100 | 100 |
| III-44 | 100 | 100 |
| III-45 | 100 | 100 |
| III-46 | 100 | 100 |
| III-49 | 100 | 100 |
| III-50 | 100 | 100 |
| III-51 | 100 | 100 |
| III-52 | 100 | 100 |
| III-53 | 100 | 100 |
| III-54 | 100 | 100 |
| III-55 | 100 | 100 |
| III-56 | 100 | 100 |
| III-57 | 100 | 100 |
| III-58 | 100 | 100 |
| III-60 | 100 | 100 |
| III-62 | 100 | 100 |
| III-63 | 100 | 100 |
| III-64 | 100 | 100 |
| III-69 | 100 | 100 |
| III-70 | 100 | 100 |
| III-71 | 100 | 100 |
| III-72 | 100 | 100 |
| III-76 | 100 | 100 |
| III-77 | 100 | 100 |
| III-78 | 100 | 100 |
| III-79 | 100 | 100 |
| III-84 | 100 | 100 |
| III-89 | 100 | 100 |

Table 7 (continued)

| Compound No. | Concentration (ppm) | rate of dead pests (%) |
|---|---|---|
| III-91 | 100 | 100 |
| III-148 | 100 | 100 |
| III-149 | 100 | 100 |
| III-150 | 100 | 100 |
| III-152 | 100 | 100 |
| III-153 | 100 | 100 |
| III-154 | 100 | 100 |
| III-156 | 100 | 100 |
| III-160 | 100 | 100 |
| III-161 | 100 | 100 |
| III-162 | 100 | 100 |
| III-164 | 100 | 100 |
| III-165 | 100 | 100 |
| III-166 | 100 | 100 |
| III-184 | 100 | 100 |
| III-188 | 100 | 100 |
| III-455 | 100 | 100 |
| V-1 | 100 | 100 |
| V-3 | 100 | 100 |
| V-4 | 100 | 100 |

Test Example 3

Insecticidal effect for *Plutella xylostella* by immersion treatment of host crop root

Roots of 4-leave stage cabbage was washed with tap water to remove soil, and then immersed in a pesticidal solution which was prepared by dissolving 5 mg of test compound (represented by the compound Nos. in the above Examples) in 0.5 ml of acetone containing Tween 20 (trade name) and then diluting it to the prescribed concentration (25 ppm) with ion-exchange water, and which was filled in a light-shielded Erlenmeyer flask (100 ml). On the fifth day after immersion of root, the root was cut off, and the stem and leaves were put into an ice cream cup (180 ml). Ten second-instar larvae of *Plutella xylostella* were set free therein, and the cup was left in a temperature-controlled breeding room (24°C). On the fifth day after, the number of surviving *Plutella xylostella* was examined. The rate of dead pests was calculated by the following formula.

$$\text{rate of dead pests (\%)} = (\text{number of dead pests/ number of test pests}) \times 100$$

The result was shown in table 8.

Table 8

| Compound No. | Concentration (ppm) | rate of dead pests (%) |
|---|---|---|
| I-4 | 25 | 95 |

Table 8 (continued)

| Compound No. | Concentration (ppm) | rate of dead pests (%) |
|---|---|---|
| I-8 | 25 | 100 |
| I-11 | 25 | 100 |
| I-14 | 25 | 100 |
| I-20 | 25 | 100 |
| I-23 | 25 | 95 |
| I-26 | 25 | 100 |
| I-28 | 25 | 100 |
| I-29 | 25 | 100 |
| I-34 | 25 | 100 |
| I-41 | 25 | 100 |
| I-42 | 25 | 100 |
| I-44 | 25 | 100 |
| I-69 | 25 | 100 |
| I-71 | 25 | 95 |
| I-72 | 25 | 100 |
| I-75 | 25 | 95 |
| I-78 | 25 | 90 |
| I-81 | 25 | 100 |
| I-84 | 25 | 100 |
| I-102 | 25 | 100 |
| I-105 | 25 | 100 |
| I-111 | 25 | 100 |
| I-123 | 25 | 100 |
| I-126 | 25 | 100 |
| I-129 | 25 | 100 |
| I-144 | 25 | 95 |
| I-205 | 25 | 90 |
| I=222 | 25 | 100 |
| I=249 | 25 | 95 |
| I-321 | 25 | 95 |
| III-38 | 25 . | 90 |
| III-58 | 25 | 90 |
| III-64 | 25 | 90 |
| III-89 | 25 | 100 |
| III-91 | 25 | 100 |
| III-148 | 25 | 100 |
| III-149 | 25 | 95 |
| III-152 | 25 | 100 |

Table 8 (continued)

| Compound No. | Concentration (ppm) | rate of dead pests (%) |
|---|---|---|
| III-153 | 25 | 100 |
| III-154 | 25 | 100 |
| III-160 | 25 | 90 |
| V-4 | 25 | 95 |

Test Example 4

Insecticidal effect for *Spodoptera litura* by immersion treatment of host crop root

Roots of primary leaves developing stage soybean was washed with tap water to remove soil, and then immersed in a pesticidal solution which was prepared by dissolving 5 mg of test compound (represented by the compound Nos. in the above Examples) in 0.5 ml of acetone containing Tween 20 (trade name) and then diluting it to the prescribed concentration (25 ppm) with ion-exchange water, and which was filled in a light-shielded Erlenmeyer flask (100 ml). On the fifth day after immersion of root, two primary leaves were cut off, and the leaves were put into an ice cream cup (180 ml). Ten second-instar larvae of *Spodoptera litura* were set free therein, and the cup was left in a temperature-controlled breeding room (25°C). On the fifth day after, the number of surviving *Spodoptera litura* was examined. The rate of dead pests was calculated by the following formula.

rate of dead pests (%) = (number of dead pests/ number of test pests) x 100

The result was shown in table 9.

Table 9

| Compound No. | Concentration (ppm) | rate of dead pests (%) |
|---|---|---|
| I-1 | 25 | 100 |
| I-4 | 25 | 100 |
| I-8 | 25 | 100 |
| I-11 | 25 | 100 |
| I-14 | 25 | 100 |
| I-17 | 25 | 100 |
| I-20 | 25 | 95 |
| I-23 | 25 | 100 |
| I-26 | 25 | 100 |
| I-28 | 25 | 100 |
| I-29 | 25 | 100 |
| I-32 | 25 | 90 |
| I-37 | 25 | 100 |
| I-40 | 25 | 100 |
| I-41 | 25 | 100 |
| I-42 | 25 | 100 |
| I-43 | 25 | 100 |

Table 9 (continued)

| Compound No. | Concentration (ppm) | rate of dead pests (%) |
|---|---|---|
| I-44 | 25 | 100 |
| I-45 | 25 | 100 |
| I-47 | 25 | 100 |
| I-48 | 25 | 100 |
| I-50 | 25 | 100 |
| I-53 | 25 | 100 |
| I-59 | 25 | 100 |
| I-62 | 5 | 100 |
| I-71 | 25 | 90 |
| I-74 | 25 | 100 |
| I-75 | 25 | 100 |
| I-78 | 25 | 100 |
| I-93 | 25 | 100 |
| I-98 | 25 | 95 |
| I-101 | 25 | 100 |
| I-104 | 25 | 100 |
| I-105 | 25 | 100 |
| I-122 | 25 | 95 |
| I-128 | 25 | 100 |
| I-129 | 25 | 100 |
| I-205 | 25 | 100 |
| I-206 | 25 | 100 |
| I-218 | 25 | 100 |
| I-219 | 25 | 100 |
| I-221 | 25 | 100 |
| I-222 | 25 | 100 |
| I-251 | 25 | 90 |
| I-252 | 25 | 100 |
| I-255 | 25 | 100 |
| I-275 | 25 | 100 |
| I-278 | 25 | 90 |
| I-281 | 25 | 100 |
| I-288 | 25. | 100 |
| I-306 | 25 . | 100 |
| I-307 | 25 | 90 |
| I-321 | 25 | 100 |
| I-396 | 25 | 100 |
| I-399 | 25 | 100 |

Table 9 (continued)

| Compound No. | Concentration (ppm) | rate of dead pests (%) |
|---|---|---|
| II-10 | 25 | 100 |
| II-11 | 25 | 100 |
| II-64 | 25 | 95 |
| III-2 | 25 | 90 |
| III-8 | 25 | 100 |
| III-9 | 25 | 95 |
| III-12 | 25 | 100 |
| III-13 | 25 | 100 |
| III-15 | 25 | 95 |
| III-37 | 25 | 95 |
| III-38 | 25 | 100 |
| III-49 | 25 | 95 |
| III-58 | 25 | 100 |
| III-61 | 25 | 100 |
| III-64 | 25 | 90 |
| III-79 | 25 | 100 |
| III-91 | 25 | 95 |
| III-144 | 25 | 100 |
| III-148 | 25 | 100 |
| III-149 | 25 | 100 |
| III-152 | 25 | 100 |
| III-153 | 25 | 100 |
| III-154 | 25 | 100 |
| III-164 | 25 | 100 |
| IV-4 | 25 | 100 |
| V-1 | 25 | 100 |

Test Example 5

Acaricidal effect for Tetranychus *urticae*

Ten female imagoes *Tetranychus urticae* were set free on a kidney bean wherein primary leaves had just developed. On the following day, a pesticidal solution, which was prepared by dissolving 5 mg of test compound (represented by the compound Nos. in the above Examples) in 0.5 ml of acetone containing Tween 20 (trade name) and then diluting it to the prescribed concentration (500 ppm) with Dain (trade name, sticker) water diluted 5000-fold, was sprayed with spray gun in the amounts wherein the pesticidal solution dripped. The kidney bean was left in a temperature-controlled breeding room (25°C). On the second and seventh day after treatment, the number of surviving *Tetranychus urticae* was examined. The rate of decrease was calculated by the following formula.

$$\text{rate of decrease (\%)} = (1-(S_2/(5.59\times(10+(10+S_1)/2+S_1)))) \times 100$$

In the above formula,

$S_1$; the number of surviving imagoes on the second day after
$S_2$; the number of surviving pests on the seventh day after

The result was shown in table 10.

Table 10

| Compound No. | Concentration (ppm) | rate of decrease (%) |
|---|---|---|
| III-21 | 100 | 99 |
| III-24 | 100 | 95 |
| III-25 | 100 | 97 |
| III-27 | 100 | 96 |
| III-31 | 100 | 99 |
| III-35 | 100 | 97 |
| III-37 | 100 | 97 |
| III-38 | 100 | 98 |
| III-39 | 100 | 97 |
| III-41 | 100 | 97 |
| III-42 | 100 | 98 |
| III-44 | 100 | 96 |
| III-45 | 100 | 100 |
| III-46 | 100 | 97 |
| III-48 | 100 | 95 |
| III-49 | 100 | 96 |
| III-184 | 100 | 97 |

Test Example 6

Pest control effect for *Plutella xylostella* by plant seedling foot soil surface irrigation treatment with pesticidal solution

A pesticidal solution which was prepared by dissolving 20 mg of test compound (represented by the compound Nos. in the above Examples) in 0.2 ml of acetone containing Tween 20 (trade name) and then diluting to 3 ml with ion-exchange water, was irrigated on the plant foot soil surface of 2.5-leave stage cabbage. This cabbage plant was transplanted to 1/5000a pot, and left in a facility wherein imagoes of *Plutella xylostella* were set free to lay eggs successively. On the 24th day after, the number of lavae on the plant was examined. The rate of pest control was calculated by the following formula.

$$\text{rate of pest control (\%)} = (1\text{- number of pests in treated plant/ number of pests in non-treated plant}) \times 100$$

The result was shown in table 11.

Table 11

| Compound No. | Dose (mg/plant) | rate of pest control (%) |
|---|---|---|
| I-4 | 20 | 92 |

Table 11 (continued)

| Compound No. | Dose (mg/plant) | rate of pest control (%) |
|---|---|---|
| I-25 | 20 | 88 |
| I-26 | 20 | 100 |
| I-29 | 20 | 92 |
| II-10 | 20 | 88 |
| III-78 | 20 | 83 |
| III-84 | 20 | 100 |
| III-89 | 20 | 92 |

Test Example 7

Pest control effect for *Plutella Xylostella* by plant seedling foot soil surface irrigation treatment with pesticidal solution

A pesticidal solution which was prepared by dissolving 20 mg of test compound (represented by the compound Nos. in the above Examples) in 0.2 ml of acetone containing Tween 20 (trade name) and then diluting to 3 ml with ion-exchange water, was irrigated on the plant foot soil surface of 2.5-leave stage cabbage. This cabbage plant was transplanted to 1/5000a pot, and left. in a facility wherein imagoes of *Plutella xylostella* were set free to lay eggs successively. On the 30th day after, the number of lavae on the plant was examined. The rate of pest control was calculated by the. following formula.

$$\text{rate of pest control (\%)} = (1 - \text{number of pests in treated plant/ number of pests in non-treated plant}) \times 100$$

The result was shown in table 12.

Table 12

| Compound No. | Dose (mg/plant) | rate of pest control (%) |
|---|---|---|
| I-42 | 20 | 94 |
| I-44 | 20 | 96 |
| I-45 | 20 | 98 |
| III-160 | 20 | 91 |
| III-164 | 20 | 95 |

Test Example 8

Insecticidal effect for *Plutella xylostella* by seed treatment

4% aqueous solution of PVA (polyvinylalcohol) .was applied to seeds of cabbage. Then the seeds were powdered with test compound (represented by the compound Nos. in the above Examples), and sowed in a Φ 6 cm in diameter Jiffy Pot (trade name). On the 31th day after sowing, the stem and leaves were cut off, and were put into an ice cream cup (180 ml). Fifteen first-instar larvae of *Plutella xylostella* were set free therein, and the cup was left in a temperature-controlled breeding room (24°C). On the third day after, the number of surviving *Plutella* xylostella was examined. The rate of dead pests was calculated by the following formula.

$$\text{rate of dead pests (\%)} = (\text{number of dead pests/ number of test pests}) \times 100$$

The result was shown in table 13.

Table 13

| Compound No. | Dose (mg/seed) | rate of dead pests (%) |
|---|---|---|
| I-44 | 1 | 100 |
| I-44 | 5 | 97 |

Industrial Applicability.

As described above, since the compound (I) of the present invention has a superior insecticidal activity, and also has a systemic action (penetration and translocation ability), it can be used for soil treatment and seed treatment, and therefore can make a significant contribution to the laborsaving in agriculture and improvement of safety in workers and environment.

## Claims

**1.** A compound represented by the formula:

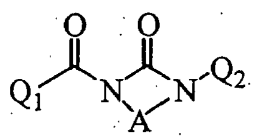

(I)

wherein, A represents a group represented by the formula:

1) $CY_1Y_2OCY_3Y_4$
2) $CY_1Y_2SO_nCY_3Y_4$
3) $CY_1Y_2NRCY_3Y_4$
4) $CY_1{=}NCY_3Y_4$ or
5) $CY_1Y_2N{=}CY_3$

wherein n represents 0, 1 or 2, and $Y_1$, $Y_2$, $Y_3$ and $Y_4$ represent independently a hydrogen atom, a $C_{1-6}$ alkyl group or a halo$C_{1-6}$ alkyl group; R represents a hydrogen atom, a halogen atom, a group via optionally oxidized sulfur atom, a nitro group, a nitroso group, a formyl group, an optionally substituted hydroxy group, a cyano. group, an optionally substituted hydrocarbon group, an optionally substituted amino group, an optionally substituted heterocyclic group, an optionally substituted $C_{2-7}$ acyl group, an optionally substituted $C_{1-6}$ alkoxycarbonyl group, an optionally substituted $C_{7-11}$ aralkyloxycarbonyl group, an optionally substituted $C_{6-10}$ aryloxycarbonyl group, an optionally substituted carbamoyl group, an optionally substituted thiocarbamoyl group, an optionally substituted hydroxyoxalyl group, an optionally substituted aminooxalyl group or an optionally substituted hydroxyaminooxalyl group; $Q_1$ represents a substituted phenyl group or a substituted aromatic heterocyclic group; and $Q_2$ represents a substituted phenyl group, a $C_{1-6}$ alkyl group or a halo$C_{1-6}$ alkyl group; provided that when A is a group represented by $CH_2OCH_2$, $Q_1$ is a group other than dichlorophenyl; or a salt thereof.

**2.** The compound according to claim 1, which is any one of the compounds represented by the formulas:

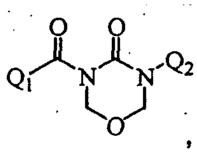
(Ia) ,

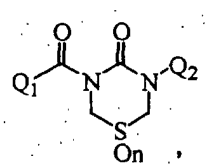
(Ib) ,

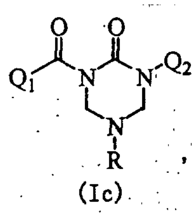
(Ic) ,

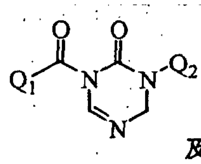
(Id) 及び

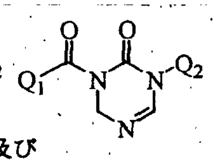
(Ie)

wherein respective symbols are as defined in claim 1.

**3.** The compound according to claim 2, wherein R is a hydrogen atom, a nitroso group, a hydroxyoxalyl group, an aminooxalyl group, or a $C_{1-6}$ alkyl group, a $C_{3-10}$ cycloalkyl group, a $C_{2-6}$ alkenyl group, a $C_{2-6}$ alkynyl group, a $C_{6-10}$ aryl group, a $C_{7-11}$ aralkyl group, a $C_{1-6}$ alkoxycarbonyl group, a $C_{6-10}$ aryloxycarbonyl group, a $C_{7-11}$ aralkyloxycarbonyl group, a $C_{1-6}$ alkoxycarbonylthio group, a $C_{2-7}$ acyl group, a $C_{1-6}$ alkylsulfonyl group, a $C_{6-10}$ arylsulfonyl group, a $C_{1-6}$ alkoxyoxalyl group, a N-$C_{1-6}$ alkylcarbamoyl group, a N,N-di($C_{1-6}$ alkyl) carbamoyl group, a N-($C_{1-6}$ alkoxy, $C_{7-11}$ aralkyloxy or $C_{1-6}$ alkyl)aminooxalyl group, a N,N-di($C_{1-6}$ alkyl)aminooxalyl group, or a N,O-di($C_{1-6}$ alkyl)hydroxyaminooxalyl group, each of which may be substituted.

**4.** The compound according to claim 1, wherein the substituent of the substituted phenyl group for $Q_2$ is at. least one selected from the group consisting of a halogen atom, a cyano group, a $C_{1-6}$ alkyl group, a halo$C_{1-6}$ alkyl group, a $C_{1-6}$ alkoxycarbonyl group, a group represented by the formula: $Z_1$-W- (wherein $Z_1$ represents a benzene ring or an aromatic heterocyclic ring, each of which is substituted with a halogen atom, a $C_{1-6}$ alkyl group or a halo$C_{1-6}$ alkyl group, and -W- represents -O-, -S-, -SO- or -$SO_2$-), and a group represented by the formula: $Z_2$-W- (wherein $Z_2$ represents a $C_{1-6}$ alkyl, a $C_{2-6}$ alkenyl or a $C_{2-6}$ alkynyl, each of which may be substituted with a halogen atom, and -W- is as defined above).

**5.** The compound according to claim 1, wherein $Q_1$ is a phenyl group or an aromatic heterocyclic group, each of which is substituted with a halogen atom, a $C_{1-6}$ alkyl group, a halo$C_{1-6}$ alkyl group, a $C_{1-6}$ alkoxy group or a halo$C_{1-6}$ alkoxy group.

**6.** The compound according to claim 2, wherein $Q_1$ is difluorophenyl, chlorofluorophenyl or 3-chloropyridin-2-yl,

$Q_2$ is a phenyl group which has at least one substituent selected from the group consisting of a. halogen atom, cyano group, $C_{1-4}$ alkoxycarbonyl group, $C_{1-3}$ alkyl group, halo$C_{1-3}$ alkyl group; phenyloxy group, phenylthio group, phenylsulfinyl group and phenylsulfonyl group, each of which is substituted with halogen atom, $C_{1-3}$ alkyl group or halo$C_{1-3}$ alkyl group; pyridyloxy group, pyridylthio group, pyridylsulfinyl group and pyridylsulfonyl group, each of which is substituted with halogen atom, $C_{1-3}$ alkyl group or halo$C_{1-3}$ alkyl group; and $C_{1-3}$ alkoxy group, $C_{1-4}$ alkylthio group, $C_{1-4}$ alkylsulfinyl group, $C_{1-4}$ alkylsulfonyl group, $C_{2-4}$ alkenylthio group, $C_{2-4}$ alkenylsulfinyl group, $C_{2-4}$ alkenylsulfonyl group, $C_{2-4}$ alkynylthio group, $C_{2-4}$ alkynylsulfinyl group and $C_{2-4}$ alkynylsulfonyl group, each of which may be substituted with halogen atom; or 2,2,2-trifluoroethyl,

R is a hydrogen atom, $C_{1-3}$ alkyl group, allyl group, propargyl group, phenyl group, benzyl group, phenethyl group, methoxycarbonyl group, benzyloxycarbonyl group, methoxycarbonylthio group, N-methylcarbamoyl group, N,N-dimethylcarbamoyl group, acetyl group (which may be substituted with halogen atom, methylthio group, methylsulfinyl group or methylsulfonyl group), methylsulfonyl group, 4-methylphenylsulfonyl group, trifluoromethylsulfonyl group, nitroso group, methoxyoxalyl group; hydroxyoxalyl group, aminooxalyl group, N-(methoxy, benzyloxy or methyl)aminooxalyl group, N,N-dimethylaminooxalyl group or N,O-dimethylhydroxyaminooxalyl group.

**7.** A process for producing the compound according to claim 1 or a salt thereof, which comprises reacting a compound represented by the formula:

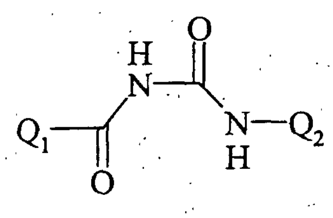

(II)

wherein respective symbols are as defined in claim 1, or a salt thereof, with a compound represented by the formula:

$$L\text{-}A\text{-}L \quad (III)$$

wherein L represents a leaving group, or

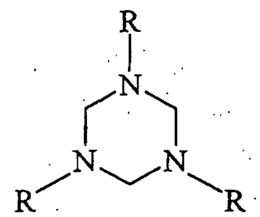

(IV)

wherein R is as defined in claim 1; and if desired, converting the substituent of the obtained compound to other substituent.

**8.** A process for producing the compound represented by the formula:

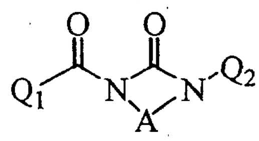

(I)

wherein, A represents a group represented by the formula:

1) $CY_1Y_2OCY_1Y_2$
2) $CY_1Y_2SO_nCY_1Y_2$ or
3) $CY_1Y_2NRCY_1Y_2$

wherein n represents 0, 1 or 2, and $Y_1$ and $Y_2$ represent independently a hydrogen atom, a $C_{1-6}$ alkyl group or a halo$C_{1-6}$ alkyl group; R represents a hydrogen atom, a halogen atom, a group via optionally oxidized sulfur atom, a nitro group, a nitroso group, a formyl group, an optionally substituted hydroxy group, a cyano group, an optionally substituted hydrocarbon group, an optionally substituted amino group, an optionally substituted heterocyclic group, an optionally substituted $C_{2-7}$ acyl group, an optionally substituted $C_{1-6}$ alkoxycarbonyl group, an optionally substituted $C_{7-11}$ aralkyloxycarbonyl group, an optionally substituted $C_{6-10}$ aryloxycarbonyl group, an optionally substituted carbamoyl group, an optionally substituted thiocarbamoyl group, an optionally substituted hydroxyoxalyl group, an optionally substituted aminooxalyl group or an optionally substituted hydroxyaminooxalyl group; $Q_1$ represents a substituted phenyl group or a substituted aromatic heterocyclic group; and $Q_2$ represents a substituted phenyl group, a $C_{1-6}$ alkyl group or a halo$C_{1-6}$ alkyl group; provided that when A is a group represented by $CH_2OCH_2$, $Q_1$ is a group other than dichlorophenyl; or a salt thereof, which comprises subjecting the compound represented by the formula:

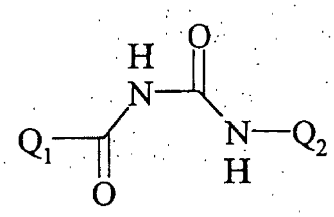

(II)

wherein respective symbols are as defined in claim 1, or a salt thereof, to dehydration reaction with an aldehyde or a ketone, if necessary, in the presence of hydrogen sulfide or amine represented by the formula $RNH_2$ (wherein R is as. defined in claim 1); and if desired, converting the substituent of the obtained compound to other substituent.

**9.** A pest controller for soil treatment which comprises a compound represented by the formula:

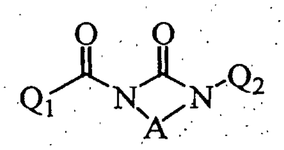 (I)

wherein, A represents a group represented by the formula:

1) $CY_1Y_2OCY_3Y_4$
2) $CY_1Y_2SO_nCY_3Y_4$
3) $CY_1Y_2NRCY_3Y_4$
4) $CY_1$=$NCY_3Y_4$ or
5) $CY_1Y_2N$=$CY_3$

wherein n represents 0, 1 or 2, and $Y_1$, $Y_2$, $Y_3$ and $Y_4$ represent independently a hydrogen atom, a $C_{1-6}$ alkyl group or a halo$C_{1-6}$ alkyl group; R represents a hydrogen atom, a halogen atom, a group via optionally oxidized sulfur atom, a nitro group, a nitroso group, a formyl group, an optionally substituted hydroxy group, a cyano group, an optionally substituted hydrocarbon group, an optionally substituted amino group, an optionally substituted heterocyclic group, an optionally substituted $C_{2-7}$ acyl group, an optionally substituted $C_{1-6}$ alkoxycarbonyl group, an optionally substituted $C_{7-11}$ aralkyloxycarbonyl group, an optionally substituted $C_{6-10}$ aryloxycarbonyl group, an optionally substituted carbamoyl group, an optionally substituted thiocarbamoyl group, an optionally substituted hydroxyoxalyl group, an optionally substituted aminooxalyl group or an optionally substituted hydroxyaminooxalyl group; $Q_1$ represents a substituted phenyl group or a substituted aromatic heterocyclic group; and $Q_2$ represents a substituted phenyl group, a $C_{1-6}$ alkyl group or a halo$C_{1-6}$ alkyl group; or a salt thereof as an active ingredient.

**10.** A pest controller according to claim 9. which is for seed treatment.

**11.** A pest controller for spraying which comprises the compound according to any one of claims 1 to 6 or a salt thereof as an active ingredient.

**INTERNATIONAL SEARCH REPORT**

International application No.
PCT/JP03/02461

A. CLASSIFICATION OF SUBJECT MATTER
Int.$Cl^7$ C07D251/22, 273/04, 285/34, 413/12, 413/06, 417/12, 417/06, 417/14, 413/14, 401/12, 401/06, 401/14, A01N43/88, 43/64, 47/16, 47/06

B. FIELDS SEARCHED
Minimum documentation searched (classification system followed by classification symbols)
Int.$Cl^7$ C07D251/22, 273/04, 285/34, 413/12, 413/06, 417/12, 417/06, 417/14, 413/14, 401/12, 401/06, 401/14, A01N43/88, 43/64, 47/16, 47/06

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X | Wellinga, Kobus; Mulder, Rudolf; Van Daalen, Jan J., Synthesis and laboratory evaluation of 1-(2,6-disubstituted benzoyl)-3-phenylureas, a new class of insecticides. I. 1-(2,5-Dichlorobenzoyl)-3-phenylureas, Journal of Agricultural and Food Chemistry (1973), 21(3), 348-54 | 1-11 |
| X | JP 50-105629 A (N.V. Philips' Gloeilampenfabrieken), 20 August, 1975 (20.08.75), Full text & US 3748356 A | 1-11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 April, 2003 (01.04.03) | 22 April, 2003 (22.04.03) |
| Name and mailing address of the ISA/ Japanese Patent Office | Authorized officer |
| Facsimile No. | Telephone No. |

Form PCT/ISA/210 (second sheet) (July 1998)